(19) Europäisches Patentamt
European Patent Office
Office européen des brevets

(11) EP 4 186 907 A1

(12) **EUROPEAN PATENT APPLICATION**
published in accordance with Art. 153(4) EPC

(43) Date of publication:
**31.05.2023 Bulletin 2023/22**

(21) Application number: **21855612.4**

(22) Date of filing: **12.08.2021**

(51) International Patent Classification (IPC):
***C07D 487/04*** (2006.01) ***C07D 519/00*** (2006.01)
***A61K 31/497*** (2006.01) ***A61K 31/5377*** (2006.01)
***A61P 17/00*** (2006.01) ***A61P 19/02*** (2006.01)
***A61P 25/00*** (2006.01)

(52) Cooperative Patent Classification (CPC):
**A61K 31/4188; A61K 31/437; A61K 31/454; A61K 31/496; A61K 31/497; A61K 31/501; A61K 31/5377; A61P 11/00; A61P 17/00; A61P 19/02; A61P 25/00; A61P 31/14; A61P 31/16; A61P 37/06; C07D 487/04;** (Cont.)

(86) International application number:
**PCT/CN2021/112351**

(87) International publication number:
**WO 2022/033562 (17.02.2022 Gazette 2022/07)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **14.08.2020 CN 202010818470**

(71) Applicant: **Henan Medinno Pharmaceutical Technology Co., Ltd.**
**Zhengzhou, Henan 450000 (CN)**

(72) Inventors:
- **LU, Liang**
  **Zhengzhou, Henan 450000 (CN)**
- **HUANG, Hai**
  **Zhengzhou, Henan 450000 (CN)**
- **ZHANG, Longzheng**
  **Zhengzhou, Henan 450000 (CN)**
- **ZHAO, Saisai**
  **Zhengzhou, Henan 450000 (CN)**
- **ZHANG, Jixuan**
  **Zhengzhou, Henan 450000 (CN)**

(74) Representative: **Slingsby Partners LLP**
**1 Kingsway**
**London WC2B 6AN (GB)**

(54) **JAK INHIBITOR COMPOUND FOR TREATING SEVERE PNEUMONIA**

(57) The present disclosure relates to a class of JAK inhibitor compounds for treating severe pneumonia. Specifically, the present disclosure discloses use of a compound represented by formula (G), isotopically labeled compound thereof, or optical isomer thereof, geometric isomer thereof, a tautomer thereof or a mixture of various isomers, or a pharmaceutically acceptable salt thereof, or a prodrug thereof, or a metabolite thereof in the manufacture of a medicament for treating severe pneumonia.

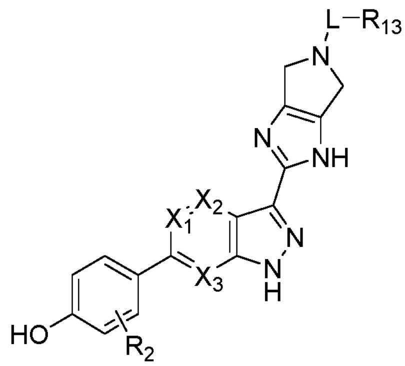

(G)

EP 4 186 907 A1

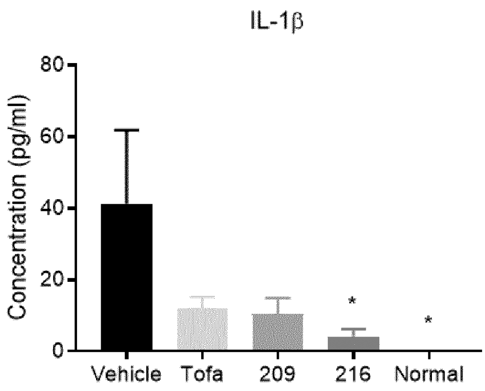


FIG. 1

(52) Cooperative Patent Classification (CPC): (Cont.)
**C07D 519/00**

## Description

This application claims priority to Chinese Patent Application No. 202010818470.4, filed on August 14, 2020, which is incorporated herein by reference in its entirety.

## TECHNICAL FIELD

The present disclosure provides compounds having inhibitory activity against Janus kinase (JAK) for treating pneumonia.

## BACKGROUND

JAK belongs to a family of intracellular non-receptor tyrosine kinases, which plays an important role in cytokine receptor signaling pathway by interacting with signal transducer and activator of transcription (STAT). Many cytokines and growth factors signal through the JAK-STAT signaling pathway, including interleukins such as IL-2-7, IL-9, IL-10, IL-15, IL-21, and the like, GM-CSF (granulocyte/macrophage colony-stimulating factor), and interferons including IFN-$\alpha$, IFN-$\beta$, IFN-$\gamma$, and the like. JAK is a very important class of drug targets, and several JAK inhibitors have been developed for this target. For example, inhibitors of JAK3 have been proposed to be suitable as immunosuppressive agents.

Pneumonia is considered one of the most common infectious diseases in humans and can be caused by a variety of pathogens such as viruses, streptococcus pneumonia, and the like. After the pathogens invade lungs, immune responses in the lungs are activated. A rapid and well-coordinated immune response can help clear viruses. However, a dysregulated and excessive immune response can lead to production of a large amount of pro-inflammatory cytokines, which can trigger a cytokine storm and lead to acute lung injury (ALI). A strong correlation between immune overstimulation and severe pneumonia has been demonstrated.

There is a need for medicaments that are effective in suppressing severe pneumonia.

## SUMMARY

In a first aspect, the present disclosure provides use of a compound of Formula (G) as a JAK inhibitor

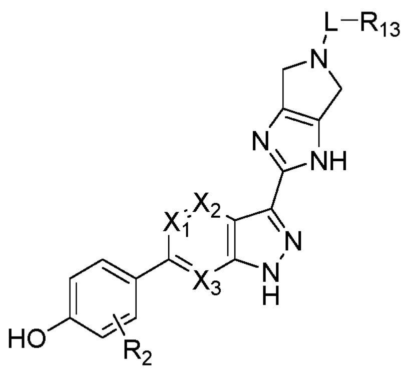

(G)

or an isotopically labeled compound thereof, or an optical isomer thereof, a geometric isomer thereof, a tautomer thereof or a mixture of various isomers, or a pharmaceutically acceptable salt thereof, or a prodrug thereof, or a metabolite thereof, in the manufacture of a medicament for treating severe pneumonia,
wherein

L is C=O, O=S=O, $CH_2$ or a covalent bond; and
$X_1$ is N or $CR_{14}$; and
$X_2$ is N or $CR_{15}$; and
$X_3$ is N or $CR_{16}$; and
$R_{14}$, $R_{15}$, $R_{16}$ are each independently selected from H, -OH, -SH, -CN, halogen, $-NO_2$, $-SF_5$, - S-$C_{1-4}$ alkyl, $C_{1-6}$ alkyl, $C_{2-8}$ alkenyl, $C_{2-8}$ alkynyl, $C_{1-6}$ alkoxy, $C_{3-7}$ cycloalkyl, 3-7 membered heterocycloalkyl, $C_{5-7}$ aryl, 5-7 membered heteroaryl, $-N(R_9)(R_{10})$, $-N(R_{11})(C(=O)R_{12})$, - $C(=O)-N(R_9)(R_{10})$, $-C(=O)-R_{12}$, $-C(=O)-OR_{12}$, $-OC(=O)R_{12}$, $-N(R_{11})(S(=O)_2R_{12})$, $-S(= O)_2-N(R_9)(R_{10})$, $-SR_{12}$ and $-OR_{12}$, in which the -S-$C_{1-4}$ alkyl, $C_{1-6}$ alkyl, $C_{1-6}$ alkoxy, $C_{3-7}$ cycloalkyl, and 3-7 membered heterocycloalkyl are optionally substituted with 1, 2 or 3 substitutes selected from halogen, -OH, $-NH_2$, $-NH(CH_3)$, $-N(CH_3)_2$, -CN, $C_{1-4}$ alkyl, $C_{3-7}$ cycloalkyl, $C_{1-4}$ hydroxyalkyl, -S-$C_{1-4}$ alkyl, -C(=O)H,

-C(=O)-$C_{1-4}$ alkyl, -C(=O)-O-$C_{1-4}$ alkyl, -C(=O)-$NH_2$, -C(=O)-N($C_{1-4}$ alkyl)$_2$, -N($C_{1-4}$ alkyl)(C(=O) $C_{1-4}$ alkyl), $C_{1-4}$ haloalkyl, $C_{1-4}$ alkoxy and $C_{1-4}$ haloalkoxy; and

$R_{13}$ is H, -N($R_{17}$)($R_{18}$), $C_{1-6}$ alkoxy, -$SR_{12}$, -$OR_{12}$, -CN, halogen, -$NO_2$, -$SF_5$, -S-$C_{1-4}$ alkyl, $C_{1-6}$ alkyl or $C_{3-7}$ cycloalkyl, 3-7 membered heterocycloalkyl, $C_{5-7}$ aryl, 5-7 membered heteroaryl, $C_{7-11}$ bicyclic aryl, 7-11 membered bicyclic heteroaryl, 11-15 membered tricyclyl, $C_{5-11}$ bicycloalkyl, or 5-11 membered bicyclic heteroalkyl, and $R_{13}$ is substituted with 0, 1, 2, 3 or 4 $R_1$(s), in which $R_{17}$ and $R_{18}$ are each independently selected from H, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, $C_{3-7}$ heterocycloalkyl, $C_{5-7}$ aryl, 5-7 membered heteroaryl, $C_{7-11}$ bicyclic aryl, 7-11 membered bicyclic heteroaryl, 11-15 membered tricyclyl, $C_{5-11}$ bicycloalkyl, and 5-11 membered bicyclic heteroalkyl and are optionally substituted with one or more substitutes each independently selected from -OH, - CN, -SH, halogen, -$NO_2$, -$SF_5$, -S-$C_{1-4}$ alkyl, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 4-10 membered heterocycloalkyl, $C_{5-7}$ aryl, 5-7 membered heteroaryl, $C_{7-11}$ bicyclic aryl, 7-11 membered bicyclic heteroaryl, -N($R_9$)($R_{10}$), - N($R_{11}$)(C(=O)$R_{12}$), -C(=O)-N($R_9$)($R_{10}$), -C(=O)-$R_{12}$, -C(=O)-$OR_{12}$, -OC(=O)$R_{12}$, - N($R_{11}$)(S(=O)$_2R_{12}$), -S(=O)$_2$-N($R_9$)($R_{10}$), -$SR_{12}$ and -$OR_{12}$, in which the -S-$C_{1-4}$ alkyl, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 4-10 membered heterocycloalkyl, $C_{5-7}$ aryl, 5-7 membered heteroaryl, $C_{7-11}$ bicyclic aryl, and 7-11 membered bicyclic heteroaryl are optionally substituted with 1, 2 or 3 substitutes each independently selected from halogen, -CN, -OH, $C_{1-4}$ alkyl, $C_{1-4}$ alkoxy, $C_{1-4}$ haloalkyl, $C_{1-4}$ haloalkoxy, $C_{3-6}$ cycloalkyl, -N($R_9$)($R_{10}$), -N($R_{11}$)(C(=O)$R_{12}$), -C(=O)-$OR_{12}$, -C(=O)H, -C(=O)$R_{12}$, -C(= O)-N($R_9$)($R_{10}$), -N($R_{11}$)(S(=O)$_2R_{12}$), -S(=O)$_2$-N($R_9$)($R_{10}$), -$SR_{12}$ and -$OR_{12}$; or $R_{17}$, $R_{18}$ and the N atom connected thereto together form a 3-14 membered ring; and

0, 1, 2, 3 or 4 $R_2$(s) are present in formula (G), and $R_2$ is selected from H, halogen, -OH, -$NO_2$, -CN, -$SF_5$, -SH, -S-$C_{1-4}$ alkyl, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 4-10 membered heterocycloalkyl, $C_{5-7}$ aryl, 5-7 membered heteroaryl, $C_{7-11}$ bicyclic aryl, 7-11 membered bicyclic heteroaryl, -N($R_9$)($R_{10}$), -N($R_{11}$)(C(=O)$R_{12}$), - C(=O)-N($R_9$)($R_{10}$), -C(=O)-$R_{12}$, -C(=O)-$OR_{12}$, -OC(=O)$R_{12}$, -N($R_{11}$)(S(=O)$_2R_{12}$), -S(= O)$_2$-N($R_9$)($R_{10}$), -$SR_{12}$ and -$OR_{12}$, in which the -S-$C_{1-4}$ alkyl, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 4-10 membered heterocycloalkyl, $C_{5-7}$ aryl, 5-7 membered heteroaryl, $C_{7-11}$ bicyclic aryl, and 7-11 membered bicyclic heteroaryl are each optionally substituted with 1, 2 or 3 substituent(s) each independently selected from the group consisting of halogen, -CN, -OH, $C_{1-4}$ alkyl, $C_{1-4}$ alkoxy, $C_{1-4}$ haloalkyl, $C_{1-4}$ haloalkoxy, $C_{3-6}$ cycloalkyl, -N($R_9$)($R_{10}$), -N($R_{11}$)(C(=O)$R_{12}$), -C(=O)-$OR_{12}$, -C(=O)H, -C(=O)$R_{12}$, - C(=O)-N($R_9$)($R_{10}$), -N($R_{11}$)(S(=O)$_2R_{12}$), -S(=O)$_2$-N($R_9$)($R_{10}$), -$SR_{12}$ and -$OR_{12}$; and

$R_1$ is selected from H, halogen, -OH, -$NO_2$, -CN, -$SF_5$, -SH, -S-$C_{1-4}$ alkyl, $C_{1-8}$ alkyl, $C_{2-8}$ alkenyl, $C_{2-8}$ alkynyl, $C_{1-8}$ alkoxy, $C_{3-7}$ cycloalkyl, 3-10 membered heterocycloalkyl, $C_{5-7}$ aryl, 5-7 membered heteroaryl, $C_{7-11}$ bicyclic aryl, 7-11 membered bicyclic heteroaryl, 11-15membered tricyclyl, Cs-nbicycloalkyl, 5-11 membered bicyclic heteroalkyl, -N($R_9$)($R_{10}$), - N($R_{11}$)(C(=O)$R_{12}$), -C(=O)-N($R_9$)($R_{10}$), -C(=O)-$R_{12}$, -C(=O)-$OR_{12}$, -OC(=O)$R_{12}$, - N($R_{11}$)(S(=O)$_2R_{12}$), -S(=O)$_2$-N($R_9$)($R_{10}$), -$SR_{12}$ and -$OR_{12}$, in which the -S-$C_{1-4}$ alkyl, $C_{1-8}$ alkyl, $C_{2-8}$ alkenyl, $C_{2-8}$ alkynyl, and $C_{1-8}$ alkoxy are optionally substituted with 1, 2, 3, or 4 $R_3$(s), and in which the $C_{3-7}$ cycloalkyl, 3-10 membered heterocycloalkyl, $C_{5-7}$ aryl, 5-7 membered heteroaryl, $C_{7-11}$ bicyclic aryl, and 7-11 membered bicyclic heteroaryl are optionally substituted with 1, 2, 3, or 4 $R_4$(s); and

$R_3$ and $R_4$ are each independently selected from H, halogen, -OH, -$NO_2$, -CN, -$SF_5$, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 3-10 membered heterocycloalkyl, $C_{5-7}$ aryl, 5-7 membered heteroaryl, $C_{7-11}$ bicyclic aryl, 7-11 membered bicyclic heteroaryl, -N($R_5$)($R_6$), -N($R_{11}$)(C(=O)$R_{12}$), -CON($R_7$)($R_8$), -C(=O)-$R_{12}$, -C(=O)-$OR_{12}$, -OC(=O)$R_{12}$, -N($R_{11}$)(S(=O)$_2R_{12}$), -S(=O)$_2$-N($R_9$)($R_{10}$), -$SR_{12}$ and -$OR_{12}$, in which the $C_{1-6}$ alkyl, $C_{3-7}$ cycloalkyl, 3-10 membered heterocycloalkyl, $C_{5-7}$ aryl, 5-7 membered heteroaryl, $C_{7-11}$ bicyclic aryl, and 7-11 membered bicyclic heteroaryl are each optionally substituted with 1, 2, 3 or 4 substituent(s) each independently selected from the group consisting of halogen, -CN, -OH, $C_{1-4}$ alkyl, $C_{1-6}$ alkoxy, $C_{1-4}$ haloalkyl, $C_{1-4}$ haloalkoxy, $C_{3-6}$ cycloalkyl, -N($R_9$)($R_{10}$), -N($R_{11}$)(C(=O)$R_{12}$), -C(=O)-$OR_{12}$, -C(=O)H, -C(=O)$R_{12}$, -C(= O)-N($R_9$)($R_{10}$), -N($R_{11}$)(S(=O)$_2R_{12}$), -S(=O)$_2$-N($R_9$)($R_{10}$), -$SR_{12}$ and -$OR_{12}$; and

$R_5$, $R_6$, $R_7$, $R_8$, $R_9$, $R_{10}$, $R_{11}$, and $R_{12}$ are each independently H or selected from the group consisting of $C_{1-6}$ alkyl, $C_{1-4}$ haloalkyl, $C_{3-7}$ cycloalkyl, 4-14 membered heterocycloalkyl, $C_{6-10}$ aryl, 5-10 membered heteroaryl, ($C_{3-7}$ cycloalkyl)-$C_{1-4}$ alkyl-, (4-10 membered heterocycloalkyl)-$C_{1-4}$ alkyl-, ($C_{6-10}$ aryl)-$C_{1-4}$ alkyl- and (5-10 membered heteroaryl)-$C_{1-4}$ alkyl-, wherein the substituents included in the above group are each optionally substituted with 1, 2, 3 or 4 substituent(s) each independently selected from the group consisting of halogen, -$CF_3$, -OH, -$NH_2$, -NH($CH_3$), -N($CH_3$)$_2$, -CN, oxo, $C_{1-4}$ alkyl, $C_{2-6}$ alkenyl, $C_{2-6}$ alkynyl, $C_{3-7}$ cycloalkyl, $C_{1-4}$ hydroxyalkyl, -S-$C_{1-4}$ alkyl, -C(=O)H, -C(=O)-$C_{1-4}$ alkyl, - C(=O)-O-$C_{1-4}$ alkyl, -C(=O)-$NH_2$, -C(=O)-N($C_{1-4}$ alkyl)$_2$, $C_{1-4}$ haloalkyl, $C_{1-4}$ alkoxy and $C_{1-4}$ haloalkoxy.

In some preferred embodiments of the present disclosure, use of an isotopically labeled compound of the above-mentioned compound of formula (G) in the manufacture of a medicament for treating severe pneumonia is provided. In some more preferred embodiments of the present disclosure, in an isotopically labeled compound of the

compound of formula (G), all Hs are each independently and optionally substituted with D.

In some preferred embodiments of the present disclosure, in formula (G), $X_1$ is N. In some preferred embodiments of the present disclosure, in formula (G), $X_2$ is N. In some preferred embodiments of the present disclosure, in formula (G), $X_3$ is N. In some preferred embodiments of the present disclosure, in formula (G), $X_1$ is $CR_{14}$, $X_2$ is N or $CR_{15}$, and $X_3$ is $CR_{16}$. In some preferred embodiments of the present disclosure, in formula (G), $X_1$ is $CR_{14}$, $X_2$ is $CR_{15}$, and $X_3$ is $CR_{16}$. In some preferred embodiments of the present disclosure, in formula (G), $X_1$ is $CR_{14}$, $X_2$ is $CR_{15}$, $X_3$ is $CR_{16}$, and $R_{14}$, $R_{15}$, and $R_{16}$ are each independently selected from H, -OH, -CN, halogen, $C_{1-6}$ alkyl, $C_{1-6}$ alkoxy, $C_{3-7}$ cycloalkyl, and 3-7 membered heterocycloalkyl. In some preferred embodiments of the present disclosure, in formula (G), $X_1$ is $CR_{14}$, $X_2$ is N, $X_3$ is $CR_{16}$, and $R_{14}$ and $R_{16}$ are each independently selected from H, -OH, -CN, halogen, $C_{1-6}$ alkyl, $C_{1-6}$ alkoxy, $C_{3-7}$ cycloalkyl, and 3-7 membered heterocycloalkyl. In some preferred embodiments of the present disclosure, in formula (G), $X_1$, $X_2$, and $X_3$ are the same. In some preferred embodiments of the present disclosure, in formula (G), $X_1$, $X_2$ and $X_3$ are CH. In some preferred embodiments of the present disclosure, in formula (G), $X_1$, $X_2$ and $X_3$ are N. In some preferred embodiments of the present disclosure, in formula (G), $X_1$ is $C(CH_3)$, $X_2$ and $X_3$ are CH. In some preferred embodiments of the present disclosure, in formula (G), $X_2$ is $C(CH_3)$, $X_1$ and $X_3$ are CH. In some preferred embodiments of the present disclosure, in formula (G), $X_3$ is $C(CH_3)$, $X_1$ and $X_2$ are CH. In some preferred embodiments of the present disclosure, in formula (G), $X_1$ is N, $X_2$ and $X_3$ are CH. In some preferred embodiments of the present disclosure, in formula (G), $X_2$ is N, $X_1$ and $X_3$ are CH. In some preferred embodiments of the present disclosure, in formula (G), $X_3$ is N, $X_1$ and $X_2$ are CH.

In some more preferred embodiments of the present disclosure, in an isotopically labeled compound of the compound of formula (G), all Hs are each independently and optionally substituted with D, and $X_1$, $X_2$ and $X_3$ are the same. In some more preferred embodiments of the present disclosure, in an isotopically labeled compound of the compound of formula (G), all Hs are each independently and optionally substituted with D, and $X_1$, $X_2$ and $X_3$ are all CH. In some more preferred embodiments of the present disclosure, in an isotopically labeled compound of the compound of formula (G), all Hs are each independently and optionally substituted with D, and $X_1$, $X_2$ and $X_3$ are N. In some more preferred embodiments of the present disclosure, in an isotopically labeled compound of the compound of formula (G), all H are each independently and optionally substituted with D, and $X_1$ is $C(CH_3)$, $X_2$ and $X_3$ are both CH. In some more preferred embodiments of the present disclosure, in an isotopically labeled compound of the compound of formula (G), all Hs are each independently and optionally substituted with D, and $X_2$ is $C(CH_3)$, $X_1$ and $X_3$ are both CH. In some more preferred embodiments of the present disclosure, in an isotopically labeled compound of the compound of formula (G), all Hs are each independently and optionally substituted with D, and $X_3$ is $C(CH_3)$, and $X_1$ and $X_2$ are both CH. In some more preferred embodiments of the present disclosure, in an isotopically labeled compound of the compound of formula (G), all Hs are each independently and optionally substituted with D, and $X_1$ is N, $X_2$ and $X_3$ are both CH. In some more preferred embodiments of the present disclosure, in an isotopically labeled compound of the compound of formula (G), all Hs are each independently and optionally substituted with D, and $X_2$ is N, $X_1$ and $X_3$ are both CH. In some more preferred embodiments of the present disclosure, in an isotopically labeled compound of the compound of formula (G), all Hs are each independently and optionally substituted with D, and $X_3$ is N, and $X_1$ and $X_2$ are both CH.

In some preferred embodiments of the present disclosure, in formula (G), L is C=O, O=S=O or $CH_2$. In some particularly preferred embodiments of the present disclosure, in formula (G), L is C=O. In some particularly preferred embodiments of the present disclosure, in formula (G), L is O=S=O. In some particularly preferred embodiments of the present disclosure, in formula (G), L is $CH_2$. In other embodiments of the present disclosure, in formula (G), L is a covalent bond.

In some particularly preferred embodiments of the present disclosure, in formula (G), $X_1$, $X_2$ and $X_3$ are all CH, and L is C=O.

In some particularly preferred embodiments of the present disclosure, in formula (G), $X_1$, $X_2$ and $X_3$ are all CH, and L is O=S=O.

In some particularly preferred embodiments of the present disclosure, in formula (G), $X_1$, $X_2$ and $X_3$ are all CH, and L is $CH_2$.

In some particularly preferred embodiments of the present disclosure, in formula (G), $X_1$, $X_2$ and $X_3$ are all CH, and L is a covalent bond.

In some particularly preferred embodiments of the present disclosure, in formula (G), $X_1$, $X_2$ and $X_3$ are all N, and L is C=O.

In some particularly preferred embodiments of the present disclosure, in formula (G), $X_1$, $X_2$ and $X_3$ are all N, and L is O=S=O.

In some particularly preferred embodiments of the present disclosure, in formula (G), $X_1$, $X_2$ and $X_3$ are all N, and L is $CH_2$.

In some particularly preferred embodiments of the present disclosure, in formula (G), $X_1$, $X_2$ and $X_3$ are all N, and L is a covalent bond.

In some particularly preferred embodiments of the present disclosure, in formula (G), $X_1$, $X_2$ and $X_3$ are all

$CR_{14}$, wherein $R_{14}$ is selected from -OH, -CN, halogen, $C_{1-6}$ alkyl, $C_{1-6}$ alkoxy, $C_{3-7}$ cycloalkyl, and 3-7 membered heterocycloalkyl, and L is C=O.

In some particularly preferred embodiments of the present disclosure, in formula (G), $X_1$, $X_2$ and $X_3$ are all $CR_{14}$, wherein $R_{14}$ is selected from -OH, -CN, halogen, $C_{1-6}$ alkyl, $C_{1-6}$ alkoxy, $C_{3-7}$ cycloalkyl, and 3-7 membered heterocycloalkyl, and L is O=S=O.

In some particularly preferred embodiments of the present disclosure, in formula (G), $X_1$, $X_2$ and $X_3$ are all $CR_{14}$, wherein $R_{14}$ is selected from -OH, -CN, halogen, $C_{1-6}$ alkyl, $C_{1-6}$ alkoxy, $C_{3-7}$ cycloalkyl, and 3-7 membered heterocycloalkyl, and L is $CH_2$.

In some particularly preferred embodiments of the present disclosure, in formula (G), $X_1$, $X_2$ and $X_3$ are all $CR_{14}$, wherein $R_{14}$ is selected from -OH, -CN, halogen, $C_{1-6}$ alkyl, $C_{1-6}$ alkoxy, $C_{3-7}$ cycloalkyl, and 3-7 membered heterocycloalkyl, and L is a covalent bond.

In some particularly preferred embodiments of the present disclosure, in formula (G), $X_1$ is $C(CH_3)$, $X_2$ and $X_3$ are both CH, and L is C=O.

In some particularly preferred embodiments of the present disclosure, in formula (G), $X_1$ is $C(CH_3)$, $X_2$ and $X_3$ are both CH, and L is O=S=O.

In some particularly preferred embodiments of the present disclosure, in formula (G), $X_1$ is $C(CH_3)$, $X_2$ and $X_3$ are both CH, and L is $CH_2$.

In some particularly preferred embodiments of the present disclosure, in formula (G), $X_1$ is $C(CH_3)$, $X_2$ and $X_3$ are both CH, and L is a covalent bond.

In some particularly preferred embodiments of the present disclosure, in formula (G), $X_2$ is $C(CH_3)$, $X_1$ and $X_3$ are both CH, and L is C=O.

In some particularly preferred embodiments of the present disclosure, in formula (G), $X_2$ is $C(CH_3)$, $X_1$ and $X_3$ are both CH, and L is O=S=O.

In some particularly preferred embodiments of the present disclosure, in formula (G), $X_2$ is $C(CH_3)$, $X_1$ and $X_3$ are both CH, and L is $CH_2$.

In some particularly preferred embodiments of the present disclosure, in formula (G), $X_2$ is $C(CH_3)$, $X_1$ and $X_3$ are both CH, and L is a covalent bond.

In some particularly preferred embodiments of the present disclosure, in formula (G), $X_3$is $C(CH_3)$, $X_1$ and $X_2$ are both CH, and L is C=O.

In some particularly preferred embodiments of the present disclosure, in formula (G), $X_3$ is $C(CH_3)$, $X_1$ and $X_2$ are both CH, and L is O=S=O.

In some particularly preferred embodiments of the present disclosure, in formula (G), $X_3$ is $C(CH_3)$, $X_1$ and $X_2$ are both CH, and L is $CH_2$.

In some particularly preferred embodiments of the present disclosure, in formula (G), $X_3$ is $C(CH_3)$, $X_1$ and $X_2$ are both CH, and L is a covalent bond.

In some particularly preferred embodiments of the present disclosure, in formula (G), $X_1$ is N, $X_2$ and $X_3$ are both CH, and L is C=O.

In some particularly preferred embodiments of the present disclosure, in formula (G), $X_1$ is N, $X_2$ and $X_3$ are both CH, and L is O=S=O.

In some particularly preferred embodiments of the present disclosure, in formula (G), $X_1$ is N, $X_2$ and $X_3$ are both CH, and L is $CH_2$.

In some particularly preferred embodiments of the present disclosure, in formula (G), $X_1$ is N, $X_2$ and $X_3$ are both CH, and L is a covalent bond.

In some particularly preferred embodiments of the present disclosure, in formula (G), $X_2$ is N, $X_1$ and $X_3$ are both CH, and L is C=O.

In some particularly preferred embodiments of the present disclosure, in formula (G), $X_2$ is N, $X_1$ and $X_3$ are both CH, and L is O=S=O.

In some particularly preferred embodiments of the present disclosure, in formula (G), $X_2$ is N, $X_1$ and $X_3$ are both CH, and L is $CH_2$.

In some particularly preferred embodiments of the present disclosure, in formula (G), $X_2$ is N, $X_1$ and $X_3$ are both CH, and L is a covalent bond.

In some particularly preferred embodiments of the present disclosure, in formula (G), $X_3$ is N, $X_1$ and $X_2$ are both CH, and L is C=O.

In some particularly preferred embodiments of the present disclosure, in formula (G), $X_3$ is N, $X_1$ and $X_2$ are both CH, and L is O=S=O.

In some particularly preferred embodiments of the present disclosure, in formula (G), $X_3$ is N, $X_1$ and $X_2$ are both CH, and L is $CH_2$.

In some particularly preferred embodiments of the present disclosure, in formula (G), $X_3$ is N, $X_1$ and $X_2$ are

both CH, and L is a covalent bond.

In some preferred embodiments of the present disclosure, in formula (G), $R_{13}$ is H, - $N(R_{17})(R_{18})$, $C_{1-6}$ alkoxy, -OH, -SH, -CN, halogen, $-NO_2$, $-SF_5$, -S-$C_{1-4}$ alkyl, $C_{1-6}$ alkyl, or $C_{3-7}$ cycloalkyl, 3-7 membered heterocycloalkyl, $C_{5-7}$ aryl, 5-7 membered heteroaryl, $C_{7-11}$ bicyclic aryl, 7-1 1membered bicyclic heteroaryl, 11-15 membered tricyclyl, Cs-nbicycloalkyl, or 5-11 membered bicyclic heteroalkyl, in which $R_{17}$ and $R_{18}$ are each independently selected from H, $C_{1-6}$ alkyl, $C_{1-6}$ alkoxy, $C_{3-7}$ cycloalkyl, $C_{3-7}$ heterocycloalkyl, $C_{5-7}$ aryl, and 5-7 membered heteroaryl, and are optionally substituted with one or more of -OH, -CN, -SH, halogen, $-NO_2$,-and $SF_5$, wherein $R_{13}$ is optionally substituted with 1, 2, 3 or 4 $R_1$(s). In some preferred embodiments of the present disclosure, in formula (G), $R_{13}$ is H, $-N(R_{17})(R_{18})$, $C_{1-6}$ alkoxy, -OH, -SH, -CN, halogen, $-NO_2$, $-SF_5$, -S-$C_{1-4}$ alkyl, $C_{1-6}$ alkyl, or $C_{3-7}$ cycloalkyl, 3-7 membered heterocycloalkyl, $C_{5-7}$ aryl, 5-7 membered heteroaryl, $C_{7-11}$ bicyclic aryl, 7-11membered bicyclic heteroaryl, or 11-15 membered tricyclyl and $R_{17}$ and $R_{18}$ are defined as above, wherein $R_{13}$ is optionally substituted with 1, 2, 3 or 4 $R_1$(s). In some preferred embodiments of the present disclosure, in formula (G), $R_{13}$ is H, $-N(R_{17})(R_{18})$, $C_{1-6}$ alkoxy, $C_{1-6}$ alkyl, or $C_{3-7}$ cycloalkyl, 3-7 membered heterocycloalkyl, $C_{5-7}$ aryl, or 5-7 membered heteroaryl, and $R_{17}$ and $R_{18}$ are defined as above, wherein $R_{13}$ is optionally substituted with 1, 2, 3 or 4 $R_1$(s). In some preferred embodiments of the present disclosure, in formula (G), $R_{13}$ is $-N(R_{17})(R_{18})$, $C_{1-6}$ alkoxy, $C_{1-6}$ alkyl, or $C_{3-7}$ cycloalkyl, 4-6 membered heterocycloalkyl, phenyl, or 5-6 membered heteroaryl, and $R_{17}$ and $R_{18}$ are defined as above, wherein $R_{13}$ is optionally substituted with 1, 2, or 3 $R_1$(s). In some preferred embodiments of the present disclosure, in formula (G), $R_{13}$ is $-N(R_{17})(R_{18})$, $C_{1-3}$ alkoxy, $C_{3-6}$ cycloalkyl, 4-6 membered heterocycloalkyl, phenyl, 5-6 membered heteroaryl or $C_{1-4}$ alkyl, and $R_{17}$ and $R_{18}$ are defined as above, wherein $R_{13}$ is optionally substituted with 1, 2, or 3 $R_1$(s). In some preferred embodiments of the present disclosure, in formula (G), $R_{13}$ is -N(H)($C_{1-3}$ alkyl), -N(H)(3-6 membered cycloalkyl), -N(H) (4-6 membered heterocycloalkyl), -N ($C_{1-3}$ alkyl) ($C_{1-3}$ alkyl), $C_{1-3}$ alkoxy, C3-6 cycloalkyl, 4-6 membered azacycloalkyl or oxacycloalkyl, phenyl, 5-6 membered azaaryl or $C_{1-4}$ alkyl; or $R_{13}$ is $-N(R_{17})(R_{18})$, and $R_{17}$ and $R_{18}$ and the N atom connected thereto together form a 4-10 membered ring (where $R_{13}$ is optionally substituted with 1, 2, or 3 $R_1$(s)). In some particularly preferred embodiments of the present disclosure, in formula (G), $R_{13}$ is cyclopropyl, cyclobutyl, cyclopentyl, cyclohexyl, methyl, ethyl, propyl, butyl, methoxy, ethoxy, propoxy, $-N(H)(CH_3)$, $-N(H)(CH_2CH_3)$, $-N(H)(CH_2CH_2OH)$, - $N(H)(CH_2CH_2CN)$, $-N(CH_3)(CH_3)$, -N(H)(cyclopropyl), -N(H)(cyclobutyl), - N(H)(tetrahydrofuranyl), pyrazinyl, pyridazinyl, pyrrolidinyl, pyrazolyl, piperidinyl, phenyl, azetidinyl, morpholinyl, piperazinyl or tetrahydropyranyl; or $R_{13}$ is $-N(R_{17})(R_{18})$, and $R_{17}$ and $R_{18}$ and the N atom connected thereto together form a 7-membered ring (where $R_{13}$ is optionally substituted with 1, 2, or 3 $R_1$(s)). In some particularly preferred embodiments of the present disclosure, in formula (G), $R_{13}$ is cyclopropyl. In some particularly preferred embodiments of the present disclosure, in formula (G), $R_{13}$ is cyclobutyl. In some particularly preferred embodiments of the present disclosure, in formula (G), $R_{13}$ is cyclopentyl. In some particularly preferred embodiments of the present disclosure, in formula (G), $R_{13}$ is cyclohexyl. In some particularly preferred embodiments of the present disclosure, in formula (G), $R_{13}$ is a methyl group. In some particularly preferred embodiments of the present disclosure, in formula (G), $R_{13}$ is an ethyl group. In some particularly preferred embodiments of the present disclosure, in formula (G), $R_{13}$ is propyl. In some particularly preferred embodiments of the present disclosure, in formula (G), $R_{13}$ is a butyl group. In some particularly preferred embodiments of the present disclosure, in formula (G), $R_{13}$ is pyrazinyl. In some particularly preferred embodiments of the present disclosure, in formula (G), $R_{13}$ is a pyridazinyl. In some particularly preferred embodiments of the present disclosure, in formula (G), $R_{13}$ is a pyrrolidinyl. In some particularly preferred embodiments of the present disclosure, in formula (G), $R_{13}$ is pyrazolyl. In some particularly preferred embodiments of the present disclosure, in formula (G), $R_{13}$ is piperidinyl. In some particularly preferred embodiments of the present disclosure, in formula (G), $R_{13}$ is phenyl. In some particularly preferred embodiments of the present disclosure, in formula (G), $R_{13}$ is azetidinyl. In some particularly preferred embodiments of the present disclosure, in formula (G), $R_{13}$ is morpholinyl. In some particularly preferred embodiments of the present disclosure, in formula (G), $R_{13}$ is piperazinyl. In some particularly preferred embodiments of the present disclosure, in formula (G), $R_{13}$ is tetrahydropyranyl. In some particularly preferred embodiments of the present disclosure, in formula (G), $R_{13}$ is methoxy. In some particularly preferred embodiments of the present disclosure, in formula (G), $R_{13}$ is ethoxy. In some particularly preferred embodiments of the present disclosure, in formula (G), $R_{13}$ is $-N(H)(CH_3)$. In some particularly preferred embodiments of the present disclosure, in formula (G), $R_{13}$ is $-N(H)(CH_2CH_3)$. In some particularly preferred embodiments of the present disclosure, in formula (G), $R_{13}$ is - $N(H)(CH_2CH_2OH)$. In some particularly preferred embodiments of the present disclosure, in formula (G), $R_{13}$ is $-N(H)(CH_2CH_2CN)$. In some particularly preferred embodiments of the present disclosure, in formula (G), $R_{13}$ is $-N(CH_3)(CH_3)$. In some particularly preferred embodiments of the present disclosure, in formula (G), $R_{13}$ is -N(H) (cyclopropyl). In some particularly preferred embodiments of the present disclosure, in formula (G), $R_{13}$ is -N(H) (cyclobutyl). In some particularly preferred embodiments of the present disclosure, in formula (G), $R_{13}$ is -N(H) (tetrahydrofuranyl). In some particularly preferred embodiments of the present disclosure, in formula (G), $R_{13}$ is $-N(R_{17})(R_{18})$, and $R_{17}$ and $R_{18}$ and the N atom connected to them together form a 7-membered ring.

In some particularly preferred embodiments of the present disclosure, in formula (G), $R_{17}$ and $R_{18}$ are each independently selected from H, $C_{1-6}$ alkyl, $C_{1-6}$ alkoxy, $C_{3-7}$ cycloalkyl, $C_{3-7}$ heterocycloalkyl, $C_{5-7}$ aryl, and 5-7 membered heteroaryl, and are optionally substituted with one or more of -OH, -CN, -SH, halogen, $-NO_2$, and $SF_5$. In some particularly

preferred embodiments of the present disclosure, in formula (G), $R_{17}$ and $R_{18}$ are each independently selected from H, $C_{1-6}$ alkyl, $C_{3-7}$ cycloalkyl, and $C_{3-7}$ heterocycloalkyl and are optionally substituted with one or more of -OH, -CN, -SH, halogen, $-NO_2$, and $SF_5$. In some particularly preferred embodiments of the present disclosure, in formula (G), $R_{17}$ and $R_{18}$ are each independently selected from H, $C_{1-6}$ alkyl, $C_{3-7}$ cycloalkyl, and $C_{3-7}$ heterocycloalkyl and are optionally substituted with one or more of -OH and -CN. In some preferred embodiments of the present disclosure, in formula (G), $R_{17}$ and $R_{18}$ are each independently selected from H, methyl, ethyl, propyl, 3-membered cycloalkyl, 4-membered cycloalkyl, 5-membered cycloalkyl, 5-membered heterocycloalkyl, and 6-membered heterocycloalkyl, and optionally substituted with one or more of -OH and -CN. In some preferred embodiments of the present disclosure, in formula (G), $R_{17}$, $R_{18}$ and the N atom connected thereto together form a 4-10 membered ring. In some preferred embodiments of the present disclosure, in formula (G), $R_{17}$, $R_{18}$ and the N atom connected thereto together form a 7-membered ring.

In some particularly preferred embodiments of the present disclosure, in formula (G), L is C=O, and $R_{13}$ is $-N(R_{17})(R_{18})$, $C_{1-6}$ alkoxy, -OH, -SH, -CN, halogen, $-NO_2$, $-SF_5$, or $-S-C_{1-4}$ alkyl, and $R_{13}$ is substituted with 0, 1, 2, 3 or 4 $R_1$(s) in which $R_{17}$ and $R_{18}$ are each independently selected from H, $C_{1-6}$ alkyl, $C_{1-6}$ alkoxy, $C_{3-7}$ cycloalkyl, $C_{3-7}$ heterocycloalkyl, $C_{5-7}$ aryl, and 5-7 membered heteroaryl, and are optionally substituted with one or more of - OH, -CN, -SH, halogen, $-NO_2$,-and $SF_5$, or $R_{17}$, $R_{18}$ and the N atom connected thereto together form a 3-14 membered ring. In some particularly preferred embodiments of the present disclosure, in formula (G), L is C=O, and $R_{13}$ is $-N(R_{17})(R_{18})$, or $C_{1-6}$ alkoxy, in which $R_{17}$ and $R_{18}$ are each independently selected from H, $C_{1-6}$ alkyl, $C_{3-7}$ cycloalkyl, and $C_{3-7}$ heterocycloalkyl and are optionally substituted with one or more of -OH, -CN, -SH, halogen, $-NO_2$,-and $SF_5$, or $R_{17}$, $R_{18}$ and the N atom connected thereto together form a 3-10 membered ring. In some particularly preferred embodiments of the present disclosure, in formula (G), L is C=O, and $R_{13}$ is methoxy, ethoxy, propoxy, $-N(H)(CH_3)$, - N(H)(CH2$CH_3$), - N(H)($CH_2CH_2OH$), -N(H)($CH_2CH_2CN$), $-N(CH_3)(CH_3)$, -N(H)(cyclopropyl), -N(H) (cyclobutyl), -N(H) (tetrahydrofuranyl); or $R_{13}$ is $-N(R_{17})(R_{18})$, and $R_{17}$, $R_{18}$ and the N atom connected thereto together form a 7-membered ring. In some particularly preferred embodiments of the present disclosure, in formula (G), L is C=O, and $R_{13}$ is methoxy. In some particularly preferred embodiments of the present disclosure, in formula (G), L is C=O, and $R_{13}$ is ethoxy. In some particularly preferred embodiments of the present disclosure, in formula (G), L is C=O, and $R_{13}$ is $-N(H)(CH_3)$. In some particularly preferred embodiments of the present disclosure, in formula (G), L is C=O, and $R_{13}$ is $-N(H)(CH_2CH_3)$. In some particularly preferred embodiments of the present disclosure, in formula (G), L is C=O, and $R_{13}$ is $-N(H)(CH_2CH_2OH)$. In some particularly preferred embodiments of the present disclosure, in formula (G), L is C=O, and $R_{13}$ is $-N(H)(CH_2CH_2CN)$. In some particularly preferred embodiments of the present disclosure, in formula (G), L is C=O, and $R_{13}$ is - $N(CH_3)(CH_3)$. In some particularly preferred embodiments of the present disclosure, in formula (G), L is C=O, and $R_{13}$ is -N(H)(cyclopropyl). In some particularly preferred embodiments of the present disclosure, in formula (G), L is C=O, and $R_{13}$ is -N(H)(cyclobutyl). In some particularly preferred embodiments of the present disclosure, in formula (G), L is C=O, and $R_{13}$ is -N(H)(tetrahydrofuranyl). In some particularly preferred embodiments of the present disclosure, in the formula (G), L is C=O, and $R_{13}$ is $-N(R_{17})(R_{18})$, and $R_{17}$, $R_{18}$ and the N atom connected thereto together form a 7-membered ring.

In some particularly preferred embodiments of the present disclosure, in formula (G), one, two or three $R_2$(s) are present and $R_2$ is selected from H, halogen, -OH, $-NO_2$, -CN, $-SF_5$, -SH, $-S-C_{1-4}$ alkyl, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, and 4-10 membered heterocycloalkyl, in which the $-S-C_{1-4}$ alkyl, $C_{1-6}$ alkyl, $C_{3-7}$ cycloalkyl, and 4-10 membered heterocycloalkyl are each optionally substituted with 1, 2 or 3 substituent(s) each independently selected from the group consisting of halogen, -OH, $-NH_2$, $-NH(CH_3)$, $-N(CH_3)_2$, -CN, $C_{1-4}$ alkyl, $C_{1-4}$ haloalkyl, $C_{1-4}$ alkoxy, and $C_{1-4}$ haloalkoxy. In some particularly preferred embodiments of the present disclosure, in formula (G), one, two or three $R_2$(s) are present and $R_2$ is selected from halogen, $C_{1-6}$ alkyl and $C_{3-6}$ cycloalkyl, in which the $C_{1-6}$ alkyl and $C_{3-6}$ cycloalkyl are each optionally substituted with 1, 2 or 3 substituent(s) each independently selected from the group consisting of halogen, -OH, $-NH_2$, $-NH(CH_3)$, $-N(CH_3)_2$, -CN, $C_{1-4}$ alkyl, $C_{1-4}$ haloalkyl, $C_{1-4}$ alkoxy, and $C_{1-4}$ haloalkoxy. In some particularly preferred embodiments of the present disclosure, in formula (G), one, two or three $R_2$(s) are present and $R_2$ is selected from halogen, and $C_{1-6}$ alkyl, in which the $C_{1-6}$ alkyl is optionally substituted with 1, 2 or 3 substituent(s) each independently selected from the group consisting of halogen, -OH, $-NH_2$, $-NH(CH_3)$, $-N(CH_3)_2$, -CN, $C_{1-4}$ alkyl, $C_{1-4}$ haloalkyl, $C_{1-4}$ alkoxy, and $C_{1-4}$ haloalkoxy. In some particularly preferred embodiments of the present disclosure, in formula (G), one, or two $R_2$(s) are present and $R_2$ is selected from halogen, and $C_{1-6}$ alkyl. In some particularly preferred embodiments of the present disclosure, in formula (G), one or two $R_2$(s) are present and $R_2$ is selected from fluorine, chlorine, bromine, methyl, ethyl, n-propyl, isopropyl, n-butyl, and isobutyl. In some preferred embodiments of the present disclosure, in formula (G), one or two $R_2$(s) are present, and $R_2$ is selected from fluorine, chlorine, methyl, ethyl, n-propyl, and isopropyl. In some preferred embodiments of the present disclosure, in formula (G), one or two $R_2$(s) are present, and $R_2$ is selected from fluorine, methyl, and ethyl. In some preferred embodiments of the present disclosure, in formula (G), one or two $R_2$ (s) are present, and $R_2$ is selected from fluorine and ethyl. In some preferred embodiments of the present disclosure, in formula (G), one $R_2$ is present, and $R_2$ is selected from fluorine and ethyl. In some preferred embodiments of the present disclosure, in formula (G), two $R_2$(s) are present, and $R_2$ is selected from fluorine and ethyl. In some particularly preferred embodiments of the present disclosure, in

formula (G), two $R_2$(s) are present which are respectively fluorine and ethyl. In some particularly preferred embodiments of the present disclosure, in formula (G), one $R_2$ is present, and $R_2$ is an ethyl group.

In some preferred embodiments of the present disclosure, in formula (G), $R_{13}$ is substituted with 0, 1, 2, 3 or 4 $R_1$(s), and each $R_1$ is independently selected from H, halogen, -OH, -$NO_2$,- CN, -$SF_5$, -SH, -S-$C_{1-4}$ alkyl, $C_{1-8}$ alkyl, $C_{2-8}$ alkenyl, $C_{2-8}$ alkynyl, $C_{1-8}$ alkoxy, $C_{3-7}$ cycloalkyl, 3 -10 membered heterocycloalkyl, $C_{5-7}$ aryl, 5-7 membered heteroaryl, $C_{7-11}$ bicyclic aryl, and 7-11 membered bicyclic heteroaryl, wherein the -S-$C_{1-4}$ alkyl, $C_{1-8}$ alkyl, $C_{2-8}$ alkenyl, $C_{2-8}$ alkynyl, and $C_{1-8}$ alkoxy are optionally substituted with 1, 2, 3, or 4 $R_3$(s), and wherein the $C_{3-7}$ cycloalkyl, 3-10 membered heterocycloalkyl, $C_{5-7}$ aryl, 5-7 membered heteroaryl, $C_{7-11}$ bicyclic aryl, and 7-11 membered bicyclic heteroaryl are optionally substituted with 1, 2 , 3 or 4 $R_4$(s). In some preferred embodiments of the present disclosure, in formula (G), $R_{13}$ is substituted with 0, 1, 2, 3 or 4 $R_1$(s), and each $R_1$ is independently selected from H, halogen, -OH, -$NO_2$,- CN, -$SF_5$, -SH, -S-$C_{1-4}$ alkyl, $C_{1-8}$ alkyl, $C_{3-7}$ cycloalkyl, 3-7 membered heterocycloalkyl, $C_{5-7}$ aryl, and 5-7 membered heteroaryl, wherein the -S-$C_{1-4}$ alkyl, and $C_{1-8}$ alkyl are optionally substituted with 1, 2, 3 or 4 $R_3$(s), and wherein the $C_{3-7}$ cycloalkyl, 3-7 membered heterocycloalkyl, $C_{5-7}$ aryl, and 5-7 membered heteroaryl are optionally substituted with 1, 2, 3, or 4 $R_4$(s). In some preferred embodiments of the present disclosure, in formula (G), $R_{13}$ is substituted with 0, 1, 2, 3 or 4 $R_1$(s), and each R1 is independently selected from halogen, -OH, -CN, $C_{1-8}$ alkyl, $C_{3-7}$ cycloalkyl, 3-7 membered heterocycloalkyl, $C_{5-7}$ aryl, 5-7 membered heteroaryl, wherein the $C_{1-8}$ alkyl is optionally substituted with 1, 2 , 3 or 4 $R_3$(s), and wherein the $C_{3-7}$ cycloalkyl, 3-7 membered heterocycloalkyl, $C_{5-7}$ aryl, 5-7 membered heteroaryl are optionally substituted with 1, 2, 3 or 4 $R_4$(s). In some preferred embodiments of the present disclosure, in formula (G), $R_{13}$ is substituted with 0, 1, 2, 3 or 4 $R_1$(s), and each $R_1$ is independently selected from halogen, - OH, -CN, $C_{1-8}$ alkyl, $C_{3-7}$ cycloalkyl, and 3-7 membered heterocycloalkyl, wherein the $C_{1-8}$ alkyl is optionally substituted with 1, 2, or 3 $R_3$(s), and wherein the $C_{3-7}$ cycloalkyl, and 3-7 membered heterocycloalkyl are optionally substituted with 1, 2, or 3 $R_4$(s). In some preferred embodiments of the present disclosure, in formula (G), $R_{13}$ is substituted with 0 or 1 $R_1$, and each $R_1$ is independently selected from halogen, -OH, -CN, $C_{1-6}$ alkyl, $C_{3-7}$ cycloalkyl, 5-7 membered heterocycloalkyl, wherein the $C_{1-6}$ alkyl is optionally substituted with 1, 2, or 3 $R_3$(s), and wherein the $C_{3-7}$ cycloalkyl, and 5-7-membered heterocycloalkyl are optionally substituted with 1, 2, or 3 $R_4$(s). In some preferred embodiments of the present disclosure, in formula (G), $R_{13}$ is substituted with 0 or 1 $R_1$, and each $R_1$ is independently selected from halogen, -OH, -CN, $C_{1-4}$ alkyl, $C_{3-6}$ cycloalkyl, and 5-7 membered heterocycloalkyl, wherein the $C_{1-4}$ alkyl is optionally substituted with 1 or 2 $R_3$(s), and wherein the $C_{3-6}$ cycloalkyl and 5-7 membered heterocycloalkyl are optionally substituted with 1, 2, or 3 $R_4$(s). In some particularly preferred embodiments of the present disclosure, in formula (G), $R_{13}$ is substituted with 0 or 1 $R_1$, and each $R_1$ is independently selected from methyl, ethyl, hydroxyl, -CN, piperidinyl, morpholinyl, piperazinyl, and cyclopropyl, wherein the piperidinyl, morpholinyl, and piperazinyl are optionally substituted with 1, 2, 3 or 4 $C_{1-3}$ alkyl. In some particularly preferred embodiments of the present disclosure, in formula (G), $R_{13}$ is substituted with 0 or 1 $R_1$, and each $R_1$ is independently selected from methyl, ethyl, hydroxy, -CN, piperidinyl, morpholinyl, 1-methylpiperazinyl, and cyclopropyl. In some particularly preferred embodiments of the present disclosure, in formula (G), $R_1$ is absent. In some particularly preferred embodiments of the present disclosure, in formula (G), $R_1$ is 1-methylpiperazinyl. In some particularly preferred embodiments of the present disclosure, in formula (G), $R_1$ is methyl. In some particularly preferred embodiments of the present disclosure, in formula (G), $R_1$ is ethyl. In some particularly preferred embodiments of the present disclosure, in formula (G), $R_1$ is piperidinyl. In some particularly preferred embodiments of the present disclosure, in formula (G), $R_1$ is morpholinyl. In some particularly preferred embodiments of the present disclosure, in formula (G), $R_1$ is hydroxyl. In some particularly preferred embodiments of the present disclosure, in formula (G), $R_1$ is -CN. In some particularly preferred embodiments of the present disclosure, in formula (G), $R_1$ is cyclopropyl.

The preferred options of the respective substituents mentioned in the above various preferred embodiments can be combined with each other in any way, and various combinations thereof are within the scope of the present disclosure. In the most preferred embodiments of the present disclosure, the compound of formula (G) is each specific compound shown in Example 1 to Example 58 herein. That is, the compound of formula (G) is selected from

(2-(6-(2-ethyl-5-fluoro-4-hydroxyphenyl)-1H-indazol-3-yl)-4,6-dihydropyrrolo[3,4-d]imidazol-5(1H)-yl)(5-(piperidin-1-yl)pyrazin-2-yl)ketone;

(2-(6-(2-ethyl-5-fluoro-4-hydroxyphenyl)-1H-indazol-3-yl)-4,6-dihydropyrrolo[3,4-d]imidazol-5(1H)-yl)(5-morpholinylpyrazin-2-yl)ketone;

(2-(6-(2-ethyl-5-fluoro-4-hydroxyphenyl)-1H-indazol-3-yl)-4,6-dihydropyrrolo[3,4-d]imidazol-5(1H)-yl)(1-methyl-1H-pyrazol-4-yl)ketone;

(2-(6-(2-ethyl-5-fluoro-4-hydroxyphenyl)-1H-indazol-3-yl)pyrrolo[3,4-d]imidazol-5(1H, 4H,6H)-yl)(1-methylpiperidin-4-yl)ketone;

(2-(6-(2-ethyl-5-fluoro-4-hydroxyphenyl)-1H-indazol-3-yl)pyrrolo[3,4-d]imidazol-5(1H, 4H,6H)-yl)(5-(4-methylpiperzin-1-yl)pyrazin-2-yl)ketone;

(2-(6-(2-ethyl-4-hydroxyphenyl)-1H-indazol-3-yl)pyrrolo[3,4-d]imidazol-5(1H,4H,6H)-yl)(5-(4-methylpiperzin-1-yl)pyrazin-2-yl)ketone;

5-ethyl-2-fluoro-4-(3-(5-(benzenesulfonyl)-1,4,5,6-tetrahydropyrrolo[3,4-d]imidazol-2-yl)-1H-indazol-6-yl)phenol;
5-ethyl-2-fluoro-4-(3-(5-(pyrazin-2ylmethyl)-1,4,5,6-tetrahydropyrrolo[3,4-d]imidazol-2-yl)-1H-indazol-6-yl)phenol;
4-(3-(5-(cyclopropylmethyl)-1,4,5,6-tetrahydropyrrolo[3,4-d]imidazol-2-yl)-1H-indazol-6-yl)-5-ethyl-2-fluorophenol;
Cyclopropyl(2-(6-(2-ethyl-5-fluoro-4-hydroxyphenyl)-1H-indazol-3-yl)pyrrolo[3,4-d]imidazol-5(1H,4H,6H)-yl)ketone;
4-(3-(5-(cyclobutylmethyl)-1,4,5,6-tetrahydropyrrolo[3,4-d]imidazol-2-yl)-1H-indazol-6-yl)-5-ethyl-2-fluorophenol;
Cyclobutyl(2-(6-(2-ethyl-5-fluoro-4-hydroxyphenyl)-1H-indazol-3-yl)pyrrolo[3,4-d]imidazol-5(1H,4H,6H)-yl)ketone;
(2-(6-(2-ethyl-5-fluoro-4-hydroxyphenyl)-1H-indazol-3-yl)pyrrolo[3,4-d]imidazol-5(1H,4H,6H)-yl)(3-hydroxylcyclobutyl)ketone;
(2-(6-(2-ethyl-5-fluoro-4-hydroxyphenyl)-1H-indazol-3-yl)-pyrrolo[3,4-d]imidazol-5-(1H,4H,6H)-yl)(pyridazin-4-yl)ketone;
(2-(6-(2-ethyl-5-fluoro-4-hydroxyphenyl)-1H-indazol-3-yl)-pyrrolo[3,4-d]imidazol-5-(1H,4H,6H)-yl)(pyridazin-3-yl)ketone;
(S)-(2-(6-(2-ethyl-5-fluoro-4-hydroxyphenyl)-1H-indazol-3-yl)-4,6-dihydropyrrolo[3,4-d]imidazol-5-(1H)-yl)(3-hydroxylpyrrolidin-1-yl)ketone;
5-ethyl-2-fluoro-4-(3 -(5-(4-hydroxylcyclohexyl)-1,4,5,6-tetrahydropyrrolo[3,4-d]imidazol-2-yl)-1H-indazol-6-yl)phenol;
4-(3-(5-(cyclopropanesulfonyl)-1,4,5,6-tetrahydropyrrolo[3,4-d]imidazol-2-yl)-1H-indazol-6-yl)-5-ethyl-2-fluorophenol;
4-(3-(5-(cyclobutylsulfonyl)-1,4,5,6-tetrahydropyrrolo[3,4-d]imidazol-2-yl)-1H-indazol-6-yl)-5-ethyl-2-fluorophenol;
4-(3-(5-(cyclopentylsulfonyl)-1,4,5,6-tetrahydropyrrolo[3,4-d]imidazol-2-yl)-1H-indazol-6-yl)-5-ethyl-2-fluorophenol;
5-ethyl-2-fluoro-4-(3-(5-((1-methyl-1H-pyrazol-4-yl)methyl)-1,4,5,6-tetrahydropyrrolo[3,4-d]imidazol-2-yl)-1H-indazol-6-yl)phenol;
4-(3-(5-(cyclopentyl-1,4,5,6-tetrahydropyrrolo[3,4-d]imidazol-2-yl)-1H-indazol-6-yl)-5-ethyl-2-fluorophenol;
5-ethyl-2-fluoro-4-(3-(5-(tetrahydro-2H-pyran-4-yl)-1,4,5,6-tetrahydropyrrolo[3,4-d]imidazol-2-yl)-1H-indazol-6-yl)phenol;
1-(2-(6-(2-ethyl-5-fluoro-4-hydroxyphenyl)-1H-indazol-3-yl)-4,6-dihydropyrrolo[3,4-d]imidazol-5-(1H)-yl)ethan-1-one;
1-(2-(6-(2-ethyl-5-fluoro-4-hydroxyphenyl)-1H-indazol-3-yl)-4,6-dihydropyrrolo[3,4-d]imidazol-5-(1H)-yl)propan-1-one;
(1-(2-(6-(2-ethyl-5-fluoro-4-hydroxyphenyl)-1H-indazol-3-yl)-4,6-dihydropyrrolo[3,4-d]imidazol-5-(1H)-yl)-2-methylpropan-1-one);
2-cyclopropyl-1-(2-(6-(2-ethyl-5-fluoro-4-hydroxyphenyl)-1H-indazol-3-yl)-4,6-dihydropyrrolo[3,4-d]imidazol-5-(1H)-yl)ethan-1-one;
1-(2-(6-(2-ethyl-5-fluoro-4-hydroxyphenyl)-1H-indazol-3-yl)-4,6-dihydropyrrolo[3,4-d]imidazol-5-(1H)-yl)-3-methylbutan-1-one;
(2-(6-(2-ethyl-5-fluoro-4-hydroxyphenyl)-1H-indazol-3-yl)-4,6-dihydropyrrolo[3,4-d]imidazol-5-(1H)-yl)(pyrrolidin-1-yl)ketone;
(2-(6-(2-ethyl-5-fluoro-4-hydroxyphenyl)-1H-indazol-3-yl)-4,6-dihydropyrrolo[3,4-d]imidazol-5-(1H)-yl)(piperidin-1-yl)ketone;
(2-(6-(2-ethyl-5-fluoro-4-hydroxyphenyl)-1H-indazol-3-yl)-4,6-dihydropyrrolo[3,4-d]imidazol-5-(1H)-yl)(morpholino)ketone;
(2-(6-(2-ethyl-5-fluoro-4-hydroxyphenyl)-1H-indazol-3-yl)-4,6-dihydropyrrolo[3,4-d]imidazol-5-(1H)-yl)(4-methylpiperzin-1-yl)ketone;
(2-(6-(2-ethyl-5-fluoro-4-hydroxyphenyl)-1H-indazol-3-yl)-4,6-dihydropyrrolo[3,4-d]imidazol-5-(1H)-yl)(4-ethylpiperzin-1-yl)ketone;
Cyclopropyl(2-(6-(2-ethyl-5-fluoro-4-hydroxyphenyl)-1H-pyrazolo [4,3-b]pyridin-3-yl)-4,6-dihydropyrrolo[3,4-d]imidazol-5(1H)-yl)ketone;
Cyclopropyl(2-(6-(2-ethyl-5-fluoro-4-hydroxyphenyl)-4-methyl-1H-indazol-3-yl)-4,6-dihydropyrrolo[3,4-d]imidazol-5(1H)-yl)ketone;
(S)-(2-(6-(2-ethyl-5-fluoro-4-hydroxyphenyl)-4-methyl-1H-indazol-3-yl)-4,6-dihydropyrrolo[3,4-d]imidazol -5(1H)-yl)(3-hydroxylpyrrolidin-1-yl)ketone;
Cyclopropyl(2-(6-(2-ethyl-5-fluoro-4-hydroxyphenyl)-1H-pyrazolo[4,3-c]pyridin-3-yl)-4,6-dihydropyrrolo[3,4-d]imidazol-5(1H)-yl)ketone;
(R)-(2-(6-(2-ethyl-5-fluoro-4-hydroxyphenyl)-1H-indazol-3-yl)-4,6-dihydropyrrolo[3,4-d]imidazol-5(1H)-yl)(3-hydroxylpyrrolidin-1-yl)ketone;
(2-(6-(2-ethyl-5-fluoro-4-hydroxyphenyl)-1H-indazol-3-yl)-4,6-dihydropyrrolo[3,4-d]imidazol-5(1H)-yl)(4-hydroxylpiperidin-1-yl)ketone;

2-(6-(2-ethyl-5-fluoro-4-hydroxyphenyl)-1H-indazol-3-yl)-N-methyl-4,6-dihydropyrrolo[3,4-d]imidazol-5-(1H)-carboxamide;
2-(6-(2-ethyl-5-fluoro-4-hydroxyphenyl)-1H-indazol-3-yl)-N-ethyl-4,6-dihydropyrrolo[3,4-d]imidazol-5-(1H)-carboxamide;
2-(6-(2-ethyl-5-fluoro-4-hydroxyphenyl)-1H-indazol-3-yl)-N-(2-hydroxylethyl)-4,6-dihydropyrrolo[3,4-d]imidazol-5(1H)-carboxamide;
1-(2-(6-(2-ethyl-5-fluoro-4-hydroxyphenyl)-1H-indazol-3 -yl)-1,4, 5, 6-tetrahydropyrrolo[3,4-d]imidazol-5 -carbonyl)pyrrolidin-3-nitrile;
2-(6-(2-ethyl-5-fluoro-4-hydroxyphenyl)-1H-indazol-3-yl)-N-(tetrahydrofuran-3-yl)-4,6-dihydropyrrolo[3,4-d]imidazol-5(1H)-carboxamide;
Methyl 2-(6-(2-ethyl-5-fluoro-4-hydroxyphenyl)-1H-indazol-3-yl)-4,6-dihydropyrrolo[3,4-d]imidazol-5(1H)-carboxylate;
Ethyl 2-(6-(2-ethyl-5-fluoro-4-hydroxyphenyl)-1H-indazol-3-yl)-4,6-dihydropyrrolo[3,4-d]imidazol-5(1H)-carboxylate;
(S)-(2-(6-(2-ethyl-5-fluoro-4-hydroxyphenyl)-1H-pyrazolo[3,4-b]pyridin-3-yl)-4,6-dihydropyrrolo[3,4-d]imidazol-5(1H)-yl)(3-hydroxylpyrrolidin-1-yl)ketone;
3-(2-(6-(2-ethyl-5-fluoro-4-hydroxyphenyl)-1H-indazol-3-yl) -4,6-dihydropyrrolo[3,4-d]imidazol-5(1H)-yl)-3-oxypropionitrile;
2-(6-(2-ethyl-5-fluoro-4-hydroxyphenyl)-1H-indazol-3-yl)-N,N-dimethyl-4,6-dihydropyrrolo[3,4-d]imidazol-5(1H)-carboxamide;
N-(2-cyanoethyl)-2-(6-(2-ethyl-5-fluoro-4-hydroxyphenyl)-1H-indazol-3-yl)-4,6-dihydropyrrolo[ 3,4-d]imidazol-5(1H)-carboxamide;
N-cyclopropyl-2-(6-(2-ethyl-5-fluoro-4-hydroxyphenyl)-1H-indazol-3-yl)-4,6-dihydropyrrolo[3,4-d]imidazol-5(1H)-carboxamide;
N-cyclobutyl-2-(6-(2-ethyl-5-fluoro-4-hydroxyphenyl)-1H-indazol-3-yl) -4,6-dihydropyrrolo[3,4- d]imidazol-5(1H)-carboxamide;
(S)-6-(2-ethyl-5-fluoro-4-hydroxyphenyl)-3-(5-prolyl-1,4,5,6-tetrahydropyrrolo[3,4-d]imidazol-2-yl)- 1H-indazol; and
(R)-6-(2-ethyl-5-fluoro-4-hydroxyphenyl)-3-(5-prolyl-1,4,5,6-tetrahydropyrrolo[3,4-d]imidazol-2-yl)- 1H-indazol.

In the compound of formula (G), when $X_1$, $X_2$, and $X_3$ are the same, the compound of formula (G) can also be represented as a compound of formula (G'):

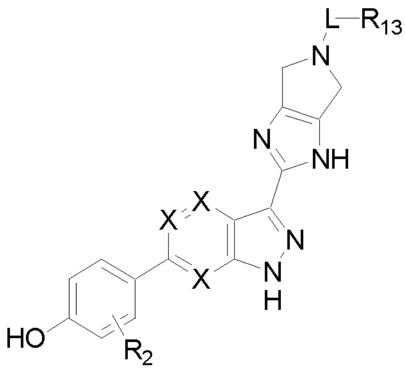

(G'),

wherein X is N or $CR_{14}$, and $R_{14}$, $R_{13}$, $R_1$, L, and $R_2$ are as defined in the compound of formula (G).
In a preferred embodiment, the present disclosure provides use of a compound of Formula (G)'

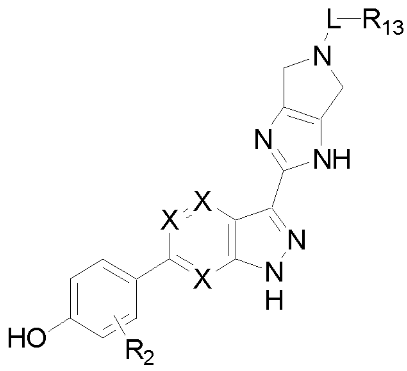

(G')

or an isotopically labeled compound thereof, or an optical isomer thereof, a geometric isomer thereof, a tautomer thereof or a mixture of various isomers, or a pharmaceutically acceptable salt thereof, or a prodrug thereof, or a metabolite thereof, in the manufacture of a medicament for treating severe pneumonia,
wherein

X is N or CH;
L is C=O, O=S=O, $CH_2$ or a covalent bond; and
$R_{13}$ is H, -N($R_{17}$)($R_{18}$), $C_{1-6}$ alkoxy, -$SR_{12}$, -$OR_{12}$, -CN, halogen, -$NO_2$, -$SF_5$, -S-$C_{1-4}$ alkyl, $C_{1-6}$ alkyl or $C_{3-7}$ cycloalkyl, 3-7 membered heterocycloalkyl, $C_{5-7}$ aryl, 5-7 membered heteroaryl, $C_{7-11}$ bicyclic aryl, 7-11 membered bicyclic heteroaryl, 11-15 membered tricyclyl, $C_{5-11}$ bicycloalkyl, or 5-11 membered bicyclic heteroalkyl, and $R_{13}$ is substituted with 0, 1, 2, 3 or 4 $R_1$(s), in which $R_{17}$ and $R_{18}$ are each independently selected from H, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, $C_{3-7}$ heterocycloalkyl, $C_{5-7}$ aryl, 5-7 membered heteroaryl, $C_{7-11}$ bicyclic aryl, 7-11 membered bicyclic heteroaryl, 11-15 membered tricyclyl, $C_{5-11}$ bicycloalkyl, and 5-11 membered bicyclic heteroalkyl and are optionally substituted with one or more substitutes each independently selected from -OH, - CN, -SH, halogen, -$NO_2$, -$SF_5$, -S-$C_{1-4}$ alkyl, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 4-10 membered heterocycloalkyl, $C_{5-7}$ aryl, 5-7 membered heteroaryl, $C_{7-11}$ bicyclic aryl, 7-11 membered bicyclic heteroaryl, -N($R_9$)($R_{10}$), - N($R_{11}$)(C(=O)$R_{12}$), -C(=O)-N($R_9$)($R_{10}$), -C(=O)-$R_{12}$, -C(=O)-$OR_{12}$, -OC(=O)$R_{12}$, - N($R_{11}$)(S(=O)$_2R_{12}$), -S(=O)$_2$-N($R_9$)($R_{10}$), -$SR_{12}$ and -$OR_{12}$, in which the -S-$C_{1-4}$ alkyl, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 4-10 membered heterocycloalkyl, $C_{5-7}$ aryl, 5-7 membered heteroaryl, $C_{7-11}$ bicyclic aryl, and 7-11 membered bicyclic heteroaryl are optionally substituted with 1, 2 or 3 substitutes each independently selected from halogen, -CN, -OH, $C_{1-4}$ alkyl, $C_{1-4}$ alkoxy, $C_{1-4}$ haloalkyl, $C_{1-4}$ haloalkoxy, $C_{3-6}$ cycloalkyl, -N($R_9$)($R_{10}$), -N($R_{11}$)(C(=O)$R_{12}$), -C(=O)-$OR_{12}$, -C(=O)H, -C(=O)$R_{12}$, -C(= O)-N($R_9$)($R_{10}$), -N($R_{11}$)(S(=O)$_2R_{12}$), -S(=O)$_2$-N($R_9$)($R_{10}$), -$SR_{12}$ and -$OR_{12}$; or $R_{17}$, $R_{18}$ and the N atom connected thereto together form a 3-14 membered ring; and
0, 1, 2, 3 or 4 $R_2$(s) are present in formula (G'), and $R_2$ is selected from H, halogen, -OH, - $NO_2$, -CN, -$SF_5$, -SH, -S-$C_{1-4}$ alkyl, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 4-10 membered heterocycloalkyl, $C_{5-7}$ aryl, 5-7 membered heteroaryl, $C_{7-11}$ bicyclic aryl, 7-11 membered bicyclic heteroaryl, -N($R_9$)($R_{10}$), -N($R_{11}$)(C(=O)$R_{12}$), - C(=O)-N($R_9$)($R_{10}$), -C(=O)-$R_{12}$, -C(=O)-$OR_{12}$, -OC(=O)$R_{12}$, -N($R_{11}$)(S(=O)$_2R_{12}$), -S(= O)$_2$-N($R_9$)($R_{10}$), -$SR_{12}$ and -$OR_{12}$, in which the -S-$C_{1-4}$ alkyl, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 4-10 membered heterocycloalkyl, $C_{5-7}$ aryl, 5-7 membered heteroaryl, $C_{7-11}$ bicyclic aryl, and 7-11 membered bicyclic heteroaryl are each optionally substituted with 1, 2 or 3 substituent(s) each independently selected from the group consisting of halogen, -CN, -OH, $C_{1-4}$ alkyl, $C_{1-4}$ alkoxy, $C_{1-4}$ haloalkyl, $C_{1-4}$ haloalkoxy, $C_{3-6}$ cycloalkyl, -N($R_9$)($R_{10}$), -N($R_{11}$)(C(=O)$R_{12}$), -C(=O)-$OR_{12}$, -C(=O)H, -C(=O)$R_{12}$, - C(=O)-N($R_9$)($R_{10}$), -N($R_{11}$)(S(=O)$_2R_{12}$), -S(=O)$_2$-N($R_9$)($R_{10}$), -$SR_{12}$ and -$OR_{12}$; and
$R_1$ is selected from H, halogen, -OH, -$NO_2$, -CN, -$SF_5$, -SH, -S-$C_{1-4}$ alkyl, $C_{1-8}$ alkyl, $C_{2-8}$ alkenyl, $C_{2-8}$ alkynyl, $C_{1-8}$ alkoxy, $C_{3-7}$ cycloalkyl, 3-10 membered heterocycloalkyl, $C_{5-7}$ aryl, 5-7 membered heteroaryl, $C_{7-11}$ bicyclic aryl, 7-11 membered bicyclic heteroaryl, 11-15membered tricyclyl, Cs-nbicycloalkyl, 5-11 membered bicyclic heteroalkyl, -N($R_9$)($R_{10}$), - N($R_{11}$)(C(=O)$R_{12}$), -C(=O)-N($R_9$)($R_{10}$), -C(=O)-$R_{12}$, -C(=O)-$OR_{12}$, -OC(=O)$R_{12}$, - N($R_{11}$)(S(=O)$_2R_{12}$), -S(=O)$_2$-N($R_9$)($R_{10}$), -$SR_{12}$ and -$OR_{12}$, in which the -S-$C_{1-4}$ alkyl, $C_{1-8}$ alkyl, $C_{2-8}$ alkenyl, $C_{2-8}$ alkynyl, and $C_{1-8}$ alkoxy are optionally substituted with 1, 2, 3, or 4 $R_3$(s), and in which the $C_{3-7}$ cycloalkyl, 3-10 membered heterocycloalkyl, $C_{5-7}$ aryl, 5-7 membered heteroaryl, $C_{7-11}$ bicyclic aryl, and 7-11 membered bicyclic heteroaryl are optionally substituted with 1, 2, 3, or 4 $R_4$(s); and
$R_3$ and $R_4$ are each independently selected from H, halogen, -OH, -$NO_2$, -CN, -$SF_5$, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 3-10 membered heterocycloalkyl, $C_{5-7}$ aryl, 5-7 membered

heteroaryl, $C_{7-11}$ bicyclic aryl, 7-11 membered bicyclic heteroaryl, $-N(R_5)(R_6)$, $-N(R_{11})(C(=O)R_{12})$, $-CON(R_7)(R_8)$, $-C(=O)-R_{12}$, $-C(=O)-OR_{12}$, $-OC(=O)R_{12}$, $-N(R_{11})(S(=O)_2R_{12})$, $-S(=O)_2-N(R_9)(R_{10})$, $-SR_{12}$ and $-OR_{12}$, in which the $C_{1-6}$ alkyl, $C_{3-7}$ cycloalkyl, 3-10 membered heterocycloalkyl, $C_{5-7}$ aryl, 5-7 membered heteroaryl, $C_{7-11}$ bicyclic aryl, and 7-11 membered bicyclic heteroaryl are each optionally substituted with 1, 2, 3 or 4 substituent(s) each independently selected from the group consisting of halogen, -CN, -OH, $C_{1-4}$ alkyl, $C_{1-6}$ alkoxy, $C_{1-4}$ haloalkyl, $C_{1-4}$ haloalkoxy, $C_{3-6}$ cycloalkyl, $-N(R_9)(R_{10})$, $-N(R_{11})(C(=O)R_{12})$, $-C(=O)-OR_{12}$, -C(=O)H, $-C(=O)R_{12}$, $-C(=O)-N(R_9)(R_{10})$, $-N(R_{11})(S(=O)_2R_{12})$, $-S(=O)_2-N(R_9)(R_{10})$, $-SR_{12}$ and $-OR_{12}$; and

$R_5$, $R_6$, $R_7$, $R_8$, $R_9$, $R_{10}$, $R_{11}$, and $R_{12}$ are each independently H or selected from the group consisting of $C_{1-6}$ alkyl, $C_{1-4}$ haloalkyl, $C_{3-7}$ cycloalkyl, 4-14 membered heterocycloalkyl, $C_{6-10}$ aryl, 5-10 membered heteroaryl, ($C_{3-7}$ cycloalkyl)-$C_{1-4}$ alkyl-, (4-10 membered heterocycloalkyl)-$C_{1-4}$ alkyl-, ($C_{6-10}$ aryl)-$C_{1-4}$ alkyl- and (5-10 membered heteroaryl)-$C_{1-4}$ alkyl-, wherein the substituents included in the above group are each optionally substituted with 1, 2, 3 or 4 substituent(s) each independently selected from the group consisting of halogen, $-CF_3$, -OH, $-NH_2$, $-NH(CH_3)$, $-N(CH_3)_2$, -CN, oxo, $C_{1-4}$ alkyl, $C_{2-6}$ alkenyl, $C_{2-6}$ alkynyl, $C_{3-7}$ cycloalkyl, $C_{1-4}$ hydroxyalkyl, $-S-C_{1-4}$ alkyl, -C(=O)H, $-C(=O)-C_{1-4}$ alkyl, - $C(=O)-O-C_{1-4}$ alkyl, $-C(=O)-NH_2$, $-C(=O)-N(C_{1-4}\ alkyl)_2$, $C_{1-4}$ haloalkyl, $C_{1-4}$ alkoxy and $C_{1-4}$ haloalkoxy.

In some preferred embodiments of the present disclosure, use of an isotopically labeled compound of the above-mentioned compound of formula (G') in the manufacture of a medicament for treating severe pneumonia is provided. In some more preferred embodiments of the present disclosure, in an isotopically labeled compound of the compound of formula (G'), all Hs are each independently and optionally substituted with D.

In some preferred embodiments of the present disclosure, in formula (G'), X is N. In some more preferred embodiments of the present disclosure, in formula (G'), X is CH.

In some more preferred embodiments of the present disclosure, in an isotopically labeled compound of the compound of formula (G'), all H are each independently and optionally substituted with D, and X is N. In some more preferred embodiments of the present disclosure, in an isotopically labeled compound of the compound of formula (G'), all Hs are each independently and optionally substituted with D, and Xis CH.

In some preferred embodiments of the present disclosure, in formula (G'), L is C=O, O=S=O or $CH_2$. In some particularly preferred embodiments of the present disclosure, in formula (G'), L is C=O. In some particularly preferred embodiments of the present disclosure, in formula (G'), L is O=S=O. In some particularly preferred embodiments of the present disclosure, in formula (G'), L is $CH_2$. In other embodiments of the present disclosure, in formula (G'), L is a covalent bond.

In some particularly preferred embodiments of the present disclosure, in formula (G'), X is CH, and L is C=O.

In some particularly preferred embodiments of the present disclosure, in formula (G'), X is CH, and L is O=S=O.

In some particularly preferred embodiments of the present disclosure, in formula (G'), X is CH, and L is CH2.

In some particularly preferred embodiments of the present disclosure, in formula (G'), X is CH, and L is a covalent bond.

In some particularly preferred embodiments of the present disclosure, in formula (G'), X is N, and L is C=O.

In some particularly preferred embodiments of the present disclosure, in formula (G'), X is N, and L is O=S=O.

In some particularly preferred embodiments of the present disclosure, in formula (G'), X is N, and L is $CH_2$.

In some particularly preferred embodiments of the present disclosure, in formula (G'), X is N, and L is a covalent bond.

In some preferred embodiments of the present disclosure, in formula (G'), $R_{13}$ is H, - $N(R_{17})(R_{18})$, $C_{1-6}$ alkoxy, -OH, -SH, -CN, halogen, $-NO_2$, $-SF_5$, $-S-C_{1-4}$ alkyl, $C_{1-6}$ alkyl, or $C_{3-7}$ cycloalkyl, 3-7 membered heterocycloalkyl, $C_{5-7}$ aryl, 5-7 membered heteroaryl, $C_{7-11}$ bicyclic aryl, 7-11membered bicyclic heteroaryl, 11-15 membered tricyclyl, Cs-nbicycloalkyl, or 5-11 membered bicyclic heteroalkyl, in which $R_{17}$ and $R_{18}$ are each independently selected from H, $C_{1-6}$ alkyl, $C_{1-6}$ alkoxy, $C_{3-7}$ cycloalkyl, $C_{3-7}$ heterocycloalkyl, $C_{5-7}$ aryl, and 5-7 membered heteroaryl, and are optionally substituted with one or more of -OH, -CN, -SH, halogen, $-NO_2$,-and $SF_5$, wherein $R_{13}$ is optionally substituted with 1, 2, 3 or 4 $R_1$(s). In some preferred embodiments of the present disclosure, in formula (G'), $R_{13}$ is H, $-N(R_{17})(R_{18})$, $C_{1-6}$ alkoxy, -OH, -SH, -CN, halogen, $-NO_2$, $-SF_5$, $-S-C_{1-4}$ alkyl, $C_{1-6}$ alkyl, or $C_{3-7}$ cycloalkyl, 3-7 membered heterocycloalkyl, $C_{5-7}$ aryl, 5-7 membered heteroaryl, $C_{7-11}$ bicyclic aryl, 7-11membered bicyclic heteroaryl, or 11-15 membered tricyclyl and $R_{17}$ and $R_{18}$ are defined as above, wherein $R_{13}$ is optionally substituted with 1, 2, 3 or 4 $R_1$(s). In some preferred embodiments of the present disclosure, in formula (G'), $R_{13}$ is H, $-N(R_{17})(R_{18})$, $C_{1-6}$ alkoxy, $C_{1-6}$ alkyl, or $C_{3-7}$ cycloalkyl, 3-7 membered heterocycloalkyl, $C_{5-7}$ aryl, or 5-7 membered heteroaryl, and $R_{17}$ and $R_{18}$ are defined as above, wherein $R_{13}$ is optionally substituted with 1, 2, 3 or 4 $R_1$(s). In some preferred embodiments of the present disclosure, in formula (G'), $R_{13}$ is $-N(R_{17})(R_{18})$, $C_{1-6}$ alkoxy, $C_{1-6}$ alkyl, or $C_{3-7}$ cycloalkyl, 4-6 membered heterocycloalkyl, phenyl, or 5-6 membered heteroaryl, and $R_{17}$ and $R_{18}$ are defined as above, wherein $R_{13}$ is optionally substituted with 1, 2, or 3 $R_1$(s). In some preferred embodiments of the present disclosure, in formula (G'), $R_{13}$ is $-N(R_{17})(R_{18})$, $C_{1-3}$ alkoxy, $C_{3-6}$ cycloalkyl, 4-6 membered heterocycloalkyl, phenyl, 5-6 membered heteroaryl or $C_{1-4}$ alkyl, and $R_{17}$ and $R_{18}$ are defined as above,

wherein $R_{13}$ is optionally substituted with 1, 2, or 3 $R_1$(s). In some preferred embodiments of the present disclosure, in formula (G'), $R_{13}$ is -N(H)($C_{1-3}$ alkyl), -N(H)(3-6 membered cycloalkyl), -N(H) (4-6 membered heterocycloalkyl), -N ($C_{1-3}$ alkyl) ($C_{1-3}$ alkyl), $C_{1-3}$ alkoxy, C3-6 cycloalkyl, 4-6 membered azacycloalkyl or oxacycloalkyl, phenyl, 5-6 membered azaaryl or $C_{1-4}$ alkyl; or $R_{13}$ is -N($R_{17}$)($R_{18}$), and $R_{17}$ and $R_{18}$ and the N atom connected thereto together form a 4-10 membered ring (where $R_{13}$ is optionally substituted with 1, 2, or 3 $R_1$). In some particularly preferred embodiments of the present disclosure, in formula (G'), $R_{13}$ is cyclopropyl, cyclobutyl, cyclopentyl, cyclohexyl, methyl, ethyl, propyl, butyl, methoxy, ethoxy, propoxy, -N(H)($CH_3$), -N(H)($CH_2CH_3$), -N(H)($CH_2CH_2OH$), - N(H)($CH_2CH_2CN$), -N($CH_3$)($CH_3$), -N(H)(cyclopropyl), -N(H)(cyclobutyl), - N(H)(tetrahydrofuranyl), pyrazinyl, pyridazinyl, pyrrolidinyl, pyrazolyl, piperidinyl, phenyl, azetidinyl, morpholinyl, piperazinyl or tetrahydropyranyl; or $R_{13}$ is -N($R_{17}$)($R_{18}$), and $R_{17}$ and $R_{18}$ and the N atom connected thereto together form a 7-membered ring (where $R_{13}$ is optionally substituted with 1, 2, or 3 $R_{1s}$). In some particularly preferred embodiments of the present disclosure, in formula (G'), $R_{13}$ is cyclopropyl. In some particularly preferred embodiments of the present disclosure, in formula (G'), $R_{13}$ is cyclobutyl. In some particularly preferred embodiments of the present disclosure, in formula (G'), $R_{13}$ is cyclopentyl. In some particularly preferred embodiments of the present disclosure, in formula (G'), $R_{13}$ is cyclohexyl. In some particularly preferred embodiments of the present disclosure, in formula (G'), $R_{13}$ is a methyl group. In some particularly preferred embodiments of the present disclosure, in formula (G'), $R_{13}$ is an ethyl group. In some particularly preferred embodiments of the present disclosure, in formula (G'), $R_{13}$ is propyl. In some particularly preferred embodiments of the present disclosure, in formula (G'), $R_{13}$ is a butyl group. In some particularly preferred embodiments of the present disclosure, in formula (G'), $R_{13}$ is pyrazinyl. In some particularly preferred embodiments of the present disclosure, in formula (G'), $R_{13}$ is a pyridazinyl. In some particularly preferred embodiments of the present disclosure, in formula (G'), $R_{13}$ is a pyrrolidinyl. In some particularly preferred embodiments of the present disclosure, in formula (G'), $R_{13}$ is pyrazolyl. In some particularly preferred embodiments of the present disclosure, in formula (G'), $R_{13}$ is piperidinyl. In some particularly preferred embodiments of the present disclosure, in formula (G'), $R_{13}$ is phenyl. In some particularly preferred embodiments of the present disclosure, in formula (G'), $R_{13}$ is azetidinyl. In some particularly preferred embodiments of the present disclosure, in formula (G'), $R_{13}$ is morpholinyl. In some particularly preferred embodiments of the present disclosure, in formula (G'), $R_{13}$ is piperazinyl. In some particularly preferred embodiments of the present disclosure, in formula (G'), $R_{13}$ is tetrahydropyranyl. In some particularly preferred embodiments of the present disclosure, in formula (G'), $R_{13}$ is methoxy. In some particularly preferred embodiments of the present disclosure, in formula (G'), $R_{13}$ is ethoxy. In some particularly preferred embodiments of the present disclosure, in formula (G'), $R_{13}$ is -N(H)($CH_3$). In some particularly preferred embodiments of the present disclosure, in formula (G'), $R_{13}$ is - N(H)($CH_2CH_3$). In some particularly preferred embodiments of the present disclosure, in formula (G'), $R_{13}$ is -N(H)($CH_2CH_2OH$). In some particularly preferred embodiments of the present disclosure, in formula (G'), $R_{13}$ is -N(H)($CH_2CH_2CN$). In some particularly preferred embodiments of the present disclosure, in formula (G'), $R_{13}$ is -N($CH_3$)($CH_3$). In some particularly preferred embodiments of the present disclosure, in formula (G'), $R_{13}$ is -N(H) (cyclopropyl). In some particularly preferred embodiments of the present disclosure, in formula (G'), $R_{13}$ is -N(H) (cyclobutyl). In some particularly preferred embodiments of the present disclosure, in formula (G'), $R_{13}$ is -N(H) (tetrahydrofuranyl). In some particularly preferred embodiments of the present disclosure, in formula (G'), $R_{13}$ is -N($R_{17}$)($R_{18}$), and $R_{17}$ and $R_{18}$ and the N atom connected to them together form a 7-membered ring.

In some particularly preferred embodiments of the present disclosure, in formula (G'), $R_{17}$ and $R_{18}$ are each independently selected from H, $C_{1-6}$ alkyl, $C_{1-6}$ alkoxy, $C_{3-7}$ cycloalkyl, $C_{3-7}$ heterocycloalkyl, $C_{5-7}$ aryl, and 5-7 membered heteroaryl, and are optionally substituted with one or more of -OH, -CN, -SH, halogen, $-NO_2$, and $SF_5$. In some particularly preferred embodiments of the present disclosure, in formula (G'), $R_{17}$ and $R_{18}$ are each independently selected from H, $C_{1-6}$ alkyl, $C_{3-7}$ cycloalkyl, and $C_{3-7}$ heterocycloalkyl and are optionally substituted with one or more of -OH, -CN, -SH, halogen, $-NO_2$, and $SF_5$. In some particularly preferred embodiments of the present disclosure, in formula (G'), $R_{17}$ and $R_{18}$ are each independently selected from H, $C_{1-6}$ alkyl, $C_{3-7}$ cycloalkyl, and $C_{3-7}$ heterocycloalkyl and are optionally substituted with one or more of -OH and -CN. In some preferred embodiments of the present disclosure, in formula (G'), $R_{17}$ and $R_{18}$ are each independently selected from H, methyl, ethyl, propyl, 3-membered cycloalkyl, 4-membered cycloalkyl, 5-membered cycloalkyl, 5-membered heterocycloalkyl, and 6-membered heterocycloalkyl, and optionally substituted with one or more of -OH and -CN. In some preferred embodiments of the present disclosure, in formula (G'), $R_{17}$, $R_{18}$ and the N atom connected thereto together form a 4-10 membered ring. In some preferred embodiments of the present disclosure, in formula (G'), $R_{17}$, $R_{18}$ and the N atom connected thereto together form a 7-membered ring.

In some particularly preferred embodiments of the present disclosure, in formula (G'), L is C=O, and $R_{13}$ is -N($R_{17}$)($R_{18}$), $C_{1-6}$ alkoxy, -OH, -SH, -CN, halogen, $-NO_2$, $-SF_5$, or -S-$C_{1-4}$ alkyl, and $R_{13}$ is substituted with 0, 1, 2, 3 or 4 $R_1$(s) in which $R_{17}$ and $R_{18}$ are each independently selected from H, $C_{1-6}$ alkyl, $C_{1-6}$ alkoxy, $C_{3-7}$ cycloalkyl, $C_{3-7}$ heterocycloalkyl, $C_{5-7}$ aryl, and 5-7 membered heteroaryl, and are optionally substituted with one or more of - OH, -CN, -SH, halogen, $-NO_2$,-and $SF_5$, or $R_{17}$, $R_{18}$ and the N atom connected thereto together form a 3-14 membered ring. In some particularly preferred embodiments of the present disclosure, in formula (G'), L is C=O, and $R_{13}$ is -N($R_{17}$)($R_{18}$), or $C_{1-6}$ alkoxy, in which $R_{17}$ and $R_{18}$ are each independently selected from H, $C_{1-6}$ alkyl, $C_{3-7}$ cycloalkyl, and $C_{3-7}$ heterocycloalkyl and are optionally substituted with one or more of -OH, -CN, -SH, halogen, $-NO_2$,-and $SF_5$, or $R_{17}$, $R_{18}$

and the N atom connected thereto together form a 3-10 membered ring. In some particularly preferred embodiments of the present disclosure, in formula (G'), L is C=O, and $R_{13}$ is methoxy, ethoxy, propoxy, -N(H)($CH_3$), - N(H)(CH2$CH_3$), -N(H)($CH_2CH_2OH$), -N(H)($CH_2CH_2CN$), -N($CH_3$)($CH_3$), -N(H)(cyclopropyl), -N(H) (cyclobutyl), -N(H) (tetrahydrofuranyl); or $R_{13}$ is -N($R_{17}$)($R_{18}$), and $R_{17}$, $R_{18}$ and the N atom connected thereto together form a 7-membered ring. In some particularly preferred embodiments of the present disclosure, in formula (G'), L is C=O, and $R_{13}$ is methoxy. In some particularly preferred embodiments of the present disclosure, in formula (G'), L is C=O, and $R_{13}$ is ethoxy. In some particularly preferred embodiments of the present disclosure, in formula (G'), L is C=O, and $R_{13}$ is -N(H)($CH_3$). In some particularly preferred embodiments of the present disclosure, in formula (G'), L is C=O, and $R_{13}$ is -N(H)($CH_2CH_3$). In some particularly preferred embodiments of the present disclosure, in formula (G'), L is C=O, and $R_{13}$ is -N(H)($CH_2CH_2OH$). In some particularly preferred embodiments of the present disclosure, in formula (G'), L is C=O, and $R_{13}$ is -N(H)($CH_2CH_2CN$). In some particularly preferred embodiments of the present disclosure, in formula (G'), L is C=O, and $R_{13}$ is - N($CH_3$)($CH_3$). In some particularly preferred embodiments of the present disclosure, in formula (G'), L is C=O, and $R_{13}$ is -N(H)(cyclopropyl). In some particularly preferred embodiments of the present disclosure, in formula (G'), L is C=O, and $R_{13}$ is - N(H)(cyclobutyl). In some particularly preferred embodiments of the present disclosure, in formula (G'), L is C=O, and $R_{13}$ is -N(H)(tetrahydrofuranyl). In some particularly preferred embodiments of the present disclosure, in the formula (G'), L is C=O, and $R_{13}$ is -N($R_{17}$)($R_{18}$), and $R_{17}$, $R_{18}$ and the N atom connected thereto together form a 7-membered ring.

In some particularly preferred embodiments of the present disclosure, in formula (G'), one, two or three $R_2$(s) are present and $R_2$ is selected from H, halogen, -OH, -$NO_2$, -CN, -$SF_5$, -SH, -S-$C_{1-4}$ alkyl, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, and 4-10 membered heterocycloalkyl, in which the -S-$C_{1-4}$ alkyl, $C_{1-6}$ alkyl, $C_{3-7}$ cycloalkyl, and 4-10 membered heterocycloalkyl are each optionally substituted with 1, 2 or 3 substituent(s) each independently selected from the group consisting of halogen, -OH, -$NH_2$, -NH($CH_3$), -N($CH_3$)$_2$, -CN, $C_{1-4}$ alkyl, $C_{1-4}$ haloalkyl, $C_{1-4}$ alkoxy, and $C_{1-4}$ haloalkoxy. In some particularly preferred embodiments of the present disclosure, in formula (G'), one, two or three $R_2$(s) are present and $R_2$ is selected from halogen, $C_{1-6}$ alkyl and $C_{3-6}$ cycloalkyl, in which the $C_{1-6}$ alkyl and $C_{3-6}$ cycloalkyl are each optionally substituted with 1, 2 or 3 substituent(s) each independently selected from the group consisting of halogen, -OH, -$NH_2$, -NH($CH_3$), -N($CH_3$)$_2$, -CN, $C_{1-4}$ alkyl, $C_{1-4}$ haloalkyl, $C_{1-4}$ alkoxy, and $C_{1-4}$ haloalkoxy. In some particularly preferred embodiments of the present disclosure, in formula (G'), one, two or three $R_2$(s) are present and $R_2$ is selected from halogen, and $C_{1-6}$ alkyl, in which the $C_{1-6}$ alkyl is optionally substituted with 1, 2 or 3 substituent(s) each independently selected from the group consisting of halogen, -OH, -$NH_2$, -NH($CH_3$), -N($CH_3$)$_2$, -CN, $C_{1-4}$ alkyl, $C_{1-4}$ haloalkyl, $C_{1-4}$ alkoxy, and $C_{1-4}$ haloalkoxy. In some particularly preferred embodiments of the present disclosure, in formula (G'), 1, or two $R_2$(s) are present and $R_2$ is selected from halogen, and $C_{1-6}$ alkyl. In some particularly preferred embodiments of the present disclosure, in formula (G'), one or two $R_2$(s) are present and $R_2$ is selected from fluorine, chlorine, bromine, methyl, ethyl, n-propyl, isopropyl, n-butyl, and isobutyl. In some preferred embodiments of the present disclosure, in formula (G'), one or two $R_2$(s) are present, and $R_2$ is selected from fluorine, chlorine, methyl, ethyl, n-propyl, and isopropyl. In some preferred embodiments of the present disclosure, in formula (G'), one or two $R_2$ (s) are present, and $R_2$ is selected from fluorine, methyl, and ethyl. In some preferred embodiments of the present disclosure, in formula (G'), one or two $R_2$(s) are present, and $R_2$ is selected from fluorine and ethyl. In some preferred embodiments of the present disclosure, in formula (G'), one $R_2$ is present, and $R_2$ is selected from fluorine and ethyl. In some preferred embodiments of the present disclosure, in formula (G'), two $R_2$(s) are present, and $R_2$ is selected from fluorine and ethyl. In some particularly preferred embodiments of the present disclosure, in formula (G'), two $R_2$(s) are present which are respectively fluorine and ethyl. In some particularly preferred embodiments of the present disclosure, in formula (G'), one $R_2$ is present, and $R_2$ is an ethyl group.

In some preferred embodiments of the present disclosure, in formula (G'), $R_{13}$ is substituted with 0, 1, 2, 3 or 4 $R_1$(s), and each $R_1$ is independently selected from H, halogen, -OH, -$NO_2$,- CN, -$SF_5$, -SH, -S-$C_{1-4}$ alkyl, $C_{1-8}$ alkyl, $C_{2-8}$ alkenyl, $C_{2-8}$ alkynyl, $C_{1-8}$ alkoxy, $C_{3-7}$ cycloalkyl, 3 -10 membered heterocycloalkyl, $C_{5-7}$ aryl, 5-7 membered heteroaryl, $C_{7-11}$ bicyclic aryl, 7-11 membered bicyclic heteroaryl, wherein the -S-$C_{1-4}$ alkyl, $C_{1-8}$ alkyl, $C_{2-8}$ alkenyl, $C_{2-8}$ alkynyl, and $C_{1-8}$ alkoxy are optionally substituted with 1, 2, 3, or 4 $R_3$(s), and wherein the $C_{3-7}$ cycloalkyl, 3-10 membered heterocycloalkyl, $C_{5-7}$ aryl, 5-7 membered heteroaryl, $C_{7-11}$ bicyclic aryl, and 7-11 membered bicyclic heteroaryl are optionally substituted with 1, 2 , 3 or 4 $R_4$(s). In some preferred embodiments of the present disclosure, in formula (G'), $R_{13}$ is substituted with 0, 1, 2, 3 or 4 $R_1$(s), and each R1 is independently selected from H, halogen, -OH, -$NO_2$,- CN, -$SF_5$, -SH, -S-$C_{1-4}$ alkyl, $C_{1-8}$ alkyl, $C_{3-7}$ cycloalkyl, 3-7 membered heterocycloalkyl, $C_{5-7}$ aryl, 5-7 membered heteroaryl, wherein the -S-$C_{1-4}$ alkyl, and $C_{1-8}$ alkyl are optionally substituted with 1, 2, 3 or 4 $R_3$(s), and wherein the $C_{3-7}$ cycloalkyl, 3-7 membered heterocycloalkyl, $C_{5-7}$ aryl, and 5-7 membered heteroaryl are optionally substituted with 1, 2, 3, or 4 $R_4$(s). In some preferred embodiments of the present disclosure, in formula (G'), $R_{13}$ is substituted with 0, 1, 2, 3 or 4 $R_1$(s), and each R1 is independently selected from halogen, -OH, -CN, $C_{1-8}$ alkyl, $C_{3-7}$ cycloalkyl, 3-7 membered heterocycloalkyl, $C_{5-7}$ aryl, 5-7 membered heteroaryl, wherein the $C_{1-8}$ alkyl is optionally substituted with 1, 2 , 3 or 4 $R_3$(s), and wherein the $C_{3-7}$ cycloalkyl, 3-7 membered heterocycloalkyl, $C_{5-7}$ aryl, 5-7 membered heteroaryl are optionally substituted with 1, 2, 3 or 4 $R_4$(s). In some preferred embodiments of the present disclosure, in formula (G'), $R_{13}$ is

substituted with 0, 1, 2, 3 or 4 $R_1$(s), and each $R_1$ is independently selected from halogen, -OH, -CN, $C_{1-8}$ alkyl, $C_{3-7}$ cycloalkyl, and 3-7 membered heterocycloalkyl, wherein the $C_{1-8}$ alkyl is optionally substituted with 1, 2, or 3 $R_3$(s), and wherein the $C_{3-7}$ cycloalkyl, and 3-7 membered heterocycloalkyl are optionally substituted with 1, 2, or 3 $R_4$. In some preferred embodiments of the present disclosure, in formula (G'), $R_{13}$ is substituted with 0 or 1 $R_1$, and each R1 is independently selected from halogen, -OH, -CN, $C_{1-6}$ alkyl, $C_{3-7}$ cycloalkyl, 5-7 membered heterocycloalkyl, wherein the $C_{1-6}$ alkyl is optionally substituted with 1, 2, or 3 $R_3$(s), and wherein the $C_{3-7}$ cycloalkyl, and 5-7-membered heterocycloalkyl are optionally substituted with 1, 2, or 3 $R_4$(s). In some preferred embodiments of the present disclosure, in formula (G'), $R_{13}$ is substituted with 0 or 1 $R_1$, and each $R_1$ is independently selected from halogen, - OH, -CN, $C_{1-4}$ alkyl, $C_{3-6}$ cycloalkyl, and 5-7 membered heterocycloalkyl, wherein the $C_{1-4}$ alkyl is optionally substituted with 1 or 2 $R_3$, and wherein the $C_{3-6}$ cycloalkyl and 5-7 membered heterocycloalkyl are optionally substituted with 1, 2, or 3 $R_4$(s). In some particularly preferred embodiments of the present disclosure, in formula (G'), $R_{13}$ is substituted with 0 or 1 $R_1$, and each $R_1$ is independently selected from methyl, ethyl, hydroxyl, -CN, piperidinyl, morpholinyl, piperazinyl, and cyclopropyl, wherein the piperidinyl, morpholinyl, and piperazinyl are optionally substituted with 1, 2, 3 or 4 $C_{1-3}$ alkyl. In some particularly preferred embodiments of the present disclosure, in formula (G'), $R_{13}$ is substituted with 0 or 1 $R_1$, and each $R_1$ is independently selected from methyl, ethyl, hydroxy, -CN, piperidinyl, morpholinyl, 1-methylpiperazinyl, and cyclopropyl. In some particularly preferred embodiments of the present disclosure, in formula (G'), $R_1$ is absent. In some particularly preferred embodiments of the present disclosure, in formula (G'), $R_1$ is 1-methylpiperazinyl. In some particularly preferred embodiments of the present disclosure, in formula (G'), $R_1$ is methyl. In some particularly preferred embodiments of the present disclosure, in formula (G'), $R_1$ is ethyl. In some particularly preferred embodiments of the present disclosure, in formula (G'), $R_1$ is piperidinyl. In some particularly preferred embodiments of the present disclosure, in formula (G'), $R_1$ is morpholinyl. In some particularly preferred embodiments of the present disclosure, in formula (G'), $R_1$ is hydroxyl. In some particularly preferred embodiments of the present disclosure, in formula (G'), $R_1$ is - CN. In some particularly preferred embodiments of the present disclosure, in formula (G'), $R_1$ is cyclopropyl.

The preferred options of the respective substituents mentioned in the above various preferred embodiments can be combined with each other in any way, and various combinations thereof are within the scope of the present disclosure.

In the compound of formula (G'), when $R_{13}$ is a ring, the compound of formula (G') can also be represented as a compound of the following formula (I):

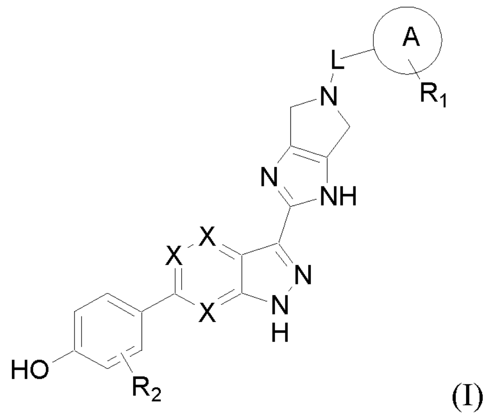

(I)

wherein the ring A is $C_{3-7}$ cycloalkyl, $_{3-7}$ membered heterocycloalkyl, $C_{5-7}$ aryl, 5-7 membered heteroaryl, $C_{7-11}$ bicyclic aryl, 7-11 membered bicyclic heteroaryl, 11-15 membered tricyclyl, $C_{5-11}$ bicyclic alkyl group or 5-11 membered bicyclic heteroalkyl, which may be optionally substituted with $R_1$, and L, $R_1$, $R_2$, and X are defined as above in the compound of formula (G').

In particular, the present disclosure provides use of a compound of formula (I) as a JAK inhibitor:

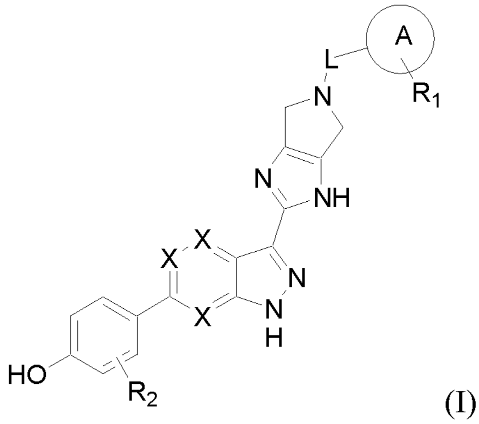

(I)

or an isotopically labeled compound thereof, or an optical isomer thereof, a geometric isomer thereof, a tautomer thereof or a mixture of various isomers, or a pharmaceutically acceptable salt thereof, or a prodrug thereof, or a metabolite thereof, in the manufacture of a medicament for treating severe pneumonia

in which
L is C=O, O=S=O, $CH_2$ or a covalent bond; and
X is CH or N;
The ring A is $C_{3-7}$ cycloalkyl, 3-7 membered heterocycloalkyl, $C_{5-7}$ aryl, 5-7 membered heteroaryl, $C_{7-11}$ bicyclic aryl, 7-11 membered bicyclic heteroaryl, or 11 -15 membered tricyclyl;
0, 1, 2, 3 or 4 $R_1$(s) are present in formula (I), and $R_1$ is selected from H, halogen, $C_{1-8}$ alkyl, $C_{2-8}$ alkenyl, $C_{2-8}$ alkynyl, $C_{1-8}$ alkoxy, $C_{3-7}$ cycloalkyl, 3-7 membered heterocycloalkyl, $C_{5-7}$ aryl, 5-7 membered heteroaryl, $C_{7-11}$ bicyclic aryl, and 7-11 membered bicyclic heteroaryl, in which the $C_{1-8}$ alkyl, $C_{2-8}$ alkenyl, $C_{2-8}$ alkynyl, and $C_{1-8}$ alkoxy are optionally substituted with 1, 2, 3 or 4 $R_3$(s), and in which the $C_{3-7}$ cycloalkyl, 3-7 membered heterocycloalkyl, $C_{5-7}$ aryl, 5-7 membered heteroaryl, $C_{7-11}$ bicyclic aryl, 7-11 membered bicyclic heteroaryl are optionally substituted with 1, 2, 3 or 4 $R_4$(s),
0, 1, 2, 3 or 4 $R_2$(s) are present in formula (I), and $R_2$ is selected from H, halogen, -OH, $-NO_2$, -CN, $-SF_5$, $C_{1-6}$alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 4-10 membered heterocycloalkyl, $-N(R_9)(R_{10})$, $-N(R_{11})(C(=O)R_{12})$, $-C(=O)-N(R_9)(R_{10})$, - $C(=O)-R_{12}$, $-C(=O)-OR_{12}$, $-OC(=O)R_{12}$, $-N(R_{11})(S(=O)_2R_{12})$, $-S(=O)_2-N(R_9)(R_{10})$, $-SR_{12}$ and $-OR_{12}$, in which the $C_{1-6}$ alkyl, $C_{3-7}$ cycloalkyl and 4-10 membered heterocycloalkyl are each optionally substituted with 1, 2 or 3 substituent(s) each independently selected from the group consisting of halogen, -CN, -OH, $C_{1-4}$ alkyl, $C_{1-4}$ alkoxy, $C_{1-4}$ haloalkyl, $C_{1-4}$ haloalkoxy, $C_{3-6}$ cycloalkyl, $-N(R_9)(R_{10})$, $-N(R_{11})(C(=O)R_{12})$, $-C(=O)-OR_{12}$, -C(=O)H, $-C(=O)R_{12}$, - $C(=O)-N(R_9)(R_{10})$, $-N(R_{11})(S(=O)_2R_{12})$, $-S(=O)_2-N(R_9)(R_{10})$, $-SR_{12}$ and $-OR_{12}$;
$R_3$ is selected from halogen, cyano, $C_{1-3}$ alkyl, hydroxy, $C_{1-6}$ alkoxy, $-N(R_5)(R_6)$, - $CON(R_7)(R_8)$ or 3-7 membered heterocycloalkyl, in which the 3-7 membered heterocycloalkyl is optionally substituted with 1, 2, 3 or 4 $R_4$(s);
$R_4$ is selected from halogen, $C_{1-3}$ alkyl, hydroxyl, $C_{1-6}$ alkoxy, $-NH_2$, $-NHCH_3$ or $-N(CH_3)_2$; $R_5$, $R_6$, $R_7$, $R_8$ are each independently hydrogen or $C_{1-4}$ alkyl;
$R_9$ is selected from H, $C_{1-4}$ alkyl, $C_{1-4}$ haloalkyl or $C_{3-7}$ cycloalkyl;
$R_{10}$ is H or selected from the group consisting of $C_{1-4}$ alkyl, $C_{1-4}$ haloalkyl, $C_{3-7}$ cycloalkyl, 4-10 membered heterocycloalkyl, $C_{6-10}$ aryl, 5-10 membered heteroaryl, ($C_{3-7}$ cycloalkyl)-$C_{1-4}$ alkyl-, (4-10 membered heterocycloalkyl)-$C_{1-4}$ alkyl-, ($C_{6-10}$ aryl)-$C_{1-4}$ alkyl- and (5-10 membered heteroaryl)-$C_{1-4}$ alkyl-, wherein each substituent included in the above group is optionally substituted with 1, 2, 3 or 4 substituent(s) each independently selected from the group consisting of -OH, $-NH_2$, $-NH(CH_3)$, $-N(CH_3)_2$, -CN, $C_{1-4}$ alkyl, $C_{3-7}$ cycloalkyl, $C_{1-4}$ hydroxyalkyl, $-S-C_{1-4}$ alkyl, -C(=O)H, $-C(=O)-C_{1-4}$ alkyl, $-C(=O)-O-C_{1-4}$ alkyl, $-C(=O)-NH_2$, $-C( =O)-N(C_{1-4}$ alkyl$)_2$, $C_{1-4}$ haloalkyl, $C_{1-4}$ alkoxy and $C_{1-4}$ haloalkoxy;
$R_{11}$ is selected from H, $C_{1-4}$ alkyl and $C_{3-7}$ cycloalkyl; and
$R_{12}$ is selected from the group consisting of $C_{1-6}$ alkyl, $C_{3-7}$ cycloalkyl, 4- to 14-membered heterocycloalkyl, $C_{6-10}$ aryl, 5-10 membered heteroaryl, ($C_{3-7}$ cycloalkyl)-$C_{1-4}$ alkyl-, (4-10 membered heterocycloalkyl)-$C_{1-4}$ alkyl-, ($C_{6-10}$ aryl)-$C_{1-4}$ alkyl- and (5-10 membered heteroaryl)-$C_{1-4}$ alkyl-, wherein each substituent included in the above group is optionally substituted with 1, 2 or 3 substituent(s) each independently selected from the group consisting of halogen, $-CF_3$, -CN, -OH, $-NH_2$, $-NH(CH_3)$, $-N(CH_3)_2$, oxo, $-S-C_{1-4}$ alkyl, $C_{1-4}$ alkyl, $C_{1-4}$ haloalkyl, $C_{2-6}$ alkenyl, $C_{2-6}$ alkynyl , $C_{3-7}$ cycloalkyl, $C_{1-4}$ alkoxy and $C_{1-4}$ haloalkoxy.

In some preferred embodiments of the present disclosure, in formula (I), L is C=O, O=S=O or $CH_2$. In some particularly preferred embodiments of the present disclosure, in formula (I), L is C=O. In some particularly preferred embodiments of the present disclosure, in formula (I), L is O=S=O. In some particularly preferred embodiments of the present disclosure, in formula (I), L is $CH_2$. In other embodiments of the present disclosure, in formula (I), L is a covalent bond.

In some particularly preferred embodiments of the present disclosure, in formula (I), X is CH. In other some embodiments of the present disclosure, in formula (I), X is N.

In some particularly preferred embodiments of the present disclosure, in formula (I), X is CH, and L is C=O.

In some particularly preferred embodiments of the present disclosure, in formula (I), X is CH, and L is O=S=O.

In some particularly preferred embodiments of the present disclosure, in formula (I), X is CH, and L is CH2.

In some particularly preferred embodiments of the present disclosure, in formula (I), X is CH, and L is a covalent bond.

In some particularly preferred embodiments of the present disclosure, in formula (I), X is N, and L is C=O.

In some particularly preferred embodiments of the present disclosure, in formula (I), X is N, and L is O=S=O.

In some particularly preferred embodiments of the present disclosure, in formula (I), X is N, and L is $CH_2$.

In some particularly preferred embodiments of the present disclosure, in formula (I), X is N, and L is a covalent bond.

In some preferred embodiments of the present disclosure, in formula (I), the ring A is $C_{3-7}$ cycloalkyl, 3-7 membered heterocycloalkyl, $C_{5-7}$ aryl, 5-7 membered heteroaryl, where the ring A is optionally substituted with 1, 2, 3 or 4 $R_1$(s). In some preferred embodiments of the present disclosure, in formula (I), the ring A is $C_{5-6}$ cycloalkyl, 5-6 membered heterocycloalkyl, phenyl, or 5-6 membered heteroaryl, wherein the ring A is optionally substituted with 1, 2, 3 or 4 $R_1$(s). In some preferred embodiments of the present disclosure, in formula (I), the ring A is 5-6 membered heterocycloalkyl, phenyl, or 5-6 membered heteroaryl, wherein the ring A is optionally substituted with 1, 2, 3 or 4 $R_1$(s). In some preferred embodiments of the present disclosure, in formula (I), the ring A is 5-6 membered azacycloalkyl, or phenyl, 5-6 membered azaaryl, where the ring A is optionally substituted with 1, 2, 3 or 4 $R_1$(s). In some preferred embodiments of the present disclosure, in formula (I), the ring A is pyrazinyl, pyrazolyl, piperidinyl or phenyl, where the ring A is optionally substituted with 1, 2, 3 or 4 $R_1$(s). In some particularly preferred embodiments of the present disclosure, in formula (I), the ring A is pyrazinyl. In some particularly preferred embodiments of the present disclosure, in formula (I), the ring A is pyrazolyl. In some particularly preferred embodiments of the present disclosure, in formula (I), the ring A is piperidinyl. In some particularly preferred embodiments of the present disclosure, in formula (I), the ring A is phenyl.

In some preferred embodiments of the present disclosure, in formula (I), $R_1$ is absent or $R_1$ is selected from $C_{1-8}$ alkyl, $C_{2-8}$ alkenyl, $C_{2-8}$ alkynyl, $C_{1-8}$ alkoxy, $C_{3-7}$ cycloalkyl, 3-7 membered heterocycloalkyl, $C_{5-7}$ aryl, and 5-7 membered heteroaryl, wherein the $C_{1-8}$ alkyl, $C_{2-8}$ alkenyl, $C_{2-8}$ alkynyl, and $C_{1-8}$ alkoxy are optionally substituted with 1, 2, 3 or 4 $R(s)_3$, and wherein the $C_{3-7}$ cycloalkyl, 3-7 membered heterocycloalkyl, $C_{5-7}$ aryl, and 5-7 membered heteroaryl group are optionally substituted with 1, 2, 3, or 4 $R_4$(s). In some preferred embodiments of the present disclosure, in formula (I), $R_1$ is absent or $R_1$ is selected from $C_{1-8}$ alkyl, $C_{3-7}$ cycloalkyl, 3-7 membered heterocycloalkyl, $C_{5-7}$ aryl, 5-7 membered heteroaryl, wherein the $C_{1-8}$ alkyl group is optionally substituted with 1, 2, 3 or 4 $R_3$(s), and wherein the $C_{3-7}$ cycloalkyl group, 3-7 membered heterocycloalkyl, $C_{5-7}$ aryl, 5-7 membered heteroaryl are optionally substituted with 1, 2, 3, or 4 $R_4$(s). In some preferred embodiments of the present disclosure, in formula (I), $R_1$ is absent or $R_1$ is selected from $C_{1-8}$ alkyl, and 3-7 membered heterocycloalkyl, wherein the C1-8 alkyl is optionally substituted with 1, 2, 3, or 4 $R_3$(s), and wherein the 3-7 membered heterocycloalkyl is optionally substituted with 1, 2, 3, or 4 $R_4$(s). In some preferred embodiments of the present disclosure, in formula (I), $R_1$ is absent or $R_1$ is selected from $C_{1-6}$ alkyl, 5-7 membered heterocycloalkyl, wherein the $C_{1-6}$ alkyl is optionally substituted with 1, 2, 3, or 4 $R_3$(s), and wherein the 5-7 membered heterocycloalkyl is optionally substituted with 1, 2, 3, or 4 $R_4$(s). In some preferred embodiments of the present disclosure, in formula (I), $R_1$ is absent or $R_1$ is selected from $C_{1-6}$ alkyl, 5-7 membered heterocycloalkyl, wherein the C1-6 alkyl is optionally substituted with 1 or 2 $R_3$, and wherein the 5-7 membered heterocycloalkyl is optionally substituted with 1, 2, 3 or 4 $C_{1-3}$ alkyl. In some preferred embodiments of the present disclosure, in formula (I), $R_1$ is absent or $R_1$ is selected from $C_{1-4}$ alkyl, and 5-7 membered heterocycloalkyl, wherein the $C_{1-4}$ alkyl is optionally substituted with 1 or 2 $R_3$, and wherein the 5-7 membered heterocycloalkyl is optionally substituted with 1, 2, 3 or 4 $C_{1-3}$ alkyl. In some preferred embodiments of the present disclosure, in formula (I), $R_1$ is absent or $R_1$ is selected from methyl, piperidinyl, morpholinyl, piperazinyl, wherein the piperidinyl, morpholinyl, and piperazinyl are optionally substituted with 1, 2, 3 or 4 $C_{1-3}$ alkyl groups. In some preferred embodiments of the present disclosure, in formula (I), $R_1$ is absent or $R_1$ is selected from methyl, piperidinyl, morpholinyl, and 1-methylpiperazinyl. In some particularly preferred embodiments of the present disclosure, in formula (I), $R_1$ is absent. In some particularly preferred embodiments of the present disclosure, in formula (I), $R_1$ is 1-methylpiperazinyl. In some particularly preferred embodiments of the present disclosure, in formula (I), $R_1$ is methyl. In some particularly preferred embodiments of the present disclosure, in formula (I), R1 is piperidinyl. In some particularly preferred embodiments of the present disclosure, in formula (I), R1 is morpholinyl.

In some particularly preferred embodiments of the present disclosure, in formula (I), one, two or three $R_2$(s)

are present and $R_2$ is selected from halogen, $C_{1-6}$ alkyl, and $C_{3-6}$ cycloalkyl, in which the $C_{1-6}$ alkyl, and $C_{3-6}$ cycloalkyl are each optionally substituted with 1, 2 or 3 substituent(s) each independently selected from the group consisting of halogen, - OH, $-NH_2$, $-NH(CH_3)$, $-N(CH_3)_2$, -CN, $C_{1-4}$ alkyl, $C_{1-4}$ haloalkyl, $C_{1-4}$ alkoxy, and $C_{1-4}$ haloalkoxy. In some particularly preferred embodiments of the present disclosure, in formula (I), one, two or three $R_2$(s) are present and $R_2$ is selected from halogen, and $C_{1-6}$ alkyl, in which the $C_{1-6}$ alkyl is optionally substituted with 1, 2 or 3 substituent(s) each independently selected from the group consisting of halogen, -OH, $-NH_2$, $-NH(CH_3)$, $-N(CH_3)_2$, -CN, $C_{1-4}$ alkyl, $C_{1-4}$ haloalkyl, $C_{1-4}$ alkoxy, and $C_{1-4}$ haloalkoxy. In some particularly preferred embodiments of the present disclosure, in formula (I), one or two $R_2$(s) are present and $R_2$ is selected from fluorine, chlorine, bromine, methyl, ethyl, n-propyl, isopropyl, n-butyl, and isobutyl. In some preferred embodiments of the present disclosure, in formula (I), one or two $R_2$(s) are present, and $R_2$ is selected from fluorine, chlorine, methyl, ethyl, n-propyl, and isopropyl. In some preferred embodiments of the present disclosure, in formula (I), one or two $R_2$ (s) are present, and $R_2$ is selected from fluorine, methyl, and ethyl. In some preferred embodiments of the present disclosure, in formula (I), one or two $R_2$ (s) are present, and $R_2$ is selected from fluorine and ethyl. In some preferred embodiments of the present disclosure, in formula (I), one $R_2$ is present, and $R_2$ is selected from fluorine and ethyl. In some preferred embodiments of the present disclosure, in formula (I), two $R_2$(s) are present, and $R_2$ is selected from fluorine and ethyl. In some particularly preferred embodiments of the present disclosure, in formula (I), two $R_2$(s) are present which are respectively fluorine and ethyl. In some particularly preferred embodiments of the present disclosure, in formula (I), one $R_2$ is present, and $R_2$ is an ethyl group.

The preferred options of the respective substituents mentioned in the above various preferred embodiments can be combined with each other in any way, and various combinations thereof are within the scope of the present disclosure.

In particular, the present disclosure provides use of a compound of formula (I) as a JAK inhibitor:

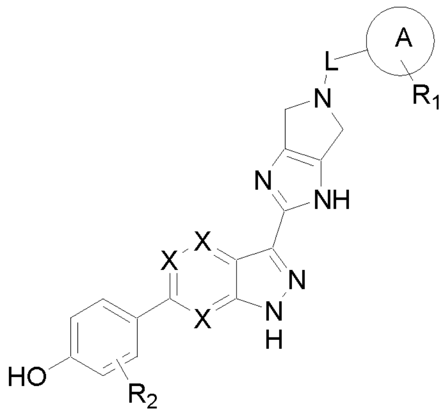

(I)

or an isotopically labeled compound thereof, or an optical isomer thereof, a geometric isomer thereof, a tautomer thereof or a mixture of various isomers, or a pharmaceutically acceptable salt thereof, or a prodrug thereof, or a metabolite thereof, in the manufacture of a medicament for treating severe pneumonia

in which

L is C=O, and O=S=O;

X is CH;

The ring A is 5-7 membered heteroaryl or $C_{5-7}$ aryl;

0, 1, 2, 3 or 4 $R_1$(s) are present in formula (I), and $R_1$ is selected from $C_{1-8}$ alkyl, and 3-7 membered heterocycloalkyl, in which the $C_{1-8}$ alkyl is optionally substituted with 1, 2, 3 or 4 $R_3$(s), and in which the 3-7 membered heterocycloalkyl is optionally substituted with 1, 2, 3 or 4 $R_4$(s),

1, 2, or 3 $R_2$(s) are present in formula (I), and $R_2$ is selected from H, halogen, -OH, $-NO_2$, - CN, $-SF_5$, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 4-10 membered heterocycloalkyl, $-N(R_9)(R_{10})$, $-N(R_{11})(C(=O)R_{12})$, $-C(=O)-N(R_9)(R_{10})$, - $C(=O)-R_{12}$, $-C(=O)-OR_{12}$, $-OC(=O)R_{12}$, $-N(R_{11})(S(=O)_2R_{12})$, $-S(=O)_2-N(R_9)(R_{10})$, $-SR_{12}$ and $-OR_{12}$, in which the $C_{1-6}$ alkyl, $C_{3-7}$ cycloalkyl and 4-10 membered heterocycloalkyl are each optionally substituted with 1, 2 or 3 substituent(s) each independently selected from the group consisting of halogen, -CN, -OH, $C_{1-4}$ alkyl, $C_{1-4}$ alkoxy, $C_{1-4}$ haloalkyl, $C_{1-4}$ haloalkoxy, $C_{3-6}$ cycloalkyl, $-N(R_9)(R_{10})$, $-N(R_{11})(C(=O)R_{12})$, $-C(=O)-OR_{12}$, -C(=O)H, $-C(=O)R_{12}$, - $C(=O)-N(R_9)(R_{10})$, $-N(R_{11})(S(=O)_2R_{12})$, $-S(=O)_2-N(R_9)(R_{10})$, $-SR_{12}$ and $-OR_{12}$;

$R_3$ is selected from halogen, cyano, $C_{1-3}$ alkyl, hydroxy, $C_{1-6}$ alkoxy, $-N(R_5)(R_6)$, - $CON(R_7)(R_8)$ or 3-7 membered heterocycloalkyl, in which the 3-7 membered heterocycloalkyl is optionally substituted with 1, 2, 3 or 4 $R_4$(s);

$R_4$ is selected from halogen, $C_{1-3}$ alkyl, hydroxyl, $C_{1-6}$ alkoxy, $-NH_2$, $-NHCH_3$ or $-N(CH_3)_2$; $R_5$, $R_6$, $R_7$, $R_8$ are each independently hydrogen or $C_{1-4}$ alkyl;

$R_9$ is selected from H, $C_{1-4}$ alkyl, $C_{1-4}$ haloalkyl or $C_{3-7}$ cycloalkyl;

$R_{10}$ is H or selected from the group consisting of $C_{1-4}$ alkyl, $C_{1-4}$ haloalkyl, $C_{3-7}$ cycloalkyl, 4-10 membered heterocycloalkyl, $C_{6-10}$ aryl, 5-10 membered heteroaryl, ($C_{3-7}$ cycloalkyl)-$C_{1-4}$ alkyl-, (4-10 membered heterocycloalkyl)-$C_{1-4}$ alkyl-, ($C_{6-10}$ aryl)-$C_{1-4}$ alkyl- and (5-10 membered heteroaryl)-$C_{1-4}$ alkyl-, wherein each substituent included in the above group is optionally substituted with 1, 2, 3 or 4 substituent(s) each independently selected from the group consisting of -OH, $-NH_2$, $-NH(CH_3)$, $-N(CH_3)_2$, -CN, $C_{1-4}$ alkyl, $C_{3-7}$ cycloalkyl, $C_{1-4}$ hydroxyalkyl, -S-$C_{1-4}$ alkyl, -C(=O)H, -C(=O)-$C_{1-4}$ alkyl, -C(=O)-O-$C_{1-4}$ alkyl, $-C(=O)-NH_2$, -C( =O)-N($C_{1-4}$ alkyl$)_2$, $C_{1-4}$ haloalkyl, $C_{1-4}$ alkoxy and $C_{1-4}$ haloalkoxy;

$R_{11}$ is selected from H, $C_{1-4}$ alkyl and $C_{3-7}$ cycloalkyl; and

$R_{12}$ is selected from the group consisting of $C_{1-6}$ alkyl, $C_{3-7}$ cycloalkyl, 4- to 14-membered heterocycloalkyl, $C_{6-10}$ aryl, 5-10 membered heteroaryl, ($C_{3-7}$ cycloalkyl)-$C_{1-4}$ alkyl-, (4-10 membered heterocycloalkyl)-$C_{1-4}$ alkyl-, ($C_{6-10}$ aryl)-$C_{1-4}$ alkyl- and (5-10 membered heteroaryl)-$C_{1-4}$ alkyl-, wherein each substituent included in the above group is optionally substituted with 1, 2 or 3 substituent(s) each independently selected from the group consisting of halogen, $-CF_3$, -CN, -OH, $-NH_2$, $-NH(CH_3)$, $-N(CH_3)_2$, oxo, -S-$C_{1-4}$ alkyl, $C_{1-4}$ alkyl, $C_{1-4}$ haloalkyl, $C_{2-6}$ alkenyl, $C_{2-6}$ alkynyl , $C_{3-7}$ cycloalkyl, $C_{1-4}$ alkoxy and $C_{1-4}$ haloalkoxy.

In some preferred embodiments of the present disclosure, in formula (I), L is O=S=O. In some preferred embodiments of the present disclosure, in formula (I), L is C=O.

In some preferred embodiments of the present disclosure, in formula (I), the ring A is 5-6 membered heteroaryl or phenyl wherein the ring A is optionally substituted with 1, 2, 3 or 4 $R_1$(s). In some preferred embodiments of the present disclosure, in formula (I), the ring A is pyrazinyl, pyrazolyl, or phenyl, where the ring A is optionally substituted with 1, 2, 3 or 4 $R_1$(s). In some particularly preferred embodiments of the present disclosure, in formula (I), the ring A is pyrazinyl. In some particularly preferred embodiments of the present disclosure, in formula (I), the ring A is phenyl.

In some preferred embodiments of the present disclosure, in formula (I), $R_1$ is absent or $R_1$ is selected from $C_{1-8}$ alkyl, and 3-7 membered heterocycloalkyl, wherein the C1-8 alkyl is optionally substituted with 1, 2, 3, or 4 $R_3$(s), and wherein the 3-7 membered heterocycloalkyl is optionally substituted with 1, 2, 3, or 4 $R_4$(s). In some preferred embodiments of the present disclosure, in formula (I), $R_1$ is absent or $R_1$ is selected from $C_{1-6}$ alkyl, 5-7 membered heterocycloalkyl, wherein the $C_{1-6}$ alkyl is optionally substituted with 1, 2, 3, or 4 $R_3$(s), and wherein the 5-7 membered heterocycloalkyl is optionally substituted with 1, 2, 3, or 4 $R_4$(s). In some preferred embodiments of the present disclosure, in formula (I), $R_1$ is absent or $R_1$ is selected from $C_{1-6}$ alkyl, 5-7 membered heterocycloalkyl, wherein the C1-6 alkyl is optionally substituted with 1 or 2 $R_3$(s), and wherein the 5-7 membered heterocycloalkyl is optionally substituted with 1, 2, 3 or 4 $C_{1-3}$ alkyl. In some preferred embodiments of the present disclosure, in formula (I), $R_1$ is absent or $R_1$ is selected from $C_{1-4}$ alkyl, and 5-7 membered heterocycloalkyl, wherein the $C_{1-4}$ alkyl is optionally substituted with 1 or 2 $R_3$, and wherein the 5-7 membered heterocycloalkyl is optionally substituted with 1, 2, 3 or 4 $C_{1-3}$ alkyl. In some preferred embodiments of the present disclosure, in formula (I), $R_1$ is absent or $R_1$ is selected from methyl, piperidinyl, morpholinyl, wherein the piperidinyl, and morpholinyl are optionally substituted with 1, 2, 3 or 4 $C_{1-3}$ alkyl groups. In some preferred embodiments of the present disclosure, in formula (I), $R_1$ is absent or $R_1$ is selected from methyl, piperidinyl, and morpholinyl. In some particularly preferred embodiments of the present disclosure, in formula (I), $R_1$ is absent. In some particularly preferred embodiments of the present disclosure, in formula (I), $R_1$ is methyl. In some particularly preferred embodiments of the present disclosure, in formula (I), R1 is piperidinyl. In some particularly preferred embodiments of the present disclosure, in formula (I), R1 is morpholinyl.

In some particularly preferred embodiments of the present disclosure, in formula (I), one or two $R_2$(s) are present and $R_2$ is selected from halogen, $C_{1-6}$ alkyl, and $C_{3-6}$ cycloalkyl, in which the $C_{1-6}$ alkyl, and $C_{3-6}$ cycloalkyl are each optionally substituted with 1, 2 or 3 substituent(s) each independently selected from the group consisting of halogen, -OH, $-NH_2$, $-NH(CH_3)$, $-N(CH_3)_2$, -CN, $C_{1-4}$ alkyl, $C_{1-4}$ haloalkyl, $C_{1-4}$ alkoxy, and $C_{1-4}$ haloalkoxy. In some particularly preferred embodiments of the present disclosure, in formula (I), one, or two $R_2$(s) are present and $R_2$ is selected from halogen, and $C_{1-6}$ alkyl, in which the $C_{1-6}$ alkyl is optionally substituted with 1, 2 or 3 substituent(s) each independently selected from the group consisting of halogen, -OH, $-NH_2$, $-NH(CH_3)$, $-N(CH_3)_2$, -CN, $C_{1-4}$ alkyl, $C_{1-4}$ haloalkyl, $C_{1-4}$ alkoxy, and $C_{1-4}$ haloalkoxy. In some particularly preferred embodiments of the present disclosure, in formula (I), two $R_2$s are present and $R_2$ is selected from fluorine, chlorine, bromine, methyl, ethyl, n-propyl, isopropyl, n-butyl, and isobutyl. In some preferred embodiments of the present disclosure, in formula (I), two $R_2$s are present, and $R_2$ is selected from fluorine, chlorine, methyl, ethyl, n-propyl, and isopropyl. In some preferred embodiments of the present disclosure, in formula (I), two $R_2$s are present, and $R_2$ is selected from fluorine, methyl, and ethyl. In some preferred embodiments of the present disclosure, in formula (I), two $R_2$s are present, and $R_2$ is selected from fluorine and ethyl. In some preferred embodiments of the present disclosure, in formula (I), two $R_2$(s) are present, and $R_2$ is selected from fluorine and ethyl. In some particularly preferred embodiments of the present disclosure, in formula (I), two $R_2$(s) are present which are

respectively fluorine and ethyl.

The preferred options of the respective substituents mentioned in the above various preferred embodiments can be combined with each other in any way, and various combinations thereof are within the scope of the present disclosure.

In a more preferred embodiment of the present disclosure, the compound of formula (I) is selected from

(2-(6-(2-ethyl-5-fluoro-4-hydroxyphenyl)-1H-indazol-3-yl)-4,6-dihydropyrrolo[3,4-d]imidazol-5(1H)-yl)(5-(piperidin-1-yl)pyrazin-2-yl)ketone;
(2-(6-(2-ethyl-5-fluoro-4-hydroxyphenyl)-1H-indazol-3-yl)-4,6-dihydropyrrolo[3,4-d]imidazol-5(1H)-yl)(5-morpholinylpyrazin-2-yl)ketone;
(2-(6-(2-ethyl-5-fluoro-4-hydroxyphenyl)-1H-indazol-3-yl)-4,6-dihydropyrrolo[3,4-d]imidazol-5(1H)-yl)(1-methyl-1H-pyrazol-4-yl)ketone;
(2-(6-(2-ethyl-5-fluoro-4-hydroxyphenyl)-1H-indazol-3-yl)pyrrolo[3,4-d]imidazol-5(1H, 4H,6H)-yl)(1-methylpiperidin-4-yl)ketone;
(2-(6-(2-ethyl-5-fluoro-4-hydroxyphenyl)-1H-indazol-3-yl)pyrrolo[3,4-d]imidazol-5(1H,4H,6H)-yl)(5-(4-methylpiperzin-1-yl)pyrazin-2-yl)ketone;
(2-(6-(2-ethyl-4-hydroxyphenyl)-1H-indazol-3-yl)pyrrolo[3,4-d]imidazol-5(1H,4H,6H)-yl)(5-(4-methylpiperzin-1-yl)pyrazin-2-yl)ketone;
5-ethyl-2-fluoro-4-(3-(5-(benzenesulfonyl)-1,4,5,6-tetrahydropyrrolo[3,4-d]imidazol-2-yl)-1H-indazol-6-yl)phenol;
5-ethyl-2-fluoro-4-(3 -(5-(pyrazin-2ylmethyl)-1,4, 5, 6-tetrahydropyrrolo[3,4-d]imidazol-2-yl)-1H-indazol-6-yl)phenol;
4-(3-(5-(cyclopropylmethyl)-1,4,5,6-tetrahydropyrrolo[3,4-d]imidazol-2-yl)-1H-indazol-6-yl)-5-ethyl-2-fluorophenol;
Cyclopropyl (2-(6-(2-ethyl-5-fluoro-4-hydroxyphenyl)-1H-indazol-3-yl)pyrrolo[3,4-d]imidazol-5(1H,4H,6H)-yl)ketone;
4-(3-(5-(cyclobutylmethyl)-1,4,5,6-tetrahydropyrrolo[3,4-d]imidazol-2-yl)-1H-indazol-6-yl)-5-ethyl-2-fluorophenol;
Cyclobutyl (2-(6-(2-ethyl-5-fluoro-4-hydroxyphenyl)-1H-indazol-3-yl)pyrrolo[3,4-d]imidazol-5(1H,4H,6H)-yl)ketone;
(2-(6-(2-ethyl-5-fluoro-4-hydroxyphenyl)-1H-indazol-3-yl)-pyrrolo[3,4-d]imidazol-5-(1H,4H,6H)-yl)(pyridazin-4-yl)ketone;
(2-(6-(2-ethyl-5-fluoro-4-hydroxyphenyl)-1H-indazol-3-yl)-pyrrolo[3,4-d]imidazol-5-(1H,4H,6H)-yl)(pyridazin-3-yl)ketone;
4-(3-(5-(cyclopropanesulfonyl)-1,4,5,6-tetrahydropyrrolo[3,4-d]imidazol-2-yl)-1H-indazol-6-yl)-5-ethyl-2-fluorophenol;
4-(3-(5-(cyclobutylsulfonyl)-1,4,5,6-tetrahydropyrrolo[3,4-d]imidazol-2-yl)-1H-indazol-6-yl)-5-ethyl-2-fluorophenol;
4-(3-(5-(cyclopentylsulfonyl)-1,4, 5, 6-tetrahydropyrrolo[3,4-d]imidazol-2-yl)-1H-indazol-6-yl)-5-ethyl-2-fluorophenol;
5-ethyl-2-fluoro-4-(3-(5-((1-methyl-1H-pyrazol-4-yl)methyl)-1,4,5,6-tetrahydropyrrolo[3,4-d]imidazol-2-yl)-1H-indazol-6-yl)phenol;
4-(3-(5-(cyclopentyl-1,4,5,6-tetrahydropyrrolo[3,4-d]imidazol-2-yl)-1H-indazol-6-yl)-5-ethyl-2-fluorophenol;
5-ethyl-2-fluoro-4-(3 -(5-(tetrahydro-2H-pyran-4-yl)-1,4, 5, 6-tetrahydropyrrolo[3,4-d]imidazol-2-yl)-1H-indazol-6-yl)phenol;
(2-(6-(2-ethyl-5-fluoro-4-hydroxyphenyl)-1H-indazol-3-yl)-4,6-dihydropyrrolo[3,4-d]imidazol-5-(1H)-yl)(pyrrolidin-1-yl)ketone;
(2-(6-(2-ethyl-5-fluoro-4-hydroxyphenyl)-1H-indazol-3-yl)-4,6-dihydropyrrolo[3,4-d]imidazol-5-(1H)-yl)(piperidin-1-yl)ketone;
(2-(6-(2-ethyl-5-fluoro-4-hydroxyphenyl)-1H-indazol-3-yl)-4,6-dihydropyrrolo[3,4-d]imidazol-5-(1H)-yl)(morpholino)ketone;
(2-(6-(2-ethyl-5-fluoro-4-hydroxyphenyl)-1H-indazol-3-yl)-4,6-dihydropyrrolo[3,4-d]imidazol-5-(1H)-yl)(4-methylpiperzin-1-yl)ketone;
(2-(6-(2-ethyl-5-fluoro-4-hydroxyphenyl)-1H-indazol-3-yl)-4,6-dihydropyrrolo[3,4-d]imidazol-5-(1H)-yl)(4-ethylpiperzin-1-yl)ketone;
Cyclopropyl(2-(6-(2-ethyl-5-fluoro-4-hydroxyphenyl)-1H-pyrazolo [4,3-b]pyridin-3-yl)-4,6-dihydropyrrolo[3,4-d]imidazol-5(1H)-yl)ketone;
Cyclopropyl(2-(6-(2-ethyl-5-fluoro-4-hydroxyphenyl)-4-methyl-1H-indazol-3-yl)-4,6-dihydropyrrolo[3,4-d]imidazol-5(1H)-yl)ketone;
(S)-(2-(6-(2-ethyl-5-fluoro-4-hydroxyphenyl)-4-methyl-1H-indazol-3-yl)-4,6-dihydropyrrolo[3,4-d]imidazol -5(1H)-yl)(3-hydroxylpyrrolidin-1-yl)ketone;
Cyclopropyl(2-(6-(2-ethyl-5-fluoro-4-hydroxyphenyl)-1H-pyrazolo[4,3-c]pyridin-3-yl)-4,6-dihydropyrrolo[3,4-d]imidazol-5(1H)-yl)ketone;
(R)-(2-(6-(2-ethyl-5-fluoro-4-hydroxyphenyl)-1H-indazol-3-yl)-4,6-dihydropyrrolo[3,4-d]imidazol-5(1H )-yl)(3-hydroxylpyrrolidin-1-yl)ketone;
(2-(6-(2-ethyl-5-fluoro-4-hydroxyphenyl)-1H-indazol-3-yl)-4,6-dihydropyrrolo[3,4-d]imidazol-5(1H)-yl)(4-hydroxylpiperidin-1-yl)ketone;
1-(2-(6-(2-ethyl-5-fluoro-4-hydroxyphenyl)-1H-indazol-3 -yl)-1,4, 5, 6-tetrahydropyrrolo[3,4-d]imidazol-5 -carbonyl)pyrrolidin-3-nitrile;
(S)-(2-(6-(2-ethyl-5-fluoro-4-hydroxyphenyl)-1H-pyrazolo[3,4-b]pyridin-3-yl)-4,6-dihydropyrrolo[3,4-d]imidazol-5(1H)-yl)(3-hydroxylpyrrolidin-1-yl)ketone;
(S)-6-(2-ethyl-5-fluoro-4-hydroxyphenyl)-3-(5-prolyl-1,4,5,6-tetrahydropyrrolo[3,4-d]imidazol-2-yl)- 1H-indazol; and
(R)-6-(2-ethyl-5-fluoro-4-hydroxyphenyl)-3-(5-prolyl-1,4,5,6-tetrahydropyrrolo[3,4-d]imidazol-2-yl)- 1H-indazol.

In some of the most preferred embodiments of the present disclosure, the compound of formula (I) is each specific compound shown in Example 1 to Example 8 herein. That is, the compound of formula (I) is selected from

(2-(6-(2-ethyl-5-fluoro-4-hydroxyphenyl)-1H-indazol-3-yl)-4,6-dihydropyrrolo[3,4-d]imidazol-5(1H)-yl)(5-(piperidin-1-yl)pyrazin-2-yl)ketone;
(2-(6-(2-ethyl-5-fluoro-4-hydroxyphenyl)-1H-indazol-3-yl)-4,6-dihydropyrrolo[3,4-d]imidazol-5(1H)-yl)(5-morpholinpyrazin-2-yl)ketone;
(2-(6-(2-ethyl-5-fluoro-4-hydroxyphenyl)-1H-indazol-3-yl)-4,6-dihydropyrrolo[3,4-d]imidazol-5(1H)-yl)(1-methyl-1H-pyrazol-4-yl)ketone;
5-ethyl-2-fluoro-4-{3-[5-(1-methylpiperidin-4-carbonyl)-1H,4H,5H,6H-pyrrolo[3,4-d]imidazol-2-yl]-1H-indazol-6-yl} phenol;
5-ethyl-2-fluoro-4-{3-[5-(4-methylpiperazin-1-carbonyl)-1H,4H,5H,6H-pyrrolo[3,4-d]imidazol-2-yl]-1H-indazol-6-yl} phenol;
3-ethyl-4-{3-[5-(4-methylpiperazin-1-carbonyl)-1H,4H,5H,6H-pyrrolo[3,4-d]imidazol-2-yl]-1H-indazol-6-yl} phenol;
5-ethyl-2-fluoro-4-(3-(5-(bemzenesulfonyl)-1,4,5,6-tetrahydropyrrolo[3,4-d]imidazol-2-yl)-1H-indazol-6-yl)phenol; and
5-ethyl-2-fluoro-4-(3-(5-(pyrazin-2-methyl)-1,4,5,6-tetrahydropyrrolo[3,4-d]imidazol-2-yl)-1H-indazol-6-yl)phenol.

For simplicity, hereinafter, the term "a compound as shown by Formula (G)" or "a compound of Formula (G)" or "a compound of the invention" or "a compound according to the invention" also encompasses any optical isomer, geometric isomer, tautomer or a mixture of various isomers of the compound of Formula (G); the term "a compound as shown by Formula (G')" or "a compound of Formula (G')" or "a compound of the invention" or "a compound according to the invention" also encompasses any optical isomer, geometric isomer, tautomer or a mixture of various isomers of the compound of Formula (G'); and the term "a compound as shown by Formula (I)" or "a compound of Formula (I)" or "a compound of the invention" or "a compound according to the invention" also encompasses any optical isomer, geometric isomer, tautomer or a mixture of various isomers of the compound of Formula (I).

The term "optical isomer" refers that when a compound has one or more chiral centers, each chiral center may have an R configuration or an S configuration, and the various isomers thus constituted are known as an optical isomer. Optical isomers comprise all diastereomers, enantiomers, meso forms, racemates or mixtures thereof. For example, optical isomers can be separated by a chiral chromatography or by chiral synthesis.

The term "geometric isomer" refers that when a double bond is present in a compound, the compound may exist as a cis isomer, a trans isomer, an E isomer, or a Z isomer. A geometric isomer comprises a cis isomer, trans isomer, E isomer, Z isomer, or a mixture thereof.

The term "tautomer" refers to an isomer that is formed by rapid movement of an atom at two positions in a single molecule. It will be understood by those skilled in the art that tautomers can be mutually transformed, and in a certain state, may coexist by reaching an equilibrium state. As used herein, the term "a compound as shown by Formula (G)" also encompasses any tautomer of the compound of Formula (G); "a compound as shown by Formula (G')" also encompasses any tautomer of the compound of Formula (G'); and "a compound as shown by Formula (I)" also encompasses any tautomer of the compound of Formula (I).

Unless otherwise indicated, reference to "a compound as shown by Formula (G)" or "a compound of Formula (G)" or "a compound of the invention" or "a compound according to the invention" herein also encompasses isotopically-labeled compounds obtained by replacing any atom of the compound with its isotopic atom; reference to "a compound as shown by Formula (G')" or "a compound of Formula (G')" or "a compound of the invention" or "a compound according to the invention" herein also encompasses isotopically-labeled compounds obtained by replacing any atom of the compound with its isotopic atom; and reference to "a compound as shown by Formula (I)" or "a compound of Formula (I)" or "a compound of the invention" or "a compound according to the invention" herein also encompasses isotopically-labeled compounds obtained by replacing any atom of the compound with its isotopic atom.

The invention comprises all pharmaceutically acceptable isotopically-labeled compounds of Formula (G) wherein one or more atoms are replaced by atoms having the same atomic number but different atomic mass or mass number than those normally found in nature. The invention comprises all pharmaceutically acceptable isotopically-labeled compounds of Formula (G') wherein one or more atoms are replaced by atoms having the same atomic number but different atomic mass or mass number than those normally found in nature. The invention comprises all pharmaceutically acceptable isotopically-labeled compounds of Formula (I) wherein one or more atoms are replaced by atoms having the same atomic number but different atomic mass or mass number than those normally found in nature.

Examples of isotopes suitable for inclusion in the compounds of the invention include isotopes of hydrogen, such as $^{2}H$ (D) and $^{3}H$ (T), of carbon, such as $^{11}C$, $^{13}C$ and $^{14}C$, of chlorine, such as $^{36}C1$, of fluorine, such as $^{18}F$, of iodine, such as $^{123}I$ and $^{125}I$, of nitrogen, such as $^{13}N$ and $^{15}N$, of oxygen, such as $^{15}O$, $^{17}O$ and $^{18}O$, and of sulphur, such as $^{35}S$.

Certain isotopically-labelled compounds of formula (G), for example, those incorporating a radioactive isotope, are useful in drug and/or substrate tissue distribution studies. Certain isotopically-labelled compounds of formula (G'), for example, those incorporating a radioactive isotope, are useful in drug and/or substrate tissue distribution studies. Certain isotopically-labelled compounds of formula (I), for example, those incorporating a radioactive isotope, are useful in drug and/or substrate tissue distribution studies. The radioactive isotopes deuterium, i.e. $^{2}H$, and carbon-14, i.e. $^{14}C$, are particularly useful for this purpose in view of their ease of incorporation and ready means of detection.

Substitution with heavier isotopes such as deuterium, i.e. $^{2}H$, may afford certain therapeutic advantages resulting from greater metabolic stability, for example, increased in vivo half-life or reduced dosage requirements, and hence may be preferred in some circumstances.

Substitution with positron emitting isotopes, such as $^{11}C$, $^{18}F$, $^{15}O$ and $^{13}N$, can be useful in Positron Emission Topography (PET) studies for examining substrate receptor occupancy.

Isotopically-labeled compounds of formula (G) can generally be prepared by conventional techniques known to those skilled in the art or by processes analogous to those described in the accompanying Examples and Preparations using an appropriate isotopically-labeled reagents in place of the non-labeled reagent previously employed. Isotopically-labeled compounds of formula (G') can generally be prepared by conventional techniques known to those skilled in the art or by processes analogous to those described in the accompanying Examples and Preparations using an appropriate isotopically-labeled reagents in place of the non-labeled reagent previously employed. Isotopically-labeled compounds of formula (I) can generally be prepared by conventional techniques known to those skilled in the art or by processes analogous to those described in the accompanying Examples and Preparations using an appropriate isotopically-labeled reagents in place of the non-labeled reagent previously employed.

The compound of formula (G) may exist in the form of a pharmaceutically acceptable salt, for example, an acid addition salt and/or a base addition salt of the compound of formula (G). Unless otherwise indicated, "a pharmaceutically acceptable salt" as used herein includes acid addition salts or base addition salts that may appear in the compound of formula (G). The compound of formula (G') may exist in the form of a pharmaceutically acceptable salt, for example, an acid addition salt and/or a base addition salt of the compound of formula (G'). Unless otherwise indicated, "a pharmaceutically acceptable salt" as used herein includes acid addition salts or base addition salts that may appear in the compound of formula (G'). The compound of formula (I) may exist in the form of a pharmaceutically acceptable salt, for example, an acid addition salt and/or a base addition salt of the compound of formula (I). Unless otherwise indicated, "a pharmaceutically acceptable salt" as used herein includes acid addition salts or base addition salts that may appear in the compound of formula (I).

The pharmaceutically acceptable salt of the compound of formula (G), the compound of formula (G') and the compound of formula (I) include acid addition salts and base addition salts thereof. Suitable acid addition salts are formed from acids that form non-toxic salts. Examples include but are not limited to: acetate, adipate, aspartate, benzoate, benzenesulfonate, bicarbonate/carbonate, bisulfate/sulfate, borate, camphor sulfonate, citrate, cyclohexamine sulfonate, ethanedisulfonate, formate, fumarate, glucoheptonate, gluconate, glucuronate, hexafluorophosphate, 2-(4-hydroxybenzyl) benzoate, hydrochloride/chloride, hydrobromide/bromide, hydroiodide/iodide, 2-isethionate, lactate, malate, maleate, malonate, methanesulfonate, methyl sulfate, naphthalate, 2-naphthalenesulfonate, nicotinate, nitrate, orotate, oxalate, palmitate, phosphate/hydrogen phosphate/dihydrogen phosphate, pyroglutamate, glucarate, stearate, salicylate, tannate, tartrate, tosylate and trifluoroacetate. Suitable base addition salts are formed from bases that form non-toxic salts. Examples thereof include, but are not limited to: aluminum, arginine, calcium, choline, diethylamine, diethanolamine, glycine, lysine, magnesium, meglumine, ethanolamine, potassium, sodium, tromethamine, and zinc salts. It is also possible to form half salts of acids and bases, such as hemisulfate and hemicalcium salts. For a review of suitable salts, please refer to Handbook of Pharmaceutical Salts: Properties, Selection and Use by Stahl and Wermuth (Wiley-VCH, 2002). Methods for preparing pharmaceutically acceptable salts of the compounds described herein are known to those skilled in the art.

Certain compounds of the invention may exist in unsolvated form as well as solvated forms, including hydrated forms. In general, the compounds of formula (G), the compounds of formula (G') and the compounds of formula (I), whether present in solvated form or in unsolvated form, are included within the scope of the invention.

Certain compounds of the invention may exist in different crystalline or amorphous forms, and the compounds of formula (G), the compounds of formula (G') and the compounds of Formula (I) present in any forms, are included within the scope of the invention.

To avoid ambiguity, the definitions of some terms used herein are given below. Unless otherwise stated, the meanings of the terms used herein are as follows.

The term "pharmaceutically acceptable" means that the corresponding compound, carrier or molecule is suitable for administration to humans. Preferably, the term refers to it is approved by regulatory agencies such as CFDA (China), EMEA (Europe), FDA (United States), and other national regulatory agencies to be suitable for mammals, preferably humans.

The "prodrug" refers to a derivative that is converted into a compound of the present disclosure by a reaction with enzymes, gastric acid, and the like in a living body under physiological conditions, for example, through oxidation, reduction, hydrolysis, and the like catalyzed by enzymes.

The "metabolite" refers to all molecules derived from any compound of the present disclosure in a cell or organism, preferably a human.

The term "hydroxy" refers to -OH.

The term "halogen" or "halo" refers to -F, -Cl, -Br, or -I.

The term "cyano" refers to -CN.

In the present disclosure, when there are multiple substituents of a certain type, each substituent is independently selected, and these substituents may be the same or different. For example, when there are 2, 3, or 4 $R_1$s, these $R_1$s may be the same or different. For example, when there are 2, 3, or 4 $R_2$s, these $R_2$s may be the same or different. For example, when $R_1$ and $R_2$ are both $-N(R_9)(R_{10})$, $R_9$ and $R_{10}$ contained in $R_1$ and $R_2$ can be independently selected, that is, $R_9$ in $R_1$ and $R_9$ in $R_2$ can be the same or different, and $R_{10}$ in $R_1$ and $R_{10}$ in $R_2$ may be the same or different. For example, when there are two $R_1$s, and the two $R_1$s are both $-N(R_9)(R_{10})$, $R_9$ and $R_{10}$ in the two $R_1$s can be selected independently, that is, $R_9$ in the first $R_1$ and $R_9$ in the second $R_1$ may be the same or different, and $R_{10}$ in the first $R_1$ and $R_{10}$ in the second $R_1$ may be the same or different. The above statement applies to $R_1$, $R_2$, $R_3$, $R_4$, $R_5$, $R_6$, $R_7$, $R_8$, $R_9$, $R_{10}$, $R_{11}$, $R_{12}$, $R_{13}$, $R_{14}$, $R_{15}$, $R_{16}$, $R_{17}$, and $R_{18}$.

As used herein, the term "substituted" means that one or more (preferably 1 to 5, more preferably 1 to 3) hydrogen atoms in a group are independently replaced by a corresponding number of substituents.

As used herein, the term "independently" means that when the number of substituents is more than one, these substituents may be the same or different.

As used herein, the term "optional" or "optionally" means that the event described therein may or may not occur. For example, an "optionally substituted" group means that the group may be unsubstituted or substituted.

As used herein, the term "heteroatom" as used herein refers to oxygen (O), nitrogen (N), or $S(O)_m$in which m may be 0, 1 or 2, i.e. a sulfur atom S, or a sulfoxide group SO, or a sulfonyl group $S(O)_2$).

As used herein, the term "alkyl" refers to saturated aliphatic hydrocarbons, including straight and branched chains. In some embodiments, the alkyl group has 1-8, or 1-6, or 1-3 carbon atoms. For example, the term "$C_{1-8}$ alkyl" refers to a straight or branched chain group of atoms having 1-8 carbon atoms. The term "$C_{1-8}$ alkyl" includes the terms "$C_{1-6}$ alkyl", "$C_1$-$C_3$ alkyl" and "$C_1$-$C_4$ alkyl" in its definition. Examples of alkyl groups include, but are not limited to, methyl, ethyl, propyl, isopropyl, butyl, isobutyl, sec-butyl, tert-butyl, pentyl, 2-pentyl, 3-pentyl, isopentyl, neopentyl, (R)-2-methylbutyl, (S)-2-methylbutyl, 3-methylbutyl, 2,3-dimethylpropyl, 2,3-dimethylbutyl, hexyl, and the like. The alkyl group may be optionally substituted with one or more (for example, 1 to 5) suitable substituent(s).

As used herein, the term "alkenyl" refers to an aliphatic hydrocarbon having at least one carbon-carbon double bond, including straight and branched chains having at least one carbon-carbon double bond. In some embodiments, alkenyl groups have 2-8 carbon atoms, 2-6 carbon atoms, 3-6 carbon atoms, or 2-4 carbon atoms. For example, the term "$C_{2-8}$ alkenyl" refers to a linear or branched unsaturated atomic group (having at least one carbon-carbon double bond) having 2-8 carbon atoms. The double bond may or may not be the point of attachment of another group. Alkenyl groups include, but are not limited to, vinyl, 1-propenyl, 2-propenyl, 2-methyl-2-propenyl, butenyl, pentenyl, 3-hexenyl, and the like. Alkenyl groups may be optionally substituted with one or more (for example, 1 to 5) suitable substituent(s). When the compound of formula (I) contains an alkenyl group, the alkenyl group may be present in the pure E form, the pure Z form, or any mixture thereof.

As used herein, the term "alkynyl" refers to an aliphatic hydrocarbon having at least one carbon-carbon triple bond, including straight and branched chains having at least one carbon-carbon triple bond. In some embodiments, an alkynyl group has 2-8 carbon atoms, 2-6 carbon atoms, 3-6 carbon atoms, or 2-4 carbon atoms. For example, the term "$C_{2-8}$ alkynyl" refers to a linear or branched unsaturated atomic group (having at least one carbon-carbon triple bond) having 2-8 carbon atoms. The triple bond may or may not be the point of attachment of another group. Alkynyl groups include, but are not limited to, ethynyl, 1-propynyl, 2-propynyl, 2-methyl-2-propynyl, butynyl, pentynyl, 3-hexynyl, and the like. The alkynyl group may be optionally substituted with one or more (for example, 1 to 5) suitable substituent(s).

As used herein, the term "$C_{3-7}$ cycloalkyl" refers to a cycloalkyl group having 3-7 carbon atoms forming a ring,

such as cyclopropyl, cyclobutyl, cyclopentyl, cyclohexyl, cyclohexenyl, cycloheptyl. The cycloalkyl may be optionally substituted with one or more suitable substituent(s).

As used herein, the term "n-membered heterocycloalkyl" refers to a cycloalkyl group having m ring-forming carbon atoms and (n-m) ring-forming heteroatoms, the heteroatoms being selected from O, S and N. For example, 3-7 membered heterocycloalkyl includes, but not limited to, oxetane, thietane, azetidine, tetrahydrofuran, tetrahydrothiophene, pyrrolidine, tetrahydropyran, tetrahydrothiopyran, piperidine, morpholine, piperazine, oxepane, thiepane, and azepine. The heterocycloalkyl may be optionally substituted with one or more suitable substituent(s).

As used herein, the term "$C_{5-7}$ aryl" refers to an aryl group having an aromatic ring containing 5-7 carbon atoms, preferably phenyl.

As used herein, the term "n-membered heteroaryl" refers to a heteroaryl group having m carbon atoms forming an aromatic ring and (n-m) heteroatoms forming an aromatic ring, the heteroatoms being selected from O, S and N. For example, 5-7 membered heteroaryl includes but not limited to pyrazine, pyrazole, pyrrole, furan, thiophene, thiazole, and pyridine. The heteroaryl may be optionally substituted with one or more suitable substituent(s).

As used herein, the term "$C_{7-11}$ bicyclic aryl" refers to a bicyclic aryl group having 7-11 carbon atoms, such as naphthalene, indene and the like. The bicyclic aryl may be optionally substituted with one or more suitable substituent(s).

As used herein, the term "n-membered bicyclic heteroaryl" refers to a bicyclic heteroaryl group having m carbon atoms forming an aromatic bicyclic ring and (n-m) heteroatoms forming an aromatic bicyclic ring, and the heteroatoms are selected from O, S and N. For example, 7-11 membered bicyclic heteroaryl includes, but not limited to, quinoline, isoquinoline, benzothiazole, and the like. The bicyclic heteroaryl may be optionally substituted with one or more suitable substituent(s).

As used herein, the term "11-15 membered tricyclyl" includes but not limited to acridine and the like. The 11-15 membered tricyclyl may be optionally substituted with one or more suitable substituent(s).

As used herein, the term "haloalkyl" refers to an alkyl group having one or more halogen substituent(s) (up to perhaloalkyl, that is, each hydrogen atom of the alkyl is replaced by a halogen atom). For example, the term "$C_{1-6}$ haloalkyl" refers to a $C_{1-6}$ alkyl group with one or more halogen substituent(s) (up to perhaloalkyl, that is, each hydrogen atom of the alkyl group is replaced by a halogen atom). As another example, the term "$C_{1-4}$ haloalkyl" refers to a $C_{1-4}$ alkyl group with one or more halogen substituent(s) (up to perhaloalkyl, that is, each hydrogen atom of the alkyl group is replaced by a halogen atom); the term "$C_{1-3}$ haloalkyl" refers to a $C_{1-3}$ alkyl group with one or more halogen substituent(s) (up to perhaloalkyl, that is, each hydrogen atom of the alkyl group is replaced by a halogen atom); and the term "$C_{1-2}$ haloalkyl" refers to a $C_{1-2}$ alkyl group (i.e. methyl or ethyl) with one or more halogen substituent(s) (up to perhaloalkyl, that is, each hydrogen atom of the alkyl group is replaced by a halogen atom). As another example, the term "$C_1$ haloalkyl" refers to a methyl group with 1, 2, or 3 halogen substituent(s). Examples of haloalkyl groups include: $CF_3$, $C_2F_5$, $CHF_2$, $CH_2F$, $CH_2CF_3$, $CH_2Cl$, and the like.

As used herein, the term "alkoxy" refers to alkyl with a single bond attached to an oxygen atom. The point of attachment of the alkoxy group to a molecule is through the oxygen atom. Alkoxy can be described as alkyl-O-. The term "$C_{1-6}$ alkoxy" refers to a linear or branched alkoxy group containing 1 to 6 carbon atoms. The term "$C_{1-6}$ alkoxy" includes the term "$C_{1-3}$ alkoxy" in its definition. Alkoxy includes, but not limited to, methoxy, ethoxy, propoxy, isopropoxy, butoxy, hexoxy, and the like. The alkoxy group may be optionally substituted with one or more suitable substituent(s).

Herein, a numerical range relating to the number of substituents, the number of carbon atoms, or the number of ring members represents an enumeration of all integers in the range, and the range is only a simplified representation thereof. For example:

"1-4 substituent(s)" means 1, 2, 3 or 4 substituent(s);
"1-3 substituent(s)" means a 1, 2 or 3 substituent(s);
"3 to 12-membered ring" means a 3, 4, 5, 6, 7, 8, 9, 10, 11 or 12-membered ring;
"3 to 14-membered ring" means a 3, 4, 5, 6, 7, 8, 9, 10, 11, 12, 13 or 14-membered ring;
"3 to 8 membered ring" means a 3, 4, 5, 6, 7, or 8 membered ring;
"1-12 carbon atoms" or "$C_{1-12}$" means 1 ($C_1$), 2 ($C_2$), 3 ($C_3$), 4 ($C_4$), 5 ($C_5$), 6 ($C_6$), 7 ($C_7$), 8 ($C_8$), 9 ($C_9$), 10 ($C_{10}$), 11 ($C_{11}$) or 12 ($C_{12}$) carbon atoms;
"1-6 carbon atoms" or "$C_{1-6}$" means 1 ($C_1$), 2 ($C_2$), 3 ($C_3$), 4 ($C_4$), 5 ($C_5$) or 6 ($C_6$) carbon atoms;
"1-4 carbon atoms" or "$C_{1-4}$" means 1 ($C_1$), 2 ($C_2$), 3 ($C_3$), 4 ($C_4$) carbon atoms;
"2-6 carbon atoms" or "$C_{2-6}$" means 2 ($C_2$), 3 ($C_3$), 4 ($C_4$), 5 ($C_5$) or 6 ($C_6$) carbon atoms;
"$C_{3-8}$" means 3 ($C_3$), 4 ($C_4$), 5 ($C_5$), 6 ($C_6$), 7 ($C_7$), 8 ($C_8$) carbon atoms; and
"3 to 8 ring members" means 3, 4, 5, 6, 7, or 8 ring members.

Thus, a numerical range associated with the number of substituents, the number of carbon atoms, or the number of ring members also encompasses any one of its subranges, and each subrange is also considered to be disclosed herein.

The medicament for treating severe pneumonia of the present disclosure invention may be suitable for oral,

parenteral (including subcutaneous, intramuscular, intradermal and intravenous), bronchial or nasal administration as desire.

If a solid carrier is used, the dosage may be tableted, or placed in a hard gelatin capsule in powder or pellet form, or in the form of a troche or lozenge. The solid carrier may contain conventional excipients such as binding agents, fillers, tableting lubricants, disintegrants, wetting agents and the like. The tablet may, if desired, be film coated by conventional techniques. If a liquid carrier is employed, the dosage may be in the form of a syrup, emulsion, paste, soft gelatin capsule, sterile vehicle for injection, an aqueous or non-aqueous liquid suspension, or may be a dry product for reconstitution with water or other suitable vehicle before use. Liquid preparations may contain conventional additives such as suspending agents, emulsifying agents, wetting agents, non-aqueous vehicle (including edible oils), preservatives, as well as flavoring and/or coloring agents. For parenteral administration, a vehicle normally will comprise sterile water, at least in large part, although saline solutions, glucose solutions and like may be utilized. Injectable suspensions also may be used, in which case conventional suspending agents may be employed. Conventional preservatives, buffering agents and the like also may be added to the parenteral dosage forms. The medicament are prepared by conventional techniques appropriate to the desired preparation containing appropriate amounts of the active ingredient, that is, the compound of Formula (G), the compound of Formula (G') or the compound of Formula (I) according to the invention.

Medicament dosage suitable for parenteral injection may comprise physiologically acceptable sterile aqueous or nonaqueous solutions, dispersions, suspensions or emulsions, and sterile powders for reconstitution into sterile injectable solutions or dispersions. Examples of suitable aqueous and nonaqueous carriers, diluents, solvents or vehicles include water, ethanol, polyols (propyleneglycol, polyethyleneglycol, glycerol, and the like), suitable mixtures thereof, vegetable oils (such as olive oil), and injectable organic esters such as ethyl oleate.

The medicament may also contain excipients such as preserving, wetting, emulsifying, and dispensing agents. Prevention of the action of microorganisms can be ensured by various antibacterial and antifungal agents, for example, parabens, chlorobutanol, phenol, sorbic acid, and the like. It may also be desirable to include isotonic agents, for example sugars, sodium chloride, and the like. Prolonged absorption of the injectable pharmaceutical form can be brought about by the use of agents delaying absorption, for example, aluminum monostearate and gelatin.

Solid dosage forms for oral administration include capsules, tablets, pills, powders, and granules. In such solid dosage forms, the active compound is admixed with at least one inert customary excipient (or carrier) such as sodium citrate or dicalcium phosphate or (a) fillers or extenders, as for example, starches, lactose, sucrose, glucose, mannitol, and silicic acid; (b) binders, as for example, carboxymethylcellulose, alignates, gelatin, polyvinylpyrrolidone, sucrose, and acacia; (c) humectants, as for example, glycerol; (d) disintegrating agents, as for example, agar-agar, calcium carbonate, potato or tapioca starch, alginic acid, certain complex silicates, and sodium carbonate; (e) solution retarders, as for example paraffin; (f) absorption accelerators, as for example, quaternary ammonium compounds; (g) wetting agents, as for example, cetyl alcohol and glycerol monostearate; (h) adsorbents, as for example, kaolin and bentonite; and (i) lubricants, as for example, talc, calcium stearate, magnesium stearate, solid polyethylene glycols, sodium lauryl sulfate, or mixtures thereof.

Solid compositions of a similar type may also be employed as fillers in soft and hard-filled gelatin capsules using such excipients as lactose or milk sugar as well as high molecular weight polyethyleneglycols, and the like.

Solid dosage forms such as tablets, dragees, capsules, pills, and granules can be prepared with coatings and shells, such as enteric coatings and others well-known in the art. They may contain opacifying agents, and can also be of such composition that they release the active compound or compounds in a certain part of the intestinal tract in a delayed manner. Examples of embedding compositions which can be used are polymeric substances and waxes. The active compounds can also be in micro-encapsulated form, if appropriate, with one or more of the above-mentioned excipients.

Liquid dosage forms for oral administration include pharmaceutically acceptable emulsions, solutions, suspensions, syrups, and elixirs. In addition to the active compounds, the liquid dosage forms may contain inert diluents commonly used in the art, such as water or other solvents, solubilizing agents and emulsifiers, as for example, ethyl alcohol, isopropyl alcohol, ethyl carbonate, ethyl acetate, benzyl alcohol, benzyl benzoate, propyleneglycol, 1,3-butyleneglycol, dimethylformamide, oils, in particular, cottonseed oil, groundnut oil, corn germ oil, olive oil, castor oil and sesame oil, glycerol, tetrahydrofurfuryl alcohol, polyethyleneglycols and fatty acid esters of sorbitan or mixtures of these substances, and the like.

Besides such inert diluents, the composition can also include adjuvants, such as wetting agents, emulsifying and suspending agents, sweetening, flavoring, and perfuming agents.

Suspensions, in addition to the active compounds, may contain suspending agents, as for example, ethoxylatedisostearyl alcohols, polyoxyethylene sorbitol and sorbitan esters, microcrystalline cellulose, aluminum metahydroxide, bentonite, agar-agar and tragacanth, or mixtures of these substances, and the like.

Dosage forms for topical administration of a compound of the invention include paste, powders, sprays, and inhalants. The active component is admixed under sterile conditions with a physiologically acceptable carrier and any preservatives, buffers, or propellants as may be required.

Sprays may contain excipients such as lactose, talc, silicic acid, aluminum hydroxide, calcium silicate, and polyamide powders, or mixtures of these substances. The sprays may additionally contain conventional propellants, such as chlorofluorocarbons and volatile unsubstituted hydrocarbons, such as butane and propane. Inhalants may contain excipients such as lactoses, or may be aqueous solutions containing such as polyethylene oxide)-9-lauryl ether, glycocholate and deoxycholate, or oil-based solutions administered as nasal drops or sprays, or as gels.

The external dosage form of the compound of the present disclosure may be in the form of a water-in-oil (W/O) or oil-in-water (O/W) emulsion, a multi-emulsion form, such as a water-in-oil-in-water (W/O/W) form or an oil-in-water-oil (O/W/O) emulsion, or in the form of water dispersion or lipid dispersion, gel or aerosol.

The amount of the compound of formula (G), the compound of formula (G') or the compound of formula (I) in medicament, the pharmaceutical composition and dosage form can be appropriately determined by those skilled in the art as needed. For example, the compound of formula (G), the compound of formula (G') or the compound of formula (I) can be present in the medicament, pharmaceutical composition or dosage form in a therapeutically effective amount.

The term "severe pneumonia" as used herein has the usual meaning in the art. For example, severe pneumonia is pneumonia that is considered to be severe pneumonia according to the guidelines issued by the Chinese Medical Association's Branch of Respiratory Diseases or the guidelines issued by the American thoracic society (ATS) or the British Thoracic Society (BTS). For example, pneumonia that meets one of the following primary criteria or at least three secondary criteria is considered to be severe pneumonia, the primary criteria being (1) the need for mechanical ventilation with tracheal intubation, and (2) septic shock requiring vasoactive drug therapy despite aggressive fluid resuscitation, and the secondary criteria being (1) respiratory rate greater than or equal to 30 breaths/min, (2) oxygenation index less than or equal to 250 mmHg, (3) multiple lobe infiltrates, (4) impaired consciousness and/or disorientation, (5) blood urea nitrogen greater than or equal to 7.14 mmol/L, and (6) systolic blood pressure less than 90 mmHg.

In some embodiments, the "severe pneumonia" described in the present disclosure is a community-acquired severe pneumonia. In some embodiments, said "severe pneumonia" is an adult community-acquired severe pneumonia. In some embodiments, said "severe pneumonia" is a children community-acquired severe pneumonia.

Patients with severe pneumonia can be identified by methods known in the art. These methods include, but are not limited to, detection of pathogenic organisms in blood or other routinely sterile body fluids or tissue cultures by, for example, Gram staining, culture, histochemical staining, immunochemical testing, or nucleic acid testing. Patients with severe pneumonia may also be identified by methods consistent with any clinical signs, such as chest radiography.

Dysregulated and excessive immune responses are commonly present in patients with severe pneumonia, and result in production of large amounts of pro-inflammatory cytokines which may trigger a cytokine storm and lead to acute lung injury (ALI).

In some embodiments, the compounds or drugs of the present disclosure can be used to inhibit cytokine storm in severe pneumonia. A cytokine storm is a phenomenon or condition in which the body's immune system produces excessive inflammatory signals. Said inflammatory signals are, for example, pro-inflammatory cytokines, such as IL-1$\beta$, IL-6, TNF-$\alpha$ and/or IFN-$\gamma$, among others. In some embodiments, the compounds or drugs of the present disclosure can be used to inhibit pro-inflammatory cytokines, such as IL-1 $\beta$ , IL-6, TNF-$\alpha$, and/or IFN-$\gamma$.

The severe pneumonia may be caused by viruses, bacteria, pathogens such as mycoplasma, and the like. In some embodiments, said severe pneumonia is caused by viruses. In some embodiments, said viruses are selected from: severe acute respiratory syndrome coronaviruses (SARS-CoV), severe acute respiratory syndrome coronavirusus-2 (SARS-CoV-2), influenza viruses, or Middle East respiratory syndrome coronaviruses (MERS-CoV).

The present disclosure further provides a method for treating severe pneumonia, the method comprising administrating a therapeutically effective amount of the compound as described above or an isotopically labeled compound thereof, or an optical isomer thereof, a geometric isomer thereof, a tautomer thereof or a mixture of various isomers, or a pharmaceutically acceptable salt thereof, or a prodrug thereof, or a metabolite thereof, or the composition as described above to patients in need. Among them, the patient is preferably a mammal, and more preferably a human patient. The route of administration can be oral, topical (including but not limited to external application, spraying, and the like), parenteral (including subcutaneous, intramuscular, cortical, and intravenous) administration, bronchial administration, or nasal administration. Among them, it is preferably administered orally or topically. It is more preferably administered orally.

Unexpectedly, the compounds of the present disclosure demonstrated in experiments excellent efficacy as a JAK kinase inhibitor that is superior to existing JAK kinase inhibitors, such as Filgotinib, and excellent inhibition of pro-inflammatory cytokines, and showed good safety potentially.

Preferably, the present disclosure provides the following embodiments:

1. Use of a compound of Formula (G),

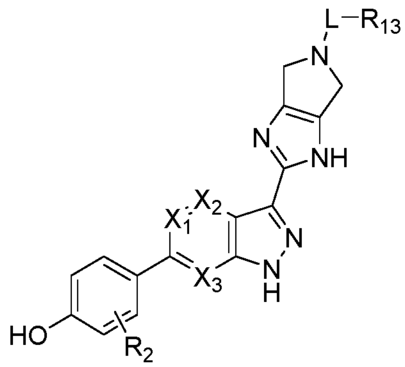

(G)

or an isotopically labeled compound thereof, or an optical isomer thereof, a geometric isomer thereof, a tautomer thereof or a mixture of various isomers, or a pharmaceutically acceptable salt thereof, or a prodrug thereof, or a metabolite thereof, in the manufacture of a medicament for treating severe pneumonia,
in which

L is C=O, O=S=O, $CH_2$ or a covalent bond; and
$X_1$ is N or $CR_{14}$; and
$X_2$ is N or $CR_{15}$; and
$X_3$ is N or $CR_{16}$; and
$R_{14}$, $R_{15}$, $R_{16}$ are each independently selected from H, -OH, -SH, -CN, halogen, $-NO_2$, $-SF_5$,-S-$C_{1-4}$ alkyl, $C_{1-6}$ alkyl, $C_{2-8}$ alkenyl, $C_{2-8}$ alkynyl, $C_{1-6}$ alkoxy, $C_{3-7}$ cycloalkyl, 3-7 membered heterocycloalkyl, $C_{5-7}$ aryl, 5-7 membered heteroaryl, $-N(R_9)(R_{10})$, $-N(R_{11})(C(=O)R_{12})$,$-C(=O)-N(R_9)(R_{10})$, $-C(=O)-R_{12}$, $-C(=O)-OR_{12}$, $-OC(=O)R_{12}$, $-N(R_{11})(S(=O)_2R_{12})$, $-S(= O)_2-N(R_9)(R_{10})$, $-SR_{12}$ and $-OR_{12}$, in which the -S-$C_{1-4}$ alkyl, $C_{1-6}$ alkyl, $C_{1-6}$ alkoxy, $C_{3-7}$ cycloalkyl, and 3-7 membered heterocycloalkyl are optionally substituted with 1, 2 or 3 substitutes selected from halogen, -OH, $-NH_2$, $-NH(CH_3)$, $-N(CH_3)_2$, -CN, $C_{1-4}$ alkyl, $C_{3-7}$ cycloalkyl, $C_{1-4}$ hydroxyalkyl, -S-$C_{1-4}$ alkyl, -C(=O)H, -C(=O)-$C_{1-4}$ alkyl, -C(=O)-O-$C_{1-4}$ alkyl, $-C(=O)-NH_2$, -C(=O)-N($C_{1-4}$ alkyl)$_2$, -N($C_{1-4}$ alkyl)(C(=O) $C_{1-4}$ alkyl), $C_{1-4}$ haloalkyl, $C_{1-4}$ alkoxy and $C_{1-4}$ haloalkoxy; and
$R_{13}$ is H, $-N(R_{17})(R_{18})$, $C_{1-6}$ alkoxy, $-SR_{12}$, $-OR_{12}$, -CN, halogen, $-NO_2$, $-SF_5$, -S-$C_{1-4}$ alkyl, $C_{1-6}$ alkyl or $C_{3-7}$ cycloalkyl, 3-7 membered heterocycloalkyl, $C_{5-7}$ aryl, 5-7 membered heteroaryl, $C_{7-11}$ bicyclic aryl, 7-11 membered bicyclic heteroaryl, 11-15 membered tricyclyl, $C_{5-11}$bicycloalkyl, or 5-11 membered bicyclic heteroalkyl, and $R_{13}$ is substituted with 0, 1, 2, 3 or 4 $R_1$(s), in which $R_{17}$, and $R_{18}$ are each independently selected from H, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, $C_{3-7}$ heterocycloalkyl, $C_{5-7}$ aryl, 5-7 membered heteroaryl, $C_{7-11}$ bicyclic aryl, 7-11 membered bicyclic heteroaryl, 11-15membered tricyclyl, Cs-nbicycloalkyl, and 5-11 membered bicyclic heteroalkyl and are optionally substituted with one or more substitutes each independently selected from -OH,-CN, -SH, halogen, $-NO_2$, $-SF_5$, -S-$C_{1-4}$ alkyl, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 4-10 membered heterocycloalkyl, $C_{5-7}$ aryl, 5-7 membered heteroaryl, $C_{7-11}$ bicyclic aryl, 7-11 membered bicyclic heteroaryl, $-N(R_9)(R_{10})$,$-N(R_{11})(C(=O)R_{12})$, $-C(=O)-N(R_9)(R_{10})$, $-C(=O)-R_{12}$, $-C(=O)-OR_{12}$, $-OC(=O)R_{12}$,$-N(R_{11})(S(=O)_2R_{12})$, $-S(=O)_2-N(R_9)(R_{10})$, $-SR_{12}$ and $-OR_{12}$, wherein the -S-$C_{1-4}$ alkyl, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 4-10 membered heterocycloalkyl, $C_{5-7}$ aryl, 5-7 membered heteroaryl, $C_{7-11}$ bicyclic aryl, and 7-11 membered bicyclic heteroaryl are optionally substituted with 1, 2 or 3 substitutes each independently selected from halogen, -CN, -OH, $C_{1-4}$ alkyl, $C_{1-4}$ alkoxy, $C_{1-4}$ haloalkyl, $C_{1-4}$ haloalkoxy, $C_{3-6}$ cycloalkyl, $-N(R_9)(R_{10})$, $-N(R_{11})(C(=O)R_{12})$, $-C(=O)-OR_{12}$, -C(=O)H, $-C(=O)R_{12}$, $-C(= O)-N(R_9)(R_{10})$, $-N(R_{11})(S(=O)_2R_{12})$, $-S(=O)_2-N(R_9)(R_{10})$, $-SR_{12}$ and $-OR_{12}$; or $R_{17}$, $R_{18}$ and the N atom connected thereto together form a 3-14 membered ring; and
0, 1, 2, 3 or 4 $R_2$(s) are present in formula (G), and $R_2$ is selected from H, halogen, -OH, $-NO_2$, -CN, $-SF_5$, -SH, -S-$C_{1-4}$ alkyl, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 4-10 membered heterocycloalkyl, $C_{5-7}$ aryl, 5-7 membered heteroaryl, $C_{7-11}$ bicyclic aryl, 7-11 membered bicyclic heteroaryl, $-N(R_9)(R_{10})$, $-N(R_{11})(C(=O)R_{12})$,$-C(=O)-N(R_9)(R_{10})$, $-C(=O)-R_{12}$, $-C(=O)-OR_{12}$, $-OC(=O)R_{12}$, $-N(R_{11})(S(=O)_2R_{12})$, $-S(= O)_2-N(R_9)(R_{10})$, $-SR_{12}$ and $-OR_{12}$, in which the -S-$C_{1-4}$ alkyl, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 4-10 membered heterocycloalkyl, $C_{5-7}$ aryl, 5-7 membered heteroaryl, $C_{7-11}$ bicyclic aryl, and 7-11 membered bicyclic heteroaryl are each optionally substituted with 1, 2 or 3 substituent(s) each independently selected from the group consisting of halogen, -CN, -OH, $C_{1-4}$ alkyl, $C_{1-4}$ alkoxy, $C_{1-4}$ haloalkyl, $C_{1-4}$ haloalkoxy, $C_{3-6}$ cycloalkyl, $-N(R_9)(R_{10})$, $-N(R_{11})(C(=O)R_{12})$, $-C(=O)-OR_{12}$, -C(=O)H, $-C(=O)R_{12}$,$-C(=O)-N(R_9)(R_{10})$, $-N(R_{11})(S(=O)_2R_{12})$, $-S(=O)_2-N(R_9)(R_{10})$, $-SR_{12}$ and $-OR_{12}$; and

$R_1$ is selected from H, halogen, -OH, $-NO_2$, -CN, $-SF_5$, -SH, $-S-C_{1-4}$ alkyl, $C_{1-8}$ alkyl, $C_{2-8}$ alkenyl, $C_{2-8}$ alkynyl, $C_{1-8}$ alkoxy, $C_{3-7}$ cycloalkyl, 3-10 membered heterocycloalkyl, $C_{5-7}$ aryl, 5-7 membered heteroaryl, $C_{7-11}$ bicyclic aryl, 7-11 membered bicyclic heteroaryl, 11-15membered tricyclyl, Cs-nbicycloalkyl, 5-11 membered bicyclic heteroalkyl, $-N(R_9)(R_{10})$,$-N(R_{11})(C(=O)R_{12})$, $-C(=O)-N(R_9)(R_{10})$, $-C(=O)-R_{12}$, $-C(=O)-OR_{12}$, $-OC(=O)R_{12}$,$-N(R_{11})(S(=O)_2R_{12})$, $-S(=O)_2-N(R_9)(R_{10})$, $-SR_{12}$ and $-OR_{12}$, in which the $-S-C_{1-4}$ alkyl, $C_{1-8}$ alkyl, $C_{2-8}$ alkenyl, $C_{2-8}$ alkynyl, and $C_{1-8}$ alkoxy are optionally substituted with 1, 2, 3, or 4 $R_3$(s), and in which the $C_{3-7}$ cycloalkyl, 3-10 membered heterocycloalkyl, $C_{5-7}$ aryl, 5-7 membered heteroaryl, $C_{7-11}$ bicyclic aryl, and 7-11 membered bicyclic heteroaryl are optionally substituted with 1, 2, 3, or 4 $R_4$(s); and

$R_3$ and $R_4$ are each independently selected from H, halogen, -OH, $-NO_2$, -CN, $-SF_5$, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 3-10 membered heterocycloalkyl, $C_{5-7}$ aryl, 5-7 membered heteroaryl, $C_{7-11}$ bicyclic aryl, 7-11 membered bicyclic heteroaryl, $-N(R_5)(R_6)$, $-N(R_{11})(C(=O)R_{12})$, $-CON(R_7)(R_8)$, $-C(=O)-R_{12}$, $-C(=O)-OR_{12}$, $-OC(=O)R_{12}$, $-N(R_{11})(S(=O)_2R_{12})$, $-S(=O)_2-N(R_9)(R_{10})$, $-SR_{12}$ and $-OR_{12}$, in which the $C_{1-6}$ alkyl, $C_{3-7}$ cycloalkyl, 3-10 membered heterocycloalkyl, $C_{5-7}$ aryl, 5-7 membered heteroaryl, $C_{7-11}$ bicyclic aryl, and 7-11 membered bicyclic heteroaryl are each optionally substituted with 1, 2, 3 or 4 substituent(s) each independently selected from the group consisting of halogen, -CN, -OH, $C_{1-4}$ alkyl, $C_{1-6}$ alkoxy, $C_{1-4}$ haloalkyl, $C_{1-4}$ haloalkoxy, $C_{3-6}$ cycloalkyl, $-N(R_9)(R_{10})$, $-N(R_{11})(C(=O)R_{12})$, $-C(=O)-OR_{12}$, -C(=O)H, $-C(=O)R_{12}$, $-C(= O)-N(R_9)(R_{10})$, $-N(R_{11})(S(=O)_2R_{12})$, $-S(=O)_2-N(R_9)(R_{10})$, $-SR_{12}$ and $-OR_{12}$; and

$R_5$, $R_6$, $R_7$, $R_8$, $R_9$, $R_{10}$, $R_{11}$, and $R_{12}$ are each independently H or selected from the group consisting of $C_{1-6}$ alkyl, $C_{1-4}$ haloalkyl, $C_{3-7}$ cycloalkyl, 4-14 membered heterocycloalkyl, $C_{6-10}$ aryl, 5-10 membered heteroaryl, ($C_{3-7}$ cycloalkyl)-$C_{1-4}$ alkyl-, (4-10 membered heterocycloalkyl)-$C_{1-4}$ alkyl-, ($C_{6-10}$ aryl)-$C_{1-4}$ alkyl- and (5-10 membered heteroaryl)-$C_{1-4}$ alkyl-, wherein the substituents included in the above group are each optionally substituted with 1, 2, 3 or 4 substituent(s) each independently selected from the group consisting of halogen, $-CF_3$, -OH, $-NH_2$, $-NH(CH_3)$, $-N(CH_3)_2$, -CN, oxo, $C_{1-4}$ alkyl, $C_{2-6}$ alkenyl, $C_{2-6}$ alkynyl, $C_{3-7}$ cycloalkyl, $C_{1-4}$ hydroxyalkyl, $-S-C_{1-4}$ alkyl, -C(=O)H, $-C(=O)-C_{1-4}$ alkyl,$-C(=O)-O-C_{1-4}$ alkyl, $-C(=O)-NH_2$, $-C(=O)-N(C_{1-4}\ alkyl)_2$, $C_{1-4}$ haloalkyl, $C_{1-4}$ alkoxy and $C_{1-4}$ haloalkoxy.

2. The use according to embodiment 1, wherein said drug is used to inhibit a cytokine storm in severe pneumonia.
3. The use according to embodiment 1 or 2, wherein said severe pneumonia is caused by viruses.
4. The use according to embodiment 3, wherein said viruses are severe acute respiratory syndrome coronaviruses (SARS-CoV), severe acute respiratory syndrome coronavirusus-2 (SARS-CoV-2), influenza viruses, or Middle East respiratory syndrome coronaviruses (MERS-CoV).
5. The use according to embodiment 1, wherein an isotopically labeled compound of the compound of formula (G) is used to manufacture a medicament for treating severe pneumonia, wherein all Hs are each independently optionally substituted with D.
6. The use according to embodiment 1, wherein $X_1$ is N.
7. The use according to embodiment 1, wherein $X_2$ is N.
8. The use according to embodiment 1, wherein $X_3$ is N.
9. The use according to embodiment 1, wherein $X_1$ is $CR_{14}$, $X_2$ is N or $CR_{15}$, and $X_3$ is $CR_{16}$.
10. The use according to embodiment 1, wherein $X_1$, $X_2$ and $X_3$ are the same.
11. The use according to embodiment 2, wherein $X_1$, $X_2$ and $X_3$ are the same.
12. The use according to embodiment 7, wherein $X_1$, $X_2$ and $X_3$ are CH.
13. The use according to embodiment 8, wherein $X_1$, $X_2$ and $X_3$ are CH.
14. The use according to any one of embodiments 1 to 10, wherein L is C=O, O=S=O or $CH_2$.
15. The use according to any one of embodiments 1 to 10, wherein $R_{13}$ is H,$-N(R_{17})(R_{18})$, $C_{1-6}$ alkoxy, -OH, -SH, -CN, halogen, $-NO_2$, $-SF_5$, $-S-C_{1-4}$ alkyl, $C_{1-6}$ alkyl, or $C_{3-7}$ cycloalkyl, 3-7 membered heterocycloalkyl, $C_{5-7}$ aryl, 5-7 membered heteroaryl, $C_{7-11}$ bicyclic aryl, 7-11 membered bicyclic heteroaryl, 11-15 membered tricyclyl, Cs-nbicycloalkyl, or 5-11 membered bicyclic heteroalkyl, and $R_{13}$ is substituted with 0, 1, 2, 3 or 4 $R_1$(s), in which $R_{17}$ and $R_{18}$ are each independently selected from H, $C_{1-6}$ alkyl, $C_{1-6}$ alkoxy, $C_{3-7}$ cycloalkyl, $C_{3-7}$ heterocycloalkyl, $C_{5-7}$ aryl, and 5-7 membered heteroaryl, and are optionally substituted with one or more of -OH, -CN, -SH, halogen, $-NO_2$,-and $SF_5$.
16. The use according to any one of embodiments 1 to 10, wherein $R_{13}$ is H,$-N(R_{17})(R_{18})$, $C_{1-6}$ alkoxy, -OH, -SH, -CN, halogen, $-NO_2$, $-SF_5$, $-S-C_{1-4}$ alkyl, $C_{1-6}$ alkyl, $C_{3-7}$ cycloalkyl, 3-7 membered heterocycloalkyl, $C_{5-7}$ aryl, 5-7 membered heteroaryl, $C_{7-11}$ bicyclic aryl, 7-11 membered bicyclic heteroaryl, or 11-15 membered tricyclyl, and $R_{13}$ is substituted with 0, 1, 2, 3 or 4 $R_1$(s).
17. The use according to any one of embodiments 1 to 10, wherein $R_{13}$ is H,$-N(R_{17})(R_{18})$, $C_{1-6}$ alkoxy, $C_{1-6}$ alkyl, $C_{3-7}$ cycloalkyl, 3-7 membered heterocycloalkyl, $C_{5-7}$ aryl, or 5-7 membered heteroaryl, and $R_{13}$ is substituted with 0, 1, 2, 3, or 4 $R_1$(s).
18. The use according to any one of embodiments 1 to 10, wherein $R_{13}$ is$-N(R_{17})(R_{18})$, $C_{1-6}$ alkoxy, $C_{1-6}$ alkyl, $C_{3-7}$

cycloalkyl, 4-6 membered heterocycloalkyl, phenyl, or 5-6 membered heteroaryl, and $R_{13}$ is substituted with 0, 1, 2, or 3 $R_1$(s).

19. The use according to any one of embodiments 1 to 10, wherein $R_{17}$ and $R_{18}$ are each independently selected from H, $C_{1-6}$ alkyl, $C_{3-7}$ cycloalkyl, and $C_{3-7}$ heterocycloalkyl, and are optionally substituted with one or more of -OH, -CN, -SH, halogen, $-NO_2$,-and $SF_5$.

20. The use according to any one of embodiments 1 to 10, wherein $R_{17}$, $R_{18}$ and the N atom connected thereto together form a 4-10 membered ring.

21. The use according to any one of embodiments 1 to 10, wherein L is C=O, and $R_{13}$ is $-N(R_{17})(R_{18})$, $C_{1-6}$ alkoxy, -OH, -SH, -CN, halogen, $-NO_2$, $-SF_5$, or $-S-C_{1-4}$ alkyl, and $R_{13}$ is substituted with 0, 1, 2, 3 or 4 $R_1$(s) in which $R_{17}$ and $R_{18}$ are each independently selected from H, $C_{1-6}$ alkyl, $C_{1-6}$ alkoxy, $C_{3-7}$ cycloalkyl, $C_{3-7}$ heterocycloalkyl, $C_{5-7}$ aryl, and 5-7 membered heteroaryl, and are optionally substituted with one or more of -OH, -CN, -SH, halogen, $-NO_2$,-and $SF_5$, or $R_{17}$, $R_{18}$ and the N atom connected thereto together form a 4-10 membered ring.

22. The use according to any one of embodiments 1 to 10, wherein 1, 2 or 3 $R_2$(s) are present and $R_2$ is selected from H, halogen, -OH, $-NO_2$, -CN, $-SF_5$, -SH, $-S-C_{1-4}$ alkyl, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, and 4-10 membered heterocycloalkyl, in which the $-S-C_{1-4}$ alkyl, $C_{1-6}$ alkyl, $C_{3-7}$ cycloalkyl, and 4-10 membered heterocycloalkyl are each optionally substituted with 1, 2 or 3 substituent(s) each independently selected from the group consisting of halogen, -OH, $-NH_2$, $-NH(CH_3)$,$-N(CH_3)_2$, -CN, $C_{1-4}$ alkyl, $C_{1-4}$ haloalkyl, $C_{1-4}$ alkoxy, and $C_{1-4}$ haloalkoxy.

23. The use according to any one of embodiments 1 to 10, wherein 1, 2 or 3 $R_2$(s) are present, and $R_2$ is selected from halogen, $C_{1-6}$ alkyl, and $C_{3-6}$ cycloalkyl in which the $C_{1-6}$ alkyl and $C_{3-6}$ cycloalkyl are each optionally substituted with 1, 2 or 3 substituent(s) each independently selected from the group consisting of halogen, -OH, $-NH_2$, $-NH(CH_3)$,$-N(CH_3)_2$, -CN, $C_{1-4}$ alkyl, $C_{1-4}$ haloalkyl, $C_{1-4}$ alkoxy, and $C_{1-4}$ haloalkoxy.

24. The use according to embodiment 15, wherein 1 or 2 $R_2$(s) are present, and $R_2$ is selected from halogen, and $C_{1-6}$ alkyl.

25. The use according to any one of embodiments 1 to 10, wherein $R_{13}$ is substituted with 0 or 1 $R_1$, and $R_1$ is selected from halogen, -OH, $C_{1-6}$ alkyl, 5-7 membered heterocycloalkyl, and $C_{3-7}$ cycloalkyl, in which the $C_{1-6}$ alkyl is optionally substituted with 1, 2, or 3 $R_3$(s) and in which the 5-7 membered heterocycloalkyl, and $C_{3-7}$ cycloalkyl is optionally substituted with 1, 2, 3 or 4 $C_{1-3}$ alkyl.

26. The use according to any one of embodiments 1 to 10, wherein the compound is selected from a group consisting of:

(2-(6-(2-ethyl-5-fluoro-4-hydroxyphenyl)-1H-indazol-3-yl)-4,6-dihydropyrrolo[3,4-d]imidazol-5(1H)-yl)(5-(piperidin-1-yl)pyrazin-2-yl)ketone;
(2-(6-(2-ethyl-5-fluoro-4-hydroxyphenyl)-1H-indazol-3-yl)-4,6-dihydropyrrolo[3,4-d]imidazol-5(1H)-yl)(5-morpholinylpyrazin-2-yl)ketone;
(2-(6-(2-ethyl-5-fluoro-4-hydroxyphenyl)-1H-indazol-3-yl)-4,6-dihydropyrrolo[3,4-d]imidazol-5(1H)-yl)(1-methyl-1H-pyrazol-4-yl)ketone;
(2-(6-(2-ethyl-5-fluoro-4-hydroxyphenyl)-1H-indazol-3-yl)pyrrolo[3,4-d]imidazol-5(1H, 4H,6H)-yl)(1-methylpiperidin-4-yl)ketone;
(2-(6-(2-ethyl-5-fluoro-4-hydroxyphenyl)-1H-indazol-3-yl)pyrrolo[3,4-d]imidazol-5(1H, 4H,6H)-yl)(5-(4-methylpiperzin-1-yl)pyrazin-2-yl)ketone;
(2-(6-(2-ethyl-4-hydroxyphenyl)-1H-indazol-3-yl)pyrrolo[3,4-d]imidazol-5(1H,4H,6H)-yl)(5-(4-methylpiperzin-1-yl)pyrazin-2-yl)ketone;
5-ethyl-2-fluoro-4-(3-(5-(benzenesulfonyl)-1,4,5,6-tetrahydropyrrolo[3,4-d]imidazol-2-yl)-1H-indazol-6-yl)phenol;
5-ethyl-2-fluoro-4-(3-(5-(pyrazin-2ylmethyl)-1,4,5,6-tetrahydropyrrolo[3,4-d]imidazol-2-yl)-1H-indazol-6-yl)phenol;
4-(3-(5-(cyclopropylmethyl)-1,4,5,6-tetrahydropyrrolo[3,4-d]imidazol-2-yl)-1H-indazol-6-yl)-5-ethyl-2-fluorophenol;
Cyclopropyl(2-(6-(2-ethyl-5-fluoro-4-hydroxyphenyl)-1H-indazol-3-yl)pyrrolo[3,4-d]imidazol-5(1H,4H,6H)-yl)ketone;
4-(3-(5-(cyclobutylmethyl)-1,4,5,6-tetrahydropyrrolo[3,4-d]imidazol-2-yl)-1H-indazol-6-yl)-5-ethyl-2-fluorophenol;
Cyclobutyl(2-(6-(2-ethyl-5-fluoro-4-hydroxyphenyl)-1H-indazol-3-yl)pyrrolo[3,4-d]imidazol-5(1H,4H,6H)-yl)ketone;
(2-(6-(2-ethyl-5-fluoro-4-hydroxyphenyl)-1H-indazol-3-yl)pyrrolo[3,4-d]imidazol-5(1H,4H,6H)-yl)(3-hydroxylcyclobutyl)ketone;
(2-(6-(2-ethyl-5-fluoro-4-hydroxyphenyl)-1H-indazol-3-yl)-pyrrolo[3,4-d]imidazol-5-(1H,4H,6H)-yl)(pyridazin-4-yl)ketone;
(2-(6-(2-ethyl-5-fluoro-4-hydroxyphenyl)-1H-indazol-3-yl)-pyrrolo[3,4-d]imidazol-5-(1H,4H,6H)-yl)(pyridazin-3-yl)ketone;
(S)-(2-(6-(2-ethyl-5-fluoro-4-hydroxyphenyl)-1H-indazol-3-yl)-4,6-dihydropyrrolo[3,4-d]imidazol-5-(1H)-yl)(3-hydroxylpyrrolidin-1-yl)ketone;
5-ethyl-2-fluoro-4-(3-(5-(4-hydroxylcyclohexyl)-1,4,5,6-tetrahydropyrrolo[3,4-d]imidazol-2-yl)-1H-indazol-6-yl)phenol;
4-(3-(5-(cyclopropanesulfonyl)-1,4,5,6-tetrahydropyrrolo[3,4-d]imidazol-2-yl)-1H-indazol-6-yl)-5-ethyl-2-fluorophenol;
4-(3-(5-(cyclobutylsulfonyl)-1,4,5,6-tetrahydropyrrolo[3,4-d]imidazol-2-yl)-1H-indazol-6-yl)-5-ethyl-2-fluorophenol;
4-(3-(5-(cyclopentylsulfonyl)-1,4, 5, 6-tetrahydropyrrolo[3,4-d]imidazol-2-yl)-1H-indazol-6-yl)-5-ethyl-2-fluorophenol;
5-ethyl-2-fluoro-4-(3-(5-((1-methyl-1H-pyrazol-4-yl)methyl)-1,4,5,6-tetrahydropyrrolo[3,4-d]imidazol-2-yl)-1H-indazol-6-yl)phenol;
4-(3-(5-(cyclopentyl-1,4, 5, 6-tetrahydropyrrolo[3,4-d]imidazol-2-yl)-1H-indazol-6-yl)-5-ethyl-2-fluorophenol;
5-ethyl-2-fluoro-4-(3 -(5-(tetrahydro-2H-pyran-4-yl)-1,4, 5, 6-tetrahydropyrrolo[3,4-d]imidazol-2-yl)-1H-indazol-6-yl)phenol;
1-(2-(6-(2-ethyl-5-fluoro-4-hydroxyphenyl)-1H-indazol-3-yl)-4,6-dihydropyrrolo[3,4-d]imidazol-5-(1H)-yl)ethan-1-one;
1-(2-(6-(2-ethyl-5-fluoro-4-hydroxyphenyl)-1H-indazol-3-yl)-4,6-dihydropyrrolo[3,4-d]imidazol-5-(1H)-yl)propan-1-one;
1-(2-(6-(2-ethyl-5-fluoro-4-hydroxyphenyl)-1H-indazol-3-yl)-4,6-dihydropyrrolo[3,4-d]imidazol-5-(1H)-yl)-2-methylpropan-1-one;
2-cyclopropyl-1-(2-(6-(2-ethyl-5-fluoro-4-hydroxyphenyl)-1H-indazol-3-yl)-4,6-dihydropyrrolo[3,4-d]imidazol-5-(1H)-yl)ethan-1-one;
1-(2-(6-(2-ethyl-5-fluoro-4-hydroxyphenyl)-1H-indazol-3-yl)-4,6-dihydropyrrolo[3,4-d]imidazol-5-(1H)-yl)-3-methylbutan-1-one;
(2-(6-(2-ethyl-5-fluoro-4-hydroxyphenyl)-1H-indazol-3-yl)-4,6-dihydropyrrolo[3,4-d]imidazol-5-(1H)-yl)(pyrrolidin-1-yl)ketone;
(2-(6-(2-ethyl-5-fluoro-4-hydroxyphenyl)-1H-indazol-3-yl)-4,6-dihydropyrrolo[3,4-d]imidazol-5-(1H)-yl)(piperidin-1-yl)ketone;
(2-(6-(2-ethyl-5-fluoro-4-hydroxyphenyl)-1H-indazol-3-yl)-4,6-dihydropyrrolo[3,4-d]imidazol-5-(1H)-yl)(morpholino)ketone;
(2-(6-(2-ethyl-5-fluoro-4-hydroxyphenyl)-1H-indazol-3-yl)-4,6-dihydropyrrolo[3,4-d]imidazol-5-(1H)-yl)(4-methylpiperzin-1-yl)ketone;
(2-(6-(2-ethyl-5-fluoro-4-hydroxyphenyl)-1H-indazol-3-yl)-4,6-dihydropyrrolo[3,4-d]imidazol-5-(1H)-yl)(4-ethylpiperzin-1-yl)ketone;
Cyclopropyl(2-(6-(2-ethyl-5-fluoro-4-hydroxyphenyl)-1H-pyrazolo [4,3-b]pyridin-3-yl)-4,6-dihydropyrrolo[3,4-d]imidazol-5(1H)-yl)ketone;
Cyclopropyl(2-(6-(2-ethyl-5-fluoro-4-hydroxyphenyl)-4-methyl-1H-indazol-3-yl)-4,6-dihydropyrrolo[3,4-d]imidazol-5(1H)-yl)ketone;
(S)-(2-(6-(2-ethyl-5-fluoro-4-hydroxyphenyl)-4-methyl-1H-indazol-3-yl)-4,6-dihydropyrrolo[3,4-d]imidazol -5(1H)-yl)(3-hydroxylpyrrolidin-1-yl)ketone;
Cyclopropyl(2-(6-(2-ethyl-5-fluoro-4-hydroxyphenyl)-1H-pyrazolo[4,3-c]pyridin-3-yl)-4,6-dihydropyrrolo[3,4-d]imidazol-5(1H)-yl)ketone;
(R)-(2-(6-(2-ethyl-5-fluoro-4-hydroxyphenyl)-1H-indazol-3-yl)-4,6-dihydropyrrolo[3,4-d]imidazol-5(1H )-yl)(3-hydroxylpyrrolidin-1-yl)ketone;
(2-(6-(2-ethyl-5-fluoro-4-hydroxyphenyl)-1H-indazol-3-yl)-4,6-dihydropyrrolo[3,4-d]imidazol-5(1H)-yl)(4-hydroxylpiperidin-1-yl)ketone;
2-(6-(2-ethyl-5-fluoro-4-hydroxyphenyl)-1H-indazol-3-yl)-N-methyl-4,6-dihydropyrrolo[3,4-d]imidazol-5-(1H)-carboxamide;
2-(6-(2-ethyl-5-fluoro-4-hydroxyphenyl)-1H-indazol-3-yl)-N-ethyl-4,6-dihydropyrrolo[3,4-d]imidazol-5-(1H)-carboxamide;
2-(6-(2-ethyl-5-fluoro-4-hydroxyphenyl)-1H-indazol-3-yl)-N-(2-hydroxylethyl)-4,6-dihydropyrrolo[3,4-d]imidazol-5(1H)-carboxamide;
1-(2-(6-(2-ethyl-5-fluoro-4-hydroxyphenyl)-1H-indazol-3 -yl)-1,4, 5, 6-tetrahydropyrrolo[3,4-d]imidazol-5 -carbonyl)pyrrolidin-3-nitrile;

2-(6-(2-ethyl-5-fluoro-4-hydroxyphenyl)-1H-indazol-3-yl)-N-(tetrahydrofuran-3-yl)-4,6-dihydropyrrolo[3,4-d]imidazol-5(1H)-carboxamide;
Methyl 2-(6-(2-ethyl-5-fluoro-4-hydroxyphenyl)-1H-indazol-3-yl)-4,6-dihydropyrrolo[3,4-d]imidazol-5(1H)-carboxylate;
Ethyl 2-(6-(2-ethyl-5-fluoro-4-hydroxyphenyl)-1H-indazol-3-yl)-4,6-dihydropyrrolo[3,4-d]imidazol-5(1H)-carboxylate;
(S)-(2-(6-(2-ethyl-5-fluoro-4-hydroxyphenyl)-1H-pyrazolo[3,4-b]pyridin-3-yl)-4,6-dihydropyrrolo[3,4-d]imidazol-5(1H)-yl)(3-hydroxylpyrrolidin-1-yl)ketone;
3-(2-(6-(2-ethyl-5-fluoro-4-hydroxyphenyl)-1H-indazol-3-yl) -4,6-dihydropyrrolo[3,4-d]imidazol-5(1H)-yl)-3-oxypropionitrile;
2-(6-(2-ethyl-5-fluoro-4-hydroxyphenyl)-1H-indazol-3-yl)-N,N-dimethyl-4,6-dihydropyrrolo[3,4-d]imidazol-5(1H)-carboxamide;
N-(2-cyanoethyl)-2-(6-(2-ethyl-5-fluoro-4-hydroxyphenyl)-1H-indazol-3-yl)-4,6-dihydropyrrolo[ 3,4-d]imidazol-5(1H)-carboxamide;
N-cyclopropyl-2-(6-(2-ethyl-5-fluoro-4-hydroxyphenyl)-1H-indazol-3-yl)-4,6-dihydropyrrolo[3,4-d]imidazol-5(1H)-carboxamide;
N-cyclobutyl-2-(6-(2-ethyl-5-fluoro-4-hydroxyphenyl)-1H-indazol-3-yl) -4,6-dihydropyrrolo[3,4- d]imidazol-5(1H)-carboxamide;
(S)-6-(2-ethyl-5-fluoro-4-hydroxyphenyl)-3-(5-prolyl-1,4,5,6-tetrahydropyrrolo[3,4-d]imidazol-2-yl)- 1H-indazol; and
(R)-6-(2-ethyl-5-fluoro-4-hydroxyphenyl)-3-(5-prolyl-1,4,5,6-tetrahydropyrrolo[3,4-d]imidazol-2-yl)- 1H-indazol.

27. Use of a compound of Formula (I),

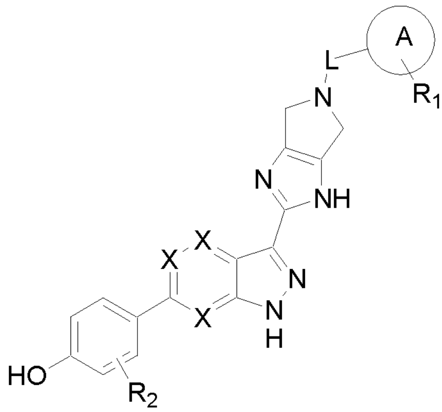

(I)

or an isotopically labeled compound thereof, or an optical isomer thereof, a geometric isomer thereof, a tautomer thereof or a mixture of various isomers, or a pharmaceutically acceptable salt thereof, or a prodrug thereof, or a metabolite thereof, in the manufacture of a medicament for treating severe pneumonia,
in which

L is C=O, O=S=O, $CH_2$ or a covalent bond; and
X is CH or N;
The ring A is $C_{3-7}$ cycloalkyl, 3-7 membered heterocycloalkyl, $C_{5-7}$ aryl, 5-7 membered heteroaryl, $C_{7-11}$ bicyclic aryl, 7-11 membered bicyclic heteroaryl, or 11 -15 membered tricyclyl;
0, 1, 2, 3 or 4 $R_1$(s) are present in formula (I), and $R_1$ is selected from H, halogen, $C_{1-8}$ alkyl, $C_{2-8}$ alkenyl, $C_{2-8}$ alkynyl, $C_{1-8}$ alkoxy, $C_{3-7}$ cycloalkyl, 3-7 membered heterocycloalkyl, $C_{5-7}$ aryl, 5-7 membered heteroaryl, $C_{7-11}$ bicyclic aryl, and 7-11 membered bicyclic heteroaryl, in which the $C_{1-8}$ alkyl, $C_{2-8}$ alkenyl, $C_{2-8}$ alkynyl, and $C_{1-8}$ alkoxy are optionally substituted with 1, 2, 3 or 4 $R_3$(s), and in which the $C_{3-7}$ cycloalkyl, 3-7 membered heterocycloalkyl, $C_{5-7}$ aryl, 5-7 membered heteroaryl, $C_{7-11}$ bicyclic aryl, 7-11 membered bicyclic heteroaryl are optionally substituted with 1, 2, 3 or 4 $R_4$(s),
0, 1, 2, 3 or 4 $R_2$(s) are present in formula (I), and $R_2$ is selected from H, halogen, -OH, $-NO_2$, -CN, $-SF_5$, $C_{1-6}$alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 4-10 membered heterocycloalkyl, $-N(R_9)(R_{10})$, $-N(R_{11})(C(=O)R_{12})$, $-C(=O)-N(R_9)(R_{10})$,$-C(=O)-R_{12}$, $-C(=O)-OR_{12}$, $-OC(=O)R_{12}$, $-N(R_{11})(S(=O)_2R_{12})$, $-S(=O)_2-N(R_9)(R_{10})$, $-SR_{12}$ and $-OR_{12}$, in which the $C_{1-6}$ alkyl, $C_{3-7}$ cycloalkyl and 4-10

membered heterocycloalkyl are each optionally substituted with 1, 2 or 3 substituent(s) each independently selected from the group consisting of halogen, -CN, -OH, $C_{1-4}$ alkyl, $C_{1-4}$ alkoxy, $C_{1-4}$ haloalkyl, $C_{1-4}$ haloalkoxy, $C_{3-6}$ cycloalkyl, $-N(R_9)(R_{10})$, $-N(R_{11})(C(=O)R_{12})$, $-C(=O)-OR_{12}$, -C(=O)H, $-C(=O)R_{12}$,$-C(=O)-N(R_9)(R_{10})$, $-N(R_{11})(S(=O)_2R_{12})$, $-S(=O)_2-N(R_9)(R_{10})$, $-SR_{12}$ and $-OR_{12}$;

$R_3$ is selected from halogen, cyano, $C_{1-3}$ alkyl, hydroxy, $C_{1-6}$ alkoxy, $-N(R_5)(R_6)$,$-CON(R_7)(R_8)$ or 3-7 membered heterocycloalkyl, in which the 3-7 membered heterocycloalkyl is optionally substituted with 1, 2, 3 or 4 $R_4$(s);

$R_4$ is selected from halogen, $C_{1-3}$ alkyl, hydroxyl, $C_{1-6}$ alkoxy, $-NH_2$, $-NHCH_3$ or $-N(CH_3)_2$; $R_5$, $R_6$, $R_7$, $R_8$ are each independently hydrogen or $C_{1-4}$ alkyl;

$R_9$ is selected from H, $C_{1-4}$ alkyl, $C_{1-4}$ haloalkyl or $C_{3-7}$ cycloalkyl;

$R_{10}$ is H or selected from the group consisting of $C_{1-4}$ alkyl, $C_{1-4}$ haloalkyl, $C_{3-7}$ cycloalkyl, 4-10 membered heterocycloalkyl, $C_{6-10}$ aryl, 5-10 membered heteroaryl, ($C_{3-7}$ cycloalkyl)-$C_{1-4}$ alkyl-, (4-10 membered heterocycloalkyl)-$C_{1-4}$ alkyl-, ($C_{6-10}$ aryl)-$C_{1-4}$ alkyl- and (5-10 membered heteroaryl)-$C_{1-4}$ alkyl-, wherein each substituent included in the above group is optionally substituted with 1, 2, 3 or 4 substituent(s) each independently selected from the group consisting of -OH, $-NH_2$, $-NH(CH_3)$, $-N(CH_3)_2$, -CN, $C_{1-4}$ alkyl, $C_{3-7}$ cycloalkyl, $C_{1-4}$ hydroxyalkyl, $-S-C_{1-4}$ alkyl, -C(=O)H, $-C(=O)-C_{1-4}$ alkyl, $-C(=O)-O-C_{1-4}$ alkyl, $-C(=O)-NH_2$, $-C( =O)-N(C_{1-4}$ alkyl$)_2$, $C_{1-4}$ haloalkyl, $C_{1-4}$ alkoxy and $C_{1-4}$ haloalkoxy;

$R_{11}$ is selected from H, $C_{1-4}$ alkyl and $C_{3-7}$ cycloalkyl; and

$R_{12}$ is selected from the group consisting of $C_{1-6}$ alkyl, $C_{3-7}$ cycloalkyl, 4- to 14-membered heterocycloalkyl, $C_{6-10}$ aryl, 5-10 membered heteroaryl, ($C_{3-7}$ cycloalkyl)-$C_{1-4}$ alkyl-, (4-10 membered heterocycloalkyl)-$C_{1-4}$ alkyl-, ($C_{6-10}$ aryl)-$C_{1-4}$ alkyl- and (5-10 membered heteroaryl)-$C_{1-4}$ alkyl-, wherein each substituent included in the above group is optionally substituted with 1, 2 or 3 substituent(s) each independently selected from the group consisting of halogen, $-CF_3$, -CN, -OH, $-NH_2$, $-NH(CH_3)$, $-N(CH_3)_2$, oxo, $-S-C_{1-4}$ alkyl, $C_{1-4}$ alkyl, $C_{1-4}$ haloalkyl, $C_{2-6}$ alkenyl, $C_{2-6}$ alkynyl, $C_{3-7}$ cycloalkyl, $C_{1-4}$ alkoxy and $C_{1-4}$ haloalkoxy.

28. The use according to embodiment 27, wherein L is C=O, O=S=O or $CH_2$.

29. The use according to embodiment 27, wherein X is CH.

30. The use according to any one of embodiments 27 to 29, wherein the ring A is $C_{3-7}$ cycloalkyl, 3-7 membered heterocycloalkyl, $C_{5-7}$ aryl, or 5-7 membered heteroaryl.

31. The use according to any one of embodiments 27 to 29, wherein the ring A is 5-6 membered heteroaryl, or phenyl.

32. The use according to any one of embodiments 27 to 29, wherein 0, or 1 $R_1$ is present, and $R_1$ is selected from $C_{1-6}$ alkyl, and 5-7 membered heterocycloalkyl in which the $C_{1-6}$ alkyl is optionally substituted with 1 or 2 $R_3$, and in which the 5-7 membered heterocycloalkyl is optionally substituted with 1, 2, 3 or 4 $C_{1-3}$ alkyl.

33. The use according to any one of embodiments 27 to 29, wherein 1 or 2 $R_2$(s) are present, and $R_2$ is selected from halogen, $C_{1-6}$ alkyl and $C_{3-6}$ cycloalkyl, in which the $C_{1-6}$ alkyl and $C_{3-6}$ cycloalkyl are each optionally substituted with 1, 2 or 3 substituent(s) each independently selected from the group consisting of halogen, -OH, $-NH_2$, $-NH(CH_3)$,$-N(CH_3)_2$, -CN, $C_{1-4}$ alkyl, $C_{1-4}$ haloalkyl, $C_{1-4}$ alkoxy, and $C_{1-4}$ haloalkoxy.

34. The use according to any one of embodiments 27 to 29, wherein

L is C=O, and O=S=O;

X is CH;

The ring A is 5-7 membered heteroaryl or $C_{5-7}$ aryl;

0, 1, 2, 3 or 4 $R_1$(s) are present in formula (I), and $R_1$ is selected from $C_{1-8}$ alkyl, and 3-7 membered heterocycloalkyl, in which the $C_{1-8}$ alkyl is optionally substituted with 1, 2, 3 or 4 $R_3$(s), and in which the 3-7 membered heterocycloalkyl is optionally substituted with 1, 2, 3 or 4 $R_4$(s),

1, 2, or 3 $R_2$(s) are present in formula (I), and $R_2$ is selected from H, halogen, -OH, $-NO_2$,-CN, $-SF_5$, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 4-10 membered heterocycloalkyl, $-N(R_9)(R_{10})$, $-N(R_{11})(C(=O)R_{12})$, $-C(=O)-N(R_9)(R_{10})$,$-C(=O)-R_{12}$, $-C(=O)-OR_{12}$, $-OC(=O)R_{12}$, $-N(R_{11})(S(=O)_2R_{12})$, $-S(=O)_2-N(R_9)(R_{10})$, $-SR_{12}$ and $-OR_{12}$, in which the $C_{1-6}$ alkyl, $C_{3-7}$ cycloalkyl and 4-10 membered heterocycloalkyl are each optionally substituted with 1, 2 or 3 substituent(s) each independently selected from the group consisting of halogen, -CN, -OH, $C_{1-4}$ alkyl, $C_{1-4}$ alkoxy, $C_{1-4}$ haloalkyl, $C_{1-4}$ haloalkoxy, $C_{3-6}$ cycloalkyl, $-N(R_9)(R_{10})$, $-N(R_{11})(C(=O)R_{12})$, $-C(=O)-OR_{12}$, -C(=O)H, $-C(=O)R_{12}$,$-C(=O)-N(R_9)(R_{10})$, $-N(R_{11})(S(=O)_2R_{12})$, $-S(=O)_2-N(R_9)(R_{10})$, $-SR_{12}$ and $-OR_{12}$;

$R_3$ is selected from halogen, cyano, $C_{1-3}$ alkyl, hydroxy, $C_{1-6}$ alkoxy, $-N(R_5)(R_6)$,$-CON(R_7)(R_8)$ or 3-7 membered heterocycloalkyl, in which the 3-7 membered heterocycloalkyl is optionally substituted with 1, 2, 3 or 4 $R_4$(s);

$R_4$ is selected from halogen, $C_{1-3}$ alkyl, hydroxyl, $C_{1-6}$ alkoxy, $-NH_2$, $-NHCH_3$ or $-N(CH_3)_2$;

$R_5$, $R_6$, $R_7$, $R_8$ are each independently hydrogen or $C_{1-4}$ alkyl;

$R_9$ is selected from H, $C_{1-4}$ alkyl, $C_{1-4}$ haloalkyl or $C_{3-7}$ cycloalkyl;

$R_{10}$ is H or selected from the group consisting of $C_{1-4}$ alkyl, $C_{1-4}$ haloalkyl, $C_{3-7}$ cycloalkyl, 4-10 membered

heterocycloalkyl, $C_{6-10}$ aryl, 5-10 membered heteroaryl, ($C_{3-7}$ cycloalkyl)-$C_{1-4}$ alkyl-, (4-10 membered heterocycloalkyl)-$C_{1-4}$ alkyl-, ($C_{6-10}$ aryl)-$C_{1-4}$ alkyl- and (5-10 membered heteroaryl)-$C_{1-4}$ alkyl-, wherein each substituent included in the above group is optionally substituted with 1, 2, 3 or 4 substituent(s) each independently selected from the group consisting of -OH, $-NH_2$, $-NH(CH_3)$, $-N(CH_3)_2$, -CN, $C_{1-4}$ alkyl, $C_{3-7}$ cycloalkyl, $C_{1-4}$ hydroxyalkyl, -S-$C_{1-4}$ alkyl, -C(=O)H, -C(=O)-$C_{1-4}$ alkyl, -C(=O)-O-$C_{1-4}$ alkyl, $-C(=O)-NH_2$, -C( =O)-N($C_{1-4}$ alkyl$)_2$, $C_{1-4}$ haloalkyl, $C_{1-4}$ alkoxy and $C_{1-4}$ haloalkoxy;
$R_{11}$ is selected from H, $C_{1-4}$ alkyl and $C_{3-7}$ cycloalkyl; and
$R_{12}$ is selected from the group consisting of $C_{1-6}$ alkyl, $C_{3-7}$ cycloalkyl, 4- to 14-membered heterocycloalkyl, $C_{6-10}$ aryl, 5-10 membered heteroaryl, ($C_{3-7}$ cycloalkyl)-$C_{1-4}$ alkyl-, (4-10 membered heterocycloalkyl)-$C_{1-4}$ alkyl-, ($C_{6-10}$ aryl)-$C_{1-4}$ alkyl- and (5-10 membered heteroaryl)-$C_{1-4}$ alkyl-, wherein each substituent included in the above group is optionally substituted with 1, 2 or 3 substituent(s) each independently selected from the group consisting of halogen, $-CF_3$, -CN, -OH, $-NH_2$, $-NH(CH_3)$, $-N(CH_3)_2$, oxo, -S-$C_{1-4}$ alkyl, $C_{1-4}$ alkyl, $C_{1-4}$ haloalkyl, $C_{2-6}$ alkenyl, $C_{2-6}$ alkynyl, $C_{3-7}$ cycloalkyl, $C_{1-4}$ alkoxy and $C_{1-4}$ haloalkoxy.

35. The use according to embodiment 34, wherein the ring A is 5-6 membered heteroaryl, or phenyl.
36. The use according to embodiment 34, wherein 0 or 1 $R_1$ is present, and $R_1$ is selected from $C_{1-6}$ alkyl, and 5-7 membered heterocycloalkyl, wherein the C1-6 alkyl is optionally substituted by 1 or 2 $R_3$, and wherein the 5-7 membered heterocycloalkyl group is optionally substituted with 1, 2, 3, or 4 $C_{1-3}$ alkyl.
37. The use according to embodiment 34, wherein 1 or 2 $R_2$(s) are present, and $R_2$ is selected from halogen, $C_{1-6}$ alkyl and $C_{3-6}$ cycloalkyl, wherein the $C_{1-6}$ alkyl and $C_{3-6}$ cycloalkyl are each optionally substituted with 1, 2 or 3 substituent(s) each independently selected from the group consisting of halogen, -OH, $-NH_2$, $-NH(CH_3)$, $-N(CH_3)_2$, -CN, $C_{1-4}$ alkyl, $C_{1-4}$ haloalkyl, $C_{1-4}$ alkoxy and $C_{1-4}$ haloalkoxy.
38. The use according to any one of embodiments 27 to 29, wherein the compound is selected from a group consisting of:

(2-(6-(2-ethyl-5-fluoro-4-hydroxyphenyl)-1H-indazol-3-yl)-4,6-dihydropyrrolo[3,4-d]imidazol-5(1H)-yl)(5-(piperidin-1-yl)pyrazin-2-yl)ketone;
(2-(6-(2-ethyl-5-fluoro-4-hydroxyphenyl)-1H-indazol-3-yl)-4,6-dihydropyrrolo[3,4-d]imidazol-5(1H)-yl)(5-morpholinpyrazin-2-yl)ketone;
(2-(6-(2-ethyl-5-fluoro-4-hydroxyphenyl)-1H-indazol-3-yl)-4,6-dihydropyrrolo[3,4-d]imidazol-5(1H)-yl)(1-methyl-1H-pyrazol-4-yl)ketone;
5-ethyl-2-fluoro-4-{3-[5-(1-methylpiperidin-4-carbonyl)-1H,4H,5H,6H-pyrrolo[3,4-d]imidazol-2-yl]-1H-indazol-6-yl}phenol;
5-ethyl-2-fluoro-4-{3-[5-(4-methylpiperazin-1-carbonyl)-1H,4H,5H,6H-pyrrolo[3,4-d]imidazol-2-yl]-1H-indazol-6-yl}phenol;
3-ethyl-4-{3-[5-(4-methylpiperazin-1-carbonyl)-1H,4H,5H,6H-pyrrolo[3,4-d]imidazol-2-yl]-1H-indazol-6-yl} phenol;
5-ethyl-2-fluoro-4-(3-(5-(bemzenesulfonyl)-1,4,5,6-tetrahydropyrrolo[3,4-d]imidazol-2-yl)-1H-indazol-6-yl)phenol; and
5-ethyl-2-fluoro-4-(3-(5-(pyrazin-2-methyl)-1,4,5,6-tetrahydropyrrolo[3,4-d]imidazol-2-yl)-1H-indazol-6-yl)phenol.

The present application will be further illustrated and described below in conjunction with the drawings and specific examples.

## DESCRIPTION OF THE DRAWINGS

FIG. 1 illustrates the effects of different compounds on IL-1β levels in mice.
FIG. 2 illustrates the effects of different compounds on IFN-γ levels in mice.
FIG. 3 illustrates the effects of different compounds on IL-6 levels in mice.
FIG. 4 illustrates the effects of different compounds on TNF-α levels in mice.

In each figure, at the horizontal coordinates, the reference sign "Vehicle" denotes the solvent control group, "Tofa" denotes the Tofacitinib group, "209" denotes the MDI- 209 group, "216" denotes MDI-216 group, and "Normal" denotes normal mice without special treatment.

## EXAMPLES

The compounds of formula (G), the compounds of formula (G') or the compounds of formula (I) of the present disclosure can be synthesized by various methods familiar to those skilled in the art of organic synthesis. The following specific examples give some exemplary synthesis methods of the compounds of formula (G), the compounds of formula (G') or the compounds of formula (I), and these methods are well-known in the field of synthetic chemistry. Obviously, referring to the exemplary embodiments of the present disclosure, those skilled in the art can appropriately adjust reactants, reaction conditions, and protective groups to easily design other synthetic routes for compounds of formula (G), formula (G') or formula (I).

The following further describes the present disclosure in conjunction with examples. Nevertheless, these examples do not limit the scope of the present disclosure. Unless otherwise stated, all reactants used in the examples were obtained from commercial sources; and the instruments and equipment used in the synthesis experiments and product analysis assays were the conventional instruments and equipment normally used in organic synthesis.

### Example 1:(2-(6-(2-ethyl-5-fluoro-4-hydroxyphenyl)-1H-indazol-3-yl)-4,6-dihydropyrrolo[3,4-d]imidazol-5(1H)-yl)(5-(piperidin-1-yl)pyrazin-2-yl)ketone (MDI-2)

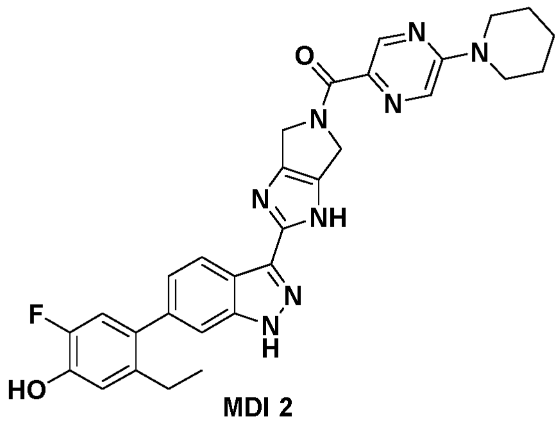

MDI 2

**Synthetic route of target compound 8 (i.e. MDI-2):**

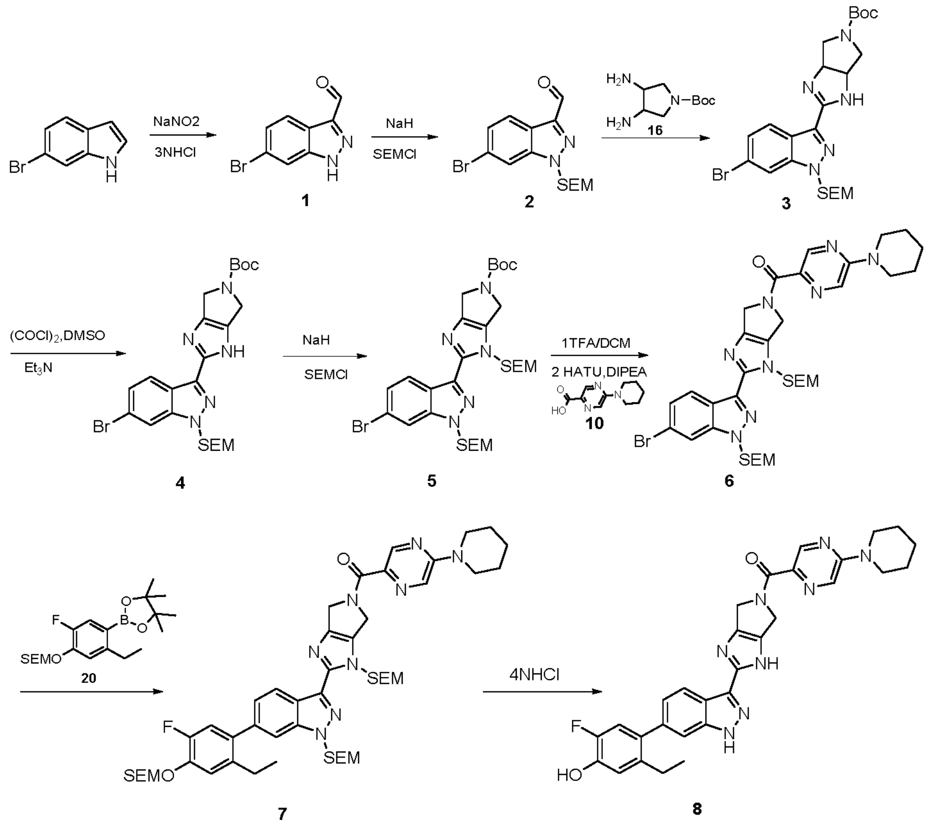


1 2 3 4 5 6 7 8

**Synthetic route of intermediate 10**

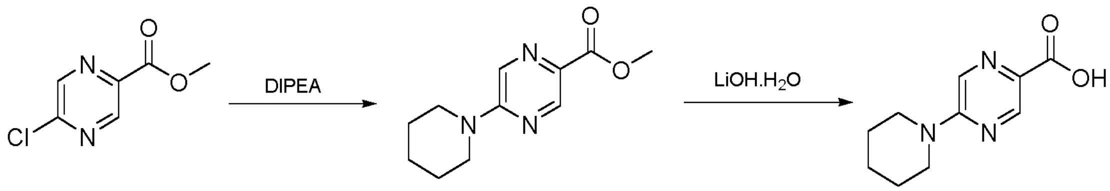


9 10

**Synthetic route of intermediate 16**

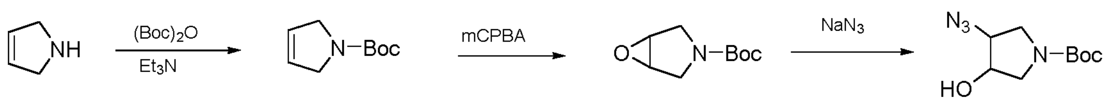


11 12 13

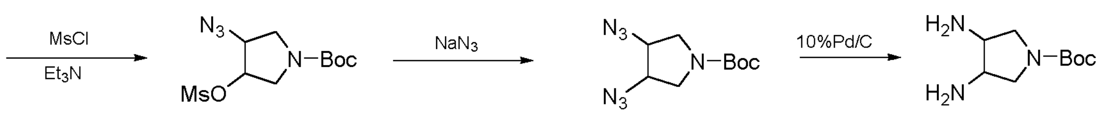


14 15 16

**Synthetic route of intermediate 20**

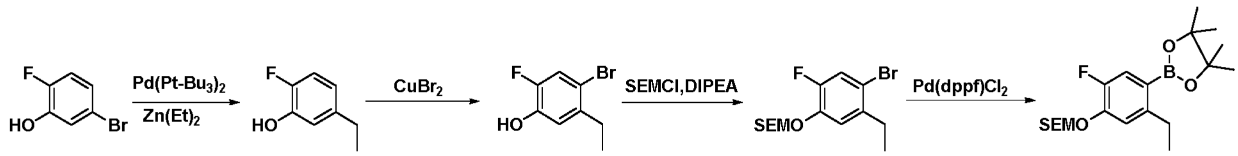


17 18 19 20

**Synthesis method:**

**Synthesis of intermediate 1:6-bromo-1H-indazole-3-formaldehyde**

Sodium nitrite (14.00 g, 200 mmol) was dissolved in 75ml DMF and 100 ml water, and then cooled to 0°C. Under nitrogen protection, 3N HCl (23 ml, 68.9 mmol) was slowly added dropwise and after addition, the reaction was carried out for 10 minutes. At 0°C, to the reaction solution, 6-bromoindole (5.00 g, 25.5 mmol) in DMF (35ml) was slowly added dropwise. After the dropwise addition was completed, the reaction was continued at room temperature overnight. The resulting mixture was extracted with ethyl acetate 3 times, and then the organic phases were combined, washed 3 times with water, washed with saturated brine, dried over anhydrous sodium sulfate, concentrated, and purified by silica gel column to afford the intermediate 1, with a yield of 83.6%.

$^1$H NMR (400 MHz, CDCl3) δ 10.29 (s, 1H), 8.24 (d, J = 8.0 Hz, 1H), 7.80 (d, J = 4.0 Hz, 1H), 7.52 (dd, J = 8.0 Hz, J = 4.0 Hz, 1H).

**Synthesis of intermediate 2: 6-bromo-1-((2-(trimethylsilyl)ethoxy) methyl) -1H-indazole-3-formaldehyde**

Intermediate 1 (1.56 g, 6.93 mmol) was dissolved in dry tetrahydrofuran, and then cooled to 0°C. Sodium hydride (0.33 g, 8.32 mmol) was added slowly, the reaction was carried out at room temperature for 1 hour, and then cooled to 0°C. After that, 2-(trimethylsilyl)ethoxymethyl chloride (1.73 g, 10.40 mmol) was added dropwise and the reaction was carried out at room temperature overnight. The reaction was quenched by adding water. The resulting mixture was extracted twice with ethyl acetate, and the organic phases were combined and washed with water and saturated brine, dried over anhydrous sodium sulfate, concentrated, and purified by silica gel column to afford Intermediate 2, with a yield of 49.2%.

$^1$H NMR (400 MHz, CDCl3) δ 10.25 (s, 1H), 8.22 (dd, J = 8.0 Hz, J = 4.0 Hz 1H), 7.88 (dd, J = 4.0 Hz, J = 4.0 Hz, 1H), 7.52 (dd, J = 4.0 Hz, J = 4.0 Hz, 1H), 5.81 (s, 2H), 3.63-3.58 (m, 2H), 0.97-0.93 (m, 2H), 0.04 (s, 9H).

**Synthesis of intermediate 16: Tert-butyl 3,4-diaminopyrrolidinyl-1-carboxylate**

**1. Synthesis of intermediate 11: Tert-butyl 2,5-dihydro-1H-pyrrole-1-carboxylate**

3-pyrroline (10.0 g, 0.15 mol) was dissolved in 400ml dichloromethane and triethylamine (40.6 ml, 0.29 mol), and then cooled to 0°C. $(Boc)_2O$ (37.9 g, 0.17 mol) was slowly added. The reaction was carried out at room temperature overnight. Water was added and the mixture was extracted twice with dichloromethane. The organic phases was combined, washed with water three times, washed with saturated brine, dried over anhydrous sodium sulfate, concentrated, and purified by silica gel column to afford Intermediate 11 with a yield of 91.0%.

**2. Synthesis of intermediate 12: Tert-butyl 6-oxa-3-azabicyclo[3.1.0] hexane-3-carboxylate**

Intermediate 11 (24.5 g, 0.15 mol) was dissolved in 450ml of dichloromethane, and then cooled to 0°C. M-chloroperoxybenzoic acid (37.5 g, 0.22 mol) was slowly added in batches. The reaction was carried out at room temperature overnight. After that, saturated sodium thiosulfate (40ml) was added, and then stirred for 30 minutes. The aqueous phase was extracted twice with dichloromethane, washed with saturated potassium carbonate solution, water and saturated brine, dried over anhydrous sodium sulfate, concentrated, and purified by silica gel column to afford Intermediate 12 with a yield of 84.9%.

$^1$H NMR (400 MHz, CDCl3) δ 3.85 (d, J = 12.0 Hz, 1H), 3.77 (d, J = 12.0 Hz, 1H), 3.69-3.67 (m, 2H), 3.36-3.30 (m, 2H), 1.45 (s, 9H).

**3. Synthesis of intermediate 13: Tert-butyl 3-azido-4-hydroxyl pyrrolidinyl-1-carboxylate**

Intermediate 12 (20.8 g, 0.12 mol) was dissolved in 150 ml 1,4-dioxane and 50 ml water, and then sodium azide (24.0 g, 0.37 mol) was added. The mixture was heated to 106°C and reacted for 18 hours, then cooled to room temperature, followed by adding 100ml of saturated brine. The resulting mixture was extracted with dichloromethane (250ml*4), and the organic phases were combined, washed with saturated brine, dried over anhydrous sodium sulfate, and concentrated to afford Intermediate 13, with a yield of 100%.

$^1$H NMR (400 MHz, CDCl3) δ 4.27-4.24 (m, 1H), 3.94 (s, 1H), 3.73-3.59 (m, 2H), 3.41-3.36 (m, 2H), 1.47 (s, 9H).

**4. Synthesis of intermediate 14: Tert-butyl 3-azido-4-((methanesulfonyl) oxy)pyrrolidinyl-1-carboxylate**

Intermediate 13 (28.0 g, 0.12 mol) was dissolved in 350ml of dichloromethane and triethylamine (37.3 g, 0.37 mol), and cooled to 0°C, followed by slowly adding methanesulfonyl chloride (16.9 g, 0.15 mol) dropwise. After the addition, the reaction was carried out at room temperature for 2 hours, the reaction was quenched with water, and the resulting mixture was extracted twice with dichloromethane. The organic phases was combined, washed with saturated sodium bicarbonate solution, water and saturated brine, dried over anhydrous sodium sulfate, and concentrated to afford Intermediate 14, with a yield of 98.0%.

**5. Synthesis of intermediate 15: Tert-butyl 3,4-diazidopyrrolidinyl-1-carboxylate**

Intermediate 14 (36.9 g, 0.12 mol) was dissolved in 250 ml DMF, to which sodium azide (23.5 g, 0.36 mol) was added. The mixture was heated to 90°C, reacted for 2 days, and cooled to room temperature, following by adding 750 ml of water. The resulting mixture was extracted with butyl tert-butyl ether (400ml*4), and the organic phases were combined, washed with saturated brine, dried with anhydrous sodium sulfate, and purified by silica gel column to afford Intermediate 15 with a yield of 62.2%.

**6. Synthesis of intermediate 16: Tert-butyl 3,4-diaminopyrrolidinyl-1-carboxylate**

Intermediate 15 (18.9 g, 0.08 mol) was dissolved in 200ml methanol, and 10% Pd/C was added where it was replaced with hydrogen 3 times. The mixture was heated to 40 °C, and reacted for 2 days. The resulting mixture was filtered and concentrated to afford Intermediate 16, with a yield of 78 %.

$^1$H NMR (400 MHz, CDCl3) δ 3.51-3.49 (m, 2H), 3.40-3.36 (m, 2H), 3.21-3.11 (m, 2H), 1.47 (s, 9H).

**Synthesis of intermediate 3: Tert-butyl 2-(6-bromo1-((2-(trimethylsilyl)ethoxy)methyl)-1H-indazol-3-yl)3,4,6,6a-tetrahydropyrrolo[3,4-d]imidazole -5(1H)-carboxylate**

Intermediate 2 (1.56 g, 6.93 mmol) and tert-butyl 3,4-diaminopyrroline-1-carboxylate (1.56 g, 6.93 mmol) were dissolved in 5ml of hexafluoroisopropanol and heated to 40°C for 2 days. The resulting mixture was concentrated and purified by a silica gel column to afford Intermediate 3 with a yield of 54.7%.

**Synthesis of intermediate 4: Tert-butyl 2-(6-bromo-1-((2-(trimethylsilyl) ethoxy)methyl)-1H-indazol-3-yl)4,6-dihydropyrrolo[3,4-d]imidazole-5(1H)-carboxylate**

Oxalyl chloride (0.53 g, 4.20 mmol) was dissolved in dry 15ml dichloromethane, and cooled to -78°C under the protection of nitrogen. DMSO (0.61 g, 7.84 mmol) was slowly added dropwise. After the addition was completed, it was allowed to react for 30 minutes. Intermediate 3 (1.00 g, 1.87 mmol) in dichloromethane was slowly added dropwise. After the dropwise addition, it was allowed to react for 30 minutes. Dry triethylamine (1.89 g, 18.66 mmol) was added slowly dropwise, and it was allowed to react for 10 minutes. The temperature was increased slowly and the reaction was carried out at room temperature for 2 hours. The reaction was quenched with saturated ammonium chloride solution and the resulting mixture was extracted twice with dichloromethane, and the organic layers were combined, washed with water and saturated brine, dried over anhydrous sodium sulfate, concentrated, and purified by silica gel column to afford Intermediate 4 with a yield of 36.3%.

$^1$H NMR (400 MHz, CDCl3) δ 8.36 (d, J = 4.0 Hz, 1H), 7.78 (d, J = 4.0 Hz, 1H), 7.44 (dd, J = 8.0 Hz, J = 4.0 Hz, 1H), 5.69 (s, 2H), 4.64-4.52 (m, 4H), 3.67-3.56 (m, 2H), 1.56 (s, 9H), 0.95-0.89 (m, 2H), 0.03 (s, 9H).

**Synthesis of intermediate 5: Tert-butyl 2-(6-bromo1-((2-(trimethylsilyl)ethoxy)methyl)-1H-indazol-3-yl)-1-((2-(trimethylsilyl)ethoxy)methyl)-4,6-dihydropyrrolo[3,4-d]imidazole-5(1H)-carboxylate**

Intermediate 4 (110 mg, 0.21 mmol) was dissolved in dry tetrahydrofuran, and cooled to 0°C. Sodium hydride (12.3 mg, 0.31 mmol) was added and it allowed to react at room temperature for 30 minutes. The mixture was cooled to 0°C. 2-(tri methylsilyl)ethoxymethyl chloride (41.2 mg, 0.25 mmol) was added slowly dropwise, and it allowed to react at room temperature for 4 hours. The reaction was quenched with water and the resulting mixture was extracted twice with ethyl acetate. The organic phases were combined, washed with water and saturated brine, dried over anhydrous sodium sulfate, concentrated, and purified by silica gel column to intermediate 5 with a yield of 73.1%.

$^1H$ NMR (400 MHz, CDCl3) δ 8.41-8.36 (m, 1H), 7.79 (s, 1H), 7.44 (dd, J = 8.0 Hz, J = 4.0 Hz, 1H), 5.94 (d, J = 12.0 Hz, 2H), 5.73 (s, 2H), 4.65-4.52 (m, 4H), 3.63-3.57 (m, 4H), 1.56 (s, 9H), 0.96-0.91 (m, 4H), 0.03 (s, 18H) ).

**Synthesis of intermediate 10: 5-(piperidin-1-yl)pyrazine-2 carboxylic acid**

**1. Synthesis of intermediate 9: Methyl 5-(piperidin-1-yl)pyrazine-2-carboxylate**

Methyl 5-chloro-pyrazine-2-carboxylate (1.72 g, 10 mmol) was dissolved in 10ml DMF, and N,N-diisopropylethylamine (4.3 ml, 25.0 mmol) and piperidine hydrochloride (1.45 g, 12.0 mmol) were added. The mixture was stirred overnight at room temperature. Under vigorous stirring, water was added. A solid was precipitated, filtered, and the filter cake was washed with water, and dried to afford Intermediate 9 with a yield of 80.0%.

**2. Synthesis of intermediate 10: 5-(piperidin-1-yl)pyrazin-2-carboxylic acid**

Intermediate 9 (430 mg, 1.95 mmol) was dissolved in 20 ml of tetrahydrofuran and 20 ml of water, to which lithium hydroxide (163 mg, 3.88 mmol) was added. The reaction was carried out at room temperature for 4 hours. The mixture was concentrated by distilling off tetrahydrofuran under reduced pressure, and the pH was adjusted to 4 with 1N HCl. A solid precipitated, filtered, and the filter cake was washed with water, and dried to afford Intermediate 10 with a yield of 91.5%.

$^1H$ NMR (400 MHz, CDCl3) δ 8.84 (s, 1H), 8.02 (s, 1H), 3.76-3.73 (m, 4H), 1.78-1.65 (m, 6H).

**Synthesis of intermediate 6 : (2-(6-bromo1-((2-(trimethylsilyl)ethoxy)methyl)-1H-indazol-3-yl)-1-((2-(trimethylsilyl)ethoxy)methyl)-4,6-dihydropyrrolo[3,4-d]imidazol-5(1H)-yl)(5-(piperidin-1-yl)pyrazin-2-yl)ketone**

Intermediate 10 (34.3 mg, 0.17 mmol) and N,N-diisopropylethylamine (58.2 mg, 0.45 mmol) were dissolved in DMF, HATU (85.7 mg, 0.22 mmol) was added, and the reaction was carried out at room temperature for 10 minutes. Intermediate 5 (100 mg, 0.15 mmol) was dissolved in 5ml of dichloromethane, to which 1ml of trifluoroacetic acid was added. The mixture was stirred at room temperature for 30 minutes, and concentrated to give a residue. The residue was dissolved in dichloromethane and was concentrated to dryness, which was repeated 3 times. The resulting residue was dissolved in DMF and was then slowly added to the previous reaction solution. It was allowed to react overnight at room temperature. The reaction was quenched with water, and the resulting mixture was extracted twice with ethyl acetate. The organic phases were combined, washed with water and saturated brine, dried over anhydrous sodium sulfate, concentrated, and purified by silica gel column to afford intermediate 6 with a yield of 57.3%.

$^1H$ NMR (400 MHz, $CDCl_3$) δ 8.87 (d, J = 8.0 Hz, 1H), 8.41-8.37 (m, 1H), 8.09-8.04 (m, 1H), 7.80 (s, 1H), 7.44-7.41 ( m, 1H), 5.96 (s, 2H), 5.75 (d, J = 8.0 Hz, 2H), 5.28 (s, 1H), 5.19 (s, 1H), 4.99 (s, 1H), 4.91 (s, 1H), 3.74-3.68 (m, 4H), 3.67-3.64 (m, 2H), 3.63-3.59 (m, 2H), 1.71-1.68 (m, 6H), 0.95-0.91 (m, 4H), 0.03 (s , 9H), 0.02 (s, 9H).

**Synthesis of intermediate 20: (2-((5-ethyl-2-fluoro-4-(4,4,5,5-tetramethyl-1,3,2-dioxaboran-2-yl)phenoxy)methoxy)ethyl)trimethylsilane**

**1. Synthesis of intermediate 17: 5-ethyl-2-fluorophenol**

5-bromo-2-fluorophenol (200.0 mg, 1.05 mmol) and bis(tri-tert-butylphosphorus) palladium (10.7 mg, 0.02 mmol) was dissolved in 10 ml THF. The atmosphere was replaced with nitrogen, which was repeated 3 times. The temperature was lowered to 10-20°C. 1 mol/L diethyl zinc solution (2.3 ml, 2.30mmol) was added dropwise. After the addition was completed, the temperature was heated up to 50°C. It was allowed to react overnight, and the temperature was cooled to 0°C. The reaction was quenched with water, and filtered with celite. The celite pad was washed with ethyl acetate. The resulting filtrate was extracted with ethyl acetate, and the organic phases were combined, washed with saturated sodium chloride solution, and dried over anhydrous sodium sulfate. After drying, it was concentrated and

separated by column chromatography to afford an oily liquid with a yield of 65.1%.

$^1$H NMR (400 MHz, $CDCl_3$) δ6.97 (d, J = 8.0 Hz, 1H), 6.85 (d, J = 12.0 Hz, 1H), 6.69 - 6.65 (m, 1H), 2.61 - 2.55 (m, 2H), 1.21 (t, J =8.0 Hz, 3H).

**2. Synthesis of intermediate 18: 4-bromo-5-ethyl-2-fluorophenol**

Intermediate 17 (200.1 mg, 1.43mmol) was dissolved in 6ml of acetonitrile, to which $CuBr_2$ (957.5 mg, 4.29mmol) was added. The mixture was stirred at room temperature for 3 hours. The reaction was quenched with water, extracted with ethyl acetate, and the organic phase was washed with saturated sodium chloride solution and dried over anhydrous sodium sulfate. It was concentrated and separated by column chromatography to afford a colorless oil, yield: 78.1%.

$^1$H NMR (400 MHz, CDCl3) δ7.25 (d, J = 12.0 Hz, 1H), 6.89 (d, J = 12.0 Hz, 1H), 2.69 - 2.63 (m, 2H), 1.19 (t, J = 12.0 Hz, 3H).

**3. Synthesis of intermediate 19: (2-((4-bromo-5-ethyl-2-fluorophenoxy)methoxy)ethyl)trimethylsilane**

Intermediate 18 (220.0 mg, 1.00mmol) was dissolved in 6ml DCM, DIPEA (130.5 mg, 1.10mmol) was added, and the temperature was reduced to 0°C. SEMCl (168.2 mg, 1.10 mmol) was added dropwise at 0°C. After the addition, the temperature was raised to room temperature, and it was allowed to react for 8 hours. The reaction was quenched with water, and extracted with DCM. The organic phase was washed with saturated sodium chloride solution, and dried over anhydrous sodium sulfate. It was concentrated to afford a colorless oil, the crude yield: 99.1%.

$^1$H NMR (400 MHz, CDCl3) δ7.26 (d, J = 12.0 Hz, 1H), 6.89 (d, J = 12.0 Hz, 1H), 5.24 (s, 2H) 3.82 - 3.78 (m, 2H) 2.67 - 2.62 (m, 2H), 1.19 (t, J = 12.0 Hz, 3H), 0.98 - 0.94 (m, 2H), 0.01 (s, 9H).

**4. Synthesis of intermediate 20: (2-((5-ethyl-2-fluoro-4-(4,4,5,5-tetramethyl-1,3,2-dioxaboran-2-yl)phenoxy)methoxy)ethyl)trimethylsilane**

Compound 19 (280.0mg, 0.80mmol), pinacol borate (206.1mg, 0.80mmol), Pd(dppf)$Cl_2$ (59.2mg, 0.08mmol) and KOAc (237.5mg, 2.40mmol) were dissolved in 1, 4-dioxane (6 ml). The atmosphere was replaced with nitrogen, which was repeated 3 times. The mixture was heated to 100°C and it was allowed to react overnight. After the reaction was completed, it was quenched with water, extracted with ethyl acetate, and the organic phase was washed with saturated sodium chloride solution, and dried over anhydrous sodium sulfate. It was concentrated and separated by column chromatography to afford a colorless oil, yield: 56.2%.

$^1$H NMR (400 MHz, CDCl3) δ7.48 (d, J = 12.0 Hz, 1H), 7.02 (d, J = 8.0 Hz, 1H), 5.28 (s, 2H), 3.82 - 3.78 (m, 2H) 2.89 - 2.83 (m, 2H), 1.35 (s, 12H), 1.17 (t, J = 8.0 Hz, 3H), 0.98 - 0.94 (m, 2H), 0.01 (s, 9H).

**Synthesis of intermediate 7: (2-(6-(2-ethyl-5-fluoro-4-((2-(trimethylsilyl)ethoxy)methyl)hydroxyphenyl)1-((2-(trimethylsilyl)ethoxy)methyl)-1H-indazol-3-yl)-1-((2-(trimethylsilyl)ethoxy)methyl)-4,6-dihydropyrrolo[3,4-d]imidazol-5(1H)-yl)(5-(piperidin-1-yl)pyrazin-2-yl)ketone**

Intermediate 6 (65.0 mg, 0.09 mmol), (2-((5-ethyl-2-fluoro-4-(4,4,5,5-tetramethyl-1,3,2-dioxaboran-2-yl)phenoxy)methoxy)ethyl)trimethylsilane (40.9 mg, 0.10 mmol), Pd(dppf)$Cl_2$ (6.3 mg, 0.01 mmol) and potassium phosphate (25.3 mg, 0.26 mmol) were dissolved in 1,4-dioxane (10ml) and water (2ml). The atmosphere was replaced with nitrogen 3 times. The mixture was heated to 100°C, reacted overnight, and cooled to room temperature. Water was added and the mixture was extracted 2 times with ethyl acetate. The organic phases were combined, washed with water and saturated brine, dried over anhydrous sodium sulfate, concentrated, and purified by silica gel column to afford Intermediate 7 with a yield of 52.8%.

$^1$H NMR (400 MHz, CDCl3) δ 8.87 (dd, J = 8.0 Hz, J = 4.0 Hz, 1H), 8.54 (dd, J = 8.0 Hz, J = 20.0 Hz, 1H), 8.10 (dd, J = 8.0 Hz, 1H), 7.48 (s, 1H), 7.27 (s, 1H), 7.20 (d, J = 8.0 Hz, 1H), 7.06 (d, J = 12.0 Hz, 1H), 6.00 (s, 2H), 5.79 (d, J = 4.0 Hz, 2H), 5.35 (s, 2H), 5.33 (s, 1H), 5.29 (s, 1H), 5.20 (s, 1H), 5.01(s, 1H), 3.91 (t, J = 8.0 Hz, J = 20.0 Hz, 2H), 3.76-3.74 (m, 4H), 3.64-3.62 (m, 4H), 2.58 (t, J = 8.0 Hz, J = 16.0 Hz, 2H), 1.74 -1.72 (m, 6H), 1.10-1.06 (m, 3H), 0.95-0.91 (m, 6H), 0.06 (s, 9H), 0.04 (s, 9H), 0.03 (s, 9H).

**Synthesis of compound 8 (i.e. MDI-2): (2-(6-(2-ethyl-5-fluoro-4-hydroxyphenyl)-1H-indazol-3-yl)-4,6-dihydropyrrolo[3,4-d]imidazol-5(1H)-yl)(5-(piperidin-1-yl)pyrazin-2-yl)ketone**

Intermediate 7 (43.0 mg, 0.05 mmol) was dissolved in methanol (4ml), to which concentrated hydrochloric acid (2ml) was added. The mixture was heated to 50°C, reacted for 6 hours, and concentrated. The resulting solid was dissolved in 1ml methanol, pH was adjusted to 8-9 with sodium bicarbonate solution, and then the resulting mixture was

extracted 4 times with dichloromethane. The organic phases were combined, dried over anhydrous sodium sulfate, and purified by a preparation plate to afford 4.5mg of the final product with a yield of 18.0%.

$^1$H NMR (400 MHz, MeOD-d4) δ 8.67 (s, 1H), 8.28 (dd, J = 8.0 Hz, J = 4.0 Hz, 1H), 8.21 (s, 1H), 7.40 (s, 1H), 7.18 (dd, J = 8.0 Hz, J = 4.0 Hz, 1H), 6.96-6.89 (m, 2H), 5.14 (s, 2H), 4.82 (s, 2H), 3.76-3.73 (m, 4H), 2.58 ( dd, J = 12.0 Hz, J = 8.0 Hz, 2H), 1.76-1.66 (m, 6H), 1.10 (t, J = 8.0 Hz, 3H).

**Example 2: (2-(6-(2-ethyl-5-fluoro-4-hydroxyphenyl)-1H-indazol-3-yl)-4,6-dihydropyrrolo[3,4-d]imidazol-5(1H)-yl)(5-morpholinepyrazin-2-yl)ketone (MDI-201)**

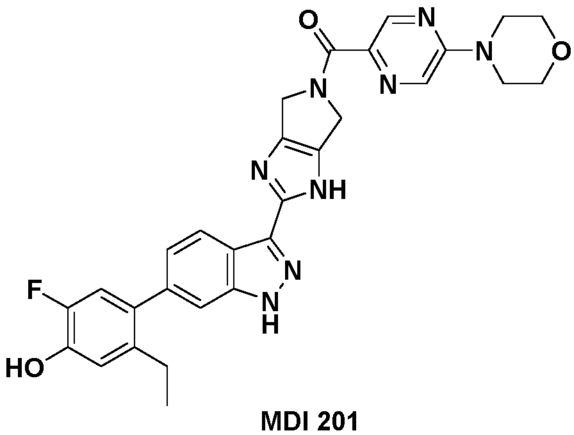

**MDI 201**

**Synthetic route of MDI-201:**

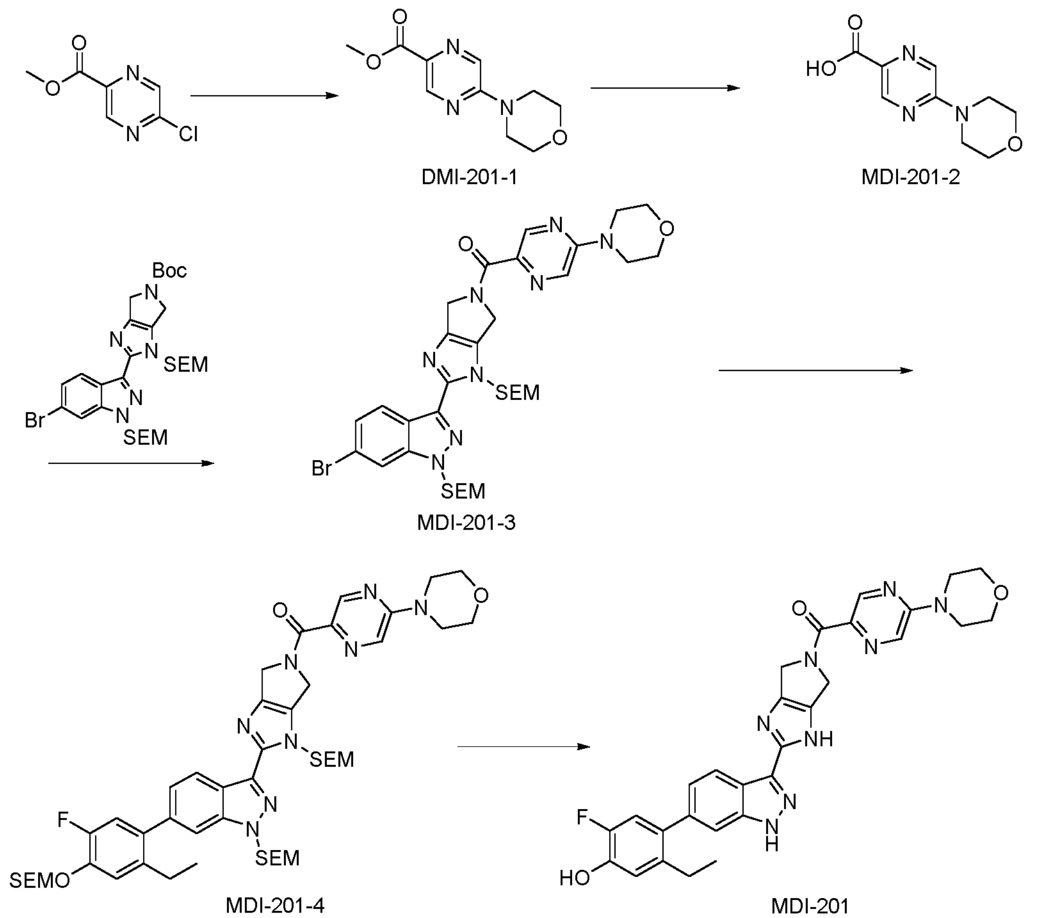

DMI-201-1

MDI-201-2

MDI-201-3

MDI-201-4

MDI-201

**Synthesis method:**

**Synthesis of intermediate MDI-201-1: Methyl 5-morpholine pyrazin-2-carboxylate**

Methyl 5-chloro-pyrazine-2-carboxylate (1.5 g, 8.7 mmol) was dissolved in 10ml DMF, and N,N-diisopropylethylamine (3.0 ml, 17.4 mmol) and morpholine (0.91 g, 10.4 mmol) were added. The mixture was stirred overnight at room temperature. Under vigorous stirring, water was added and a solid precipitated out, and filtered. The resulting filter cake was washed with water, and dried to afford the intermediate MDI-201-1 with a yield of 72.2%.

**Synthesis of intermediate MDI-201-2: 5-morpholinepyrazin-2-carboxylic acid**

The intermediate MDI-201-1 (1.4 g, 6.27 mmol) was dissolved in 20 ml of tetrahydrofuran and 20 ml of water, lithium hydroxide (0.32 g, 7.53 mmol) was added, and the reaction was carried out at room temperature for 4 hours. The reaction mixture was concentrated by distilling off tetrahydrofuran under reduced pressure and adjusted with 1N HCl to pH=4. A solid precipitated out, and filtered. The resulting filter cake was washed with water, and dried to afford the intermediate MDI-201-2 with a yield of 99.1%.

$^1$H NMR (400 MHz, CDCl3) δ 8.92 (s, 1H), 8.04 (s, 1H), 3.88-3.86 (m, 4H), 3.80-3.77 (m, 4H).

**Synthesis of intermediate MDI-201-3: (2-(6-bromo1-((2-(trimethylsilyl)ethoxy)methyl)-1H-indazol-3-yl)-1-((2-(trimethylsilyl)ethoxy)methyl)-4,6-dihydropyrrolo[3,4-d]imidazol-5(1H)-yl)(5-morpholinepyrazin-2-yl)ketone**

The intermediate MDI-201-2 (27.4 mg, 0.13 mmol) and N,N-diisopropylethylamine (46.0 mg, 0.36 mmol) was dissolved in DMF, to which HATU (67.8 mg, 0.18 mmol) was added. It was allowed to react at room temperature for 10 minutes. Intermediate tert-butyl 2-(6-bromo1-((2-(trimethylsilyl)ethoxy)methyl)-1H-indazol-3-yl)-1-((2-(trimethylsilyl)ethoxy)methyl)-4,6-dihydropyrrolo[3,4-d]imidazole-5(1H)-carboxylate (80 mg, 0.12 mmol) was dissolved in 5ml dichloromethane, to which 1ml of trifluoroacetic acid was added. The mixture was stirred at room temperature for 30 minutes, and concentrated to give a residue. The residue was dissolved in dichloromethane and concentrated to dryness, which

was repeated 3 times. The resulting residue was dissolved in DMF, and then slowly added to the previous reaction solution. It was allowed to react at room temperature overnight, and water was added to quench the reaction. The mixture was extracted twice with ethyl acetate and the organic phases were combined, washed with water and saturated brine, dried over anhydrous sodium sulfate, concentrated, and purified on a silica gel column to afford intermediate MDI-201-3 with a yield of 47.8%.

$^1$H NMR (400 MHz, $CDCl_3$) δ 8.91 (d, J = 8.0 Hz, 1H), 8.44-8.36 (m, 1H), 8.10 (d, J = 8.0 Hz, 1H), 7.80 (s, 1H), 7.46 -7.41 (m, 1H), 5.96 (s, 2H), 5.74 (d, J = 4.0 Hz, 2H), 5.27 (s, 1H), 5.19 (s, 1H), 5.00 (s, 1H), 4.92 ( s, 1H), 3.90-3.88 (m, 4H), 3.75-3.72 (m, 4H), 3.64-3.58 (m, 4H), 0.96-0.89 (m, 4H), 0.03 (s, 9H), 0.02 ( s, 9H).

**Synthesis of intermediate MDI-201-4: (2-(6-(2-ethyl-5-fluoro-4-((2-(trimethylsilyl)ethoxy)methyl)hydroxyphenyl)1-((2-(trimethylsilyl)ethoxy)methyl)-1H-indazol-3-yl)-1-((2-(trimethylsilyl)ethoxy)methyl)-4,6-dihydropyrrolo[3,4-d]imidazol-5(1H)-yl)(5-morpholinepyrazin-2-yl)ketone**

The intermediate MDI-201-3 (43.0 mg, 0.06 mmol), (2-((5-ethyl-2-fluoro-4-(4,4,5,5-tetramethyl-1,3,2-dioxaborolan-2-yl)phenoxy)methoxy)ethyl) trimethylsilane (27.1 mg, 0.07 mmol), Pd(dppf)Cl2 (4.2 mg, 0.006 mmol) and potassium phosphate ( 36.2 mg, 0.17 mmol) were dissolved in 1,4-dioxane (10ml) and water (2ml). The atmosphere was replaced with nitrogen, which was repeated 3 times. The mixture was heated to 100°C, reacted overnight, cooled to room temperature. Water was added and the resulting mixture was extracted with ethyl acetate twice. The organic phases were combined, washed with water and saturated brine, dried over anhydrous sodium sulfate, concentrated, and purified by silica gel column to afford intermediate MDI-201-4 with a yield of 40.9%.

$^1$H NMR (400 MHz, CDCl3) δ 8.91 (dd, J = 4.0 Hz, J = 4.0 Hz, 1H), 8.52 (dd, J = 8.0 Hz, J = 16.0 Hz, 1H), 8.10 (dd, J = 8.0 Hz, J = 4.0 Hz, 1H), 7.49 (s, 1H), 7.27 (s, 1H), 7.20 (d, J = 8.0 Hz, 1H), 7.06 (d, J = 12.0 Hz, 1H), 6.00 ( s, 2H), 5.79 (d, J = 4.0 Hz, 2H), 5.35 (s, 2H), 5.29 (s, 1H), 5.20 (s, 1H), 5.02(s, 1H), 4.94 (s, 1H) ), 3.91-3.86 (m, 6H), 3.76-3.72 (m, 4H), 3.65-3.61 (m, 4H), 2.58 (t, J = 8.0 Hz, 2H), 1.10-1.03 (m, 3H), 0.95-0.91 (m, 6H), 0.06 (s, 9H), 0.04 (s, 9H), 0.03 (s, 9H).

**Synthesis of MDI-201:(2-(6-(2-ethyl-5-fluoro-4-hydroxyphenyl)-1H-indazol-3-yl)-4,6-dihydropyrrolo[3,4-d]imidazol-5(1H)-yl)(5-morpholinepyrazin-2-yl)ketone**

The intermediate MDI-201-4 (22.0 mg, 0.02 mmol) was dissolved in methanol (4ml), to which concentrated hydrochloric acid (2ml) was added. The mixture was heated to 50°C, reacted for 6 hours, and concentrated. The resulting solid was dissolved with 1ml methanol, to which 2ml concentrated aqueous ammonia was added. The resulting mixture was concentrated to a residue. The residue was dissolved in methanol and concentrated to dryness, which was repeated 3 times. The resulting residue was and purified by a preparation plate to afford 8 mg of the final product, with a yield of 61.9%.

$^1$H NMR (400 MHz, DMSO-d6) δ 13.35 (s, 1H), 9.89 (s, 1H), 8.66 (d, J = 4.0 Hz, 1H), 8.38-8.33 (m, 2H), 7.42 (s, 1H), 7.15 (d, J = 8.0 Hz, 1H), 7.06 (d, J = 12.0 Hz, 1H), 6.95 (d, J = 8.0 Hz, 1H), 5.05 (s, 2H), 4.72 (s, 2H), 3.76-3.74 (m, 4H), 3.71-3.68 (m, 4H), 2.52 (dd, J = 12.0 Hz, J = 4.0 Hz, 2H), 1.05 (t, J = 8.0 Hz, 3H).

**Example 3: (2-(6-(2-ethyl-5-fluoro-4-hydroxyphenyl)-1H-indazol-3-yl)-4,6-dihydropyrrolo[3,4-d]imidazol-5(1H)-yl)(1-methyl-1H-pyrazol-4-yl)ketone (MDI-202)**

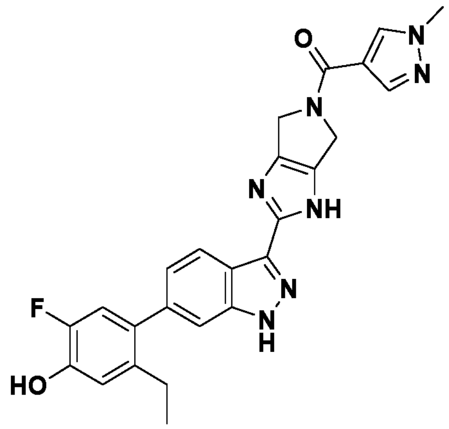

**MDI 202**

Synthetic route of MDI-202:

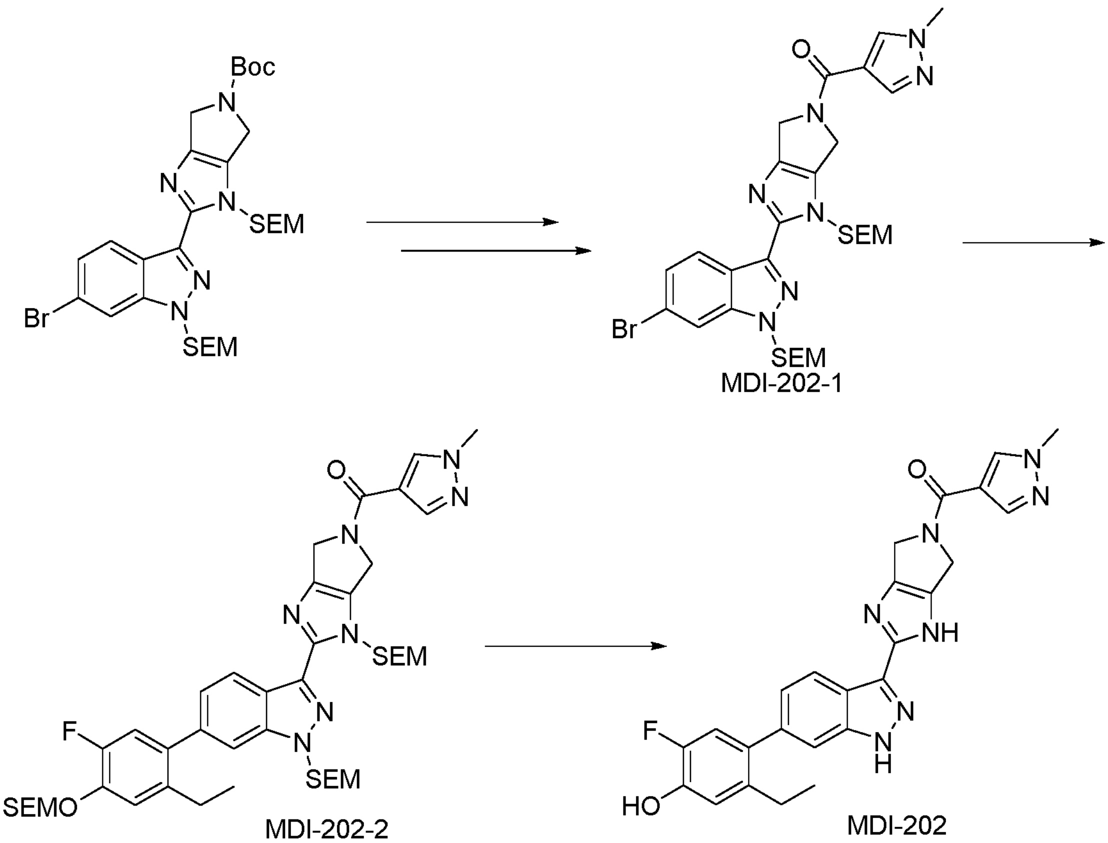

MDI-202-1

MDI-202-2

MDI-202

**Synthesis method:**

**Synthesis of intermediate MDI-202-1: (2-(6-bromo-1-((2-(trimethylsilyl)ethoxy)methyl)-1H-indazol-3-yl)-1-((2-(trimethylsilyl)ethoxy)methyl)-4,6-dihydropyrrolo [3,4-d] imidazol-5(1H)-yl)(1-methyl-1H-pyrazol-4-yl)ketone**

The intermediate 1-methyl-1H-pyrazole-4-carboxylic acid (16.5 mg, 0.13 mmol) and N,N-diisopropylethylamine (46.0 mg, 0.36 mmol) were dissolved in DMF, to which HATU (67.8 mg, 0.18 mmol) was added. It was allowed to react at room temperature for 10 minutes. Intermediate tert-butyl 2-(6-bromo-1-((2-(trimethylsilyl)ethoxy)methyl)-1H-indazol-3-yl)-1-((2-(trimethylsilyl)ethoxy)methyl)-4,6-dihydropyrrolo[3,4-d]imidazole-5(1H)-carboxylate (80 mg, 0.12 mmol) was dissolved in 5ml dichloromethane, to which 1ml of trifluoroacetic acid was added. The mixture was stirred at room

temperature for 30 minutes, and concentrated to give a residue. The residue was dissolved in dichloromethane and was concentrated to dryness, which was repeated 3 times. The resulting residue was dissolved in DMF and then slowly added to the previous reaction solution. It was allowed to react at room temperature overnight, and water was added to quench the reaction. The resulting mixture was extracted twice with ethyl acetate, and the organic phases were combined, washed with water and saturated brine, dried over anhydrous sodium sulfate, concentrated, and purified by silica gel column to afford intermediate MDI-202-1 with a yield of 41.3%.

$^1$H NMR (400 MHz, CDCl3) δ 8.43 (dd, J = 8.0 Hz, J = 20.0 Hz,1H), 7.98 (d, J = 4.0 Hz, 2H), 7.81(s, 1H), 7.45 (d, J = 8.0 Hz, 1H), 5.96 (s, 2H), 5.75 (s, 2H), 5.02-4.85 (m, 4H), 4.01 (s, 3H), 3.64-3.59 (m, 4H), 0.97-0.91 (m, 4H), 0.03 (s, 9H), 0.02 (s, 9H).

**Synthesis of intermediate MDI-202-2: (2-(6-(2-ethyl-5-fluoro-4-((2-(trimethylsilyl)ethoxy)methyl)hydroxyphenyl)1-((2-(trimethylsilyl)ethoxy)methyl)-1H-indazol-3-yl)-1-((2-(trimethylsilyl)ethoxy)methyl)-4,6-dihydropyrrolo[3,4-d]imidazol-5(1H)-yl)(1-methyl-1H-pyrazol-4-yl)ketone**

The intermediate MDI-202-1 (33 mg, 0.05 mmol), (2-((5-ethyl-2-fluoro-4-(4,4,5,5-tetramethyl-1,3,2-dioxaborolan-2-yl)phenoxy)methoxy)ethyl) trimethylsilane (23.3 mg, 0.06 mmol), Pd(dppf)$Cl_2$ (3.6 mg, 0.005 mmol) and potassium phosphate ( 31.3mg, 0.15 mmol) were dissolved in 1,4-dioxane (10ml) and water (2ml). The atmosphere was replaced with nitrogen, which was repeated 3 times and the mixture was heated to 100°C, reacted overnight, and cooled to room temperature. Water was added and the mixture was extracted with ethyl acetate twice. The organic phases were combined, washed with water and saturated brine, dried over anhydrous sodium sulfate, concentrated, and purified by silica gel column to afford intermediate MDI-202-2 with a yield of 85.0%.

$^1$H NMR (400 MHz, CDCl3) δ 8.48 (d, J = 8.0 Hz, 1H), 7.98 (d, J = 4.0 Hz, 2H), 7.49 (s, 1H), 7.20 (s, 1H), 7.18 (d, J = 8.0 Hz, 1H), 7.06 (d, J = 12.0 Hz, 1H), 6.00 (s, 2H), 5.79 (s, 2H), 5.35 (s, 2H), 5.04-4.87 (m, 4H) , 4.01 (s, 3H), 3.91 (t, J = 8.0 Hz, J = 20.0 Hz, 2H), 3.67-3.61 (m, 4H), 2.58 (d, J = 8.0 Hz, 2H), 1.11-1.07 (m, 3H), 0.95-0.91 (m, 6H), 0.06 (s, 9H), 0.03 (s, 9H), 0.02 (s, 9H).

**Synthesis of compound MDI-202: (2-(6-(2-ethyl-5-fluoro-4-hydroxyphenyl)-1H-indazol-3-yl)-4,6-dihydropyrrolo[3,4-d]imidazol-5(1H)-yl)(1-methyl-1H-pyrazol-4-yl)ketone**

The intermediate MDI-202-2 (36.0 mg, 0.04 mmol) was dissolved in methanol (4ml), to which concentrated hydrochloric acid (2ml) was added. The mixture was heated to 50°C, reacted for 6 hours, and concentrated. The solid was dissolved with 1ml methanol, to which 2ml of concentrated aqueous ammonia was added. The mixture was concentrated to give a residue. The residue was dissolved in methanol and was concentrated to dryness, which was repeated 3 times. The resulting residue was purified by a preparation plate to afford 5.0 mg of the final product with a yield of 25.4%.

$^1$H NMR (400 MHz, DMSO-d6) δ 13.33 (s, 1H), 12.87 (s, 1H), 9.89 (s, 1H), 8.35 (d, J = 8.0 Hz, 2H), 7.94 (s, 1H) , 7.42 (s, 1H), 7.15 (d, J = 8.0 Hz, 1H), 7.06 (d, J = 12.0 Hz, 1H), 6.95 (d, J = 12.0 Hz, 1H), 4.89(s, 2H) , 4.67 (s, 2H), 3.92 (s, 3H), 2.51-2.48 (m, 2H), 1.05 (t, J = 8.0 Hz, 3H).

**Example 4: (2-(6-(2-ethyl-5-fluoro-4-hydroxyphenyl)-1H-indazol-3-yl)pyrrolo[3,4-d]imidazol-5(1H,4H,6H)-yl)(1-methylpiperidin-4-yl)ketone (MDI-203)**

MDI-203 may also be named as 5-ethyl-2-fluoro-4-{3-[5-(1-methylpiperidin-4-carbonyl)-1H,4H,5H,6H-pyrrolo[3,4-d]imidazol-2-yl]-1H-indazol-6-yl}phenol.

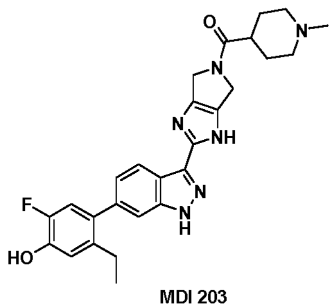

MDI 203

**Synthetic route of MDI-203:**

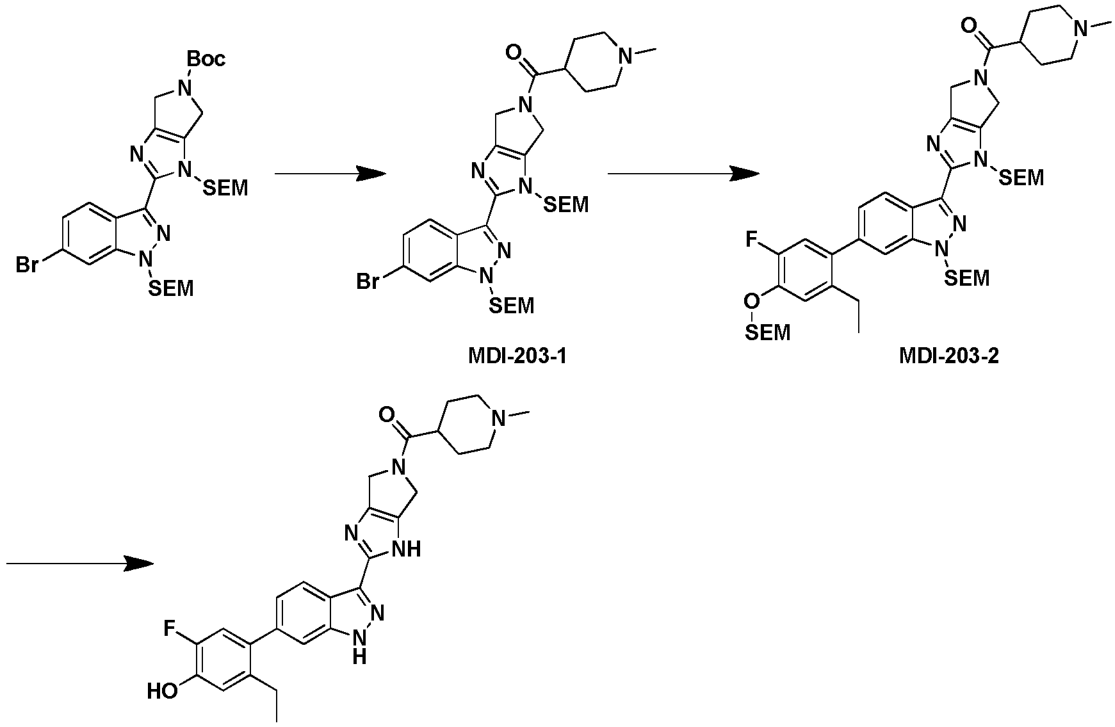

MDI-203-1

MDI-203-2

MDI 203

**Synthesis method:**

**Synthesis of intermediate MDI-203-1: (2-(6-bromo-1-((2-(trimethylsilyl)ethoxy)methyl)-1H-indazol-3-yl)-1-((2-(trimethylsilyl)ethoxy)methyl) pyrrolo[3,4-d]imidazol-5(1H,4H,6H)-yl)(1-methylpiperidin-4-yl)ketone**

1- methylpiperidine-4-carboxylic acid (18.6 mg, 0.13 mmol) and N,N-diisopropylethylamine (46.0 mg, 0.36 mmol) was dissolved in DMF, to which HATU (67.8 mg, 0.18 mmol) was added. It was allowed to react at room temperature for 10 minutes. Intermediate tert-butyl 2-(6-bromo-1-((2-(trimethylsilyl)ethoxy)methyl)-1H-indazol-3-yl)-1-((2-(trimethylsilyl)ethoxy)methyl)-4,6-dihydropyrrolo[3,4-d]imidazole-5(1H)-carboxylate (80 mg, 0.12 mmol) was dissolved in 5ml dichloromethane, to which 1ml of trifluoroacetic acid was added. The reaction mixture was stirred at room temperature for 30 minutes, and concentrated to give a residue. The residue was dissolved in dichloromethane and concentrated to dryness (to remove trifluoroacetic acid), which was repeated 3 times. Then the resulting residue was dissolved in DMF, which was slowly added to the previous reaction solution. It was allowed to react at room temperature overnight. Water was added to quench the reaction. The resulting mixture was extracted twice with ethyl acetate and the organic phases were combined, washed with water and saturated brine, dried over anhydrous sodium sulfate, concentrated, and purified by silica gel column to afford intermediate MDI-203-1 with a yield of 40.2%.

$^1$H NMR (400 MHz, CDCl3) δ8.29-8.22 (m,1H), 7.76 (s, 1H), 7.41-7.26 (m, 1H), 5.85 (s, 2H), 5.69 (s, 2H), 4.88 -4.59 (m, 4H), 3.63-3.54 (m, 6H), 3.21-2.81 (m, 5H), 2.28-2.01 (m, 4H), 0.93-0.83 (m, 5H), 0.03 (s, 9H) , 0.02 (s, 9H).

**Synthesis of intermediate MDI-203-2: (2-(6-(2-ethyl-5-fluoro-4-((2-(trimethylsilyl)ethoxy)methoxy)phenyl)1-((2-(trimethylsilyl)ethoxy)methyl)-1H-indazol-3-yl)-1-((2-(trimethylsilyl)ethoxy)methyl)pyrrolo[3,4-d]imidazol-5(1H, 4H,6H)-yl)(1-methylpiperidin-4-yl)ketone**

The intermediate MDI-203-1 (41.29 mg, 0.06 mmol), (2-((5-ethyl-2-fluoro-4-(4,4,5,5-tetramethyl-1,3,2-dioxaborolan-2-yl)phenoxy)methoxy)ethyl)trimethylsilane (27.1 mg, 0.07 mmol), Pd(dppf)Cl2 (4.2 mg, 0.006 mmol) and potassium phosphate ( 36.2 mg, 0.17 mmol) were dissolved in 1,4-dioxane (10ml) and water (2ml). The atmosphere was replaced with nitrogen, which was repeated 3 times. The mixture was heated to 100 °C, reacted overnight, and cooled to room temperature. Water was added and the resulting mixture was extracted with ethyl acetate twice. The organic phases were combined, washed with water and saturated brine, dried over anhydrous sodium sulfate, concentrated, and purified by silica gel column to afford intermediate MDI-203-2 with a yield of 40.9%.

$^1$H NMR (400 MHz, CDCl3) δ8.50-8.44 (m,1H),7.49 (s, 1H), 7.22 (dd, J = 12.0Hz,2H), 7.04 (d, J = 12.0Hz,1H), 5.98 (d, J = 12.0Hz,2H), 5.78(s, 2H), 5.34(s, 2H), 4.84-4.69 (m, 4H), 3.90-3.86 (m, 2H), 3.66-3.58 (m, 4H), 3.38-3.30

(m, 2H), 2.61-2.54 (m, 5H), 2.13-2.05 (m, 4H), 1.10-1.01 (m, 5H), 0.97-0.89 (m, 3H), 0.03 (s, 9H), 0.02 (s, 18H).

**Synthesis of compound MDI-203: (2-(6-(2-ethyl-5-fluoro-4-hydroxyphenyl)-1H-indazol-3-yl)pyrrolo[3,4-d]imidazol-5(1H,4H,6H)-yl)(1-methylpiperidin-4-yl)ketone**

Intermediate MDI-203-2 (26.4 mg, 0.03mmol) was dissolved in methanol (4ml), to which concentrated hydrochloric acid (2ml) was added. The mixture was heated to 50 °C, reacted for 6 hours, and concentrated. The solid was dissolved in 1ml methanol, to which 2ml concentrated ammonia water was added. It was concentrated to give a residue. The residue was dissolved in methanol and concentrated to dryness (to remove ammonia water), which was repeated 3 times. After separation, 5.0 mg of the final product was obtained with a yield of 34.2%.

$^1H$ NMR (400 MHz, DMSO-d6) $\delta$ 13.35 (s, 1H), 9.87 (s, 1H), 9.24 (s, 1H), 8.32 (d, J = 8.0 Hz, 1H), 7.42 (s, 1H) , 7.22 (d, J = 8.0 Hz, 1H), 7.03 (d, J = 12.0 Hz, 1H), 6.96 (d, J = 12.0 Hz, 1H), 4.80 (s, 2H), 4.48 (s, 2H) , 3.04-3.01 (m, 2H), 2.79 (s, 3H), 2.55-2.51 (m, 2H), 2.05-1.99 (m, 3H), 1.85-1.78 (m, 2H), 1.01-0.98 (m, 3H). The signals of the two H were masked by the water peak ($\delta$=3.37).

**Example 5: (2-(6-(2-ethyl-5-fluoro-4-hydroxyphenyl)-1H-indazol-3-yl)pyrrolo[3,4-d]imidazol-5(1H,4H,6H)-yl)(5-(4-methylpiperazin-1-yl)pyrazin-2-yl)ketone (MDI-204)**

MDI-204 may also be named as 5-ethyl-2-fluoro-4-{3-[5-(4-methylpiperazine-1-carbonyl)-1H,4H,5H,6H-pyrrolo[3,4-d]imidazol-2-yl]-1H-indazol-6-yl}phenol.

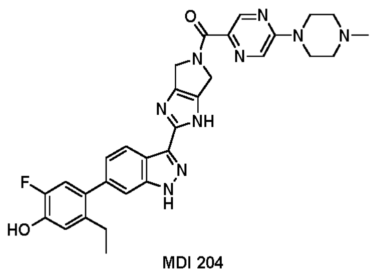

MDI 204

**Synthetic route of MDI-204:**

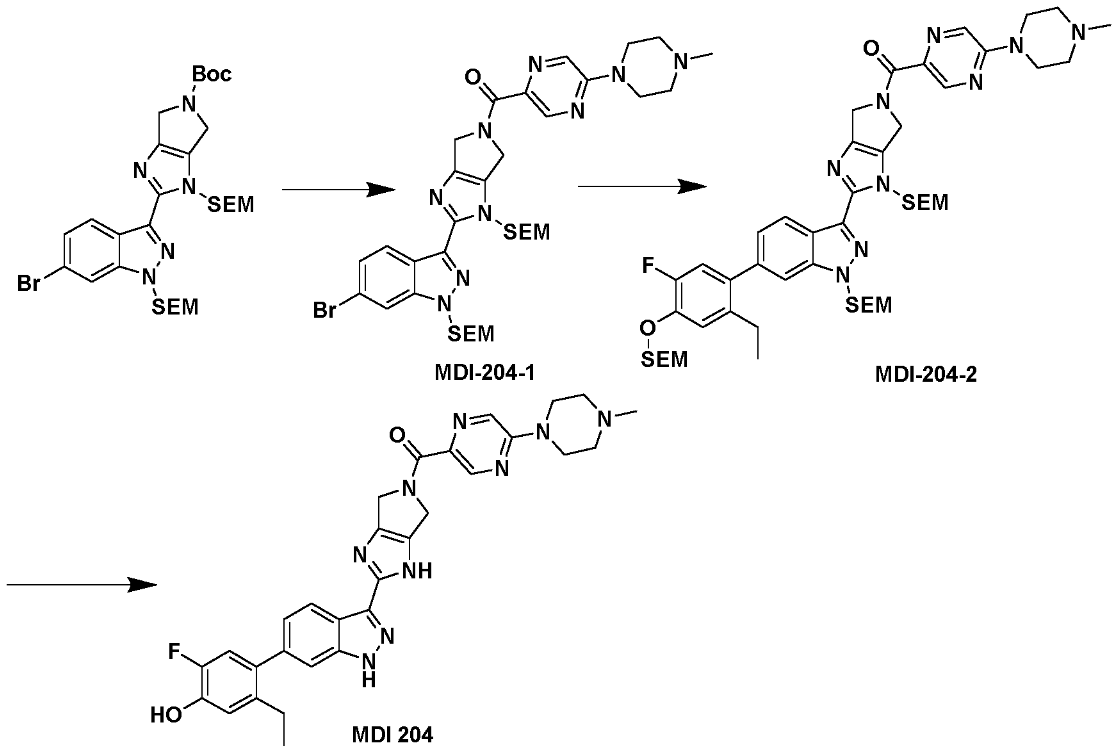

MDI-204-1

MDI-204-2

MDI 204

**Synthesis method:**

**Synthesis of intermediate MDI-204-1: (2-(6-bromol-((2-(trimethylsilyl) ethoxy)methyl)-1H-indazol-3-yl)-1-((2-(trimethylsilyl)ethoxy)methyl)pyrrolo[3,4-d]imidazol-5(1H, 4H,6H)-yl)(5-(4-methylpiperazin-1-yl)pyrazin-2-yl)ketone**

5-(4-methylpiperazin-1-yl)pyrazine-2-carboxylic acid (28.9 mg, 0.13 mmol) and N,N-diisopropylethylamine (46.0 mg, 0.36 mmol) were dissolved in DMF, to which HATU (67.8 mg, 0.18 mmol) was added. It was allowed to react at room temperature for 10 minutes. Intermediate tert-butyl 2-(6-bromo1-((2-(trimethylsilyl)ethoxy)methyl)-1H-indazol-3-yl)-1-((2-(trimethylsilyl ethoxy)methyl)-4,6-dihydropyrrolo[3,4-d]imidazole-5(1H)-carboxylate (80 mg, 0.12 mmol) was dissolved in 5ml dichloromethane, to which 1ml of trifluoroacetic acid was added. The reaction mixture was stirred at room temperature for 30 minutes, and concentrated to give a residue. The residue was dissolved in dichloromethane and concentrated to dryness (to remove trifluoroacetic acid), which was repeated 3 times. Then the resulting residue was dissolved in DMF, which was slowly added to the previous reaction solution. It was allowed to react at room temperature overnight. Water was added to quench the reaction, and the resulting mixture was extracted twice with ethyl acetate. The organic phases were combined, washed with water and saturated brine, dried over anhydrous sodium sulfate, concentrated, and purified by silica gel column to afford intermediate MDI-204-1 with a yield of 43%.

$^1$H NMR (400 MHz, CDCl3) δ8.86-8.84 (m,1H), 8.41-8.33 (m,1H), 8.07-8.05 (m,1H), 7.76 (d, J = 4.0Hz, 1H), 7.42-7.38 (m, 1H), 5.92 (s, 2H), 5.70(d, J = 4.0Hz, 2H), 5.23-4.88(m, 4H), 3.77-3.65 (m, 4H), 3.61-3.55 ( m, 4H), 2.55 (t, J = 4.0Hz, 4H), 2.37 (s, 3H), 0.94-0.83 (m, 4H), 0.03 (s, 9H), 0.02 (s, 9H).

**Synthesis of intermediate MDI-204-2: (2-(6-(2-ethyl-5-fluoro-4-((2-(trimethylsilyl)ethoxy)methoxy)phenyl)1-((2-(trimethylsilyl)ethoxy)methyl)-1H-indazol-3-yl)-1-((2-(trimethylsilyl)ethoxy)methyl)pyrrolo[3,4-d]imidazol-5(1H, 4H,6H)-yl)(5-(4-methylpiperazin-1-yl)pyrazin-2-yl)ketone**

The intermediate MDI-204-1 (46.0 mg, 0.06 mmol), (2-((5-ethyl-2-fluoro-4-(4,4,5,5-tetramethyl-1,3,2-dioxaborolan-2-yl)phenoxy)methoxy)ethyl)trimethylsilane (27.1 mg, 0.07 mmol), Pd(dppf)$Cl_2$ (4.2 mg, 0.006 mmol) and potassium phosphate ( 36.2 mg, 0.17 mmol) were dissolved in 1,4-dioxane (10ml) and water (2ml). The atmosphere was replaced with nitrogen, which was repeated 3 times. The mixture was heated to 100 °C, reacted overnight, and cooled to room temperature. Water was added and the resulting mixture was extracted with ethyl acetate twice. The organic phases were combined, washed with water and saturated brine, dried over anhydrous sodium sulfate, concentrated, and purified by silica gel column to afford intermediate MDI-204-2 with a yield of 47.2%.

$^1$H NMR (400 MHz, CDCl3) δ8.86-8.84 (m,1H), 8.51-8.43 (m,1H), 8.08-8.06 (m,1H), 7.45 (d, J = 4.0Hz, 1H),

7.26 -7.23 (m, 1H), 7.21-7.14 (m, 1H), 7.02 (d, J = 8.0Hz, 1H), 5.97 (s, 2H), 5.75(d, J = 4.0Hz, 2H), 5.32 ( s, 2H), 5.25-5.16 (m, 2H), 4.99-4.90 (m, 2H), 3.87-3.83 (m, 2H), 3.77-3.74 (m, 4H), 3.63-3.56 (m, 4H), 2.56-2.51 (m, 6H), 2.37 (s, 3H), 1.07-1.01 (m, 3H), 0.99-0.88 (m, 6H), 0.03 (s, 9H),-0.07(s, 9H),- 0.09 (s, 9H).

**Synthesis of compound MDI-204: (2-(6-(2-ethyl-5-fluoro-4-hydroxyphenyl)-1H-indazol-3-yl)pyrrolo[3,4-d]imidazol-5(1H,4H,6H)-yl)(5-(4-methylpiperazin-1-yl)pyrazin-2-yl)ketone**

Intermediate MDI-204-2 (28.7 mg, 0.03mmol) was dissolved in methanol (4ml), to which concentrated hydrochloric acid (2ml) was added. The mixture was heated to 50 °C, reacted for 6 hours, and concentrated. The resulting solid was dissolved in 1ml methanol, to which 2ml concentrated ammonia water was added. It was concentrated to give a residue. The residue was dissolved in methanol and concentrated to dryness (to remove ammonia water), which was repeated 3 times. After separation, 4.0 mg of the final product was obtained with a yield of 23.51%.

$^1$H NMR (400 MHz, DMSO-d6) δ 13.29 (s, 1H), 12.79 (d, J =16.0 Hz, 1H), 9.85 (s, 1H), 8.62 (s, 1H), 8.36 (s, 1H) ,8.34-8.30 (m, 1H),7.40 (s, 1H), 7.14-7.10 (m, 1H), 7.03 (d, J = 12.0 Hz, 1H), 6.92 (d, J = 12.0 Hz, 1H), 5.08-4.65 (m, 4H), 2.55-2.49 (m, 6H), 2.24 (s, 3H), 2.03-1.97 (m, 4H), 1.04-1.02 (m, 3H).

**Example 6: (2-(6-(2-ethyl-4-hydroxyphenyl)-1H-indazol-3-yl)pyrrolo[3,4-d]imidazol-5(1H,4H,6H)-yl)(5-(4-methylpiperazin-1-yl)pyrazin-2-yl)ketone (MDI-205)**

MDI-205 may also be named as 3-ethyl-4-{3-[5-(4-methylpiperazine-1-carbonyl)-1H,4H, SH, 6H-pyrrolo[3,4-d]imidazol-2-yl]-1H-indazol-6-yl }phenol.

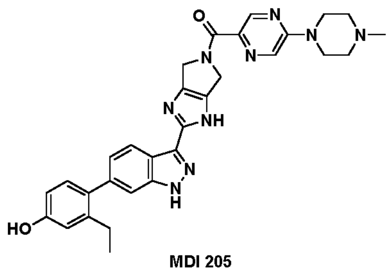

MDI 205

**Synthetic route of MDI-205:**

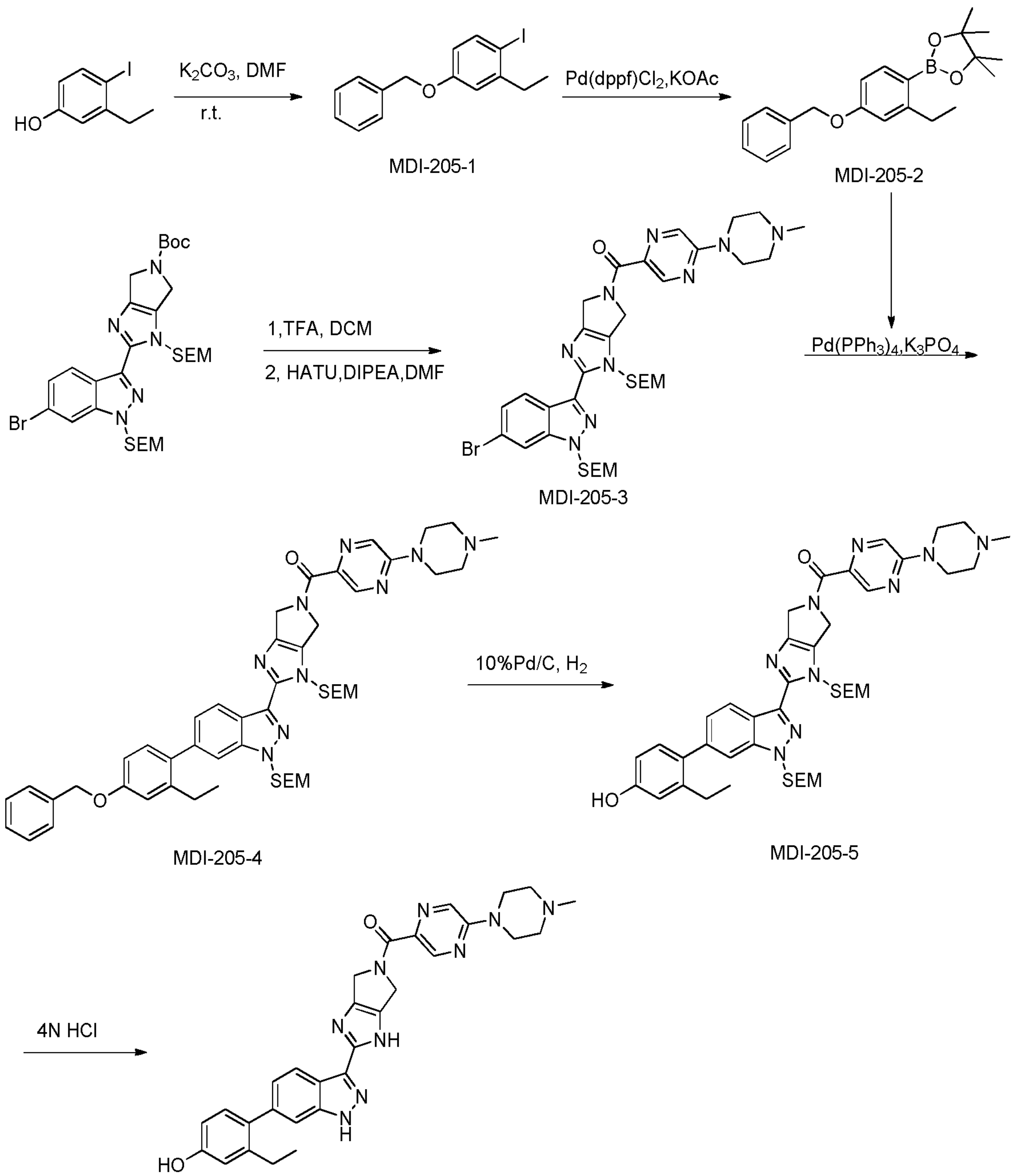


MDI-205-1

MDI-205-2

MDI-205-3

MDI-205-4

MDI-205-5

MDI-205

**Synthesis method:**

**Synthesis of intermediate MDI-205-1: 4-benzyloxy-2-ethyl-iodobenzene**

3-ethyl-4-iodophenol (200 mg, 0.81mmol), benzyl bromide (165.5mg, 0.97mmol) and potassium carbonate (222.9mg, 1.61mmol) were dissolved in DMF. It was allowed to react at room temperature for two hours. Water was added and the resulting mixture was extracted twice with EA. The organic phases were combined, washed with water, saturated brine, dried over anhydrous sodium sulfate, concentrated, and purified by silica gel column to afford 250 mg of colorless oily product with a yield of 91.7%.

$^1$H NMR (400 MHz, CDCl3) $\delta$7.70 (d, J = 8.0 Hz, 1H), 7.46-7.36(m, 5H), 6.92 (d, J = 4.0 Hz, 1H), 6.57 (dd, J = 4.0 Hz, J =8.0 Hz, 1H), 5.06 (s, 2H), 2.72 (dd, J = 8.0 Hz, J =16.0 Hz, 2H), 1.22 (t, J = 8.0 Hz, 3H).

**Synthesis of intermediate MDI-205-2: (2-(4-(phenoxy)-2-ethyl phenyl)-4,4,5,5-tetramethyl-1,3,2-dioxaborane**

MDI-205-1 (250.0mg, 0.74mmol), pinacol borate (225.1mg, 0.89mmol), Pd(dppf)$Cl_2$ (54.0mg, 0.07mmol) and KOAc (217.6mg, 2.22mmol) were dissolved in 1,4-dioxane (10 ml). The atmosphere was replaced with nitrogen, which was repeated 3 times. The mixture was heated to 100°C and reacted overnight. After the reaction was completed, it was quenched with water, extracted with ethyl acetate, and the organic phase was washed with saturated sodium chloride solution, and dried over anhydrous sodium sulfate. It was concentrated and separated by column chromatography to afford a colorless oil with a yield of 70%.

$^1$H NMR (400 MHz, CDCl3) δ 7.77 (d, J = 8.0 Hz, 1H), 7.47-7.34(m, 5H), 6.86-6.80 (m, 2H), 5.11 (s, 2H), 2.93 (dd, J = 8.0 Hz, J =16.0 Hz, 2H), 1.35 (s, 12H), 1.21 (t, J = 8.0 Hz, 3H).

**Synthesis of compound MDI-205-3: (2-(6-bromo-1-((2-(trimethylsilyl)ethoxy)methyl)-1H-indazol-3-yl)-1-((2-(trimethylsilyl)ethoxy)methyl) pyrrolo [3,4-d] imidazol-5(1H,4H,6H)-yl)(5-(4-methylpiperazin-1-yl)pyrazin-2-yl)ketone**

The intermediate 5-(4-methylpiperazin-1-yl)pyrazine-2 carboxylic acid (64.2 mg, 0.29mmol) and N,N-diisopropylethylamine (93.2 mg, 0.72mmol) were dissolved in DMF, to which HATU (109.7 mg, 0.29 mmol) was added. It was allowed to react at room temperature for 10 minutes. Intermediate tert-butyl 2-(6-bromo1-((2-(trimethylsilyl)ethoxy)methyl)-1H-indazol-3-yl)-1-((2-(trimethylsilylethoxy)methyl)-4,6-dihydropyrrolo[3,4-d]imidazole-5(1H)-carboxylate (160.0 mg, 0.24mmol) was dissolved in 5ml dichloromethane, to which 1ml of trifluoroacetic acid was added. The reaction mixture was stirred at room temperature for 30 minutes, and concentrated to give a residue. The residue was dissolved in dichloromethane and concentrated to dryness (to remove trifluoroacetic acid), which was repeated 3 times. Then the resulting residue was dissolved in DMF, which was slowly added to the previous reaction solution. It was allowed to react at room temperature overnight. Water was added to quench the reaction, and the resulting mixture was extracted twice with ethyl acetate. The organic phases were combined, washed with water and saturated brine, dried over anhydrous sodium sulfate, concentrated, and purified by silica gel column to afford intermediate MDI-205-3 with a yield of 49.2%.

$^1$H NMR (400 MHz, CDCl3) δ 8.88 (dd, J = 8.0 Hz, J = 4.0 Hz 1H), 8.42 (dd, J = 8.0 Hz, J = 20.0 Hz 1H), 8.10 (dd, J = 8.0 Hz, J = 4.0 Hz 1H), 7.80 (s, 1H), 7.41-7.46 (m, 1H), 5.96 (s, 2H), 5.74 (d, J = 4.0 Hz, 2H), 5.27 (s, 1H), 5.19 (s, 1H), 4.99 (s, 1H), 4.91 (s, 1H), 3.80-3.78 (m, 4H), 3.63-3.59 (m, 4H), 2.59-2.56 (m, 4H), 2.40 (s , 3H), 0.97-0.91(m, 4H), 0.03(s, 9H), 0.02(s, 9H).

**Synthesis of compound MDI-205-4: (2-(6-(4-(phenoxy)-2-ethyl-phenyl)1-((2-(trimethylsilyl)ethoxy)methyl)-1H-indazol-3-yl)-1-((2-(trimethylsilyl)ethoxy) methyl)pyrrolo[3,4-d]imidazol-5(1H,4H,6H)-yl)(5-(4-methylpiperazin-1-yl)pyrazin-2-yl)ketone**

Intermediate MDI-205-3 (91.0 mg, 0.12mmol), intermediate MDI-205-2 (48.1 mg, 0.14mmol), Pd(PPh3)4 (13.6 mg, 0.01mmol) and potassium phosphate (75.4 mg, 0.36mmol) were dissolved in 1,4-dioxane (20ml) and water (4ml). The atmosphere was replaced with nitrogen, which was repeated 3 times. The mixture was heated to 100°C, reacted overnight, and cooled to room temperature. Water was added, and the resulting mixture was extracted 2 times with ethyl acetate. The organic phases were combined, washed with water and saturated brine, dried over anhydrous sodium sulfate, concentrated, and purified by silica gel column to afford intermediate MDI-205-4 with a yield of 44.1%.

$^1$H NMR (400 MHz, CDCl3) δ 8.88 (dd, J = 8.0 Hz, J = 4.0 Hz 1H), 8.46 (dd, J = 4.0 Hz, J = 8.0 Hz 1H), 8.11 (dd, J = 8.0 Hz, J = 4.0 Hz 1H), 7.52-7.31 (m, 6H), 7.27-7.22 (m, 2H), 7.00 (d, J = 4.0 Hz, 1H), 6.91 (dd, J = 4.0 Hz, J = 8.0 Hz, 1H),6.00 (s, 2H), 5.77 (d, J = 4.0 Hz, 2H), 5.20 (s, 1H), 5.19 (s, 1H), 5.15 (s, 2H), 5.01 (s, 1H) , 4.93 (s, 1H), 3.81-3.77 (m, 4H), 3.65-3.61 (m, 4H), 2.62-2.57 (m, 6H), 2.40 (s, 3H), 1.10 (t, J = 8.0 Hz , 3H), 0.95-0.91(m, 4H), 0.03(s, 9H), 0.02(s, 9H).

**Synthesis of compound MDI-205-5: (2-(6-(2-ethyl-4-hydroxyphenyl)1-((2-(trimethylsilyl)ethoxy)methyl)-1H-indazol-3-yl)-1-((2-(trimethylsilyl)ethoxy)methyl) pyrrolo [3,4-d] imidazol-5(1H,4H,6H)-yl)(5-(4-methylpiperazin-1-yl)pyrazin-2-yl)ketone**

Intermediate MDI-205-4 (47.0 mg, 0.05 mmol) was dissolved in 10ml methanol, to which 5mg 10% Pd/C was added. The atmosphere was replaced with hydrogen, which was repeated three times. It was allowed to react at room temperature overnight. The palladium on carbon was filtered off and the filtrate was concentrated to afford intermediate MDI-205 -5 with a yield of 78.0%, which was directly used in the next step.

**Synthesis of compound MDI-205: (2-(6-(2-ethyl-4-hydroxyphenyl)-1H-indazol-3-yl)pyrrolo[3,4-d]imidazol-5(1H,4H,6H)-yl)(5-(4-methylpiperazin-1-yl)pyrazin-2-yl)ketone**

The intermediate MDI-205-5 (33.0mg, 0.04mmol) was dissolved in 4ML methanol, to which 2ml concentrated hydrochloric acid was added. The mixture was heated to 50°C, reacted for 6 hours, and concentrated to give a residue. The residue was dissolved in methanol and was concentrated to dryness (to remove hydrochloric acid), which was repeated 3 times. The resulting product was dissolved 1ml methanol and 2ml aqueous ammonia was added for neutralization, and the resulting mixture was concentrated to give a residue. The residue was dissolved in methanol and was concentrated to dryness (to remove aqueous ammonia), which was repeated 2 times. The obtained product was purified by a preparation plate to afford 6.2 mg of the product with a yield of 27.7%.

$^1$H NMR (400 MHz, MeOD-d4) δ 8.72 (d, J = 4.0 Hz, 1H), 8.28 (dd, J = 4.0 Hz, J = 8.0 Hz,2H), 7.40 (s, 1H), 7.18 (dd , J = 4.0 Hz, J = 8.0 Hz, 1H), 7.09 (d, J = 8.0 Hz, 1H), 6.80 (d, J = 4.0 Hz, 1H), 6.72-6.69 (m, 1H), 5.17 (s , 2H), 4.85 (s, 2H), 3.83-3.81 (m, 4H), 2.67-2.64 (m, 4H), 2.60 (dd, J = 4.0 Hz, J = 8.0 Hz, 2H), 2.43 (s, 3H), 1.10 (t, J = 8.0 Hz, 3H).

**Example 7: 5-ethyl-2-fluoro-4-(3-(5-(benzenesulfonyl)-1,4,5,6-tetrahydro pyrrolo[3,4-d]imidazol-2-yl)-1H-indazol-6-yl)phenol (MDI-206)**

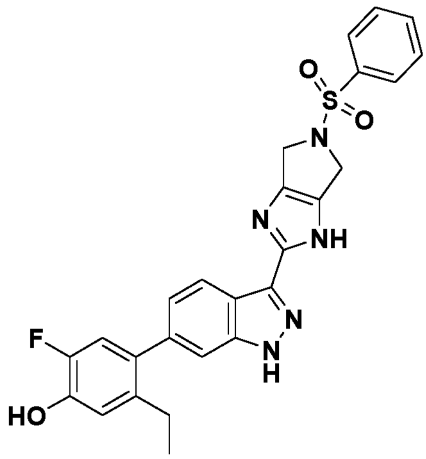

**MDI 206**

**Synthetic route of MDI-206:**

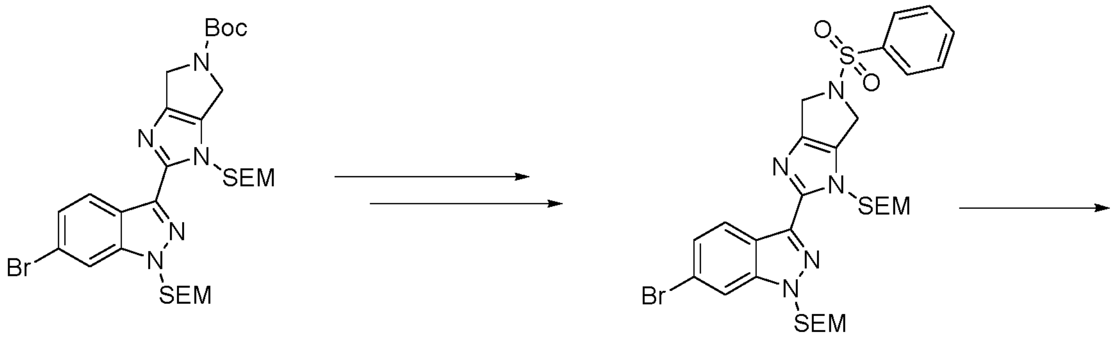

MDI-206-1

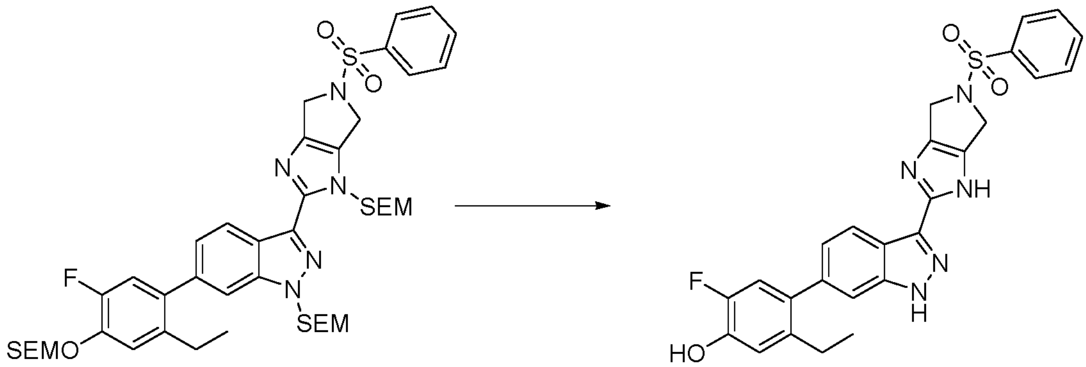

MDI-206-2 MDI-206

**Synthesis method:**

**Synthesis of intermediate MDI-206-1: (6-bromo-3-(5-(benzenesulfonyl)-1-(2-(trimethylsilyl)ethoxy)methyl)-1, 4, 5, 6-tetrahydropyrrolo[3,4-d]imidazol-2-yl)-1-((2-(trimethylsilyl)ethoxy)methyl)-1H-indazole**

Tert-butyl 2-(6-bromo-1-((2-(trimethylsilyl)ethoxy)methyl)-1H-indazol-3-yl)-1-((2-(trimethylsilyl)ethoxy)methyl)-4,6-dihydropyrrolo[3,4-d]imidazole-5(1H)-carboxylate (100 mg, 0.15 mmol) was dissolved in 5ml dichloromethane, to which 1ml of trifluoroacetic acid was added. The mixture was stirred at room temperature for 30 minutes, and concentrated to give a residue (to remove trifluoroacetic acid). The residue was dissolved in dichloromethane and was concentrated to dryness, which was repeated 3 times. The resulting residue was dissolved in 5ML DCM and $Et_3N$ (0.08ml, 0.59mmol), cooled to 0 °C, and benzenesulfonyl chloride (28.6 mg, 0.16 mmol) was slowly added. It was allowed to react at room temperature for 2 hours, and water was added to quench the reaction. The resulting mixture was extracted twice with DCM, and the organic phases were combined, washed with water and saturated brine, dried over anhydrous sodium sulfate, concentrated, and purified by silica gel column to afford intermediate MDI -206-1 with a yield of 41.4%.

$^1H$ NMR (400 MHz, CDCl3) δ 8.32 (d, J = 8.0 Hz, 1H), 7.95 (d, J = 8.0 Hz, 2H), 7.77 (s, 1H), 7.62-7.55 (m, 3H), 7.42 (d, J = 8.0 Hz, 1H), 5.85 (s, 2H), 5.70 (s, 2H), 4.66-4.58(m, 4H), 3.59-3.51 (m, 4H), 0.94-0.87 (m, 4H), 0.03 (s, 18H).

**Synthesis of intermediate MDI-206-2: (6-(2-ethyl-5-fluoro-4-((2-(trimethylsilyl)ethoxy)methyl)hydroxyphenyl)-3-(5-benzenesulfonyl)-1-((2-(trimethylsilyl)ethoxy)methyl)-1,4,5,6-tetrahydropyrrolo [3,4-d] imidazol-2-yl)-1-((2-(trimethylsilyl)ethoxy)methyl)-1H-indazole**

The intermediate MDI-206-1 (53.0 mg, 0.08 mmol), (2-((5-ethyl-2-fluoro-4-(4,4,5,5-tetramethyl-1,3,2-dioxaborolan-2-yl)phenoxy)methoxy)ethyl)trimethylsilane (35.8 mg, 0.09 mmol), Pd(dppf)$Cl_2$ (5.5 mg, 0.008 mmol) and potassium phosphate ( 47.9mg, 0.23 mmol) were dissolved in 1,4-dioxane (10ml) and water (2ml). The atmosphere was replaced with nitrogen, which was repeated 3 times. The mixture was heated to 100 °C, reacted overnight, and cooled to room temperature. Water was added and the resulting mixture was extracted with ethyl acetate twice and the organic phases were combined, washed with water and saturated brine, dried over anhydrous sodium sulfate, concentrated, and purified on a silica gel column to afford intermediate MDI-206-2 with a yield of 74.3%.

$^1H$ NMR (400 MHz, CDCl3) δ 8.42 (d, J = 8.0 Hz, 1H), 7.96-7.94 (m, 2H), 7.62-7.57 (m, 3H), 7.46 (s, 1H), 7.25 (d, J = 8.0 Hz, 1H), 7.19 (d, J = 8.0 Hz, 1H), 7.04 (d, J = 12.0 Hz, 1H), 5.89 (s, 2H), 5.75 (s, 2H), 5.34 (s, 2H), 4.67-4.60

(m, 4H), 3.90-3.86 (m, 2H), 3.62-3.51 (m, 4H), 2.58 (dd, J = 8.0 Hz, J = 16.0 Hz, 2H), 1.09-1.05 (m, 3H), 0.93-0.88 (m, 6H), 0.06 (s, 9H), 0.03 (s, 9H), 0.02 (s, 9H).

**Synthesis of compound MDI-206: 5-ethyl-2-fluoro-4-(3-(5-(benzenesulfonyl)-1,4,5,6-tetrahydropyrrolo[3,4-d]imidazol-2-yl)-1H-indazol-6-yl)phenol**

Intermediate MDI-206-2 (50.0 mg, 0.06 mmol) was dissolved in methanol (4ml), to which concentrated hydrochloric acid (2ml) was added. The mixture was heated to 50°C, reacted for 6 hours, and concentrated. The resulting solid was dissolved in 1ml methanol, and pH was adjusted with sodium bicarbonate solution to 8-9. The resulting mixture was extracted 4 times with dichloromethane, and the organic phases were combined, dried over anhydrous sodium sulfate, and purified by a preparation plate to afford 13 mg of the final product with a yield of 46.2%.

$^1H$ NMR (400 MHz, MeOD-d4) δ 8.22 (d, J = 8.0 Hz, 1H), 7.98-7.96 (m, 2H), 7.69-7.65 (m, 3H), 7.41(s, 1H), 7.16 ( d, J = 8.0 Hz, 1H), 6.96-6.89 (m, 2H), 4.61-4.52 (m, 4H), 2.57 (dd, J = 16.0 Hz, J = 8.0 Hz, 2H), 1.08 (t, J = 8.0 Hz, 3H).

**Example 8: 5-ethyl-2-fluoro-4-(3-(5-(pyrazin-2ylmethyl)-1,4,5,6-tetrahydropyrrolo[3,4-d]imidazol-2-yl)-1H-indazol-6-yl)phenol (MDI-207)**

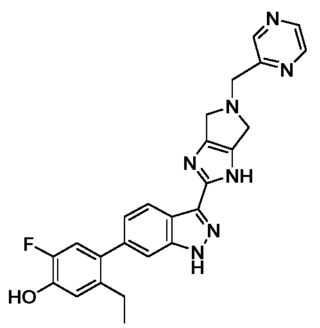

MDI 207

**Synthetic route of MDI-207:**

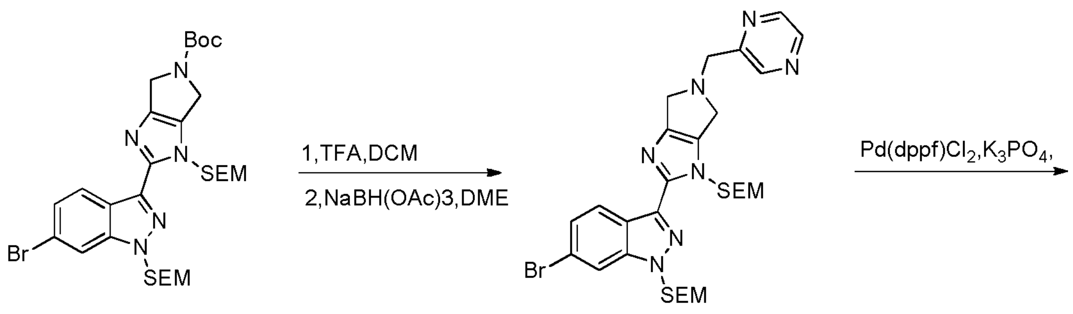


MDI-207-1

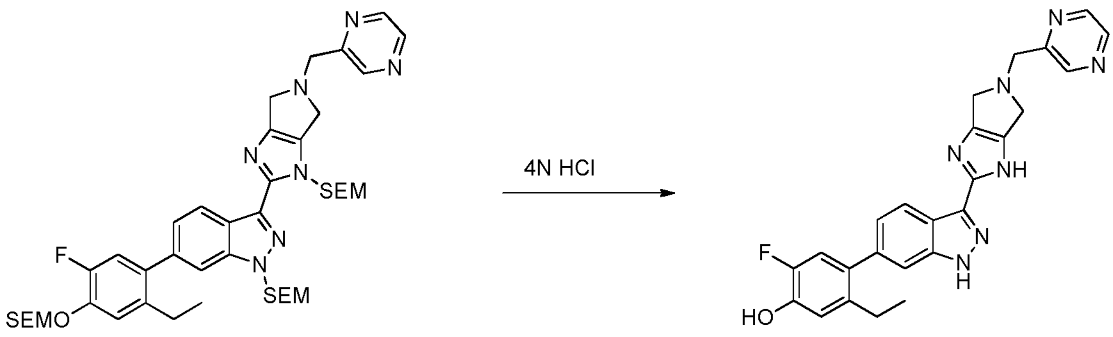


MDI-207-2

MDI-207

**Synthesis method:**

**Synthesis of intermediate MDI-207-1: (6-bromo-3-(5-(pyrazin-2ylmethyl)1-((2-(trimethylsilyl)ethoxy)methyl)-1,4,5,6-tetrahydropyrrolo[3,4-d] imidazol-2-yl)-1-((2-(trimethylsilyl)ethoxy)methyl)-1H-indazol**

Tert-butyl 2-(6-bromo-1-((2-(trimethylsilyl)ethoxy)methyl)-1H-indazol-3-yl)-1-((2-(trimethylsilyl)ethoxy)methyl)-4,6-dihydropyrrolo[3,4-d]imidazole-5(1H)-carboxylate (47.0mg, 0.07mmol) was dissolved in 5ml dichloromethane, to which 1ml of trifluoroacetic acid was added. The mixture was stirred at room temperature for 30 minutes. Water was added and saturated sodium bicarbonate solution was used to adjust pH=9. The resulting mixture was extracted with DCM. The organic phase was washed with water and saturated brine, dried over anhydrous sodium sulfate, and concentrated. The resulting solid was dissolved in 1,2-dichloroethane, to which 2-pyrazinecarboxaldehyde (30.6mg, 0.28mmol) was added. The mixture was stirred at room temperature for 1 hour, to which sodium triacetylborohydride (60.0mg, 0.28mmol) was added. It was allowed to react at room temperature for 4 hours, and water was added to quench the reaction. The resulting mixture was extracted with DCM twice, and the organic phases were combined, washed with water and saturated brine, dried over anhydrous sodium sulfate, concentrated, and purified by silica gel column to afford the product MDI-207-1 with a yield of 49.5%.

$^1$H NMR (400 MHz, CDCl3) δ 8.81 (d, J = 4.0 Hz, 1H), 8.60 (dd, J = 8.0 Hz, J = 4.0 Hz, 1H), 8.54 (d, J = 4.0 Hz, 1H), 8.39 (d, J = 8.0 Hz, 1H), 7.77 (d, J = 4.0 Hz, 1H), 7.41 (dd, J = 8.0 Hz, J = 4.0 Hz, 1H), 5.88 (s, 2H), 5.72 (s, 2H), 4.28 (s, 2H), 4.12 (dd, J = 4.0 Hz, J = 12.0 Hz, 4H), 3.62-3.55 (m, 4H), 0.96-0.87 (m, 4H), 0.03(s , 9H), 0.02(s, 9H).

**Synthesis of intermediate MDI-207-2: (6-(2-ethyl-5-fluoro-4-((2-(trimethylsilyl)ethoxy)methoxy)phenyl)-3-(5-(pyrazin-2ylmethyl)1-((2-(trimethylsilyl)ethoxy)methyl)-1,4,5,6-tetrahydropyrrolo [3,4-d] imidazol-2-yl)-1-((2-(trimethylsilyl)ethoxy)methyl)-1H-indazole**

The intermediate MDI-207-1 (20.0 mg, 0.03 mmol), the intermediate (2-((5-ethyl-2-fluoro-4-(4,4,5,5-tetramethyl-1,3,2-dioxaborolan-2-yl)phenoxy)methoxy)ethyl) trimethylsilane (14.5 mg, 0.04mmol), Pd(dppf)$Cl_2$ (2.3 mg, 0.003mmol) and potassium phosphate (19.4 mg, 0.09mmol) were dissolved in 1,4-dioxane (10ml) and water (2ml). The atmosphere was replaced with nitrogen, which was repeated 3 times. The mixture was heated to 100 °C, reacted overnight, and cooled to room temperature. Water was added and the resulting mixture was extracted with ethyl acetate twice, the organic phases were combined, washed with water and saturated brine, dried over anhydrous sodium sulfate, concentrated, and purified by silica gel column to afford intermediate MDI-207-2 with a yield of 93.0%.

$^1$H NMR (400 MHz, CDCl3) δ 8.82 (d, J = 4.0 Hz, 1H), 8.60 (dd, J = 8.0 Hz, J = 4.0 Hz, 1H), 8.54 (d, J = 4.0

Hz, 1H), 8.48 (d, J = 8.0 Hz, 1H), 7.47 (d, J = 4.0 Hz, 1H), 7.23 (dd, J = 8.0 Hz, J = 4.0 Hz, 1H), 7.19 (d, J = 8.0 Hz, 1H), 7.05 (d, J = 12.0 Hz, 1H), 5.92 (s, 2H), 5.77 (s, 2H), 5.34 (s, 2H), 4.30 (s, 2H), 4.13 (dd, J = 4.0 Hz, J = 12.0 Hz, 4H), 3.88-3.86 (m, 2H), 3.63-3.58 (m, 4H), 2.57 (d, J = 8.0 Hz,2H), 1.07 (t, J = 8.0 Hz, 3H), 0.92-0.90 (m, 6H), 0.06(s, 9H), 0.03(s, 9H), 0.02(s, 9H).

**Synthesis of compound MDI-207: 5-ethyl-2-fluoro-4-(3-(5-(pyrazin-2ylmethyl)-1,4,5,6-tetrahydropyrrolo[3,4-d]imidazol-2-yl)-1H-indazol-6-yl)phenol**

The intermediate MDI-207-2 (24.0mg, 0.03mmol) was dissolved in 4ML methanol, to which 2ml concentrated hydrochloric acid was added. The mixture was heated to 50°C, reacted for 6 hours, and concentrated to give a residue. The residue was dissolved in methanol and was concentrated to dryness, which was repeated 3 times. The resulting product was dissolved 1ml methanol and 2ml aqueous ammonia was added for neutralization, and the resulting mixture was concentrated to give a residue. The residue was dissolved in methanol and was concentrated to dryness, which was repeated 2 times. The obtained product was purified by a preparation plate to afford 8 mg of the product with a yield of 61.8%.

$^1$H NMR (400 MHz, MeOD-d4) δ 8.80 (d, J = 4.0 Hz, 1H), 8.65 (dd, J = 4.0 Hz, J = 4.0 Hz, 1H), 8.56 (d, J = 4.0 Hz, 1H ), 8.26 (dd, J = 4.0 Hz, J = 4.0 Hz, 1H), 7.41 (d, J = 4.0 Hz, 1H), 7.17(dd, J= 12.0 Hz, J = 4.0 Hz, 1H), 6.91-6.89(m, 2H), 4.30 (s, 2H), 4.07 (s, 4H), 2.56 (dd, J = 8.0 Hz, J = 16.0 Hz, 2H), 1.07 (t, J = 8.0 Hz, 3H).

**Example 9: 4-(3-(5-(cyclopropylmethyl)-1,4,5,6-tetrahydropyrrolo[3,4-d]imidazol-2-yl)-1H-indazol-6-yl)-5-ethyl-2-fluorophenol (MDI-208)**

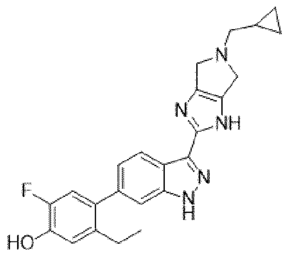

MDI-208

Intermediate 22 was first synthesized according to the following synthetic route.

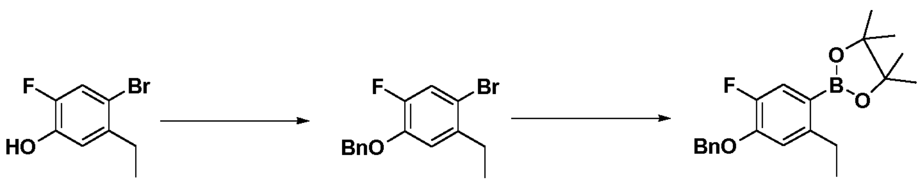

18 21 22

**Synthesis of Intermediate 21: 1-(benzyloxy)-4-bromo-5-ethyl-2-fluorobenzene**

Intermediate 18 (15.5 g, 70.8 mmol) was dissolved in 200 ml DMF, to which potassium carbonate (19.5 g, 141.5 mmol), and benzyl bromide (14.5 g, 84.9 mmol) were added, and it was allowed to raise to 60°C for reaction. After the reaction was completed, it was quenched with water, and extracted with EA. The resulting organic phase was washed with aqueous saturated solution of sodium chloride and dried over anhydrous sodium sulfate. 19.0 g of Intermediate 21 was obtained by column chromatography after concentration with a yield of 86.8%.

$^1$H NMR (400 MHz, $CDCl_3$) δ 7.44 - 7.31 (m, 5H), 7.27 (d, *J* = 8.0 Hz, 1H), 6.87 (d, *J* = 8.0 Hz, 1H), 5.12 (s, 2H), 2.68 - 2.63 (m, 2H), 1.16 (t, *J* = 8.0 Hz, 3H).

**Synthesis of Intermediate 22: 2-(4-(benzyloxy)-2-ethyl-5-fluorophenyl)-4,4,5,5-tetramethyl-1,3,2-dioxaborolane**

Intermediate 21 (1.0 g, 3.23 mmol), pinacol borate (0.82 g, 3.23 mmol), Pd(dppf)Cl2 (0.24 g, 0.32 mmol) and

KOAc (0.95 g, 9.70 mmol) were dissolved in 15 ml of 1,4-dioxane. The atmosphere was replaced with nitrogen, which was repeated 3 times. The mixture was heated to 100 °C for reaction. After the reaction was completed, water was added to quench the reaction. The resulting mixture was extracted with ethyl acetate and the organic phases were washed with saturated brine, dried over anhydrous sodium sulfate, concentrated, and purified by column chromatography to afford 0.98 g of intermediate 22 with a yield of 85.1%.

$^1$H NMR (400 MHz, $CDCl_3$) δ 7.50 - 7.30 (m, 6H), 7.82 (d, J = 8.0 Hz, 1H), 5.16 (s, 2H), 2.87 - 2.81 (m, 2H), 1.32 (s, 12H), 1.14 (t, J = 8.0 Hz, 3H).

**Synthetic route of MDI-208:**

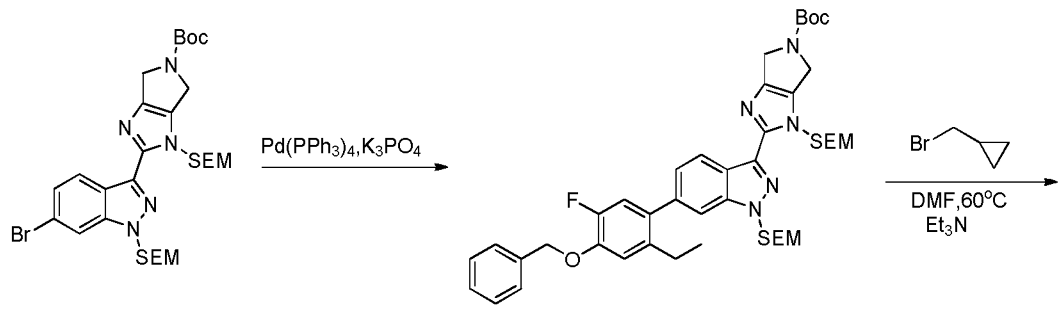


MDI-208-1

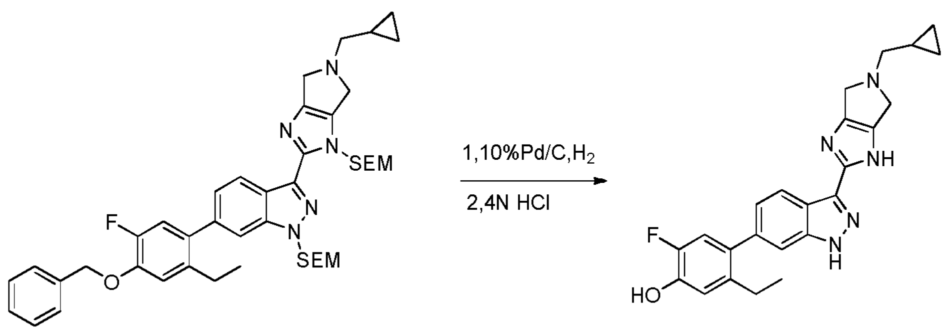


MDI-208-2

MDI-208

**Synthesis of intermediateMDI-208-1:(2-(6-(4-(benzyloxy)-2-ethyl-5-fluorophenyl)-1-((2-(trimethylsilyl)ethoxy)methyl)-1H-indazol-3-yl)-1-((2-(trimethylsilyl)ethoxy)methyl)-4,6-dihydropyrrolo[3,4-d]imidazol-5(1H)-carboxylate Tert-butyl**

Tert-butyl 2-(6-bromo1-((2-(trimethylsilyl)ethoxy)methyl)-1H-indazol-3-yl)-1-((2-(trimethylsilyl)ethoxy)methyl)-4,6-dihydropyrrolo[3,4-d]imidazol-5(1H)-carboxylate (500.0 mg, 0.75 mmol), 2-(4-(benzyloxy)-2-ethyl-5-fluorophenyl)-4,4,5,5-tetramethyl-1,3,2-dioxaboran (401.9 mg, 1.13 mmol), $Pd(PPh_3)_4$(86.9 mg, 0.08mmol) and potassium phosphate (478.9 mg, 2.26 mmol) were dissolved in 1,4-dioxane (30ml) and water (6ml). The atmosphere was replaced with nitrogen, which was repeated 3 times. The mixture was heated to 100 °C, reacted overnight, and cooled to room temperature. Water was added and the resulting mixture was exacted twice with ethyl acetate, and the organic phases were combined, washed with water and saturated brine, dried over anhydrous sodium sulfate, concentrated, and purified on a silica gel column to afford intermediate MDI-208-1 with a yield of 85.1%.

$^1$H NMR (400 MHz, CDCl3) δ 8.50-8.45 (m, 1H), 7.53-7.37 (m, 6H), 7.26 (d, J = 8.0 Hz, 1H), 7.06 (d, J = 8.0 Hz, 1H) , 6.99 (d, J = 12.0 Hz, 1H), 5.97 (d, J = 8.0 Hz, 2H), 5.77 (s, 2H), 5.23 (s, 2H), 4.67-4.54 (m, 4H), 3.65- 3.59 (m, 4H), 2.55 (d, J = 8.0 Hz, 2H), 1.57 (s, 9H), 1.05 (t, J = 8.0Hz, 3H), 0.95-0.89 (m, 4H), 0.02(s , 9H), 0.01(s, 9H).

**Synthesis of intermediate MDI-208-2: (6-(4-(benzyloxy)-2-ethyl-5-fluorophenyl)-3-(5-(cyclopropylmethyl)-1-((2-(trimethylsilyl)ethoxy)methyl)-1,4,5,6-tetrahydropyrrolo [3,4-d] imidazol-2-yl)-1-((2-(trimethylsilyl)ethoxy)methyl)-1H-indazole**

Tert-butyl 2-(6-(4-(phenoxy)-2-ethyl-5-fluorophenyl)-1-((2-(trimethylsilyl) ethoxy) methyl)-1H-indazol-3-yl)-1-((2-(trimethylsilyl)ethoxy)methyl)-4,6-dihydropyrrolo [3,4-d]imidazol-5(1H)-carboxylate (80.0 mg, 0.10 mmol) was dissolved in 5ml dichloromethane, to which 1ml trifluoroacetic acid was added. The mixture was stirred at room temperature for 30 minutes. Water was added and saturated sodium bicarbonate solution was used to adjust pH=9. The resulting mixture was extracted with DCM, and the organic phase was washed with water and saturated brine, dried over anhydrous sodium sulfate, and concentrated. The obtained solid was dissolved in 2ml DMF, and $Et_3N$ (0.1ml) and bromomethylcyclopropane (27.0 mg, 0.20mmol) were added. The mixture was heated to 60 °C, reacted overnight, and cooled to room temperature. Water was added and the resulting mixture was extracted with EA, washed with water and saturated brine, dried over anhydrous sodium sulfate, concentrated, and purified by silica gel column to afford MDI-208-2 with a yield of 33.5%.

$^1$H NMR (400 MHz, CDCl3) δ 8.48 (d, J = 8.0 Hz, 1H), 7.53-7.37 (m, 6H), 7.23(dd, J = 8.0 Hz, J = 4.0 Hz, 1H), 7.06(dd , J = 12.0 Hz, J = 4.0 Hz, 1H), 6.98 (d, J = 8.0 Hz, 1H), 5.94 (d, J =4.0 Hz, 2H), 5.76 (s, 2H), 5.23 (s, 2H), 4.13 (d, J = 36.0 Hz, 4H), 3.67-3.58 (m, 4H), 2.80(d, J = 8.0 Hz, 2H), 2.55(dd, J = 12.0 Hz, J = 8.0 Hz, 2H), 2.32-2.22 (m, 1H), 1.06(t, J = 8.0 Hz, 3H), 0.92-0.90 (m, 4H), 0.63(d, J = 8.0 Hz, 2H), 0.27(d, J = 4.0 Hz, 2H), 0.03(s, 9H), 0.02(s, 9H).

**Synthesis of compound MDI-208: 4-(3-(5-(cyclopropylmethyl)-1,4,5,6-tetrahydropyrrolo[3,4-d]imidazol-2-yl)-1H-indazol-6-yl)-5-ethyl-2-fluorophenol**

Intermediate MDI-208-2 (28.0 mg, 0.04 mmol) was dissolved in 10 ml methanol, to which 5 mg 10% Pd/C was added. The atmosphere was replaced with hydrogen, which was repeated three times. The mixture was heated to 40°C and reacted overnight, filtered to remove palladium on carbon, and concentrated. The resulting solid was dissolved in 4 ml methanol, to which 2 ml concentrated hydrochloric acid was added. The mixture was heated to 50 °C, reacted for 6 hours and concentrated to give a residue. The residue was dissolved in methanol and concentrated to dryness, which was repeated 3 times. The resulting product was dissolved 1ml methanol and 2ml aqueous ammonia was added for neutralization, and the resulting mixture was concentrated to give a residue. The residue was dissolved in methanol and was concentrated to dryness (to remove aqueous ammonia), which was repeated 2 times. The obtained product was purified by a preparation plate to afford 2 mg of the product with a yield of 13.1%.

$^1$H NMR (400 MHz, MeOD-d4) δ 8.27 (dd, J = 4.0 Hz, J = 8.0 Hz,1H), 7.42 (s, 1H), 7.17 (dd, J = 4.0 Hz, J = 8.0 Hz, 1H), 6.97-6.89 (m, 2H), 4.02 (s, 4H), 2.80(d, J = 8.0 Hz, 2H), 2.59-2.53 (m, 2H), 1.10( m, 4H), 0.66-0.61( m, 2H), 0.30-0.27(m, 2H).

**Example 10: cyclopropyl (2-(6-(2-ethyl-5-fluoro-4-hydroxyphenyl)-1H-indazol-3-yl)pyrrolo[3,4-d]imidazol-5(1H, 4H,6H)-yl)ketone (MDI-209)**

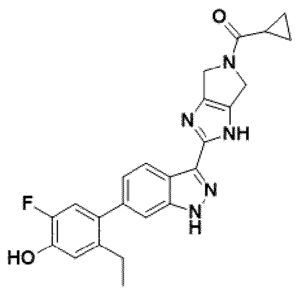

MDI 209

**Synthetic route of MDI-209:**

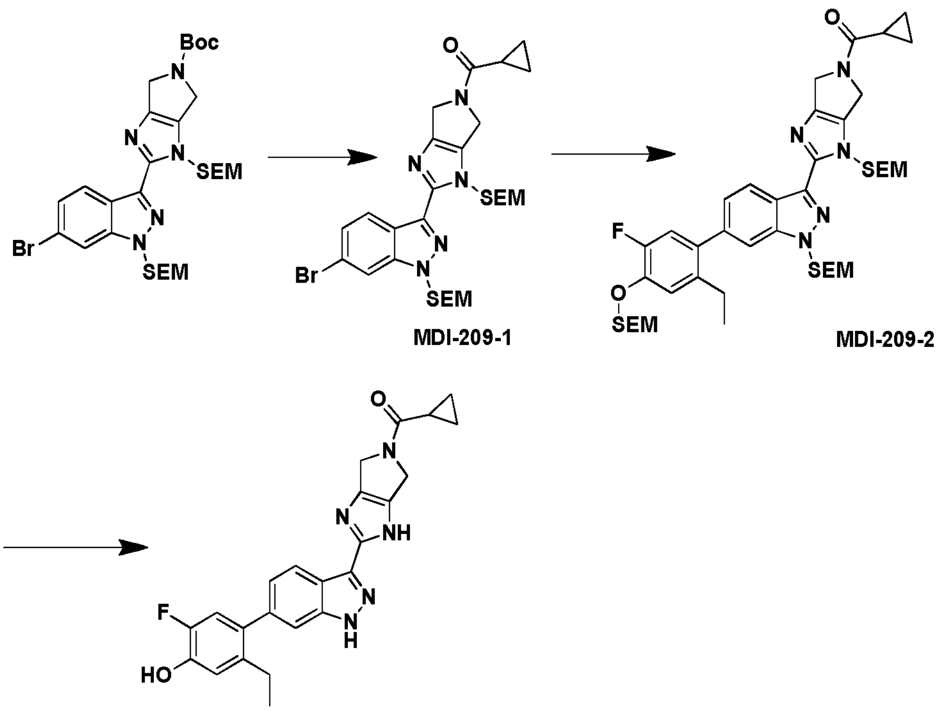

MDI-209-1

MDI-209-2

MDI 209

**Synthesis method:**

**Synthesis of intermediate MDI-209-1: (2-(6-bromo-1-((2-(trimethylsilyl)ethoxy)methyl)-1H-indazol-3-yl)-1-((2-(trimethylsilyl)ethoxy)methyl) pyrrolo[3,4-d]imidazol-5(1H,4H,6H)-yl)(cyclopropyl)ketone**

The intermediate tert-butyl 2-(6-bromo-1-((2-(trimethylsilyl)ethoxy)methyl)-1H-indazol-3-yl)-1-((2-(trimethylsilyl)ethoxy)methyl)-4,6-dihydropyrrolo[3,4-d]imidazol-5(1H)-carboxylate (80 mg, 0.12 mmol) was dissolved in 5ml of dichloromethane, to which 1ml of trifluoroacetic acid was added. The mixture was stirred at room temperature for 30 minutes, and concentrated to give a residue. The residue was dissolved in dichloromethane and was concentrated to dryness, which was repeated 3 times. The resulting residue was dissolved in 5ml of DCM, to which triethylamine (24.3 mg, 0.24mmol) was added. The temperature was lowered to 0°C, and cyclopropylformyl chloride (18.8 mg, 0.18mmol) was slowly added dropwise. After the dropwise addition was completed, the reaction was warmed up to room temperature and was allowed to react for 1-2h. Water was added to quench the reaction and liquids were separated. The organic phase was dried over sodium sulfate and concentrated by column chromatography to afford compound MDI-209-1 with a yield of 45%.

$^1$H NMR (400 MHz, CDCl3) δ 8.36 (dd, J = 17.8Hz, J =8.6 Hz, 1H), 7.80-7.79 (m, 1H), 7.41 (d, J = 8.6Hz, 1H), 5.97-5.92 (m, 2H), 5.71 (d, J = 2.4 Hz, 2H), 4.96-4.66 (m, 4H), 3.62-3.54 (m, 4H), 1.78-1.67(m, 1H), 1.10 - 1.07 (m , 2H), 0.94 - 0.84 (m, 6H), -0.05 (s, 9H), -0.08 (s, 9H).

**Synthesis of intermediate MDI-209-2: cyclopropyl(2-(6-(2-ethyl-5-fluoro-4-((2-(trimethylsilyl)ethoxy)methoxy)phenyl)1-((2-(trimethylsilyl)ethoxy) methyl)-1H-indazol-3-yl)-1-((2-(trimethylsilyl)ethoxy)methyl)pyrrolo[3,4-d]imidazol-5(1H, 4H,6H)-yl)ketone**

The intermediate MDI-209-1 (50.5 mg, 0.08mmol), (2-((5-ethyl-2-fluoro-4-(4, 4,5,5-tetramethyl-1,3,2-dioxaboran-2-yl)phenoxy)methoxy)ethyl) trimethylsilane (34.8 mg, 0.1mmol), Pd(dppf)$Cl_2$ (5.9 mg, 0.008mmol) and potassium phosphate (50.9 mg, 0.24mmol) were dissolved in 1,4-dioxane (10ml) and water (2ml). The atmosphere was replaced with nitrogen, which was repeated 3 times. The mixture was heated to 100°C, reacted overnight, and cooled to room temperature. Water was added and the resulting mixture was extracted twice with ethyl acetate and the organic phases were combined, washed with water and saturated brine, dried over anhydrous sodium sulfate, concentrated, and purified by silica gel column to afford intermediate MDI-209- 2 with a yield of 76.1%.

$^1$H NMR (400 MHz, CDCl3) δ 8.50-8.43 (m,1H), 7.46-7.45 (m,1H), 7.25-7.22 (m,1H), 7.16 (d, J = 8.0Hz, 1H), 7.02 ( d, J = 12.0Hz, 1H), 5.99-5.94 (m, 2H), 5.76(s, 2H), 5.32 (s, 2H), 4.98-4.67(m, 4H), 3.88-3.84 (m, 2H) , 3.64-3.55 (m, 4H), 2.57-2.51 (m, 2H), 1.79-1.68 (m, 1H), 1.07-1.02 (m, 6H), 0.95-0.87 (m, 5H), 0.03 (s, 9H),-0.06-0.08(m, 18H).

**Synthesis of compound MDI-209: cyclopropyl (2-(6-(2-ethyl-5-fluoro-4-hydroxyphenyl)-1H-indazol-3-yl)pyrrolo[3,4-d]imidazol-5(1H, 4H,6H)-yl)ketone**

The intermediate MDI-209-2 (50 mg, 0.06mmol) was dissolved in methanol (4ml), to which concentrated hydrochloric acid (2ml) was added. The mixture was heated to 50°C, reacted for 6 hours, and concentrated. The solid was dissolved in 1ml methanol, to which 2ml concentrated aqueous ammonia was added. The mixture was concentrated to give a residue. The residue was dissolved in methanol and was concentrated to dryness, which was repeated 3 times. The resulting residue was purified by a preparation plate to afford 10.0 mg of the final product with a yield of 38.1%.

$^1$H NMR (400 MHz, MeOD -d4) δ8.28(d, J = 8.0 Hz,1H), 7.43(s, 1H), 7.18 (dd, J = 8.4Hz, J = 1.4 Hz, 1H), 6.98 ( d, J =12.0 Hz, 1H), 6.92 (d, J =12.0 Hz, 1H), 4.95 (s, 2H), 4.65 (s, 2H), 2.59-2.53 (m, 2H), 1.98-1.89 (m , 1H), 1.08 (t, J = 8.0 Hz, 3H), 1.02-1.00 (m, 2H), 0.98-0.92 (m, 2H).

**Example 11: 4-(3-(5-(cyclobutylmethyl)-1,4,5,6-tetrahydropyrrolo[3,4-d]imidazol-2-yl)-1H-indazol-6-yl)-5-ethyl-2-fluorophenol (MDI-210)**

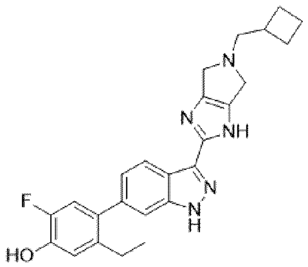

MDI-210

Synthetic route of MDI-210:

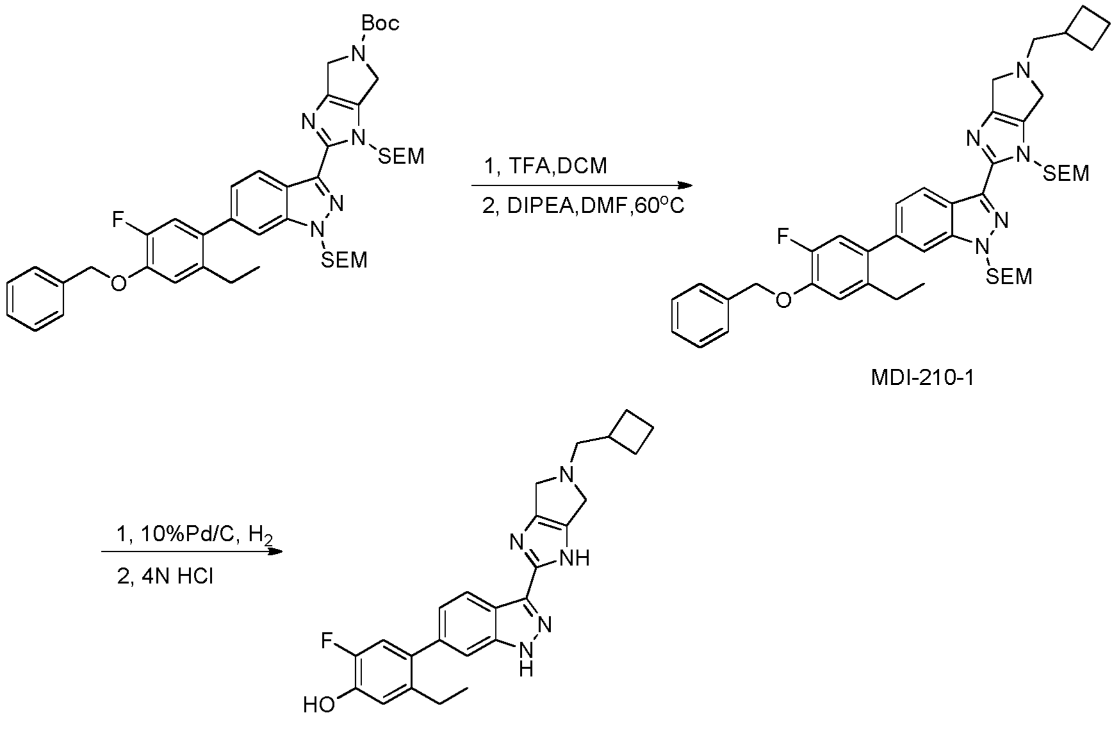


MDI-210-1

MDI-210

**Synthesis method:**

**Synthesis of intermediate MDI-210-1: (6-(4-(benzyloxy)-2-ethyl-5-fluorophenyl)-3-(5-(cyclobutylmethyl)-1-((2-(trimethylsilyl)ethoxy)methyl)-1,4,5,6-tetrahydropyrrolo [3,4-d] imidazol-2-yl)-1-((2-(trimethylsilyl)ethoxy)methyl)-1H-indazole**

Tert-butyl 2-(6-(4-(phenoxy)-2-ethyl-5-fluorophenyl)-1-((2-(trimethylsilyl) ethoxy)methyl)-1H-indazol-3-yl)-1-((2-(trimethylsilyl)ethoxy)methyl)-4,6-dihydropyrrolo [3,4-d]imidazol-5(1H)-carboxylate (80.0 mg, 0.10mmol) was dissolved in 5ml dichloromethane, to which 1ml trifluoroacetic acid was added. The mixture was stirred at room temperature for 30 minutes. Water was added and saturated sodium bicarbonate solution was used to adjust pH=9. The resulting mixture was extracted with DCM, washed with water and saturated brine, dried over sodium sulfate and concentrated. The obtained solid was dissolved in 3ml DMF, followed by addition of DIPEA (126.8 mg, 0.98mmol) and bromomethylcyclobutane (29.3 mg, 0.20mmol). The mixture was heated to 60°C, reacted overnight, and cooled to room temperature. Water was added and the resulting mixture was extracted with EA, washed with water and saturated brine, dried over anhydrous sodium sulfate, concentrated, and purified by silica gel column to afford MDI-210-1 with a yield of 35.1%.

$^1H$ NMR (400 MHz, CDCl3) δ 8.47 (dd, J = 8.0 Hz, J = 4.0 Hz, 1H), 7.53-7.51 (m, 2H), 7.46-7.37 (m, 4H), 7.23(dd, J = 8.0 Hz, J = 4.0 Hz, 1H), 7.06(d, J = 12.0 Hz, 1H), 6.98 (d, J = 8.0 Hz, 1H), 5.91 (s, 2H), 5.76 (s, 2H), 5.22 (s, 2H), 4.02 (s, 2H), 3.94 (s, 2H), 3.65-3.56 (m, 4H), 2.93(d, J = 8.0 Hz, 2H), 2.68-2.64 (m, 1H), 2.57(dd, J = 16.0 Hz, J = 8.0 Hz, 2H), 2.21-2.14(m, 2H), 1.97-1.67 (m, 4H), 1.06 (t, J = 8.0 Hz, 3H), 0.94-0.89 (m, 4H), 0.02(s, 18H).

**Synthesis of compound MDI-210: 4-(3-(5-(cyclobutylmethyl)-1,4,5,6-tetrahydropyrrolo[3,4-d]imidazol-2-yl)-1H-indazol-6-yl)-5-ethyl-2-fluorophenol**

Intermediate MDI-210-1 (35.0 mg, 0.05 mmol) was dissolved in 10 ml methanol, to which 5 mg 10% Pd/C was added. The atmosphere was replaced with hydrogen, which was repeated three times. The mixture was heated to 40°C and reacted overnight, filtered to remove palladium on carbon, and concentrated. The resulting solid was dissolved in 4 ml methanol, to which 2 ml concentrated hydrochloric acid was added. The mixture was heated to 50 °C, reacted for 6 hours and concentrated to give a residue. The residue was dissolved in methanol and was concentrated to dryness, which was repeated 3 times. The resulting product was dissolved 1ml methanol and 2ml concentrated aqueous ammonia was added for neutralization, and the resulting mixture was concentrated to give a residue. The residue was dissolved in methanol and was concentrated to dryness, which was repeated 2 times. The obtained product was purified by a preparation plate to afford 4 mg of the product with a yield of 20.7%.

$^1H$ NMR (400 MHz, MeOD-d4) δ 8.27 (dd, J = 4.0 Hz, J = 8.0 Hz,1H), 7.42 (s, 1H), 7.17 (dd, J = 4.0 Hz, J = 8.0 Hz, 1H), 6.97-6.89 (m, 2H), 3.98 (s, 4H), 3.00(d, J = 8.0 Hz, 2H), 2.72-2.68 (m, 1H), 2.59-2.53 (m, 2H), 2.21- 2.18 (m, 2H), 1.89-1.85 (m, 4H), 1.07 (t, J = 8.0 Hz, 3H).

**Example 12: cyclobutyl (2-(6-(2-ethyl-5-fluoro-4-hydroxyphenyl)-1H-indazol-3-yl)pyrrolo[3,4-d]imidazol-5(1H, 4H,6H)-yl)ketone (MDI-211)**

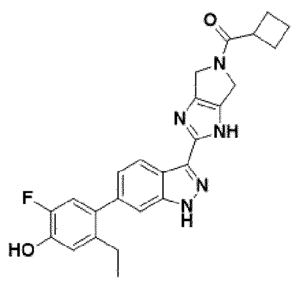

MDI 211

**Synthetic route of MDI-211:**

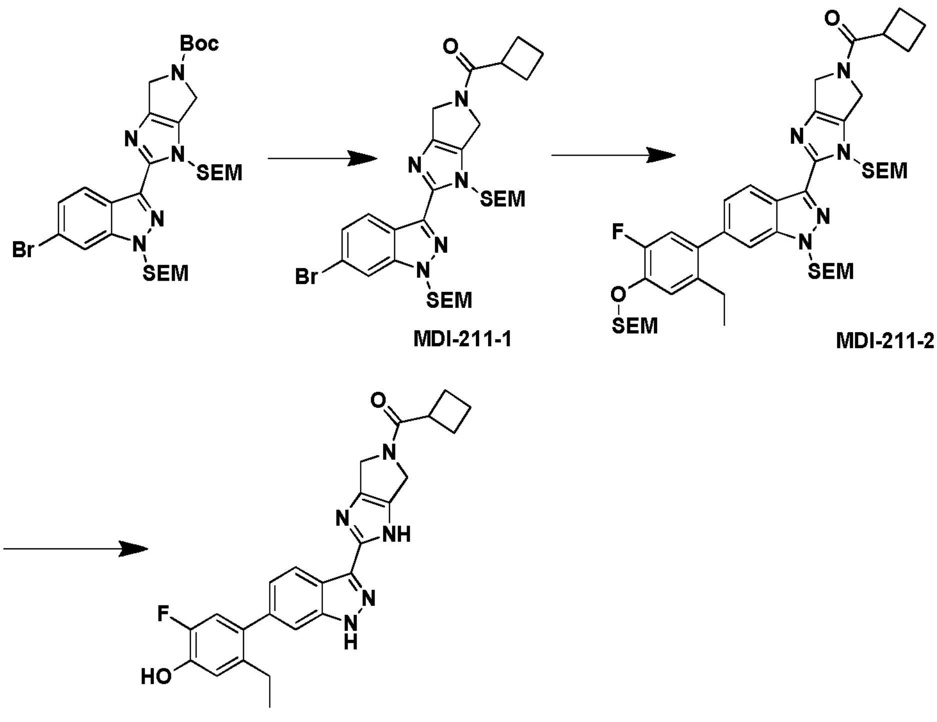

MDI-211-1

MDI-211-2

MDI 211

**Synthesis method:**

**Synthesis of intermediate MDI-211-1: (2-(6-bromo-1-((2-(trimethylsilyl)ethoxy)methyl)-1H-indazol-3-yl)-1-((2-(trimethylsilyl)ethoxy) methyl) pyrrolo[3,4-d]imidazol-5(1H, 4H,6H)-yl)(cyclobutyl)ketone**

The intermediate tert-butyl 2-(6-bromo-1-((2-(trimethylsilyl)ethoxy)methyl)-1H-indazol-3-yl)-1-((2-(trimethylsilyl)ethoxy)methyl)-4,6-dihydropyrrolo[3,4-d]imidazol-5(1H)-carboxylate (80 mg, 0.12 mmol) was dissolved in 5ml of dichloromethane, to which 1ml of trifluoroacetic acid was added. The mixture was stirred at room temperature for 30 minutes, and concentrated to give a residue. The residue was dissolved in dichloromethane and was concentrated to dryness, which was repeated 3 times. The resulting residue was dissolved in 5ml of DCM, to which triethylamine (24.3 mg, 0.24mmol) was added. The temperature was reduced to 0°C and cyclobutyl carbonyl chloride (21.3 mg, 0.18mmol) was slowly added dropwise. After the dropwise addition was completed, the reaction was warmed up to room temperature and was allowed to react for 1-2h. Water was added to quench the reaction and liquids were separated. The organic phase was dried over sodium sulfate and concentrated by column chromatography to afford compound MDI-211-1 with a yield of 43%.

$^1$H NMR (400 MHz, CDCl3) $\delta$ 8.39-8.31 (m, 1H), 7.77-7.76 (m, 1H), 7.42-7.38 (m, 1H), 5.92-5.89 (m, 2H), 5.71-5.70 (m , 2H), 4.73-4.57 (m, 4H), 3.60-3.54 (m, 4H), 3.37-3.27 (m, 1H), 2.46-2.39 (m, 2H), 2.34-2.20 (m, 3H), 2.10 -1.93 (m, 3H), 0.93-0.88 (m, 4H), -0.05 (s, 9H), -0.09 (s, 9H).

**Synthesis of intermediate MDI-211-2: cyclobutyl (2-(6-(2-ethyl-5-fluoro-4-((2-(trimethylsilyl)ethoxy)methoxy)phenyl)1-((2-(trimethylsilyl)ethoxy)methyl)-1H-indazol-3-yl)-1-((2-(trimethylsilyl)ethoxy)methyl)pyrrolo[3,4-d]imidazol-5(1H, 4H,6H)-yl)ketone**

The intermediate MDI-211-1 (51.6mg, 0.08mmol), (2-((5-ethyl-2-fluoro-4-(4, 4,5,5-tetramethyl-1,3,2-dioxaboran-2-yl)phenoxy)methoxy)ethyl)trimethylsilane (34.8 mg, 0. 1mmol), Pd(dppf)$Cl_2$ (5.9 mg, 0.008mmol) and potassium phosphate (50.9 mg, 0.24mmol) were dissolved in 1,4-dioxane (10ml) and water (2ml). The atmosphere was replaced with nitrogen, which was repeated 3 times. The mixture was heated to 100°C, reacted overnight, and cooled to room temperature. Water was added and the resulting mixture was extracted twice with ethyl acetate, and the organic phases were combined, washed with water and saturated brine, dried over anhydrous sodium sulfate, concentrated, and purified by silica gel column to afford intermediate MDI-211- 2 with a yield of 75.2%.

$^1$H NMR (400 MHz, CDCl3) $\delta$ 8.49-8.42 (m, 1H),, 7.47-7.45 (m,1H), 7.25-7.22 (m,1H), 7.16 (d, J = 8.0Hz, 1H), 7.02 (d, J = 12.0Hz, 1H), 5.96-5.93 (m, 2H), 5.75(s, 2H), 5.32 (s, 2H), 4.75-4.58(m, 4H), 3.88-3.83 (m, 2H) ), 3.63-3.55 (m, 4H), 3.38-3.28 (m, 1H), 2.57-2.51 (m, 2H), 2.48-2.20 (m, 4H), 2.06-2.00 (m, 1H), 1.96-1.92 (m, 1H), 1.08-1.05 (m,

3H), 0.93-0.85 (m, 6H), 0.03 (s, 9H), -0.06-0.08 (m, 18H).

**Synthesis of compound MDI-211: cyclobutyl (2-(6-(2-ethyl-5-fluoro-4-hydroxyphenyl)-1H-indazol-3-yl)pyrrolo[3,4-d]imidazol-5(1H, 4H,6H)-yl)ketone**

The intermediate MDI-211-2 (45 mg, 0.054mmol) was dissolved in methanol (4ml), to which concentrated hydrochloric acid (2ml) was added. The mixture was heated to 50 °C, reacted for 6 hours, and concentrated. The solid was dissolved in 1ml methanol, 2ml of concentrated aqueous ammonia was added, and the mixture was concentrated to give a residue. The residue was dissolved in methanol and was concentrated to dryness, which was repeated 3 times. The resulting residue was separated by a preparation plate to afford 8.2 mg of the final product with a yield of 34.2%.

$^1H$ NMR (400 MHz, MeOD-d4) δ 8.27(d, J = 8.0 Hz, 1H), 7.43 (s, 1H),, 7.17 (dd, J = 8.4, 1.4 Hz, 1H), 6.93 (dd, J = 20.0Hz, J =12.0 Hz, 2H), 4.68-4.63 (m, 4H), 3.54-3.46 (m, 1H), 2.57-2.53 (m, 2H), 2.43-2.26 (m, 4H), 2.16- 2.04 (m, 1H), 1.98-1.89 (m, 1H), 1.08 (t, J = 8.0 Hz, 3H).

**Example 13: (2-(6-(2-ethyl-5-fluoro-4-hydroxyphenyl)-1H-indazol-3-yl)pyrrolo[3,4-d]imidazol-5(1H,4H,6H)-yl)(3-hydroxylcyclobutyl)ketone (MDI-213)**

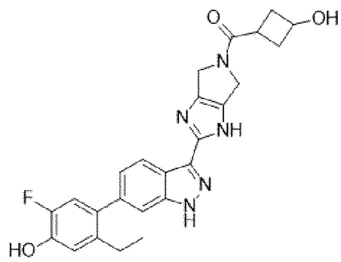

MDI-213

**Synthetic route of MDI-213:**

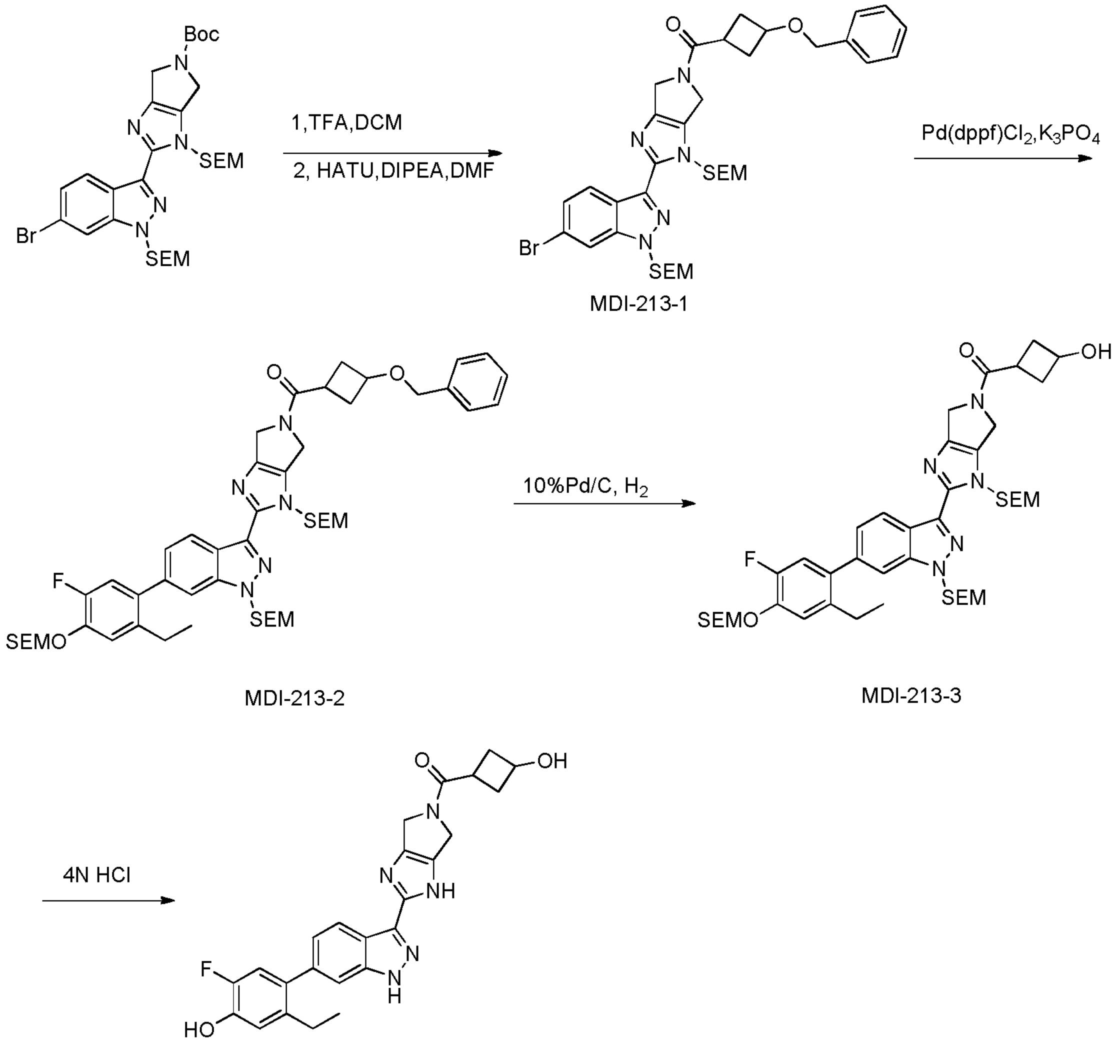


MDI-213-1

MDI-213-2

MDI-213-3

MDI-213

**Synthesis method:**

**Synthesis of intermediate MDI-213-1: (3-(benzyloxy)cyclobutyl)(2-(6-bromo-1-((2-(trimethylsilyl)ethoxy)methyl)-1H-indazol-3-yl)-1-((2-(trimethylsilyl) ethoxy)methyl)pyrrolo[3,4-d]imidazol-5(1H, 4H,6H)-yl)ketone**

The intermediate 3-benzyloxy-cyclobutanecarboxylic acid (44.6 mg, 0.22 mmol) and N,N-diisopropylethylamine (69.9 mg, 0.54 mmol) were dissolved in DMF, to which HATU (82.3 mg, 0.22 mmol) was added. The mixture was reacted at room temperature for 10 minutes. Intermediate tert-butyl 2-(6-bromo-1-((2-(trimethylsilyl)ethoxy)methyl)-1H-indazol-3-yl)-1-((2-(trimethylsilyl)ethoxy)methyl)-4,6-dihydropyrrolo[3,4-d]imidazole-5(1H)-carboxylate (120.0 mg, 0.18 mmol) was dissolved in 5ml dichloromethane, to which 1ml of trifluoroacetic acid was added. The mixture was stirred at room temperature for 30 minutes, and concentrated to give a residue. The residue was dissolved in dichloromethane and was concentrated to dryness, which was repeated 3 times. The resulting residue was dissolved in DMF, and then slowly added to the previous reaction solution. It was allowed to react at room temperature overnight, and water was added to quench the reaction. The resulting mixture was extracted twice with ethyl acetate, and the organic phases were combined, washed with water and saturated brine, dried over anhydrous sodium sulfate, concentrated, and purified by silica gel column to afford intermediate MDI-213-1 with a yield of 35.3%.

$^1H$ NMR (400 MHz, CDCl3) $\delta$ 8.41 (dd, J = 8.0 Hz, J = 20.0 Hz, 1H), 7.80-7.78 (m, 1H), 7.43-7.31 (m, 6H), 5.94 (d, J = 12.0 Hz, 2H), 5.73 (d, J = 4.0 Hz, 2H), 4.76-4.61 (m, 4H), 4.49 (s, 2H), 4.10-4.04 (m, 1H), 3.63-3.57 (m, 4H) ), 2.80-2.78 (m, 1H), 2.58-2.53 (m, 2H), 2.46-2.41 (m, 2H), 0.96-0.90 (m, 4H), 0.02(s, 9H), -0.03(s, 9H).

**Synthesis of intermediate MDI-213-2: (3-(benzyloxy)cyclobutyl)(2-(6-(2-ethyl-5-fluoro-4-((2-(trimethylsilyl)ethoxy)methoxy)phenyl)-1-((2-(trimethylsilyl) ethoxy)methyl)-1H-indazol-3-yl)-1-((2-(trimethylsilyl)ethoxy)methyl)pyrrolo[3,4-d]imidazol-5(1H, 4H,6H)-yl)ketone**

Intermediate MDI-213-1 (48.0 mg, 0.06 mmol), intermediate MDI-10-2 (30.4 mg, 0.08 mmol), Pd(dppf)Cl2 (4.7 mg, 0.01 mmol) and potassium phosphate (40.6 mg, 0.19 mmol) were dissolved in 1,4-dioxane (20ml) and water (4ml). The atmosphere was replaced with nitrogen, which was repeated 3 times. The mixture was heated to 100°C, reacted overnight, and cooled to room temperature. Water was added, and the resulting mixture was extracted 2 times with ethyl acetate, and the organic phases were combined, washed with water and saturated brine, dried over anhydrous sodium sulfate, concentrated, and purified by silica gel column to afford intermediate MDI-213-2 with a yield of 71.5%.

$^1$H NMR (400 MHz, CDCl3) δ 8.46 (dd, J = 8.0 Hz, J = 4.0 Hz, 1H), 7.49-7.48 (m, 1H), 7.38-7.32 (m, 5H), 7.28-7.24 (m, 1H), 7.19 (d, J = 8.0 Hz, 1H), 7.05 (d, J = 12.0 Hz, 1H), 5.99 (d, J = 12.0 Hz, 2H), 5.78 (d, J = 4.0 Hz, 2H), 5.34 (s, 2H), 4.78-4.62 (m, 4H), 4.50 (s, 2H), 4.12-4.05 (m, 1H), 3.90-3.86 (m, 2H), 3.66-3.58 (m, 4H) ,2.80-2.78 (m, 1H), 2.59-2.54 (m, 4H), 2.46-2.44 (m, 2H), 1.10-1.05 (m, 3H), 0.95-0.90 (m, 6H), 0.06(s, 9H),-0.04(s, 18H).

**Synthesis of intermediate MDI-213-3: (2-(6-(2-ethyl-5-fluoro-4-((2-(trimethylsilyl)ethoxy)methoxy)phenyl)-1-((2-(trimethylsilyl)ethoxy)methyl)-1H-indazol-3-yl)-1-((2-(trimethylsilyl)ethoxy)methyl)pyrrolo[3,4-d]imidazol-5(1H,4H,6H)-yl)(3-hydroxylcyclobutyl)ketone**

Intermediate MDI-213-2 (43.0 mg, 0.05 mmol) was dissolved in 10ml methanol, and 5mg of 10% Pd/C was added. The atmosphere was replaced with hydrogen, which was repeated three times. The mixture was reacted overnight at room temperature, filtered off palladium carbon, and concentrated to afford intermediate MDI-213 -3, which was directly used in the next step.

**Synthesis of compound MDI-213: (2-(6-(2-ethyl-5-fluoro-4-hydroxyphenyl)-1H-indazol-3-yl)pyrrolo[3,4-d]imidazol-5(1H,4H,6H)-yl)(3-hydroxyl cyclobutyl)ketone**

The intermediate MDI-213-3 (36.1mg, 0.05mmol) was dissolved in 4ML methanol and 2ml concentrated hydrochloric acid was added. The mixture was heated to 50°C, reacted for 6 hours, and concentrated to give a residue. The residue was dissolved in methanol and was concentrated to dryness, which was repeated 3 times. The resulting product was dissolved 1ml methanol and 2ml aqueous ammonia was added for neutralization, and the resulting mixture was concentrated to give a residue. The residue was dissolved in methanol and was concentrated to dryness, which was repeated 2 times. The obtained product was purified by a preparation plate to afford 4 mg of the product with a yield of 16.4%.

$^1$H NMR (400 MHz, MeOD-d4) δ 8.28 (dd, J = 4.0 Hz, J = 8.0 Hz, 1H), 7.43-7.42 (m, 1H), 7.19 (dd, J = 4.0 Hz, J = 8.0 Hz , 1H), 6.97-6.89 (m, 2H), 4.72-4.62 (m, 4H), 4.23-4.20 (m, 1H), 2.95-2.91 (m, 1H), 2.63-2.53 (m, 4H), 2.26 -2.18 (m, 2H), 1.10(t, J = 8.0 Hz, 3H).

**Example 14: (2-(6-(2-ethyl-5-fluoro-4-hydroxyphenyl)-1H-indazol-3-yl)-pyrrolo[3,4-d]imidazol-5-(1H,4H,6H)-yl)(pyridazin-4-yl)ketone (MDI-214)**

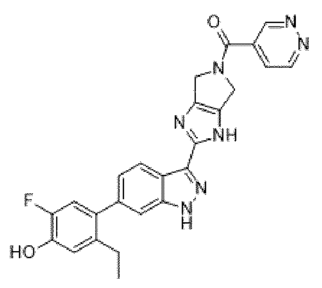

MDI-214

**Synthetic route of MDI-214:**

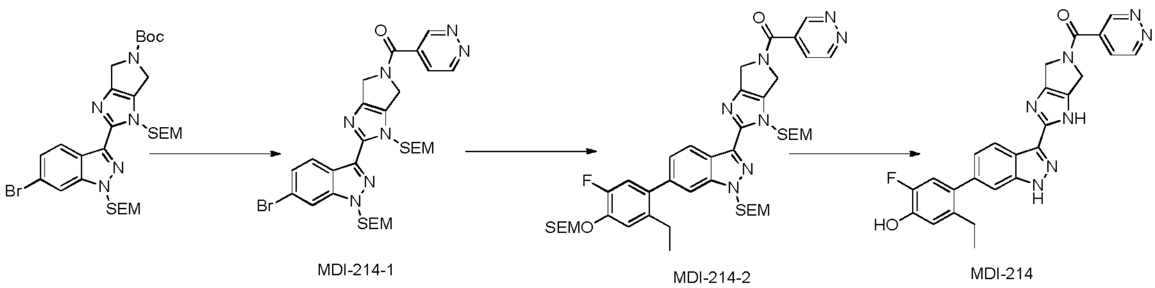


**Synthesis method:**

**Synthesis of intermediate MDI-214-1: (2-(6-bromo-1-((2-(trimethylsilyl)ethoxy)methyl)-1H-indazol-3-yl)-1-((2-(trimethylsilyl)ethoxy)methyl)-pyrrolo[3,4-d]imidazol-5-(1H, 4H,6H)-yl)(pyridazin-4-yl)ketone**

Tert-butyl 2-(6-bromo-1-((2-(trimethylsilyl)ethoxy)methyl)-1H-indazol-3-yl)-1-((2-(trimethylsilyl)ethoxy)methyl)-4,6-dihydropyrrolo[3,4-d]imidazol-5(1H)-carboxylate (45 mg, 0.06 mmol) was dissolve in 5ml of dichloromethane, to which 1ml of trifluoroacetic acid was added. The mixture was stirred at room temperature for 30 minutes, and concentrated to give a residue. The residue was dissolved in dichloromethane and was concentrated to dryness, which was repeated 3 times. The resulting residue was dissolved in 5ml of DMF, followed by addition of pyridazine-4-carboxylic acid (9mg, 0.07 mmol), HATU (32 mg, 0.08 mmol) and DIPEA (0.05ml, 0.30mmol). It was allowed to react at room temperature for 16 hours and water was added to quench the reaction. The mixture was extracted twice with EA, and the organic phases were combined, washed with water and saturated brine, dried over anhydrous sodium sulfate, concentrated, and purified by silica gel column to afford 23 mg of intermediate MDI-214-1 with a yield of 51.2%.

$^1$H NMR (400 MHz, CDCl3)δ 9.45 - 9.50 (m, 2H), 8.34 (dd, J = 38.3Hz, J = 8.6 Hz, 1H), 7.79 (t, J = 1.9 Hz, 1H), 7.69-7.66 (m, 1H), 7.45-7.41 (m, 1H), 5.92 (d, J = 31.9 Hz, 2H), 5.72 (d, J = 5.3 Hz, 2H), 4.95-4.93 (m, 2H), 4.68- 4.66 (m, 2H), 3.65-3.54 (m, 4H), 1.08-0.77 (m, 4H), 0.05-0.13 (m, 18H).

**Synthesis of intermediate MDI-214-2: (2-(6-(2-ethyl-5-fluoro-4-((2-(trimethylsilyl)ethoxy)methoxy)phenyl)-1-((2-(trimethylsilyl)ethoxy)methyl)-1H-indazol-3-yl)-1-((2-(trimethylsilyl)ethoxy)methyl)-pyrrolo [3,4-d] imidazol-5-(1H,4H,6H)-yl)(pyridazin-4-yl)ketone**

The intermediate MDI-214-1 (23 mg, 0.03 mmol), (2-((5-ethyl-2-fluoro-4-(4,4,5,5-tetramethyl-1,3,2-dioxaboran-2-yl)phenoxy)methoxy)ethyl)trimethylsilane (20 mg, 0.05 mmol), Pd(dppf)$Cl_2$ (3 mg, 0.003 mmol) and potassium phosphate (22 mg, 0.10 mmol) were dissolved in 1,4-dioxane (5 ml) and water (1 ml). The atmosphere was replaced with nitrogen, which was repeated 3 times. The mixture was heated to 100°C, reacted for 16 h, and cooled to room temperature. Water was added and the resulting mixture was extracted 2 times with ethyl acetate and the organic phases were combined, washed with water and saturated brine, dried over anhydrous sodium sulfate, concentrated, and purified by silica gel column to afford 25 mg of intermediate MDI-214-2 with a yield of 84.7%.

$^1$H NMR (400 MHz, CDCl3) δ 9.46 - 9.42 (m, 2H), 8.62 - 8.31 (m, 1H), 7.71-7.67 (m, 1H), 7.51 - 7.47 (m, 1H), 7.25 (dd, J = 8.4, 1.3 Hz, 1H), 7.18 (dd, J = 8.4, 4.0 Hz, 1H), 7.03 (dd, J = 11.6, 6.0 Hz, 1H), 5.96 (d, J = 31.6 Hz, 2H), 5.78 (d, J = 5.2 Hz, 2H), 5.34 (d, J = 2.7 Hz, 2H), 5.02 - 4.95 (m, 2H), 4.71 - 4.67 (m, 2H), 3.90 - 3.86 (m, 2H), 3.66 - 3.62 (m, 4H), 2.57 - 2.54 (m, 2H), 1.14 - 0.81 (m, 9H), 0.06 (d, J = 2.1 Hz, 9H), -0.04 (dd, J = 12.1Hz, J = 8.6 Hz, 18H).

**Synthesis of compound MDI-214: (2-(6-(2-ethyl-5-fluoro-4-hydroxyphenyl)-1H-indazol-3-yl)pyrrolo[3,4-d]imidazol-5-(1H,4H,6H)-yl)(pyridazin-4-yl)ketone**

Intermediate MDI-214-2 (25 mg, 0.03 mmol) was dissolved in methanol (4ml), to which concentrated hydrochloric acid (2ml) was added. The mixture was heated to 50 °C, reacted for 6 hours, and concentrated to give a residue. The residue was dissolved in methanol and was concentrated to dryness, which was repeated 3 times. The resulting residue was dissolve in methanol, 1ml of aqueous ammonia was added, and then the mixture was concentrated, and purified by a preparation plate to afford 4 mg of the final product with a yield of 29.4%.

$^1$H NMR (400 MHz, MeOD-d4)δ 9.48 (dd, J = 2.3, J = 1.3 Hz, 1H), 9.42 (dd, J = 5.2, J = 1.3 Hz, 1H), 8.26 (s, 1H), 8.02 (dd, J = 5.3, J = 2.2 Hz, 1H), 7.43 (d, J = 1.1 Hz, 1H), 7.17 (d, J = 8.2 Hz, 1H), 6.93 (dd, J = 19.7, J = 10.4 Hz, 2H), 4.90 (s, 2H), 4.73 (s, 2H), 2.55 (q, J = 7.5 Hz, 2H), 1.08 (t, J = 7.5 Hz, 3H).

**Example 15: (2-(6-(2-ethyl-5-fluoro-4-hydroxyphenyl)-1H-indazol-3-yl)-pyrrolo[3,4-d]imidazol-5-(1H,4H,6H)-yl)(pyridazin-3-yl)ketone (MDI-215)**

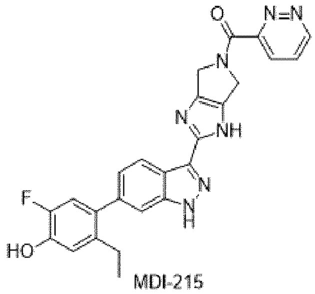

MDI-215

**Synthetic route of MDI-215:**

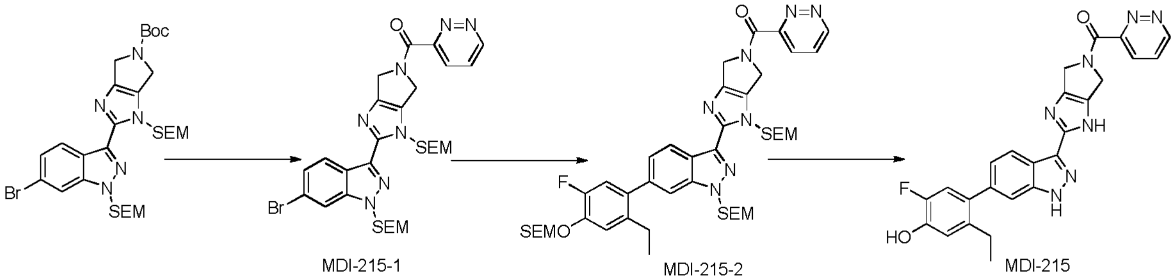

**Synthesis method:**

**Synthesis of intermediate MDI-215-1: (2-(6-bromo-1-((2-(trimethylsilyl)ethoxy)methyl)-1H-indazol-3-yl)-1-((2-(trimethylsilyl)ethoxy)methyl)-pyrrolo[3,4-d]imidazol-5-(1H,4H,6H)-yl)(pyridazin-3-yl)ketone**

Tert-butyl 2-(6-bromo1-((2-(trimethylsilyl)ethoxy)methyl)-1H-indazol-3-yl)-1-((2-(trimethylsilyl) ethoxy)methyl)-4,6-dihydropyrrolo[3,4-d]imidazole-5(1H)-carboxylate (200 mg, 0.30 mmol) was dissolved in 10 ml dichloromethane, and 2 ml of trifluoroacetic acid was added. The mixture was stirred at room temperature for 30 minutes, and concentrated to give a residue. The residue was dissolved in dichloromethane and was concentrated to dryness, which was repeated 3 times. The resulting residue was dissolved in 10 ml of DMF, followed by addition of pyridazine-3-carboxylic acid (45 mg, 0.36 mml), HATU (164 mg, 0.43 mmol) and DIPEA (0.18 ml, 1.08 mmol). It was allowed to react at room temperature for 16 hours and water was added to quench the reaction. The resulting mixture was extracted twice with EA, and the organic phases were combined, washed with water and saturated brine, dried over anhydrous sodium sulfate, concentrated and purified by silica gel column to afford 98 mg of intermediate MDI-215-1 with a yield of 49.2%.

$^1$H NMR (400 MHz, CDCl3)δ 9.35 - 9.30 (m, 1H), 8.40 (dd, J = 19.4Hz, J =8.6 Hz, 1H), 8.25 (dd, J = 4.0Hz, J =8.0 Hz, 1H ), 7.79 (dd, J = 3.1, J =1.5 Hz, 1H), 7.72-7.68 (m, 1H), 7.46-7.43 (m, 1H), 5.95 (d, J = 21.7 Hz, 2H), 5.73 ( d, J = 3.2 Hz, 2H), 5.40-5.24 (m, 2H), 5.06 - 4.98 (m, 2H), 3.66 - 3.56 (m, 4H), 0.98 - 0.87 (m, 4H), 0.00-0.05 (m, 9H),-0.09 (s, 9H).

**Synthesis of intermediate MDI-215-2: (2-(6-(2-ethyl-5-fluoro-4-((2-(trimethylsilyl)ethoxy)methoxy)phenyl)-1-((2-(trimethylsilyl)ethoxy)methyl)-1H-indazol-3-yl)-1-((2-(trimethylsilyl)ethoxy)methyl)-pyrrolo [3,4-d] imidazol-5-(1H,4H,6H)-yl)(pyridazin-3-yl)ketone**

The intermediate MDI-215-1 (98mg, 0.15 mmol), (2-((5-ethyl-2-fluoro-4-(4, 4,5,5-tetramethyl-1,3,2-dioxaboran-2-yl)phenoxy)methoxy)ethyl)trimethylsilane (100 mg, 0.25 mmol), Pd(dppf)$Cl_2$ (15 mg, 0.015 mmol) and potassium phosphate (110mg, 0.50 mmol) were dissolved in 1,4-dioxane (25 ml) and water (5ml). The atmosphere was replaced with nitrogen, which was repeated 3 times. The mixture was heated to 100°C, reacted for 16 h, and cooled to room temperature. Water was added and the resulting mixture was extracted twice with ethyl acetate, and the organic phases

were combined, washed with water and saturated brine, dried over anhydrous sodium sulfate, concentrated, and purified on a silica gel column to afford 97 mg of intermediate MDI- 215-2 with a yield of 77.1%.

$^1$H NMR (400 MHz, CDCl3)δ 9.33 - 9.32 (d, J = 4.0 Hz, 1H), 8.52 - 8.48 (m, 1H), 8.26-8.21 (m, 1H), 7.71 - 7.69 (m, 1H), 7.48 (dd, J = 2.8Hz, J =1.2 Hz, 1H), 7.26 (dd, J = 8.4Hz, J =1.3 Hz, 1H), 7.18 (dd, J = 8.4Hz, J =2.4 Hz, 1H) , 7.04 (dd, J = 11.6Hz, J = 4.2 Hz, 1H), 5.99 (d, J = 22.2 Hz, 2H), 5.78 (d, J = 3.0 Hz, 2H), 5.40 (d, J = 2.4 Hz, 1H), 5.34 (s, 2H), 5.25 (t, J = 2.1 Hz, 1H), 5.08 - 4.99 (m, 2H), 3.90-3.88 (m, 2H), 3.67 - 3.58 (m, 4H), 2.56 (q, J = 7.5 Hz, 2H), 1.10 - 0.77 (m, 9H), 0.06 (d, J = 1.2 Hz, 9H), -0.01 --0.10 (m, 18H).

**Synthesis of compound MDI-215: (2-(6-(2-ethyl-5-fluoro-4-hydroxyphenyl)-1H-indazol-3-yl)-pyrrolo[3,4-d]imidazol-5-(1H,4H,6H)-yl)(pyridazin-3-yl)ketone**

Intermediate MDI-215-2 (97 mg, 0.11 mmol) was dissolved in methanol (10ml), to which concentrated hydrochloric acid (5ml) was added. The mixture was heated to 50 °C, reacted for 6 hours, and concentrated to give a residue. The residue was dissolved in methanol and was concentrated to dryness, which was repeated 3 times. The resulting residue was dissolve in methanol, 1ml of aqueous ammonia was added, and the mixture was concentrated, and purified by a preparation plate to afford 10 mg of the final product with a yield of 18.9%.

$^1$H NMR (400 MHz,DMSO-d6) δ 13.31 (s, 1H), 12.83 (d, J = 33.0 Hz, 1H), 9.85 (s, 1H), 9.39 (dd, J = 5.0Hz, J =1.7 Hz, 1H), 8.37 - 8.31 (m, 1H), 8.07 (s, 1H), 7.92 (dd, J = 8.5Hz, J = 5.0 Hz, 1H), 7.40 (s, 1H), 7.13 (d, J = 8.1 Hz, 1H), 7.03 (d, J = 11.9 Hz, 1H), 6.92 (d, J = 9.1 Hz, 1H), 4.84 - 4.45 (m, 4H), 2.49 (q, J = 7.5 Hz, 2H), 1.02 (t, J = 7.5 Hz, 3H).

**Example 16: (S)-(2-(6-(2-ethyl-5-fluoro-4-hydroxyphenyl)-1H-indazol-3-yl)-4,6-dihydropyrrolo[3,4-d]imidazol-5-(1H)-yl)(3-hydroxylpyrrolidin-1-yl)ketone (MDI-216)**

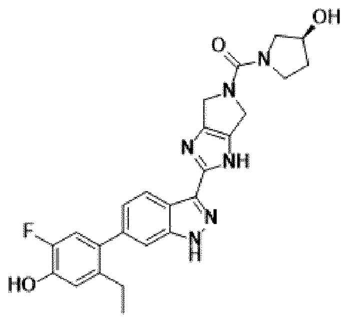

MDI-216

**Synthetic route of MDI-216:**

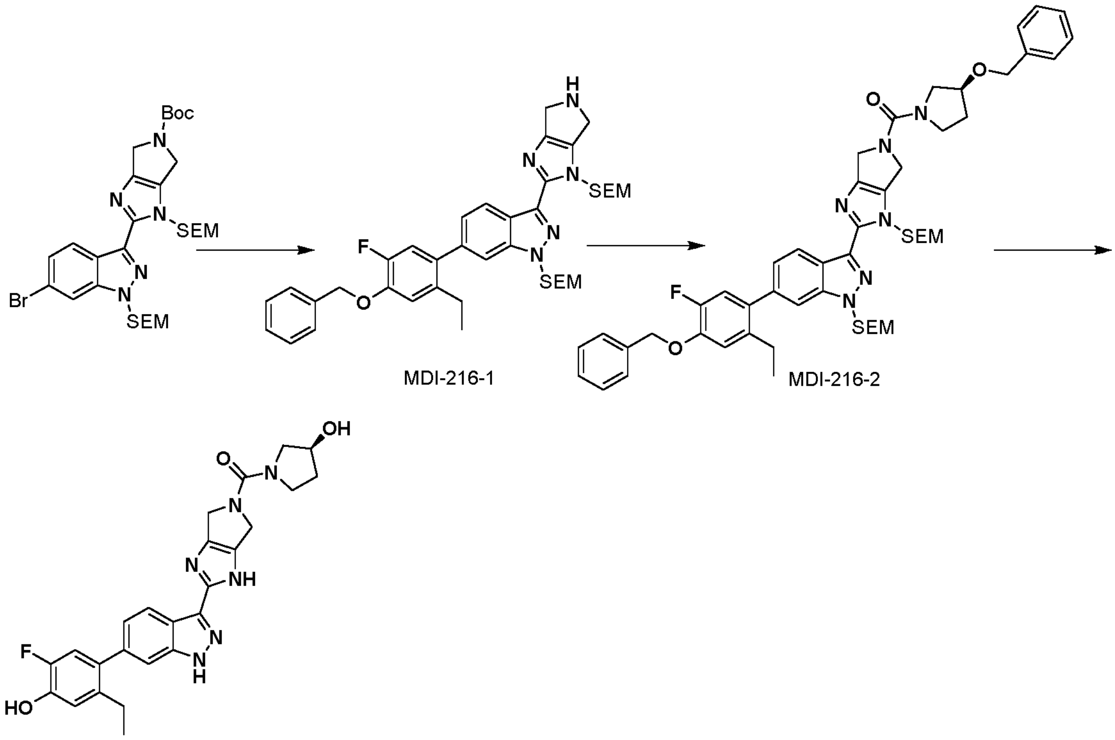

MDI-216-1

MDI-216-2

MDI-216

**Synthesis method:**

**Synthesis of intermediate MDI-216-1: 6-(4-(benzyloxy)-2-ethyl-5-fluorophenyl)-1-((2-(trimethylsilyl)ethoxy)methyl)-3-(1-((2-(trimethylsilyl)ethoxy) methyl)- 1,4,5,6-tetrahydropyrrolo[3,4-d]imidazol-2-yl)-1H-indazole**

Tert-butyl 2-(6-bromo-1-((2-(trimethylsilyl)ethoxy)methyl)-1H-indol-3-yl)-1-((2-(trimethylsilyl)ethoxy)methyl)-4,6-dihydropyrrolo[3,4-d]imidazol-5(1H)-carboxylate (500 mg, 0.75 mmol), 2-(4-(benzyloxy)-2-ethyl-5-fluorophenyl)-4,4,5,5-tetramethyl-1,3,2-dioxaborane (401 mg, 1.13 mmol), Pd(dppf)Cl2 (75 mg, 0.075 mmol) and potassium phosphate (495 mg, 2.25 mmol) were dissolved in 1,4-dioxane (30 ml) and water (6 ml).The atmosphere was replaced with nitrogen, which was repeated 3 times. The mixture was heated to 100°C, reacted for 16 hours and cooled to room temperature. Water was added and the resulting mixture was extracted twice with ethyl acetate, and the organic phases were combined, washed with water and saturated brine, dried over anhydrous sodium sulfate, concentrated, and purified by silica gel column. The purified product was dissolved in 25 ml of dichloromethane, and 5 ml of trifluoroacetic acid was added dropwise. The mixture was stirred at room temperature for 30 minutes, and concentrated to give a residue. The residue was dissolved in dichloromethane and was concentrated to dryness, which was repeated 3 times. The resulting residue was purified with silica gel column to afford 210 mg of intermediate MDI-216-1 with a yield of 39.2%.

$^1$H NMR (400 MHz, CDCl3) δ 8.48 (d, J = 8.3 Hz, 1H), 7.52 (d, J = 7.4 Hz, 1H), 7.49 - 7.37 (m, 5H), 7.25 (d, J = 8.4 Hz , 1H), 7.23-6.96(m, 2H), 5.93 (s, 2H), 5.77 (s, 2H), 5.23 (s, 2H), 4.21 (d, J = 35.1 Hz, 4H), 3.66 - 3.52 ( m, 4H), 2.54 (q, J = 7.6 Hz, 2H), 1.05 (t, J = 7.5 Hz, 3H), 0.95 - 0.89 (m, 4H), 0.02 (s, 9H), -0.05 (d, J = 3.4 Hz, 9H).

**Synthesis of intermediate MDI-216-2: (S)-(2-(6-(4-(benzyloxy)-2-ethyl-5-fluorophenyl)-1-((2-(trimethylsilyl)ethoxy)methyl)-1H-indazol-3-yl)-1-((2-(trimethylsilyl)ethoxy)methyl)-4,6-dihydropyrrolo[3,4-d]imidazol-5-(1H)-yl)(3-(benzyloxy)pyrrolidin-1-yl)ketone**

Triphosgene (25.8 mg, 0.09 mmol) was dissolved in 5 ml of tetrahydrofuran, and intermediate MDI-216-1 (80 mg, 0.09 mmol) in tetrahydrofuran (5 ml) was added dropwise at 0°C. The mixture was stirred at room temperature for 10 minutes and (S)-3-(benzyloxy) pyrrolidine (31.9 mg, 0.18 mmol) in tetrahydrofuran was added. The mixture was stirred at room temperature for 5 minutes, and water was added. The resulting mixture was extracted twice with ethyl acetate, and the organic phases were combined, washed with water and saturated brine, dried over aqueous sodium sulfate, concentrated, and purified by silica gel column to afford 71 mg of intermediate MDI-216-2 with a yield of 86.1%.

$^1$H NMR (400 MHz, CDCl3) δ 8.46 (d, J = 8.3 Hz, 1H), 7.53 - 7.51 (m, 2H), 7.47 - 7.42 (m, 3H), 7.39 - 7.29 (m, 6H), 7.24 ( dd, J = 8.4 Hz, J = 4.0 Hz,1H), 7.07 - 6.97 (m, 2H), 5.95 (s, 2H), 5.77 (s, 2H), 5.23 (s, 2H), 4.92 - 4.88 (m ,

2H), 4.76 - 4.69 (m, 2H), 4.60 (s, 2H), 4.23 (s, 1H), 3.76 - 3.70 (m, 2H), 3.66 - 3.58 (m, 6H), 2.54 (q, J = 7.5 Hz, 2H), 2.15 - 2.13 (m, 1H), 2.06 - 2.02 (m, 1H), 1.05 (t, J = 7.5 Hz, 3H), 0.95 - 0.91 (m, 4H) ,-0.01 -- 0.11(m, 18H).

**Synthesis of compound MDI-216: (S)-(2-(6-(2-ethyl-5-fluoro-4-hydroxyphenyl)-1H-indazol-3-yl)-4,6-dihydropyrrolo[3,4-d]imidazol-5-(1H)-yl)(3-hydroxylpyrrolidin-1-yl)ketone**

Intermediate MDI-216-2 (83 mg, 0.11 mmol) was dissolved in methanol (10ml), to which 10 mg Pd/C and concentrated hydrochloric acid (5ml) were added. The mixture was heated to 50 °C, reacted for 6 hours, filtered and concentrated to give a residue. The residue was dissolved in methanol and was concentrated to dryness, which was repeated 3 times. The resulting residue was dissolve in methanol, 1ml of aqueous ammonia was added, and then the mixture was concentrated, and purified by a preparation plate to afford 8 mg of the final product with a yield of 15.2%.

$^1$H NMR (400 MHz, MeOD-d4) δ 8.27 (d, *J* = 8.4 Hz, 1H), 7.43 (d, *J* = 1.0 Hz, 1H), 7.17 (d, *J* = 8.4 Hz, 1H), 6.97 - 6.90 (m, 2H), 4.81 - 4.61 (m, 4H), 4.46 - 4.44 (m, 1H), 3.79 - 3.69 (m, 2H), 3.50 - 3.43 (m, 2H), 2.56 (q, *J* = 7.5 Hz, 2H), 2.09 - 1.99 (m, 2H), 1.08 (t, *J* = 7.5 Hz, 3H).

**Example 17: 5-ethyl-2-fluoro-4-(3-(5-(4-hydroxylcyclohexyl)-1,4,5,6-tetrahydropyrrolo[3,4-d]imidazol-2-yl)-1H-indazol-6-yl)phenol (MDI-217)**

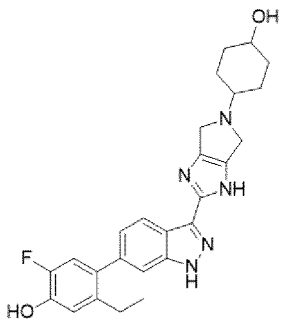

MDI-217

**Synthetic route of MDI-217:**

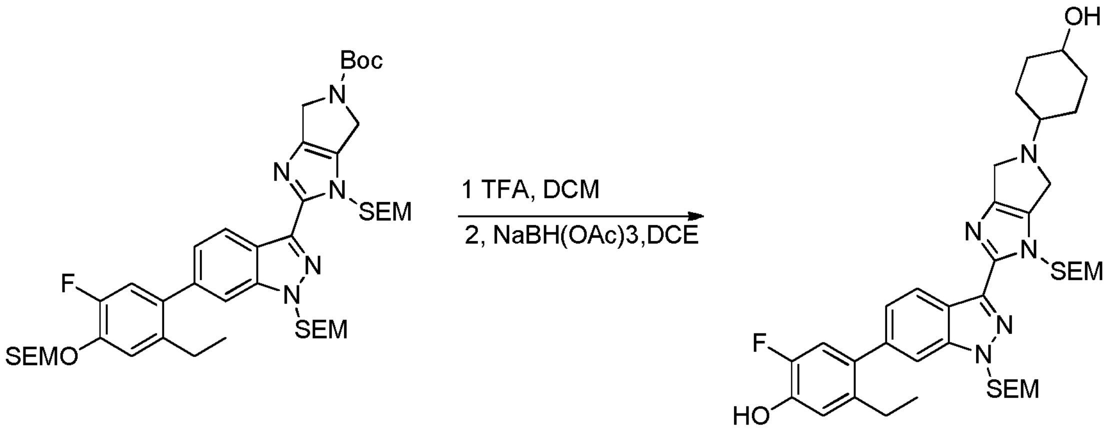


MDI-217-1

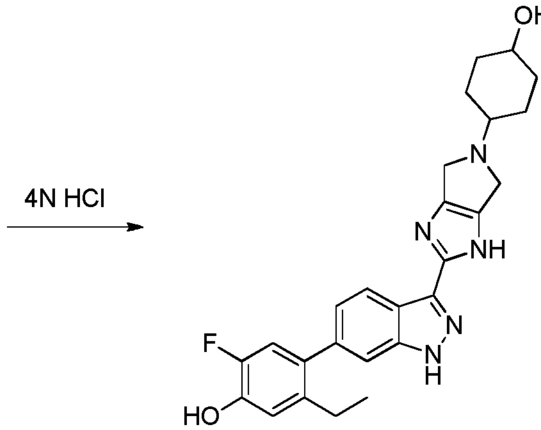


MDI-217

**Synthesis method:**

**Synthesis of intermediate MDI-217-1: 5-ethyl-2-fluoro-4-(3-(5-(4-hydroxylcyclohexyl)-1-((2-(trimethylsilyl)ethoxy)methyl)-1,4,5,6-tetrahydropyrrolo [3,4-d]imidazol-2-yl)-1-((2-(trimethylsilyl)ethoxy)methyl)-1H-indazol-6-yl)phenol**

Tert-butyl 2-(6-(2-ethyl-5-fluoro-4-((2-(trimethylsilyl)ethoxy)methoxy) phenyl) 1-((2-(trimethylsilyl)ethoxy)methyl)-1H-indazol-3-yl)-1-((2-(trimethylsilyl)ethoxy) methyl)-4,6-dihydropyrrolo[3,4-d]imidazol-5(1H)-carboxylate (65.0 mg, 0.08mmol) was dissolved in 5ml dichloromethane, and 1ml trifluoroacetic acid was added. The mixture was stirred at room temperature for 30 minutes. Water was added, pH was adjusted with saturated sodium bicarbonate to pH=9, and the resulting mixture was extracted with DCM, washed with water and saturated brine, dried over anhydrous sodium sulfate, and concentrated. The obtained solid was dissolved in 1,2-dichloroethane, and 4-hydroxycyclohexanone (17.4 mg, 0.15 mmol) was added, which was stirred at room temperature for 1 hour. And then sodium triacetyl borohydride (32.3 mg, 0.15 mmol) was added, and it was allowed to react at room temperature for 3 hours. Water was added to quench the reaction, and the resulting mixture was extracted with DCM twice, and the organic phases were combined, washed with water and saturated brine, dried over anhydrous sodium sulfate, concentrated, and purified by silica gel column to afford the product MDI-217-1 with a yield of 38.2%.

$^1$H NMR (400 MHz, CDCl3) δ 8.46 (dd, J = 8.0 Hz, J = 4.0 Hz, 1H), 7.44 (s, 1H), 7.22 (dd, J = 8.0 Hz, J = 4.0 Hz, 1H), 7.02-6.95 (m, 2H), 5.93 (d, J = 4.0 Hz, 2H), 5.77 (d, J = 4.0 Hz, 2H), 4.27-4.07 (m, 4H), 3.76-3.74 (m, 1H) , 3.65-3.56 (m, 4H), 2.76-2.74 (m, 1H), 2.55-2.49 (m, 2H), 2.11-2.03 (m, 3H), 1.88-1.86 (m, 2H), 1.74-1.66 (m, 3H), 1.08 (t, J = 4.0 Hz, 3H), 0.94-0.89 (m, 4H), 0.02(s, 9H), -0.05(s,9H).

**Synthesis of compound MDI-217: 5-ethyl-2-fluoro-4-(3-(5-(4-hydroxylcyclohexyl)-1,4,5,6-tetrahydropyrrolo[3,4-d]imidazol-2-yl)-1H-indazol-6-yl)phenol**

Intermediate MDI-217-1 (21.0 mg, 0.03 mmol) was dissolved in 4 ml of methanol, and 2 ml of concentrated hydrochloric acid was added. The mixture was heated to 50 °C, reacted for 6 hours, and concentrated to give a residue. The residue was dissolved in methanol and was concentrated to dryness, which was repeated 3 times. To the resulting residue, 1 ml of methanol was added, 2ml of concentrated aqueous ammonia was added for neutralization, and the mixture was concentrated to give a residue. The residue was dissolved in methanol and was concentrated to dryness, which was repeated 2 times. The resulting residue was purified by a preparation plate to afford 5.3 mg of the product with a yield of 39.5%.

$^1H$ NMR (400 MHz, MeOD-d4) δ 8.27 (dd, J = 4.0 Hz, J = 8.0 Hz,1H), 7.43-7.42 (m, 1H), 7.19 (dd, J = 4.0 Hz, J = 8.0 Hz , 1H), 6.96-6.88 (m, 2H), 3.98 (s, 4H), 3.93 (m, 1H), 2.74-2.72 (m, 1H), 2.58(q, J = 8.0 Hz, 2H), 2.04- 2.05 (m, 1H), 1.90-1.80 (m, 5H), 1.64-1.62 (m, 2H), 1.10 (t, J = 8.0 Hz, J = 16.0 Hz, 3H).

**Example 18: 4-(3-(5-(cyclopropanesulfonyl)-1,4,5,6-tetrahydropyrrolo [3,4-d]imidazol-2-yl)-1H-indazol-6-yl)-5-ethyl-2-fluorophenol (MDI-218)**

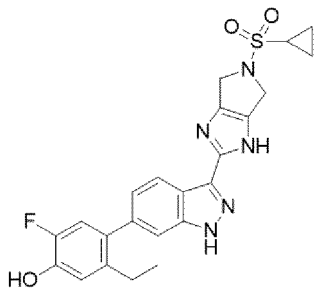

MDI-218

**Synthetic route of MDI-218:**

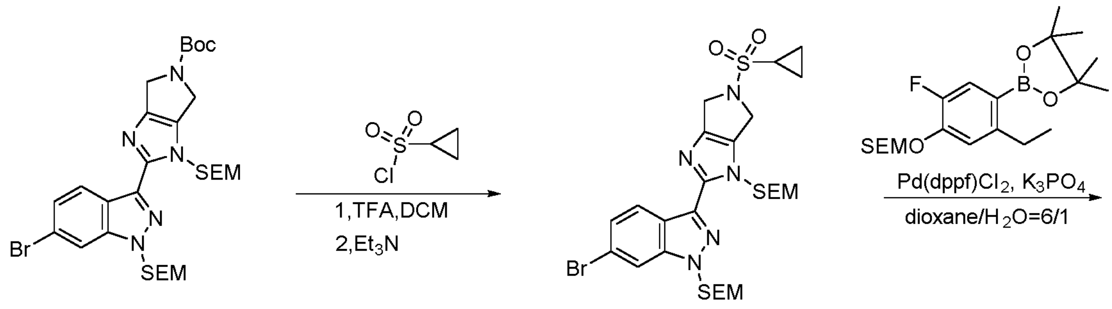


MDI-218-1

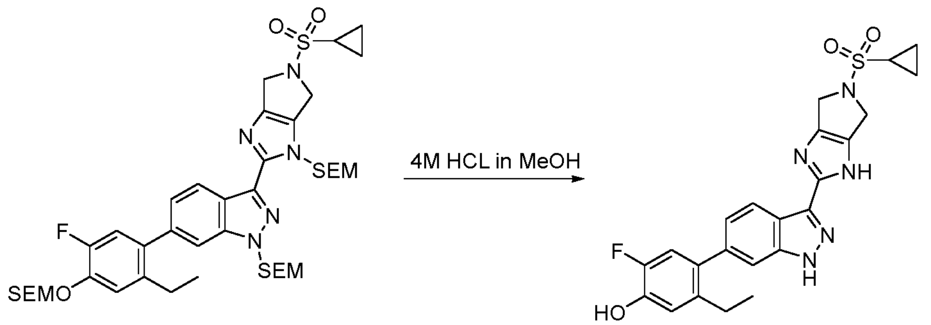


MDI-218-2

MDI-218

**Synthesis method:**

**Synthesis of intermediate MDI-218-1: 6-bromo-3-(5-(cyclopropanesulfonyl)-1-((2-(trimethylsilyl)ethoxy)methyl)-1,4,5,6-tetrahydropyrrolo [3,4-d] imidazol-2-yl)-1-((2-(trimethylsilyl)ethoxy)methyl)-1H-indazol**

Tert-butyl 2-(6-bromol-((2-(trimethylsilyl)ethoxy)methyl)-1H-indazol-3-yl)-1-((2-(trimethylsilyl)ethoxy)methyl)-4,6-dihydropyrrolo[3,4-d]imidazol-5(1H)-carboxylate (100 mg, 0.15 mmol) was dissolved in 5ml dichloromethane, and 1ml trifluoroacetic acid was added. The mixture was stirred at room temperature for 30 minutes, concentrated, quenched with sodium bicarbonate, and extracted twice with dichloromethane. The organic phases were combined, washed with water and saturated brine, dried over anhydrous sodium sulfate and concentrated. The obtained compound was dissolved in 5ml DCM and Et3N (0.08ml, 0.59mmol), and cooled to 0°C. Cyclopropylsulfonyl chloride (22.4 mg, 0.16mmol) was slowly added. It was allowed to react at room temperature for 2 hours, and water was added to quench the reaction. The resulting mixture was extracted with DCM twice, and the organic phases were combined, washed with water and saturated brine, dried over anhydrous sodium sulfate, concentrated, and purified by silica gel column to afford intermediate MDI-218-1 with a yield of 36.0%.

$^1$H NMR (400 MHz, CDCl3) δ 8.37 (d, J = 8.0 Hz, 1H), 7.80 (d, J = 4.0 Hz, 1H), 7.43 (d, J = 8.0 Hz, 1H), 5.91 (s, 2H ), 5.73 (s, 2H), 4.75-4.74 (m, 2H), 4.66-4.65 (m, 2H), 3.63-3.58 (m, 4H), 2.50-2.44 (m, 1H), 1.33-1.31 (m , 2H), 1.06-1.02 (m, 2H), 0.96-0.91 (m, 4H), 0.00-0.05 (m, 18H).

**Synthesis of intermediate MDI-218-2: 3-(5-cyclopropanesulfonyl)-1-((2-(trimethylsilyl)ethoxy)methyl)-1,4,5,6-tetrahydropyrrolo[3,4-d]imidazol-2-yl)-6-(2-ethyl-5-fluoro-4-((2-(trimethylsilyl)ethoxy)methoxy)phenyl)-1-((2-(trimethylsilyl) ethoxy)methyl)-1H-indazole**

The intermediate MDI-218-1 (36.0 mg, 0.05 mmol), (2-((5-ethyl-2-fluoro-4-(4, 4,5,5-tetramethyl-1,3,2-dioxaborolan-2-yl)phenoxy)methoxy)ethyl)trimethylsilane (25.5 mg, 0.06 mmol), Pd(dppf)Cl2 (3.9 mg, 0.005 mmol) and potassium phosphate (34.2 mg, 0.16 mmol) were dissolved in 1,4-dioxane (6 ml) and water (1 ml). The atmosphere was replaced with nitrogen, which was repeated 3 times. The mixture was heat to 100°C, reacted overnight, and cooled to room temperature. Water was added, and the resulting mixture was extracted twice with ethyl acetate, and the organic phases were combined, washed with water and saturated brine, dried over anhydrous sodium sulfate, concentrated, and purified by silica gel column to afford the intermediate MDI- 218-2, the yield was 70.0%.

$^1$H NMR (400 MHz, CDCl3) δ 8.47 (d, J = 8.0 Hz, 1H), 7.48 (s, 1H), 7.26 (d, J = 7.9 Hz, 1H), 7.18 (d, J = 8.0 Hz, 1H), 7.04 (d, J = 12.0 Hz, 1H), 5.95 (s, 2H), 5.78 (s, 2H), 5.34 (s, 2H), 4.76 (s, 2H), 4.68 (s, 2H), 3.88 (t, J = 8.0 Hz, 2H), 3.68- 3.57 (m, 4H), 2.56 (q, J = 7.6 Hz, 2H), 2.24 (t, J = 7.7 Hz, 1H), 1.12- 0.86 (m, 13H),-0.01-0.06 (m, 27H).

**Synthesis of compound MDI-218: 4-(3-(5-(cyclopropanesulfonyl)-1,4,5,6-tetrahydropyrrolo[3,4-d]imidazol-2-yl)-1H-indazol-6-yl)-5-ethyl-2-fluorophenol**

Intermediate MDI-218-2 (36.0 mg, 0.04 mmol) was dissolved in methanol (4ml), to which concentrated hydrochloric acid (2ml) was added. The mixture was heated to 50°C, reacted for 6 hours, and concentrated. The solid was dissolved in 1ml methanol, and pH was adjusted with sodium bicarbonate to 8-9, and the resulting mixture was extracted 4 times with dichloromethane. The organic phases were combined, dried over anhydrous sodium sulfate, and purified by a preparation plate to afford 16 mg of the final product with a yield of 81.4%.

$^1$H NMR (400 MHz, MeOD-d4) δ 8.27 (d, J = 8.0 Hz, 1H), 7.43 (s, 1H), 7.18 (dd, J = 8.0Hz, J = 4.0 Hz, 1H), 6.93 (dd , J = 20.0Hz, J = 12.0 Hz, 2H), 4.65 (s, 4H), 2.76-2.69(m, 1H), 2.60- 2.51 (m, 2H), 1.20- 1.18 (m, 2H), 1.10-1.06 (m, 5H).

**Example 19: 4-(3-(5-(cyclobutylsulfonyl)-1,4,5,6-tetrahydropyrrolo[3,4-d]imidazol-2-yl)-1H-indazol-6-yl)-5-ethyl-2-fluorophenol (MDI-219)**

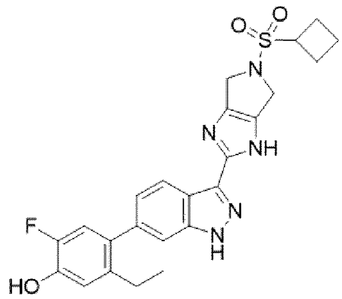

MDI-219

**Synthetic route of MDI-219:**

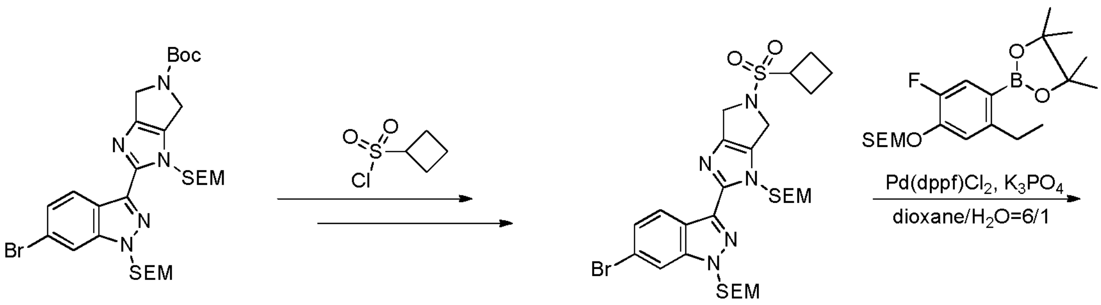


MDI-219-1

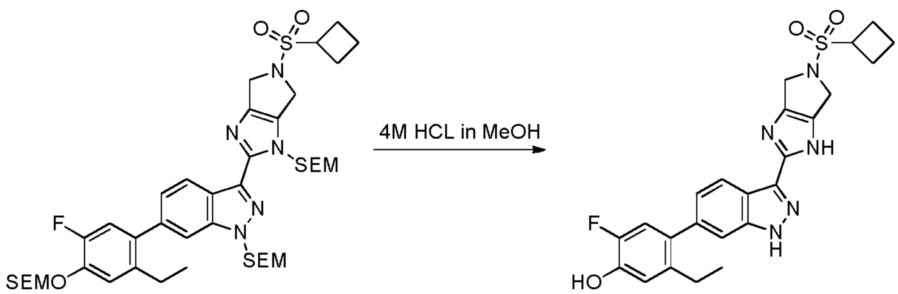


MDI-219-2

MDI-219

**Synthesis method**

**Synthesis of intermediate MDI-219-1: 6-bromo-3-(5-(cyclobutylsulfonyl)-1-(2-(trimethylsilyl)ethoxy)methyl)-1,4,5,6-tetrahydropyrrolo[3,4-d]imidazol-2-yl)-1-(2-(trimethylsilyl)ethoxy)methyl)-1H-indazole**

Tert-butyl 2-(6-bromo-1-((2-(trimethylsilyl)ethoxy)methyl)-1H-indazol-3-yl)-1-((2-(trimethylsilyl)ethoxy)methyl)-4,6-dihydropyrrolo[3,4-d]imidazol-5-(1H)-carboxylate (100 mg, 0.15 mmol) was dissolved in 5ml dichloromethane, and 1ml trifluoroacetic acid was added. The mixture was stirred at room temperature for 30 minutes, concentrated, and quenched with sodium bicarbonate. The resulting mixture was extracted twice with dichloromethane, and the organic phases were combined, washed with water and saturated brine, dried over anhydrous sodium sulfate and concentrated. The obtained compound was dissolved in 5ml DCM and Et3N (0.08ml, 0.59mmol), and cooled to 0°C. Cyclobutylsulfonyl chloride (24.6 mg, 0.16mmol) was slowly added. It was allowed to react at room temperature for 2 hours, and water was added to quench the reaction. The resulting mixture was extracted with DCM twice, and the organic phases were combined, washed with water and saturated brine, dried over anhydrous sodium sulfate, concentrated, and purified by silica gel column to afford intermediate MDI-219-1 with a yield of 32.1%.

$^1$H NMR (400 MHz, CDCl3) δ 8.36 (d, J = 8.0 Hz, 1H), 7.79 (s, 1H), 7.43 (d, J = 8 Hz, 1H), 5.92 (d, J = 4.0 Hz, 2H), 5.73 (s, 2H), 4.70 (s, 2H), 4.60 (s, 2H), 4.03- 3.95 (m, 1H), 3.65- 3.56 (m, 4H), 2.75- 2.64 (m, 2H), 2.37- 2.30 (m, 2H), 2.11- 2.04 (m, 2H), 0.95-0.90 (m, 4H), 0.00-0.05 (m, 18H).

**Synthesis of intermediate MDI-219-2: 3-(5-(cyclobutylsulfonyl)-1-((2-(trimethylsilyl)ethoxy)methyl)-1,4,5,6-tetrahydropyrrolo[3,4-d]imidazol-2-yl)-6-(2-ethyl-5-fluoro-4-((2-(trimethylsilyl)ethoxy)methoxy)phenyl)-1-((2-(trimethylsilyl)ethoxy)methyl)-1H-indazole**

The intermediate MDI-219-1 (33.0 mg, 0.05 mmol), (2-((5-ethyl-2-fluoro-4-(4, 4,5,5-tetramethyl-1,3,2-dioxaborolan-2-yl)phenoxy)methoxy)ethyl)trimethylsilane (22.6 mg, 0.06 mmol), Pd(dppf)$Cl_2$(3.5 mg, 0.005 mmol), and potassium phosphate (30.2 mg, 0.14 mmol) were dissolved in 1,4-dioxane (6 ml) and water (1 ml). The atmosphere was replaced with nitrogen, which was repeated 3 times. The mixture was heated to 100 °C, reacted overnight, and cooled to room temperature. Water was added, the resulting mixture was extracted twice with ethyl acetate, and the organic phases were combined, washed with water and saturated brine, dried over anhydrous sodium sulfate, concentrated, and purified by silica gel column to afford intermediate MDI-219-2 with a yield of 73.5%.

$^1$H NMR (400 MHz, CDCl3) δ 8.45 (d, J = 8.0 Hz, 1H), 7.48 (s, 1H), 7.26 (dd, J = 8.3Hz, J =1.3 Hz, 1H), 7.18 (d, J = 8.0 Hz, 1H), 7.07- 7.02 (m, 1H), 5.93 (s, 2H), 5.77 (s, 2H), 5.34 (s, 2H), 4.74- 4.70(m, 2H), 4.64- 4.60( m, 2H), 4.00-3.98 (m, 1H), 3.91- 3.86 (m, 2H), 3.66- 3.58 (m, 4H), 2.75 - 2.66 (m, 2H), 2.58- 2.54 (m, 2H), 2.11- 2.02 (m, 4H), 1.10-1.04 (m, 3H), 1.01- 0.96 (m, 6H), -0.02-0.05 (m, 27H).

**Synthesis of compound MDI-219: 4-(3-(5-(cyclobutylsulfonyl)-1,4,5,6-tetrahydropyrrolo[3,4-d]imidazol-2-yl)-1H-indazol-6-yl)-5-ethyl-2-fluorophenol**

Intermediate MDI-219-2 (31.0 mg, 0.04 mmol) was dissolved in methanol (4ml), to which concentrated hydrochloric acid (2ml) was added. The mixture was heated to 50°C, reacted for 6 hours, and concentrated. The solid was dissolved in 1ml methanol, and pH was adjusted with sodium bicarbonate to pH=8-9. The resulting mixture was extracted with dichloromethane 4 times, and the organic phases were combined, dried over anhydrous sodium sulfate, and purified by a preparation plate to afford 12 mg of the final product with a yield of 65.7%.

$^1$H NMR (400 MHz, MeOD-d4) δ 8.26 (d, J = 8.0 Hz, 1H), 7.43 (s, 1H), 7.17 (dd, J = 8.4Hz, J =1.4 Hz, 1H), 6.93 (dd, J = 20.0Hz, J =12.0 Hz, 2H), 4.60 (s, 4H), 4.26-4.18 (m, 1H), 2.68- 2.52 (m, 4H), 2.40- 2.31 (m, 2H), 2.13- 2.02 (m, 2H), 1.08 (t, J = 7.5 Hz, 3H).

**Example 20: 4-(3-(5-(cyclopentylsulfonyl)-1,4,5,6-tetrahydropyrrolo[3,4-d]imidazol-2-yl)-1H-indazol-6-yl)-5-ethyl-2-fluorophenol (MDI-220)**

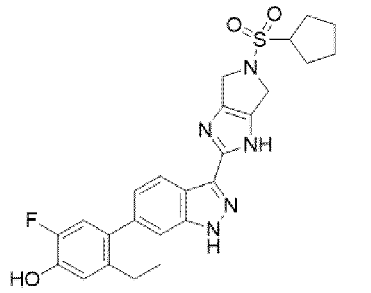

MDI-220

**Synthetic route of MDI-220:**

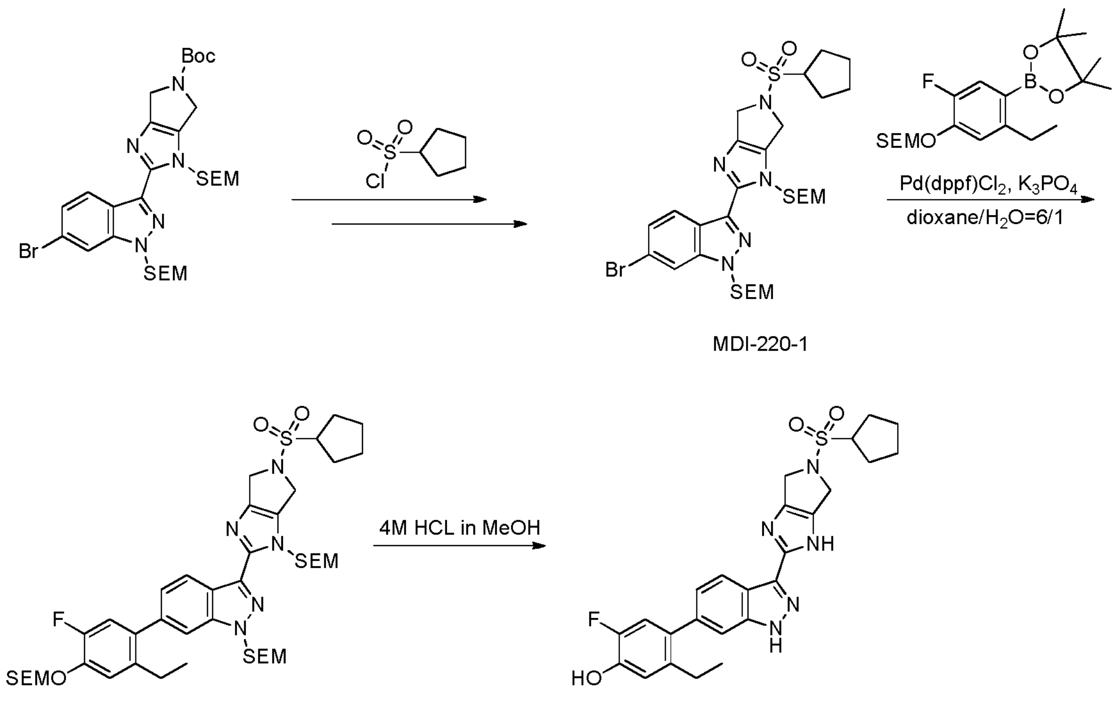


MDI-220-1

MDI-220-2

MDI-220

**Synthesis method:**

**Synthesis of intermediate MDI-220-1: 6-bromo-3-(5-(cyclopentylsulfonyl)-1-((2-(trimethylsilyl)ethoxy)methyl)-1,4,5,6-tetrahydropyrrol[3,4-d] imidazol-2-yl)-1-((2-(trimethylsilyl)ethoxy)methyl)-1H-indazol**

Tert-butyl 2-(6-bromo-1-((2-(trimethylsilyl)ethoxy)methyl)-1H-indazol-3-yl)-1-((2-(trimethylsilyl)ethoxy)methyl)-4,6-dihydropyrrolo[3,4-d]imidazol-5-(1H)-carboxylate (100 mg, 0.15 mmol) was dissolved in 5ml dichloromethane, and 1ml trifluoroacetic acid was added. The mixture was stirred at room temperature for 30 minutes, concentrated, and quenched with sodium bicarbonate. The resulting mixture was extracted twice with dichloromethane, and the organic phases were combined, washed with water and saturated brine, dried over anhydrous sodium sulfate and concentrated. The obtained compound was dissolved in 5ml DCM and Et3N (0.08ml, 0.59mmol), and cooled to 0°C. Cyclopentylsulfonyl chloride (26.8 mg, 0.16mmol) was slowly added and it was allowed to react at room temperature for 2 hours. And then water was added to quench the reaction. The resulting mixture was extracted with DCM twice, and the organic phases were combined, washed with water and saturated brine, dried over anhydrous sodium sulfate, concentrated, and purified by silica gel column to afford intermediate MDI-220-1 with a yield of 38.1%.

$^1$H NMR (400 MHz, CDCl3) δ 8.36 (d, J = 8.0 Hz, 1H), 7.80 (s, 1H), 7.43 (dd, J = 8.0Hz, J =1.6 Hz, 1H), 5.90 (s, 2H) ), 5.73 (s, 2H), 4.78- 4.72 (m, 2H), 4.68- 4.62 (m, 2H), 4.33 (t, J = 8.0 Hz, 1H), 3.63- 3.57(m, 4H), 2.14- 2.03 (m,

4H), 1.75- 1.56 (m, 4H), 0.96-0.90 (m, 4H), -0.02-0.05 (m, 18H).

**Synthesis of intermediate MDI-220-2: 3-(5-(cyclopentylsulfonyl)-1- ((2-(trimethylsilyl)ethoxy)methyl)-1,4,5,6-tetrahydropyrrolo[3,4-d]imidazol-2-yl)-6-(2-ethyl-5-fluoro-4-((2-(trimethylsilyl)ethoxy)methoxy)phenyl)-1-((2-(trimethylsilyl) ethoxy)methyl)-1H-indazole**

The intermediate MDI-220-1 (40 mg, 0.06 mmol), (2-((5-ethyl-2-fluoro-4-(4,4,5,5-tetramethyl-1,3,2-dioxaborolan-2-yl)phenoxy)methoxy)ethyl)trimethylsilane (27.3 mg, 0.07 mmol), Pd(dppf)Cl2 (4.2 mg, 0.006 mmol) and potassium phosphate ( 36.5mg, 0.17 mmol) were dissolved in 1,4-dioxane (6 ml) and water (1 ml). The atmosphere was replaced with nitrogen, which was repeated 3 times. The mixture was heated to 100 °C, reacted overnight, and cooled to room temperature. Water was added and the resulting mixture was extracted with ethyl acetate twice, and the organic phases were combined, washed with water and saturated brine, dried over anhydrous sodium sulfate, concentrated, and purified by silica gel column to afford intermediate MDI-220-2 with a yield of 62.9%.

$^1$H NMR (400 MHz, CDCl3) δ 8.46 (d, J = 8.0 Hz, 1H), 7.48 (s,1H), 7.26 (dd, J = 8.0Hz, J = 1.2 Hz, 1H), 7.18 (d, J = 8.0 Hz, 1H), 7.04 (d, J = 12.0 Hz, 1H), 5.94 (s, 2H), 5.78 (s, 2H), 5.34 (s, 2H), 4.80- 4.75 (m, 2H), 4.70 -4.65 (m, 2H), 4.33 (t, J = 8.0 Hz, 1H), 3.82- 3.80 (m, 2H), 3.73- 3.58 (m, 4H), 2.58- 2.53 (m, 2H), 2.18 - 2.05 (m, 4H), 1.79-1.58 (m, 4H), 1.11- 1.04 (m, 3H), 0.93- 0.90 (m, 6H), -0.02-0.06 (m, 27H).

**Synthesis of compound MDI-220: 4-(3-(5-(cyclopentylsulfonyl)-1,4,5,6-tetrahydropyrrolo[3,4-d]imidazol-2-yl)-1H-indazol-6-yl)-5-ethyl-2-fluorophenol**

Intermediate MDI-220-2 (32.0 mg, 0.04 mmol) was dissolved in methanol (4ml), to which concentrated hydrochloric acid (2ml) was added. The mixture was heated to 50 °C, reacted for 6 hours, and concentrated. The solid was dissolved in 1ml methanol, and pH was adjusted with sodium bicarbonate to 8-9. The resulting mixture was extracted 4 times with dichloromethane, and the organic phases were combined, dried over anhydrous sodium sulfate, and purified by a preparation plate to afford 10mg of the final product with a yield of 55.8%.

$^1$H NMR (400 MHz, MeOD-d4) δ 8.27 (dd, J = 8.0 Hz, J = 4.0 Hz,1H), 7.43 (s, 1H), 7.17 (dd, J = 8.0Hz, J = 1.4 Hz, 1H), 6.93 (dd, J = 20.0Hz, J =12.0 Hz, 2H), 4.65 (s, 4H), 3.91-3.83 (m, 1H), 2.58- 2.52 (m, 2H), 2.13 - 2.03 (m, 4H), 1.89- 1.78 (m, 2H), 1.75-1.64 (m, 2H), 1.08 (t, J = 8.0 Hz, 3H).

**Example 21: 5-ethyl-2-fluoro-4-(3-(5-((1-methyl-1H-pyrazol-4-yl)methyl) -1,4,5,6-tetrahydropyrrolo[3,4-d]imidazol-2-yl)-1H-indazol-6-yl)phenol (MDI-221)**

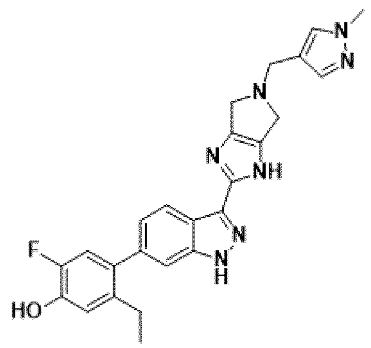

MDI-221

**Synthetic route of MDI-221:**

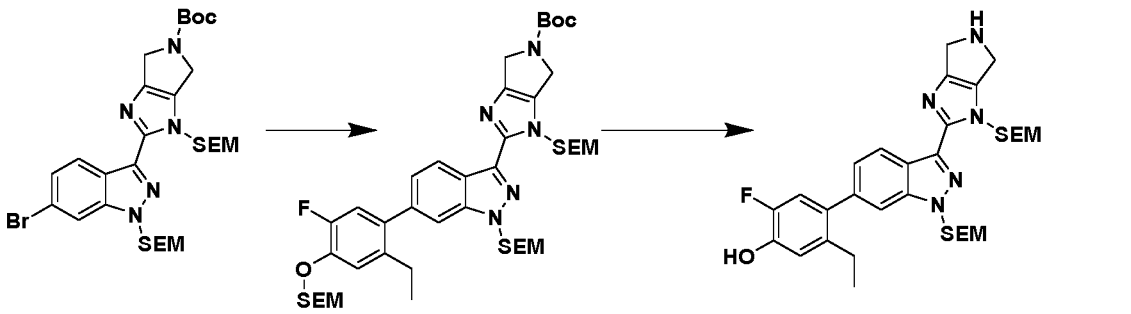

21 22

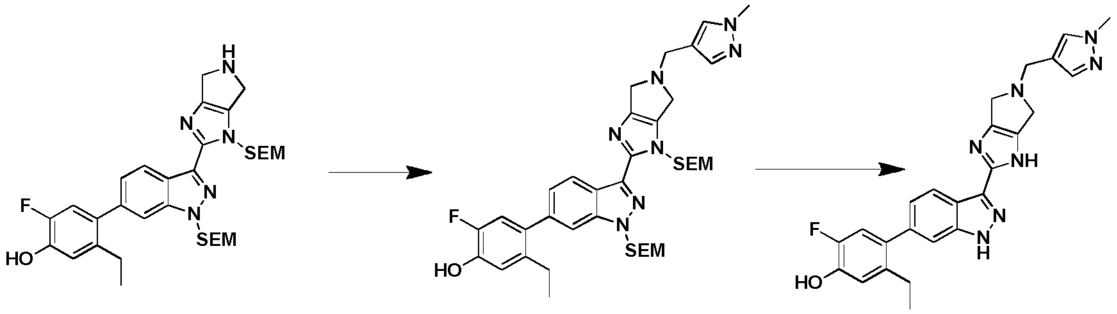

MDI-221-1 MDI-221

**Synthesis method:**

**Synthesis of intermediate 21: Tert-butyl 2-(6-(2-ethyl-5-fluoro-4-((2-(trimethylsilyl) ethoxy)methoxy)phenyl)1-((2-(trimethylsilyl)ethoxy)methyl)-1H-indazol-3-yl)-1-((2-(trimethylsilyl)ethoxy)methyl)-4,6-dihydropyrrolo[3,4-d]imidazol-5(1H)-carboxylate**

The intermediate tert-butyl 2-(6-bromo-1-((2-(trimethylsilyl)ethoxy)methyl)-1H-indazol-3-yl)-1-((2-(trimethylsilyl)ethoxy)methyl)-4,6-dihydropyrrolo[3,4-d]imidazol-5(1H)-carboxylate (39.8 mg, 0.06mmol), intermediate (2-((5-ethyl-2-fluoro-4-(4,4,5,5-tetramethyl-1,3,2-dioxaboran-2-yl)phenoxy)methoxy)ethyl)trimethylsilane (27.1 mg, 0.07 mmol), Pd(dppf)Cl2 (4.2 mg, 0.006 mmol) and potassium phosphate (36.2 mg, 0.17 mmol) were dissolved in 1,4-dioxane (10ml) and water (2ml). The atmosphere was replaced with nitrogen, which was repeated 3 times. The mixture was heated to 100 °C, reacted overnight, and cooled to room temperature. Water was added and the resulting mixture was extracted twice with ethyl acetate, and the organic phases were combined, washed with water and saturated brine, dried over anhydrous sodium sulfate, concentrated, and purified by silica gel column to afford Intermediate 21 with a yield of 78 %.

$^1$H NMR (400 MHz, CDCl3) δ8.46 (dd, J = 12.0 Hz, J = 8.0Hz, 1H), 7.45 (s, 1H), 7.24-7.21 (m,1H), 7.15 (d, J = 8.0 Hz,1H), 7.01 (d, J = 12.0Hz, 1H), 5.93 (d, J = 12.0Hz,2H), 5.75(s, 2H), 5.31(s, 2H), 4.65-4.50 (m, 4H) ), 3.88-3.84 (m, 2H), 3.63-3.55 (m, 4H), 2.57-2.51 (m, 2H), 1.54 (s, 9H), 1.07-1.03 (m, 3H), 0.90-0.85 (m , 4H), 0.03 (s, 9H), - 0.07 (s, 18H).

**Synthesis of intermediate 22: 5-ethyl-2-fluoro-4-(1-((2-(trimethylsilyl)ethoxy)methyl)-3-((2-trimethylsilyl)ethoxy)methyl)-1,4,5,6-tetrahydropyrrolo[3,4-d]imidazol-2-yl)-1H-indazol-6-yl)phenol**

The intermediate Tert-butyl 2-(6-(2-ethyl-5-fluoro-4-((2-(trimethylsilyl) ethoxy)methoxy)phenyl) 1-((2-(trimethylsilyl)ethoxy)methyl)-1H-indazol-3-yl)-1-((2-(trimethylsilyl)ethoxy)methyl)-4,6-dihydropyrrolo[3,4-d]imidazol-5(1H)-carboxylate (40 mg, 0.046mmol) was dissolved in 5ml of dichloromethane, to which 1ml of trifluoroacetic acid was added. The mixture was stirred at room temperature for 30 minutes, and concentrated to give a residue. The residue was dissolved in dichloromethane and was concentrated to dryness, which was repeated 3 times. The resulting residue was purified by silica gel column to afford Intermediate 22 with a yield of 43%.

$^1$H NMR (400 MHz, CDCl3) δ 8.44 (d, J = 8.0Hz, 1H), 7.20 (dd, J = 8.3Hz, J = 1.4Hz, 1H), 6.95 (dd, J = 20.0Hz, J = 8.0 Hz, 1H), 5.91 (s, 2H), 5.47 (s, 2H), 4.26-4.16 (m, 4H), 3.64-3.55 (m, 4H), 2.53-2.47 (m, 2H), 1.04 (t, J = 8.0Hz, 3H), 0.92-0.86 (m, 4H), -0.07 (s, 9H), -0.08 (s, 9H).

**Synthesis of intermediate MDI-221-1: 5-ethyl-2-fluoro-4-(3-(5-((1-methyl-1H-pyrazol-4-yl)methyl)-1-((2-(trimethylsilyl)ethoxy)methyl)-1,4,5,6-tetrahydropyrrolo [3,4-d] imidazol-2-yl)-1-((2-(trimethylsilyl)ethoxy)methyl)-1H-indazol-6-yl)phenol**

5-ethyl-2-fluoro-4-(1-((2-(trimethylsilyl)ethoxy)methyl)-3-((2-trimethylsilyl) ethoxy)methyl)-1,4,5,6-tetrahydropyrrolo[3,4-d]imidazol-2-yl)-1H-indazol-6-yl)phenol (40 mg, 0.064mmol) and 1-methyl-1H-pyrazol-4-formaldehyde (8.5 mg, 0.077mmol) were dissolved in 5ml 1,2-dichloroethane. It was allowed to react at room temperature for 10 minutes. The reaction was cooled to 0°C and sodium triacetoxyborohydride (26.9 mg, 0.128mmol) was added. After the addition was completed, the mixture was warmed to room temperature and reacted for 1- 2h. After the completion of the reaction, water was added to quench the reaction, the resulting mixture was extracted twice with dichloromethane, and the organic phases were combined, washed with water and saturated brine, dried over anhydrous sodium sulfate, concentrated, and purified by silica gel column to afford the intermediate MDI-221-1, yield 43.4%.

$^1$H NMR (400 MHz, CDCl3) δ 8.41 (d, J = 8.0 Hz, 1H), 7.51 (s, 1H), 7.40 (d, J = 4.0 Hz, 2H), 7.19-7.16 (m, 1H), 6.93 (dd, J = 24.0 Hz, J = 12.0 Hz, 2H), 5.87 (s, 2H), 5.73 (s, 2H), 4.01-3.98(m, 4H), 3.95 (s, 2H), 3.91 (s, 3H), 3.62-3.52(m, 4H), 2.51-2.45(m, 2H), 1.01 (t, J = 6.0Hz, 3H), 0.91-0.85 (m, 4H),-0.08 (s, 9H), -0.09 (s, 9H).

**Synthesis of compound MDI-221: 5-ethyl-2-fluoro-4-(3-(5-((1-methyl-1H-pyrazol-4-yl)methyl)-1,4,5,6-tetrahydropyrrolo [3,4-d] imidazol-2-yl)-1H-indazol-6-yl) phenol**

Intermediate MDI-221-1 (28 mg, 0.039mmol) was dissolved in methanol (4ml), to which concentrated hydrochloric acid (2ml) was added. The mixture was heated to 50 °C, reacted for 6 hours, and concentrated. The solid was dissolved in 1ml methanol, and 2ml concentrated aqueous ammonia was added and concentrated to give a residue. The residue was dissolved in methanol and was concentrated to dryness, which was repeated 3 times. The resulting residue was purified by a preparation plate to afford 5.0 mg of the final product with a yield of 28%.

$^1$H NMR (400 MHz, MeOD-d4) δ 8.25 (d, J = 8.0 Hz, 1H), 7.68 (s, 1H), 7.56 (s, 1H), 7.42 (s, 1H), 7.16 (dd, J= 8.4Hz, J=1.4 Hz, 1H), 6.93 (dd, J = 20.0Hz, J=12.0 Hz, 2H), 4.03-3.96 (m, 6H), 3.92 (s, 3H), 2.58-2.53 (m, 2H), 1.08 (t, J = 8.0 Hz, 3H).

**Example 22: 4-(3-(5-cyclopentyl-1,4,5,6-tetrahydropyrrolo[3,4-d]imidazol-2-yl)-1H-indazol-6-yl)-5-ethyl-2-fluorophenol(MDI-224)**

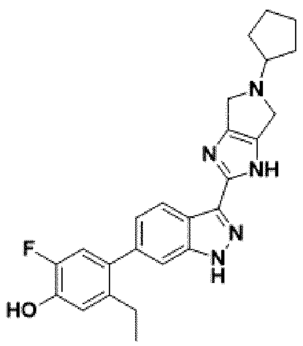

MDI-224

**Synthetic route of MDI-224:**

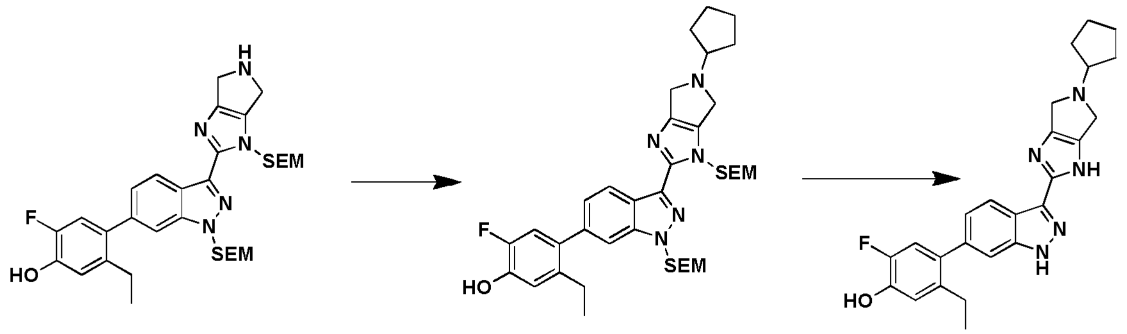

MDI-224-1

MDI-224

**Synthesis method:**

**Synthesis of intermediate MDI-224-1: 4-(3-(5-(cyclopentyl)-1-((2-(trimethylsilyl)ethoxy)methyl)-1,4,5,6-tetrahydropyrrolo [3,4-d] imidazol-2-yl)-1-((2-(trimethylsilyl)ethoxy)methyl)-1H-indazol-6-yl)-5-ethyl-2-fluorophenol**

5-ethyl-2-fluoro-4-(1-((2-(trimethylsilyl)ethoxy)methyl)-3-((2-trimethylsilyl)ethoxy)methyl)-1,4,5,6-tetrahydropyrrolo[3,4-d]imidazol-2-yl)-1H-indazol-6-yl)phenol (31.2 mg, 0.05mmol) and cyclopentanone (5.1mg, 0.06mmol) were dissolved in 5ml 1,2-dichloroethane. It was allowed to react at room temperature for 10 minutes. The reaction was cooled to 0°C, and sodium triacetoxyborohydride (21 mg, 0.1 mmol) was added. After the addition was completed, the mixture was warmed to room temperature and reacted for 1-2h. After the completion of the reaction, water was added to quench the reaction, the resulting mixture was extracted twice with dichloromethane, and the organic phases were combined, washed with water and saturated brine, dried over anhydrous sodium sulfate, concentrated, and purified by silica gel column to afford the intermediate MDI-221-1 with a yield of 56.8%.

$^1$H NMR (400 MHz, CDCl3) δ 8.40 (d, J = 8.0Hz, 1H), 7.41 (s, 1H), 7.16 (dd, J = 8.3Hz, J = 1.4Hz, 1H), 6.93 (dd, J = 24.0Hz, J = 12.0Hz, 2H), 5.89 (s, 2H), 5.73 (s, 2H), 4.08 (s, 2H), 3.99 (s, 2H), 3.62-3.55(m, 4H), 3.22 -3.19(m, 1H), 2.50-2.45(m, 2H), 1.98-1.89(m, 2H), 1.84-1.75(m, 2H),1.70-1.56(m,4H), 1.01 (t, J = 8.0Hz, 3H), 0.91-0.86 (m, 4H), -0.08 (s, 9H), -0.09 (s, 9H).

**Synthesis of compound MDI-224: 4-(3-(5-(cyclopentyl-1,4,5,6-tetrahydropyrrolo[3,4-d]imidazol-2-yl)-1H-indazol-6-yl)-5-ethyl-2-fluorophenol**

Intermediate MDI-224-1 (25 mg, 0.036 mmol) was dissolved in methanol (4ml), to which concentrated hydrochloric acid (5ml) was added. The mixture was heated to 50°C, reacted for 6 hours, and concentrated. The solid was dissolved in methanol and 2ml of concentrated aqueous ammonia was added, and the mixture was concentrated to give a residue. The residue was dissolved in methanol, and was concentrated to dryness, which was repeated 3 times. The resulting residue was purified by a preparation plate to afford 7.0 mg of the final product with a yield of 45.1%.

$^1$H NMR (400 MHz, MeOD-d4) δ 8.26(d, J= 8.0 Hz, 1H), 7.42(s, 1H),7.17 (d, *J*= 8.0 Hz, 1H),6.93 (dd, *J*= 20.0Hz, *J* =12.0 Hz, 2H),4.05-3.94 (m, 4H), 3.27-3.25 (m, 1H), 2.59-2.54 (m, 2H), 2.08-2.01(m, 2H), 1.87-1.79(m, 2H), 1.73-1.56(m,4H), 1.08 (t, *J* = 8.0 Hz, 3H).

**Example 23: 5-ethyl-2-fluoro-4-(3-(5-(tetrahydro-2H-pyran-4-yl)-1,4,5 6-tetrahydropyrrolo[3,4-d]imidazol-2-yl)-1H-indazol-6-yl)phenol (MDI-225)**

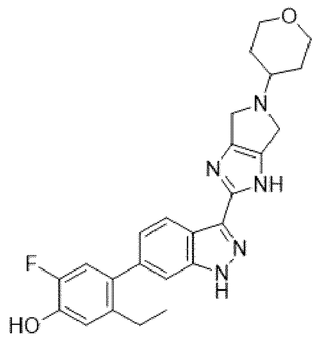

MDI-225

**Synthetic route of MDI-225:**

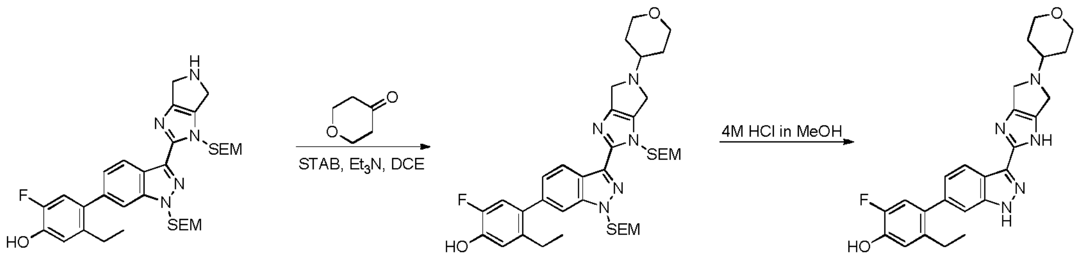


MDI-225-1

MDI-225

**Synthesis method:**

**Synthesis of intermediate MDI-225-1: 5-ethyl-2-fluoro-4-(3-(5-(tetrahydro-2H-pyran-4-yl)-1-((2-(trimethylsilyl)ethoxy)methyl)-1,4,5,6-tetrahydropyrrolo [3,4-d] imidazol-2-yl)-1-((2-(trimethylsilyl)ethoxy)methyl)-1H-indazol-6-yl)phenol**

5-ethyl-2-fluoro-4-(1-((2-(trimethylsilyl)ethoxy)methyl)-3-(1-((2-(trimethylsilyl)ethoxy)methyl)-1,4,5,6-tetrahydropyrrolo[3,4-d]imidazol-2-yl)-1H-indazol-6-yl)phenol (60 mg, 0.096 mmol) was dissolved in 5ml 1,2-dichloroethane, and tetrahydropyrone (14 mg, 0.14 mmol) was added. The mixture was stirred at room temperature for 5 minutes, and sodium triacetyl borohydride (41 mg, 0.19 mmol) was added. It was allowed to react at room temperature for 2 hours, and water was added to quench the reaction. The resulting mixture was extracted twice with DCM, and the organic phases were combined, washed with water and saturated brine, dried with anhydrous sodium sulfate, concentrated, and purified by silica gel column to afford intermediate MDI-225-1 with a yield of 51.4%.

$^1$H NMR (400 MHz, CDCl3) δ 8.43 (d, J = 8.0 Hz, 1H), 7.44 (s, 1H), 7.20 (dd, J = 8.0Hz, J =1.4 Hz, 1H), 6.96 (dd, J = 20.0Hz, J =12.0 Hz, 2H), 5.93 (s, 2H), 5.76 (s, 2H), 4.08 (s, 4H), 4.00 (s, 2H), 3.66- 3.56 (m, 4H), 3.55 -3.47 (m, 2H), 2.95- 2.86 (m, 1H), 2.54-2.46 (m, 2H), 1.99- 1.90 (m, 2H), 1.80- 1.71 (m, 2H), 1.04 (t, J = 8.0 Hz, 3H), 0.95- 0.87 (m, 4H), -0.03-0.08 (m, 18H).

**Synthesis of compound MDI-225: 5-ethyl-2-fluoro-4-(3-(5-(tetrahydro-2H-pyran-4-yl)-1,4,5,6-tetrahydropyrrolo[3,4-d]imidazol-2-yl)-1H-indazol-6-yl)phenol**

Intermediate MDI-225-1 (35.0 mg, 0.05 mmol) was dissolved in methanol (4ml), to which concentrated hydrochloric acid (2ml) was added. The mixture was heated to 50°C, reacted for 6 hours, and concentrated. The solid was dissolved in 1ml methanol, and pH was adjusted with sodium bicarbonate to 8-9. The resulting mixture was extracted 4 times with dichloromethane, and the organic phases were combined, dried over anhydrous sodium sulfate, and purified by a preparation plate to afford 12 mg of the final product with a yield of 64.1%.

$^1$H NMR (400 MHz, MeOD-d4) δ 8.26 (dd, J = 12.0 Hz, J = 4.0 Hz,1H), 7.42 (s, 1H), 7.16 (dd, J = 8.0Hz, J = 4.0 Hz, 1H ), 6.93 (dd, J = 20.0Hz, J =12.0 Hz, 2H), 4.07-3.99 (m, 6H), 3.52- 3.49 (m, 2H), 2.95- 2.90 (m, 1H), 2.58-2.53 (m, 2H), 2.00- 1.97 (m, 2H), 1.69- 1.59 (m, 2H), 1.08 (t, J = 8.0 Hz, 3H).

**Example 24: 1-(2-(6-(2-ethyl-5-fluoro-4-hydroxyphenyl)-1H-indazol-3-yl)-4,6-dihydropyrrolo[3,4-d]imidazol-5-(1H)-yl)ethan-1-one (MDI-226)**

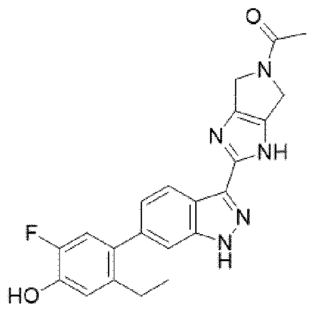

MDI-226

**Synthetic route of MDI-226:**

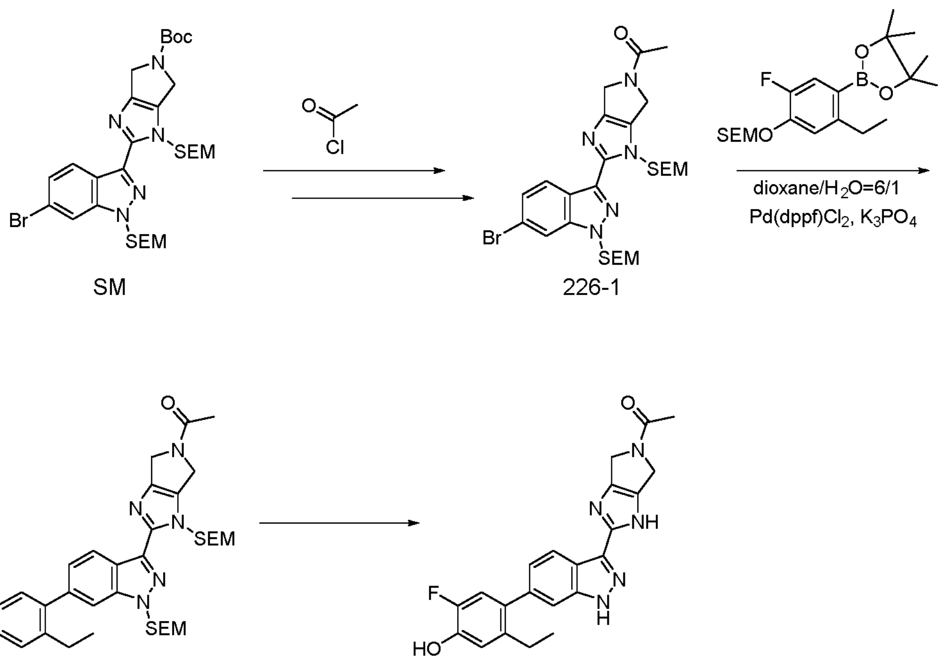


SM

226-1

226-2

MDI-226

**Synthesis method:**

**Synthesis** of **intermediate MDI-226-1: 1-(2-(6-bromo-1-((2-(trimethylsilyl)ethoxy)methyl)-1H-indazol-3-yl)-1-((2-(trimethylsilyl)ethoxy)methyl)-4,6-dihydropyrrolo[3,4-d]imidazol-5-(1H)-yl)ethan-1-one**

Tert-butyl 2-(6-bromo-1-((2-(trimethylsilyl)ethoxy)methyl)-1H-indazol-3-yl)-1-((2-(trimethylsilyl)ethoxy)methyl)-4,6-dihydropyrrolo[3,4-d]imidazol-5-(1H)-carboxylate (200 mg, 0.30 mmol) was dissolved in 10ml dichloromethane, and 2ml trifluoroacetic acid was added. The mixture was stirred at room temperature for 30 minutes, concentrated, and quenched with sodium bicarbonate. The resulting mixture was extracted twice with dichloromethane, and the organic phases were combined, washed with water and saturated brine, dried over anhydrous sodium sulfate and concentrated. The obtained compound was dissolved in 10ml DCM, and Et3N (0.16ml, 1.18mmol) was added. The mixture was cooled to 0°C, and acetyl chloride (25.0 mg, 0.32mmol) was slowly added. It was allowed to react at room temperature for 2

hours, and water was added to quench the reaction. The resulting mixture was extracted with DCM twice, and the organic phases were combined, washed with water and saturated brine, dried over anhydrous sodium sulfate, concentrated, and purified by silica gel column to afford intermediate MDI-226-1 with a yield of 54.8%.

$^1$H NMR (400 MHz, chloroform-d) δ 8.36-8.29 (m, 1H), 7.78-7.77 (m, 1H), 7.42-7.39 (m, 1H), 5.93-5.88 (m, 2H), 5.71 (d, J = 4 Hz, 2H), 4.76-4.73 (m, 2H), 4.67-4.63 (m, 2H), 3.61-3.54 (m, 4H), 2.20 (s, 3H), 0.94-0.88 (m, 4H) ), -0.03-0.09 (m, 18H).

**Synthesis of intermediate MDI-226-2: 1-(2-(6-(2-ethyl-5-fluoro-4-((2-(trimethylsilyl)ethoxy)methoxy)phenyl)-1-((2-(trimethylsilyl)ethoxy)methyl)-1H-indazol-3-yl)-1-((2-(trimethylsilyl)ethoxy)methyl)-4,6-dihydropyrrolo[3,4-d]imidazol-5-(1H)-yl)ethan-1-one**

The intermediateMDI-226-1 (100 mg, 0.16 mmol), (2-((5-ethyl-2-fluoro-4-(4, 4,5,5-tetramethyl-1,3,2-dioxaboran-2-yl)phenoxy)methoxy)ethyl)trimethylsilane (78.3 mg, 0.20 mmol) , Pd(dppf)Cl2 (12.0 mg, 0.016 mmol) and potassium phosphate (104.8 mg, 0.49 mmol) were dissolved in 1,4-dioxane (12 ml) and water (2 ml). The atmosphere was replaced with nitrogen, which was repeated 3 times. The mixture was heated to 100 °C, reacted overnight, and cooled to room temperature. Water was added and the resulting mixture was extracted twice with ethyl acetate, and the organic phases were combined, washed with water and saturated brine, dried over anhydrous sodium sulfate, concentrated, and purified by silica gel column to afford the intermediate MDI- 226-2 with a yield of 76.2%.

$^1$H NMR (400 MHz, chloroform-d) δ 8.52-8.45 (m, 1H), 7.49 (s, 1H), 7.27-7.25 (m, 1H), 7.18 (d, J = 8 Hz, 1H), 7.03 ( d, J = 12 Hz, 1H) 6.00 - 5.95 (m, 2H), 5.78 (s, 2H), 5.34 (s, 2H), 4.80-4.77 (m, 2H), 4.71-4.69 (m, 2H), 3.90-3.86 (m, 2H), 3.66-3.58 (m, 4H), 2.59-2.54 (m, 2H), 2.22 (d, J = 8 Hz, 3H), 1.10-1.06 (m, 3H), 1.03- 0.91 (m, 6H), -0.02-0.06 (m, 27H).

**Synthesis of compound MDI-226: 1-(2-(6-(2-ethyl-5-fluoro-4-hydroxyphenyl)-1H-indazol-3-yl)-4,6-dihydropyrrolo[3,4-d]imidazol-5-(1H)-yl)ethan-1-one**

Intermediate MDI-226-2 (100 mg, 0.13 mmol) was dissolved in methanol (5 ml), to which concentrated hydrochloric acid (2.5 ml) was added. The mixture was heated to 50°C, reacted for 6 hours, and concentrated. The solid was dissolved in 1 ml of methanol, and pH was adjusted to 8-9 with aqueous ammonia. The resulting mixture was concentrated, and purified by silica gel column to afford 5 mg of the final product with a yield of 9.8%.

$^1$H NMR (400 MHz, methanol-d4) δ 8.28 (d, J = 8 Hz, 1H), 7.43 (s, 1H), 7.18 (d, J = 8 Hz, 1H), 6.94 (dd, J = 22, 10 Hz, 2H), 4.79 (s, 2H), 4.65 (s, 2H), 2.59-2.53 (m, 2H), 2.23 (s, 3H), 1.08 (t, J = 7.5 Hz, 3H).

**Example 25: 1-(2-(6-(2-ethyl-5-fluoro-4-hydroxyphenyl)-1H-indazol-3-yl)-4,6-dihydropyrrolo[3,4-d]imidazol-5-(1H)-yl)propan-1-one (MDI-227)**

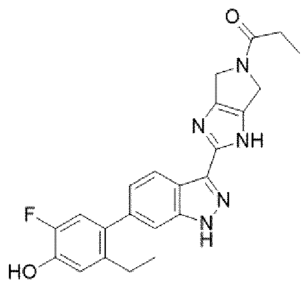

MDI-227

**Synthetic route of MDI-227:**

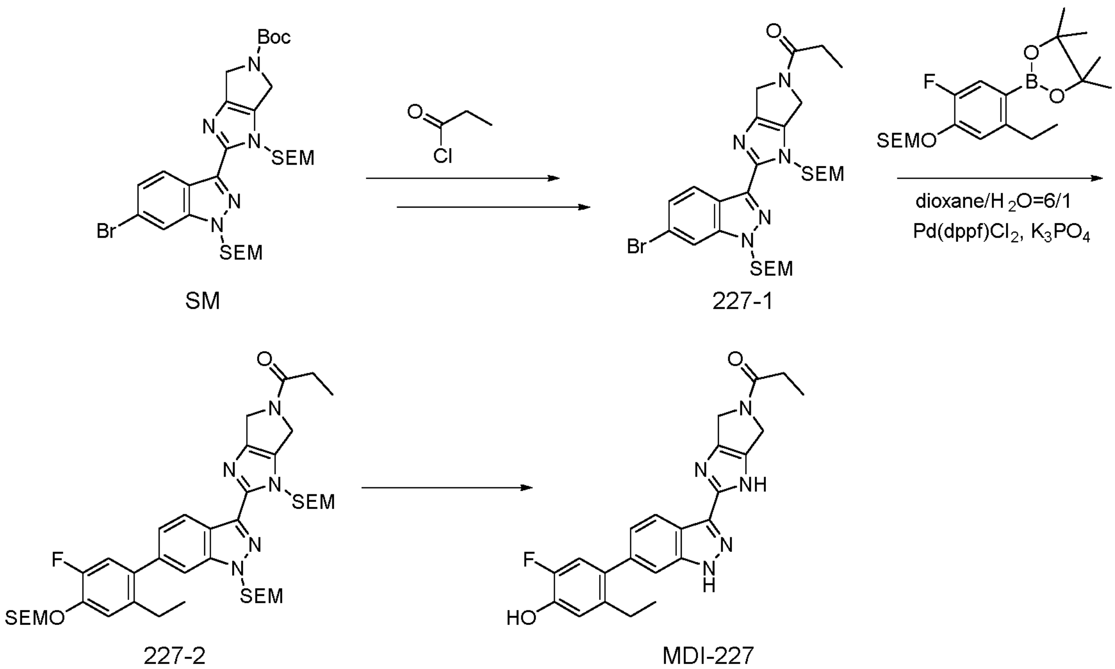


SM 227-1 227-2 MDI-227

**Synthesis method:**

**Synthesis of intermediate MDI-227-1: 1-(2-(6-bromo-1 -((2-(trimethylsilyl)ethoxy)methyl)-1H-indazol-3-yl)-1 -((2-(trimethylsilyl)ethoxy)methyl)-4,6-dihydropyrrolo[3,4-d]imidazol-5-(1H)-yl)propan-1-one**

Tert-butyl 2-(6-bromo-1 -((2-(trimethylsilyl)ethoxy)methyl)-1H-indazol-3-yl)-1 -((2-(trimethylsilyl)ethoxy)methyl)-4,6-dihydropyrrolo[3,4-d]imidazol-5-(1H)-carboxylate (200 mg, 0.30 mmol) was dissolved in 10ml dichloromethane, and 2ml trifluoroacetic acid was added. The mixture was stirred at room temperature for 30 minutes, concentrated, and quenched with sodium bicarbonate. The resulting mixture was extracted twice with dichloromethane, and the organic phases were combined, washed with water and saturated brine, dried over anhydrous sodium sulfate and concentrated. The obtained compound was dissolved in 10ml DCM and Et3N (0.16ml, 1.18mmol), and cooled to 0°C. Propionyl chloride (29 mg, 0.32mmol) was slowly added. It was allowed to react at room temperature for 2 hours, and water was added to quench the reaction. The resulting mixture was extracted with DCM twice, and the organic phases were combined, washed with water and saturated brine, dried over anhydrous sodium sulfate, concentrated, and purified by silica gel column to afford intermediate MDI-227-1 with a yield of 53.5%.

$^1$H NMR (400 MHz, chloroform-d) $\delta$ 8.39-8.32 (m, 1H), 7.77-7.76 (m, 1H), 7.42-7.38 (m, 1H), 5.93-5.89 (m, 2H), 5.71 (d, J = 4 Hz, 2H), 4.76-4.70 (m, 2H), 4.65-4.63 (m, 2H), 3.61-3.55 (m, 4H), 2.46-2.40 (m, 2H), 1.25-1.23 (m , 3H), 0.93-0.89 (m, 4H), -0.05-0.08(m, 18H).

**Synthesis of intermediate MDI-227-2: 1-(2-(6-(2-ethyl-5-fluoro-4 -((2-(trimethylsilyl)ethoxy)methoxy)phenyl)-1 -((2-(trimethylsilyl)ethoxy)methyl)-1H-indazol-3-yl)-1 -((2-(trimethylsilyl)ethoxy)methyl)-4,6-dihydropyrrolo[3,4-d]imidazol-5-(1H)-yl)propan-1-one**

The intermediate MDI-227-1 (100 mg, 0.16 mmol), (2-((5-ethyl-2-fluoro-4-(4, 4,5,5-tetramethyl-1,3,2-dioxaboran-2-yl)phenoxy)methoxy)ethyl)trimethylsilane (76.5 mg, 0.19 mmol), Pd(dppf)Cl2 (11.8 mg, 0.016 mmol) and potassium phosphate (102.4 mg, 0.48 mmol) were dissolved in 1,4-dioxane (12 ml) and water (2 ml).The atmosphere was replaced with nitrogen, which was repeated 3 times. The mixture was heated to 100 °C, reacted overnight, and cooled to room temperature. Water was added, the resulting mixture was extracted twice with ethyl acetate, and the organic phases were combined, washed with water and saturated brine, dried over anhydrous sodium sulfate, concentrated, and purified by silica gel column to afford the intermediate MDI- 227-2 with a yield of 76.7%.

$^1$H NMR (400 MHz, chloroform-d) $\delta$ 8.52-8.45 (m, 1H), 7.48 (d, J = 4 Hz, 1H), 7.28-7.25 (m, 1H), 7.18 (d, J = 8 Hz, 1H), 7.04 (d, J = 12 Hz, 1H), 5.98 (d, J = 12 Hz, 2H), 5.78 (s, 2H), 5.34 (s, 2H), 4.78 (s, 2H), 4.69- 4.68 (m, 2H), 3.90-3.86 (m, 2H), 3.66-3.58 (m, 4H), 2.59-2.54 (m, 2H), 2.49-2.40 (m, 2H), 1.31-1.26 (m, 5H) ), 1.10-1.05 (m, 3H), 0.96-0.91 (m, 4H), -0.02-0.05 (m, 27H).

**Synthesis of compound MDI-227: 1-(2-(6-(2-ethyl-5-fluoro-4-hydroxyphenyl)-1H-indazol-3-yl)-4,6-dihydropyrrolo[3,4-d]imidazol-5-(1H)-yl) propan -1-one**

Intermediate MDI-227-2 (100 mg, 0.12 mmol) was dissolved in methanol (5 ml), to which concentrated hydrochloric acid (2.5 ml) was added. The mixture was heated to 50°C, reacted for 6 hours, and concentrated. The solid was dissolved in 1 ml of methanol and pH was adjusted to 8-9 with aqueous ammonia. The resulting mixture was concentrated, and purified by silica gel column to afford 18 mg of final product with a yield of 34.8%.

$^1$H NMR (400 MHz, DMSO-d6) δ 13.29 (s, 1H), 12.80 (s, 1H), 9.85 (s, 1H), 8.33 (d, J = 8 Hz, 1H), 7.40 (s, 1H) , 7.12 (d, J = 8 Hz, 1H), 7.03 (d, J = 12 Hz, 1H), 6.92 (d, J = 12 Hz, 1H), 4.73-4.58 (m, 2H), 4.50-4.42 (m, 2H), 2.50-2.47 (m, 2H), 2.43-2.37 (m, 2H), 1.08-1.01 (m, 6H).

**Example 26: 1-(2-(6-(2-ethyl-5-fluoro-4-hydroxyphenvl)-1H-indazol-3-yl)-4,6-dihydropyrrolo[3,4-d]imidazol-5-(1H)-yl)-2-methylpropan-1-one (MDI-228)**

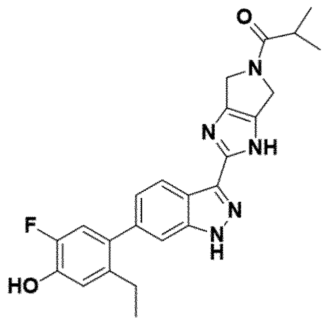

MDI 228

**Synthetic route of MDI-228:**

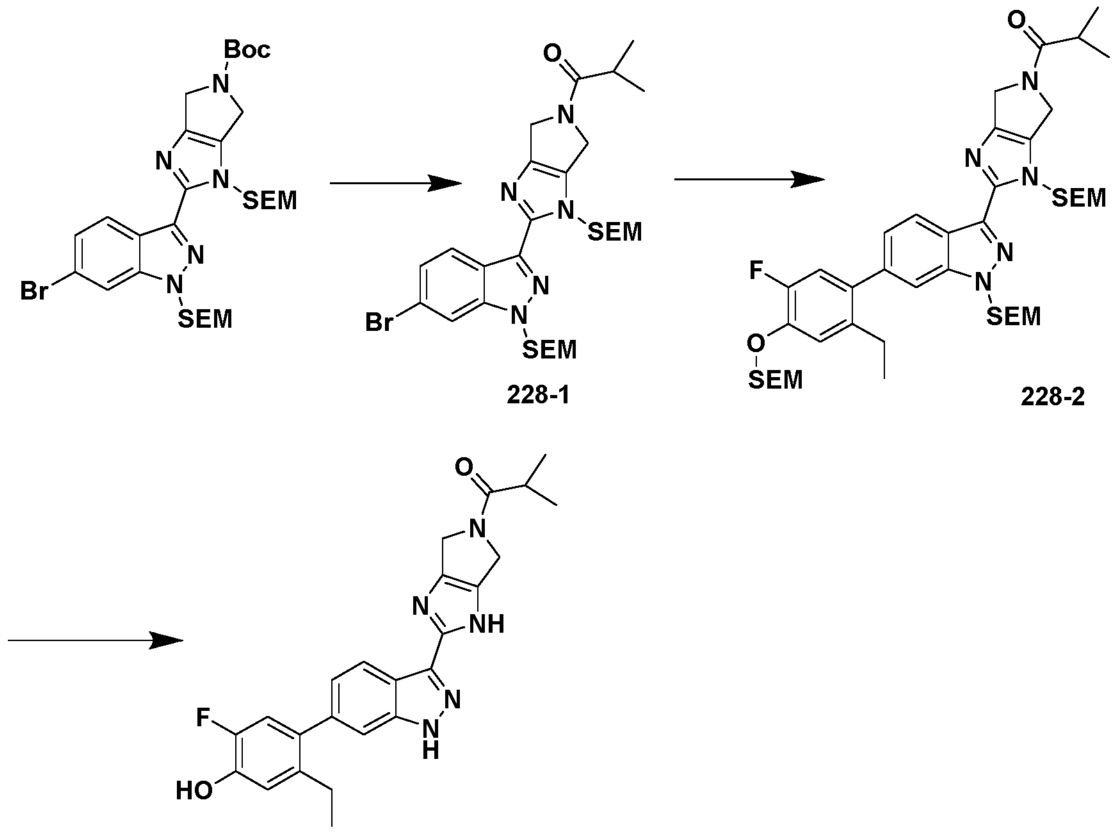

228-1

228-2

MDI 228

**Synthesis method:**

**Synthesis of intermediate MDI-228-1: (1-(2-(6-bromo-1-((2-(trimethylsilyl)ethoxy)methyl)-1H-indazol-3-yl)-1-((2-(trimethylsilyl)ethoxy)methyl)-4,6-dihydropyrrolo[3,4-d]imidazol-5-(1H)-yl)-2-methylpropan-1-one)**

The intermediate tert-butyl 2-(6-bromo1-((2-(trimethylsilyl)ethoxy)methyl)-1H-indazol-3-yl)-1-((2-(trimethylsilyl)ethoxy)methyl)-4,6-dihydropyrrolo[3,4-d]imidazol-5(1H)-carboxylate (80 mg, 0.12 mmol) was dissolved in 5ml of dichloromethane, to which 1ml of trifluoroacetic acid was added. The mixture was stirred at room temperature for 30 minutes, and concentrated to give a residue. The residue was dissolved in dichloromethane and was concentrated to dryness, which was repeated 3 times. The resulting residue was dissolved in 5ml of DCM, and then triethylamine (24.3 mg, 0.24 mmol) was added. The temperature was lowered to 0°C, and isobutyryl chloride (19.2 mg, 0.18 mmol) was slowly added dropwise. After the dropwise addition was completed, the mixture was warmed to room temperature and reacted for 1-2h. Water was added to the reaction to quench the reaction. The liquids were separated, and the organic phase was dried over sodium sulfate and concentrated by column chromatography to afford compound MDI-228-1 with a yield of 58.7%.

$^1$H NMR (400 MHz, CDCl3) $^1$H NMR (400 MHz, CDCl3) δ 8.39-8.32 (m, 1H), 7.77-7.76 (m, 1H), 7.42-7.38 (m, 1H), 5.92-5.89 (m, 2H), 5.71-5.69 (m, 2H), 4.83-4.64 (m, 4H), 3.63-3.55 (m, 4H), 2.81-2.70 (m, 1H), 1.22 (d, J = 8Hz, 6H), 0.98-0.86 (m, 4H), - 0.05-0.08 (m, 18H).

**Synthesis of intermediate MDI-228-2: 1-(2-(6-(2-ethyl-5-fluoro-4-((2-(trimethylsilyl)ethoxy)methoxy)phenyl)-1-((2-(trimethylsilyl)ethoxy)methyl)-1H-indazol-3-yl)-1-((2-(trimethylsilyl)ethoxy)methyl)-4,6-dihydropyrrolo[3,4-d]imidazol-5-(1H)-yl)-2-methylpropan-1-one**

The intermediate MDI-228-1 (50.65 mg, 0.08 mmol), (2-((5-ethyl-2-fluoro-4-(4, 4,5,5-tetramethyl-1,3,2-dioxaboran-2-yl)phenoxy)methoxy)ethyl)trimethylsilane (34.8 mg, 0. 1mmol), Pd(dppf)Cl2 (5.9 mg, 0.008 mmol) and potassium phosphate (50.9 mg, 0.24 mmol) were dissolved in 1,4-dioxane (10ml) and water (2ml). The atmosphere was replaced with nitrogen, which was repeated 3 times. The mixture was heated to 100°C, reacted overnight, and cooled to room temperature. Water was added, and the resulting mixture was extracted twice with ethyl acetate, and the organic phases were combined, washed with water and saturated brine, dried over anhydrous sodium sulfate, concentrated and purified

by silica gel column to afford intermediate MDI-228- 2 with a yield of 77.5%.

$^1$H NMR (400 MHz, CDCl3) δ 8.49-8.42 (m, 1H), 7.46-7.45 (m, 1H), 7.25-7.22 (m, 1H), 7.16 (d, J = 8Hz, 1H), 7.02 (d, J = 12Hz, 1H), 5.97-5.92 (m, 2H), 5.76(s, 2H), 5.32 (s, 2H), 4.83-4.67 (m, 4H), 3.88-3.84 (m, 2H), 3.64 -3.56 (m, 4H), 2.82-2.73 (m, 1H), 2.57-2.51 (m, 2H), 1.22 (d, J = 8Hz, 6H), 1.08-0.99 (m, 5H), 0.93-0.88 (m, 4H), 0.03 (s, 9H), -0.06-0.07(m, 18H).

**Synthesis of compound MDI-228: 1-(2-(6-(2-ethyl-5-fluoro-4-hydroxyphenyl)-1H-indazol-3-yl)-4,6-dihydropyrrolo[3,4-d]imidazol-5-(1H)-yl)-2-methylpropan-1-one**

Intermediate MDI-228-2 (50 mg, 0.061 mmol) was dissolved in methanol (4ml), to which concentrated hydrochloric acid (2ml) was added. The mixture was heated to 50°C, reacted for 6 hours, and concentrated. The solid was dissolved in 1ml methanol, 2ml concentrated aqueous ammonia was added, and the mixture was concentrated to give a residue. The residue was dissolved in methanol and was concentrated to dryness, which was repeated 3 times. The resulting residue was purified by a preparation plate to afford 15 mg of the final product with a yield of 57.0%.

$^1$H NMR (400 MHz, MeOD-d4) δ 8.26 (d, J = 8.0 Hz, 1H), 7.43 (s, 1H), 7.17 (d, J = 8Hz, 1H), 6.93 (dd, J = 20, 12 Hz, 2H), 4.83-4.59 (m, 4H), 2.94-2.90 (m, 1H), 2.58-2.52 (m, 2H), 1.22 (d, J = 8.0 Hz, 6H), 1.08 (t, J = 8.0 Hz, 3H).

**Example 27: 2-cyclopropyl-1-(2-(6-(2-ethyl-5-fluoro-4-hydroxyphenyl)-1H-indazol-3-yl)-4,6-dihydropyrrolo[3,4-d]imidazol-5-(1H)-yl)ethan-1-one (MDI-229)**

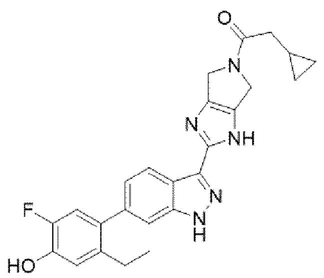

MDI-229

**Synthetic route of MDI-229:**

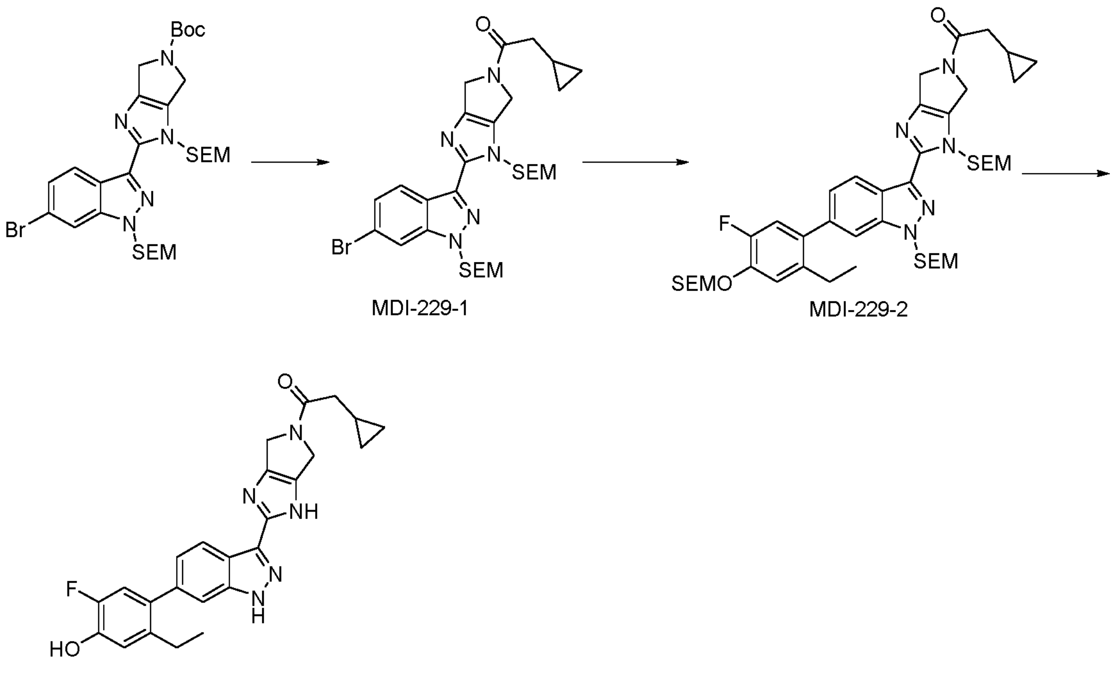

MDI-229-1

MDI-229-2

MDI-229

## Synthesis method

### Synthesis of intermediate MDI-229-1: 1-(2-(6-bromo-1-((2-(trimethylsilyl)ethoxy)methyl)-1H-indazol-3-yl)-1-((2-(trimethylsilyl)ethoxy)methyl)-4,6-dihydropyrrolo[3,4-d]imidazol-5-(1H)-yl)-2-cyclopropylethan-1-one

2-cyclopropylacetic acid (14.5 mg, 0.14 mmol) and N,N-diisopropylethylamine (46.6 mg, 0.36 mmol) were dissolved in DMF, and HATU (54.9 mg, 0.14 mmol) was added. It was allowed to react at room temperature for 10 minute. Intermediate tert-butyl 2-(6-bromo1-((2-(trimethylsilyl)ethoxy)methyl)-1H-indazol-3-yl)-1-((2-(trimethylsilyl)ethoxy)methyl)-4,6-dihydropyrrolo[3,4-d]imidazole-5(1H)-carboxylate (80.0 mg, 0.12mmol) was dissolved in 5ml dichloromethane, to which 1ml of trifluoroacetic acid was added. The mixture was stirred at room temperature for 30 minutes, and concentrated to give a residue. The residue was dissolved in dichloromethane and was concentrated to dryness, which was repeated 3 times. The resulting residue was dissolved in DMF, and then was slowly added to the previous reaction solution. It was allowed to react at room temperature overnight, and water was added to quench the reaction. The resulting mixture was extracted twice with ethyl acetate, and the organic phases were combined, washed with water and saturated brine, dried over anhydrous sodium sulfate, concentrated, and purified by silica gel column to afford intermediate MDI-229-1 with a yield of 57.8%.

$^1$H NMR (400 MHz, CDCl3) δ 8.40 (dd, J = 8.0 Hz, J = 20.0 Hz,1H), 7.80 (s, 1H), 7.44-7.41 (m, 1H), 5.92 (s, 2H), 5.74 (s, 2H), 4.78-4.66 (m, 4H), 3.63-3.58 (m, 4H), 2.39-2.35 (m, 2H), 2.04 (s, 1H), 0.96-0.91 (m, 4H), 0.66 (d, J = 8.0 Hz, 2H), 0.27 (d, J = 4.0 Hz, 2H), 0.02(s, 18H).

### Synthesis of intermediate MDI-229-2: 2-cyclopropyl-1-(2-(6-(2-ethyl-5-fluoro-4-((2-(trimethylsilyl)ethoxy)methoxy)phenyl)-1-((2-(trimethylsilyl)ethoxy) methyl)-1H-indazol-3-yl)-1-((2-(trimethylsilyl)ethoxy)methyl)-4,6-dihydropyrrolo [3,4-d]imidazol-5-(1H)-yl)ethan-1-one

The intermediate MDI-229-1 (45.0 mg, 0.07 mmol), (2-((5-ethyl-2-fluoro-4-(4, 4,5,5-tetramethyl-1,3,2-dioxaborolan-2-yl)phenoxy)methoxy)ethyl)trimethylsilane (33.1mg, 0.08 mmol), Pd(dppf)Cl2 (5.1 mg, 0.007 mmol) and potassium phosphate (44.3 mg, 0.21 mmol) were dissolved in 1,4-dioxane (20ml) and water (4ml). The atmosphere was replaced with nitrogen, which was repeated 3 times. The mixture was heated to 100°C, reacted overnight, and cooled to room temperature. Water was added, the resulting mixture was extracted twice with ethyl acetate, and the organic phases were combined, washed with water and saturated brine, dried over anhydrous sodium sulfate, concentrated, and purified by silica gel column to afford intermediate MDI-229- 1 with a yield of 55.0%.

$^1$H NMR (400 MHz, CDCl3) δ 8.50 (dd, J = 8.0 Hz, J = 16.0 Hz, 1H), 7.49 (s, 1H), 7.27 (s, 1H), 7.19 (d, J = 8.0 Hz, 1H ), 7.05 (d, J = 12.0 Hz, 1H), 5.96 (s, 2H), 5.78 (s, 2H), 5.34 (s, 2H), 4.79-4.66 (m, 4H), 3.88 (t, J = 8.0 Hz, 2H), 3.66-3.58 (m, 4H), 2.59-2.54 (m, 2H), 2.39-2.36 (m, 2H), 2.06-2.04 (m, 1H), 1.05 (t, J = 8.0 Hz , 3H), 0.96-0.91(m, 6H),

0.66-0.63(m, 2H), 0.28-0.26(m, 2H), 0.06-0.05(m, 27H).

**Synthesis of compound MDI-229: 2-cyclopropyl-1-(2-(6-(2-ethyl-5-fluoro-4-hydroxyphenyl)-1H-indazol-3-yl)-4,6-dihydropyrrolo[3,4-d]imidazol-5-(1H)-yl)ethan-1-one**

The intermediate MDI-229-4 was dissolved in 4ML methanol and 2ml concentrated hydrochloric acid was added. The mixture was heated to 50°C, reacted for 6 hours, and concentrated to give a residue. The residue was dissolved in methanol and was concentrated to dryness, which was repeated 3 times. The resulting residue was dissolved 1ml methanol and 2ml aqueous ammonia was added for neutralization, and the resulting mixture was concentrated to give a residue. The residue was dissolved in methanol and was concentrated to dryness, which was repeated 2 times, thereby obtaining 3.8 mg of the product with a yield of 12.2%.

$^1$H NMR (400 MHz, MeOD-d4) δ 8.29 (d, *J* = 8.0 Hz, 1H), 7.43 (s, 1H), 7.19-7.17 (m, 1H), 6.98-6.90 (m, 2H), 4.73-4.61 (m, 4H), 2.59-2.53 (m, 2H), 2.46 (d, *J* = 8.0 Hz, 2H), 1.17 (m, 1H), 1.08 (t, *J* = 8.0 Hz , 3H), 0.64-0.59 (m, 2H), 0.30-0.26(m, 2H).

**Example 28: 1-(2-(6-(2-ethyl-5-fluoro-4-hvdroxyphenyl)-1H-indazol-3-yl)-4,6-dihydropyrrolo[3,4-d]imidazol-5-(1H)-yl)-3-methylbutan-1-one (MDI-230)**

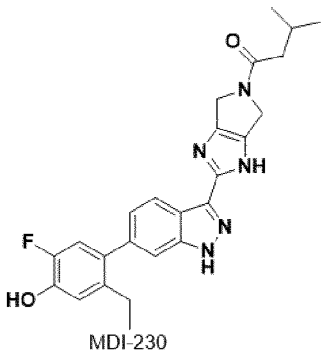

MDI-230

**Synthetic route of MDI-230:**

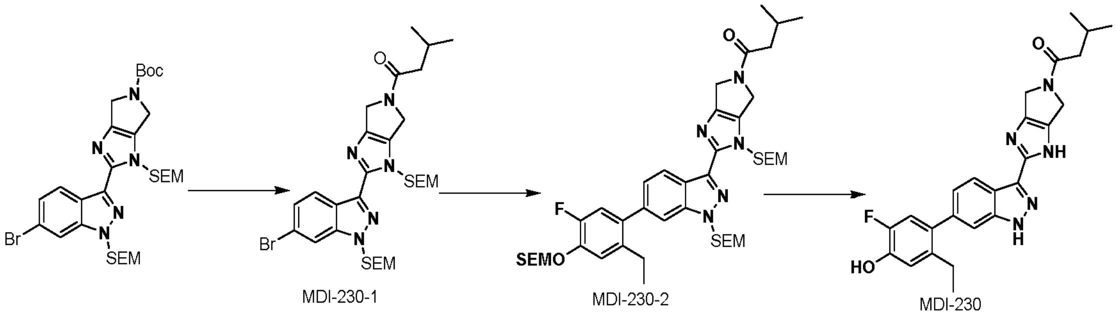

**Synthesis method:**

**Synthesis of intermediate MDI-230-1: 1-(2-(6-bromo-1-((2-(trimethylsilyl)ethoxy)methyl)-1H-indazol-3-yl)-1-((2-(trimethylsilyl)ethoxy)methyl)-4,6-dihydropyrrolo[3,4-d]imidazol-5-(1H)-yl)-3-methylbutan-1-one**

Tert-butyl 2-(6-bromo1-((2-(trimethylsilyl)ethoxy)methyl)-1H-indol-3-yl)-1-((2-(trimethylsilyl)ethoxy)methyl)-4,6-dihydropyrrolo[3,4-d]imidazol-5(1H)-carboxylate (100 mg, 0.15 mmol) was dissolve in 5 ml of dichloromethane, and 1 ml of trifluoroacetic acid was added. The mixture was stirred at room temperature for 30 minutes, and concentrated to give a residue. The residue was dissolved in dichloromethane and was concentrated to dryness, which was repeated

3 times. The resulting residue was dissolved in 5ml of dichloromethane and triethylamine (0.08ml, 0.59mmol) and cooled to 0°C. 3-methylbutyryl chloride (36.6 mg, 0.30 mmol) was slowly added. It was allowed to react at room temperature for 2 hours, and water was added to quench the reaction. The resulting mixture was extracted twice with dichloromethane, and the organic phases were combined, washed with saturated brine, dried over anhydrous sodium sulfate, concentrated, and purified by silica gel column to afford 64 mg of intermediate MDI-230-1, with a yield of 65.8%.

$^1$H NMR (400 MHz, CDCl3) δ 8.42 - 8.35 (m, 1H), 7.80-7.79 (m, 1H), 7.45-7.41 (m, 1H), 5.96 - 5.91 (m, 2H), 5.74-5.73 (m , 2H), 4.78 - 4.69 (m, 4H), 3.67 - 3.58 (m, 4H), 2.31 - 2.22 (m, 3H), 1.08 - 1.05 (m, 6H), 0.97 - 0.90 (m, 4H),- 0.02 --0.05 (m, 18H).

**Synthesis of intermediate MDI-230-2: 1-(2-(6-(2-ethyl-5-fluoro-4-((2-(trimethylsilyl)ethoxy)methoxy)phenyl)-1-((2-(trimethylsilyl)ethoxy)methyl)-1H-indazol-3-yl)-1-((2-(trimethylsilyl)ethoxy)methyl)-4,6-dihydropyrrolo[3,4-d]imidazol-5-(1H)-yl)-3-methylbutan-1-one**

The intermediate MDI-230-1 (98 mg, 0.15 mmol), (2-((5-ethyl-2-fluoro-4-(4,4,5,5-tetramethyl-1,3,2-dioxaboran-2-yl)phenoxy)methoxy)ethyl)trimethylsilane (64mg, 0.10 mmol), Pd(dppf)Cl2 (10 mg, 0.01 mmol) and potassium phosphate (70 mg, 0.30 mmol) were dissolved in 1,4-dioxane (20 ml) and water (4 ml). The atmosphere was replaced with nitrogen, which was repeated 3 times. The mixture was heat to 100 °C, reacted for 16 h, and cooled to room temperature. Water was added, the resulting mixture was extracted twice with ethyl acetate, and the organic phases were combined, washed with water and saturated brine, dried over anhydrous sodium sulfate, concentrated, and purified by silica gel column to afford 50 mg of intermediate MDI-230-2 with a yield of 59.7%.

$^1$H NMR (400 MHz, CDCl3) δ8.52 - 8.45 (m, 1H), 7.49 (s, 1H), 7.25 (d, J = 2.8 Hz, 1H), 7.18 (d, J = 8.6 Hz, 1H), 7.04 (d, J = 11.8 Hz, 1H), 5.98 (d, J = 15.6 Hz, 2H), 5.78 (s, 2H), 5.36 (s, 2H), 4.78-4.72 (m, 4H), 3.91 - 3.86 (m, 2H), 3.66 - 3.59 (m, 4H), 2.60 - 2.56 (m, 2H), 2.32 - 2.30 (m, 3H), 1.11- 1.01 (m, 6H), 0.99 - 0.89 (m, 7H), 0.03 (s, 9H),-0.03 --0.05 (m, 18H).

**Synthesis of compound MDI-230: 1-(2-(6-(2-ethyl-5-fluoro-4-hydroxyphenyl)-1H-indazol-3-yl)-4,6-dihydropyrrolo[3,4-d]imidazol-5-(1H)-yl)-3-methylbutan-1-one**

The intermediate MDI-230-2 (50 mg, 0.06 mmol) was dissolved in methanol (6 ml), to which concentrated hydrochloric acid (3 ml) was added. It was heated to 50 °C and allowed to react for 6 hours. The reaction mixture was concentrated to give a residue. The residue was dissolved in methanol and concentrated to dryness (to remove hydrochloric acid), which was repeated 3 times. The resulting residue was dissolved in methanol, to which 1 ml ammonia water was added. Then the mixture was concentrated and filtered, the filtrate was concentrated and purified by a preparation plate to afford 15 mg of the final product with a yield of 55.9%.

$^1$HNMR (400 MHz, MeOD-d4) δ 8.29 - 8.26 (m, 1H), 7.43 (s, 1H), 7.19 - 7.16 (m, 1H), 6.97 - 6.89 (m,2H), 4.75 - 4.70 (m , 4H), 2.56 (q, J = 7.5 Hz, 2H), 2.36 (m, 2H), 2.29 - 2.20 (m, 1H), 1.10 - 1.05 (m, 9H).

**Example 29: (2-(6-(2-ethyl-5-fluoro-4-hydroxyphenyl)-1H-indazol-3-yl)-4,6-dihydropyrrolo[3,4-d]imidazol-5-(1H) - yl)(pyrrolidin-1-yl)ketone (MDI-231)**

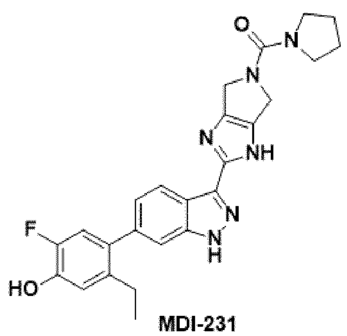

MDI-231

**Synthetic route of MDI-231:**

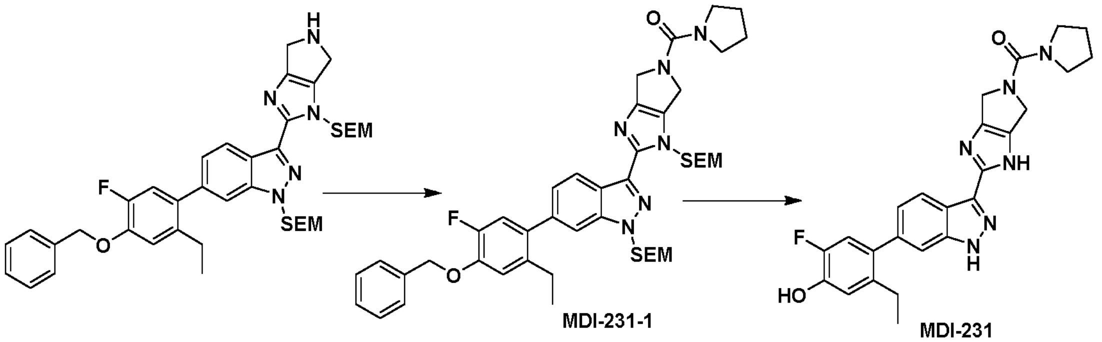


**Synthesis method:**

**Synthesis of intermediateMDI-231-1: (2-(6-(4-(benzyloxy)-2-ethyl-5-fluorophenyl)-1-((2-(trimethylsilyl)ethoxy)methyl)-1H-indazol-3-yl)-1-((2-(trimethylsilyl)ethoxy)methyl)-4,6-dihydropyrrolo [3,4-d] imidazol-5-(1H)-yl) (pyrrolidin-1-yl)ketone**

Triphosgene (64.4 mg, 0.21 mmol) was dissolved in 15 ml of dichloromethane, to which the intermediate 6-(4-(benzyloxy)-2-ethyl-5-fluorophenyl)-1- ((2-(trimethylsilyl) ethoxy)methyl)-3-(1-((2-(Trimethylsilyl)ethoxy)methyl)-1-1,4,5,6-tetrahydropyrrolo[3,4-d]imidazol-2-yl)-1H-indazole (150 mg, 0.21 mmol) in dichloromethane (5 ml) was added dropwise at 0°C, followed by addition of triethylamine (63.6 mg, 0.63 mmol). The mixture was stirred at room temperature for 5 minutes and pyrrolidine (29.8 mg, 0.42 mmol) in dichloromethane was added. The resulting mixture was stirred at room temperature for 10 minutes, and water was added. The resulting mixture was extracted twice with ethyl acetate, and the organic phases were combined, washed with water and saturated brine, dried over anhydrous sodium sulfate, concentrated, and purified by silica gel column to afford 140 mg of intermediate MDI-231-1 with a yield of 82.4%.

$^1$H NMR (400 MHz, CDCl3) δ 8.48 (d, J = 8 Hz, 1H), 7.53-7.38 (m, 6H), 7.22 (d, J = 8 Hz, 1H), 7.03 (d, J = 12 Hz , 1H), 6.95 (d, J = 8 Hz, 1H), 5.96 (s, 2H), 5.77 (s, 2H), 5.23 (s, 2H), 4.81 (s, 2H), 4.67 (s, 2H), 3.66-3.59 (m, 4H), 3.53-3.51 (m, 4H), 2.56-2.52 (m, 2H), 1.93-1.88 (m, 4H), 1.03 (t, J = 8 Hz, 3H), 0.93 -0.87 (m, 4H), -0.05-0.09 (m, 18H).

**Synthesis of compound MDI-231: (2-(6-(2-ethyl-5-fluoro-4-hydroxyphenyl)-1H-indazol-3-yl)-4,6-dihydropyrrolo[3,4-d]imidazol-5-(1H) -yl)(pyrrolidin-1-yl)ketone**

Intermediate MDI-231-1 (140 mg, 0.173 mmol) was dissolved in methanol (6 ml), to which 15 mg Pd/C was added and concentrated hydrochloric acid (3 ml) was added dropwise. The mixture was heated to 50°C, reacted for 6 hours, filtered, and concentrated to give a residue. The residue was dissolved in methanol and was concentrated to dryness, which was repeated 3 times. The resulting residue was dissolved in methanol, and 1 ml of ammonia was added. The resulting mixture was concentrated, and purified by a preparation plate to afford 21 mg of the final product with a yield of 26.3%.

$^1$H NMR (400 MHz, DMSO-d6) δ 13.25 (s, 1H), 12.69 (s, 1H), 9.83 (s, 1H), 8.31 (d, J = 8 Hz, 1H), 7.39 (s, 1H), 7.11 (d, J = 8 Hz, 1H), 7.02 (d, J = 12 Hz, 1H), 6.91 (d, J = 12 Hz, 1H), 4.57-4.56 (m, 2H), 4.49-4.48 (m, 2H), 3.32-3.31 (m, 4H), 2.48-2.44 (m, 2H), 1.85-1.79 (m, 4H), 1.02 (t, J = 7 Hz, 3H).

**Example 30: (2-(6-(2-ethyl-5-fluoro-4-hydroxyphenyl)-1H-indazol-3-yl)-4,6-dihydropyrrolo[3,4-d]imidazol-5-(1H)-yl)(piperidin-1-yl)ketone (MDI-233)**

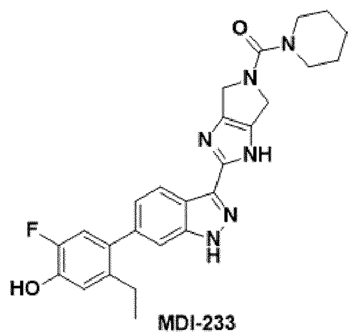

MDI-233

**Synthetic route of MDI-233:**

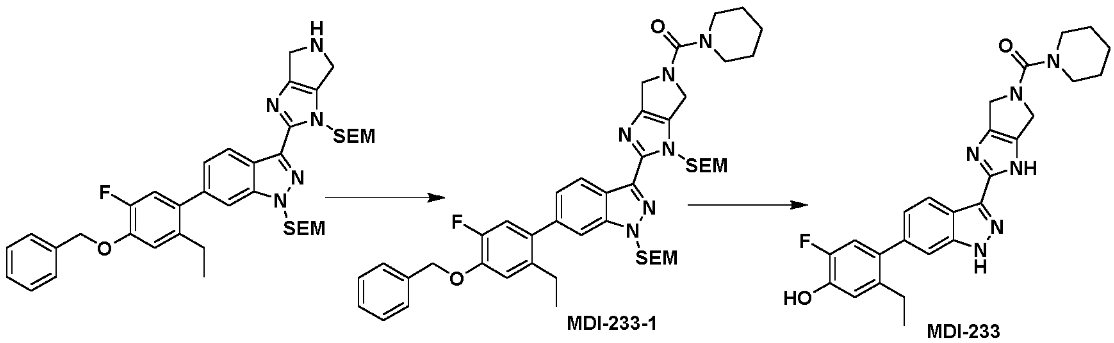

MDI-233-1

MDI-233

**Synthesis method:**

**Synthesis of intermediate MDI-233-1: (2-(6-(4-(benzyloxy)-2-ethyl-5-fluorophenyl)-1-((2-(trimethylsilyl)ethoxy)methyl)-1H-indazol-3-yl)-1-((2-(trimethylsilyl)ethoxy)methyl)-4,6-dihydropyrrolo[3,4-d]imidazol-5-(1H)-yl)(piperidin-1-yl)ketone**

Triphosgene (54.1 mg, 0.182 mmol) was dissolved in 5 ml of tetrahydrofuran, to which the intermediate 6-(4-(benzyloxy)-2-ethyl-5-fluorophenyl)-1-((2-(trimethylsilyl)ethoxy)methyl)-3-(1-((2-(trimethylsilyl)ethoxy)methyl)-1-1,4,5,6-tetrahydropyrrolo[3,4-d]imidazol-2-yl)-1H-indazole (130 mg, 0.182 mmol) in tetrahydrofuran (5 ml) was added dropwise at 0°C, followed by addition of triethylamine (55.2 mg, 0.550 mmol). The mixture was stirred at room temperature for 5 minutes, and piperidine hydrochloride (44.4 mg, 0.364 mmol) in tetrahydrofuran was added. The resulting mixture was stirred at room temperature for 10 minutes. Water was added and the resulting mixture was extracted twice with ethyl acetate, and the organic phases were combined, washed with water and saturated brine, dried over anhydrous sodium sulfate, concentrated, and purified by silica gel column to afford 105 mg of intermediate MDI-233-1, with a yield of 66.6%.

$^1$H NMR (400 MHz, $CDCl_3$) $\delta$ 8.44 (d, $J$ = 8.3 Hz, 1H), 7.50 - 7.48 (m, 2H), 7.44 - 7.35 (m, 4H), 7.23 - 7.20 (m, 1H), 7.04 - 6.94 (m, 2H), 5.93 (s, 2H), 5.74 (s, 2H), 5.20 (s, 2H), 4.69 (d, $J$ = 54.8 Hz, 4H), 3.64 - 3.56 (m, 4H), 3.31 (s, 4H) ,2.54 (q, $J$ = 7.5 Hz, 2H), 1.64 (s, 6H), 1.03 (t, $J$ = 7.5 Hz, 3H), 0.93 - 0.86 (m, 4H) , -0.07(d, $J$ = 2.7 Hz, 18H).

**Synthesis of compound MDI-233: (2-(6-(2-ethyl-5-fluoro-4-hydroxyphenyl)-1H-indazol-3-yl)-4,6-dihydropyrrolo[3,4-d]imidazol-5-(1H)-yl)(piperidin-1-yl)ketone**

Intermediate MDI-233-1 (100 mg, 0.121 mmol) was dissolved in methanol (6 ml), to which 10 mg Pd/C was added and concentrated hydrochloric acid (3 ml) was added dropwise. The mixture was heated to 50°C, reacted for 6 hours, filtered, and concentrated to give a residue. The residue was dissolved in methanol and was concentrated to dryness, which was repeated 3 times. The resulting residue was dissolved in methanol, and 1 ml of ammonia was added. The resulting mixture was concentrated, and purified by a preparation plate to afford 33 mg of the final product with a yield of 57.3%.

$^1$H NMR (400 MHz, MeOD-d4) $\delta$ 8.25 (d, $J$ = 8.4 Hz, 1H), 7.40 (s, 1H), 7.16 - 7.14 (m, 1H), 6.95 - 6.87 (m, 2H), 4.83 - 4.65 (m, 4H), 3.35 - 3.33 (m, 4H), 2.54 (q, $J$ = 7.5 Hz, 2H), 1.67 - 1.65 (m, 6H), 1.06 (t, $J$ = 7.5 Hz, 3H).

**Example 31: (2-(6-(2-ethyl-5-fluoro-4-hydroxyphenyl)-1H-indazol-3-yl)-4,6-dihydropyrrolo[3,4-d]imidazol-5-(1H)-yl)(morpholino)ketone (MDI-234)**

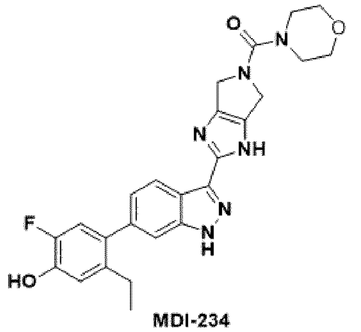

MDI-234

**Synthetic route of MDI-234:**

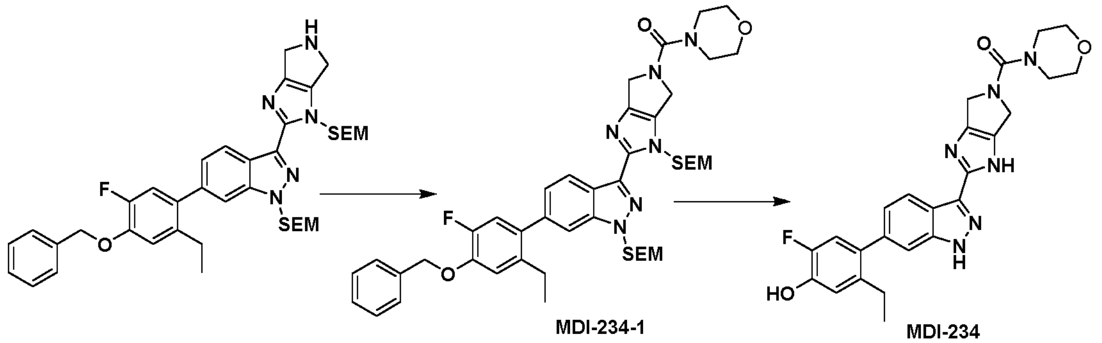

MDI-234-1

MDI-234

**Synthesis method:**

**Synthesis of intermediate MDI-234-1: (2-(6-(4-(benzyloxy)-2-ethyl-5-fluorophenyl)-1-((2-(trimethylsilyl)ethoxy)methyl)-1H-indazol-3-yl)-1-((2-(trimethylsilyl)ethoxy)methyl)-4,6-dihydropyrrolo[3,4-d]imidazol-5-(1H)-yl)(morpholino)ketone**

Triphosgene (54.1 mg, 0.182 mmol) was dissolved in 5 ml of tetrahydrofuran, to which the intermediate 6-(4-(benzyloxy)-2-ethyl-5-fluorophenyl)-1-((2-(trimethylsilyl)ethoxy)methyl)-3-(1-((2-(trimethylsilyl)ethoxy)methyl)-1-1,4,5,6-tetrahydropyrrolo[3,4-d]imidazol-2-yl)-1H-indazole (130 mg, 0.182 mmol) in tetrahydrofuran (5 ml) was added dropwise at 0°C, followed by addition of triethylamine (55.1 mg, 0.546 mmol). The mixture was stirred at room temperature for 5 minutes, and morpholine (31.7 mg, 0.364 mmol) in tetrahydrofuran was added. The resulting mixture was stirred at room temperature for 10 minutes. Water was added and the resulting mixture was extracted twice with ethyl acetate, and the organic phases were combined, washed with water and saturated brine, dried over anhydrous sodium sulfate, concentrated, and purified by silica gel column to afford 120 mg of intermediate MDI-234-1, with a yield of 79.7%.

$^1$H NMR (400 MHz, $CDCl_3$) δ 8.45 (d, *J* = 8.3 Hz, 1H), 7.53 - 7.51 (m, 2H), 7.47 - 7.35 (m, 4H), 7.26 - 7.23 (m, 1H), 7.06 - 6.97 (m, 2H), 5.96 (s, 2H), 5.77 (s, 2H), 5.23 (s, 2H), 4.68 (d, *J* = 54.8 Hz, 4H), 3.80 - 3.78 (m, 3H),3.67 - 3.59 (m, 4H), 3.43 - 3.40 (m, 3H), 3.27 - 3.21 (m, 6H), 2.54 (q, *J* = 7.5 Hz, 2H), 1.05 (t, *J* = 7.5 Hz, 3H), 0.96 - 0.89 (m, 4H) , -0.04(d, *J* = 2.7 Hz, 18H).

**Synthesis of compound MDI-234: (2-(6-(2-ethyl-5-fluoro-4-hydroxyphenyl)-1H-indazol-3-yl)-4,6-dihydropyrrolo[3,4-d]imidazol-5-(1H)-yl)(morpholino)ketone**

Intermediate MDI-234-1 (120 mg, 0.145 mmol) was dissolved in methanol (6 ml), to which 12 mg Pd/C was added and concentrated hydrochloric acid (3 ml) was added dropwise. The mixture was heated to 50°C, reacted for 6 hours, filtered, and concentrated to give a residue. The residue was dissolved in methanol and was concentrated to

dryness, which was repeated 3 times. The resulting residue was dissolved in methanol, and 1 ml of ammonia was added. The resulting mixture was concentrated, and purified by a preparation plate to afford 42 mg of the final product with a yield of 60.9%.

$^1$H NMR (400 MHz, MeOD-d4) δ 8.28 (d, *J*= 8.4 Hz, 1H), 7.43 (s, 1H), 7.19 - 7.16 (m, 1H), 6.97 - 6.90 (m, 2H), 4.71 - 4.66 (m, 4H), 3.78 - 3.75 (m, 4H), 3.41-3.39 (m, 4H), 2.54 (q, *J*= 7.5 Hz, 2H), 1.06 (t, *J*= 7.5 Hz, 3H).

**Example 32: (2-(6-(2-ethyl-5-fluoro-4-hydroxyphenyl)-1H-indazol-3-yl)-4,6-dihydropyrrolo[3,4-d]imidazol-5-(1H)-yl)(4-methylpiperazin-1-yl)ketone ( MDI-235)**

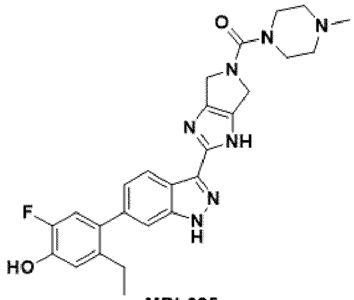

MDI-235

**Synthetic route of MDI-235:**

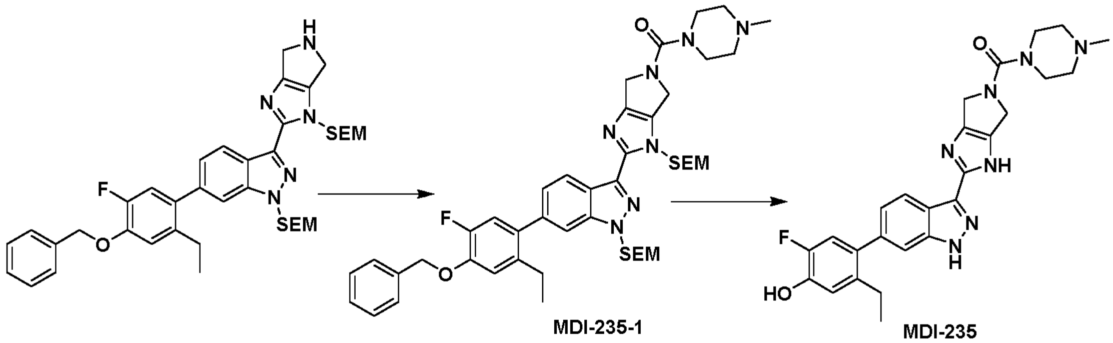

MDI-235-1

MDI-235

**Synthesis method:**

**Synthesis of intermediate MDI-235-1: (2-(6-(4-(benzyloxy)-2-ethyl-5-fluorophenyl)-1-((2-(trimethylsilyl)ethoxy)methyl)-1H-indazol-3-yl)-1-((2-(trimethylsilyl)ethoxy)methyl)-4,6-dihydropyrrolo[3,4-d]imidazol-5-(1H)-yl)(4-methylpiperazin-1-yl)ketone**

Triphosgene (8.3 mg, 0.028 mmol) was dissolved in 5 ml of tetrahydrofuran, to which the intermediate 6-(4-(benzyloxy)-2-ethyl-5-fluorophenyl)-1-((2-(trimethylsilyl)ethoxy)methyl)-3-(1-((2-(trimethylsilyl)ethoxy)methyl)-1-1,4,5,6-tetrahydropyrrolo[3,4-d]imidazol-2-yl)-1H-indazole (20 mg, 0.028 mmol) in tetrahydrofuran (5 ml) was added dropwise at 0°C, followed by addition of triethylamine (8.5 mg, 0.084 mmol). The mixture was stirred at room temperature for 5 minutes, and 1-methylpiperazine (5.60 mg, 0.056 mmol) in tetrahydrofuran was added. The resulting mixture was stirred at room temperature for 10 minutes. Water was added and the resulting mixture was extracted twice with ethyl acetate, and the organic phases were combined, washed with water and saturated brine, dried over anhydrous sodium sulfate, concentrated, and purified by silica gel column to afford 20 mg of intermediate MDI-235-1, with a yield of 85.1%.

$^1$H NMR (400 MHz, $CDCl_3$) δ 8.44 (d, *J* = 8.3 Hz, 1H), 7.50 - 7.48 (m, 2H), 7.44 - 7.33 (m, 4H), 7.23 - 7.21 (m, 1H), 7.04 - 6.94 (m, 2H), 5.93 (s, 2H), 5.75 (s, 2H), 5.20 (s, 2H), 4.70 (d, *J* = 54.8 Hz, 4H), 3.64 - 3.56 (m, 4H), 3.43 - 3.41 (m, 4H) , 2.56 - 2.49 (m, 6H) , 2.34 (s, 3H), 1.03 (t, *J* = 7.5 Hz, 3H), 0.93 - 0.86 (m, 4H) , -0.07(d, *J* = 2.7 Hz, 18H).

**Synthesis of compound MDI-235: (2-(6-(2-ethyl-5-fluoro-4-hydroxyphenyl)-1H-indazol-3-yl)-4,6-dihydropyrrolo[3,4-d]imidazol-5-(1H)-yl)(4-methylpiperazin-1-yl)ketone**

Intermediate MDI-235-1 (20 mg, 0.024 mmol) was dissolved in methanol (6 ml), to which 5 mg Pd/C was added and concentrated hydrochloric acid (3 ml) was added dropwise. The mixture was heated to 50 °C, reacted for 6 hours, filtered, and concentrated to give a residue. The residue was dissolved in methanol and was concentrated to dryness, which was repeated 3 times. The resulting residue was dissolved in methanol, and 1 ml of ammonia was added. The resulting mixture was concentrated, and purified by a preparation plate to afford 3 mg of the final product with a yield of 14.8%.

$^1$H NMR (400 MHz, MeOD-d4) δ 8.25 (d, *J* = 8.4 Hz, 1H), 7.40 (s, 1H), 7.16 - 7.14 (m, 1H), 6.95 - 6.87 (m, 2H), 4.83 - 4.66 (m, 4H), 3.44 - 3.41 (m, 4H), 2.56 - 2.51 (m, 6H), 2.35 (s, 3H), 1.06 (t, *J* = 7.5 Hz, 3H).

**Example 33: (2-(6-(2-ethyl-5-fluoro-4-hydroxyphenyl)-1H-indazol-3-yl)-4,6-dihydropyrrolo[3,4-d]imidazol-5-(1H) - yl)(4-ethylpiperazin-1-yl)ketone (MDI-236)**

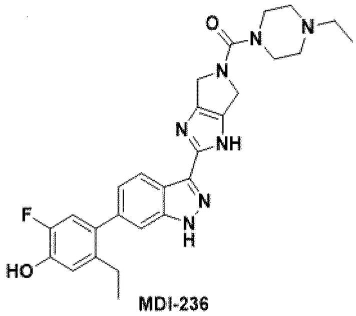

MDI-236

**Synthetic route of MDI-236:**

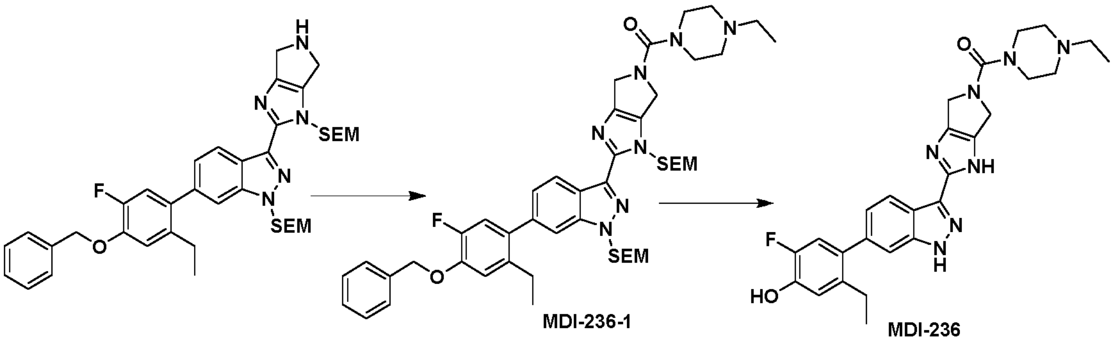

MDI-236-1

MDI-236

**Synthesis method:**

**Synthesis of intermediate MDI-236-1: (2-(6-(4-(benzyloxy)-2-ethyl-5-fluorophenyl)-1-((2-(trimethylsilyl)ethoxy)methyl)-1H-indazol-3-yl)-1-((2-(trimethylsilyl)ethoxy)methyl)-4,6-dihydropyrrolo[3,4-d]imidazol-5-(1H)-yl)(4-ethyl piperazin-1-yl)ketone**

Triphosgene (54.07 mg, 0.182 mmol) was dissolved in 15 ml of dichloromethane, to which the intermediate 6-(4-(benzyloxy)-2-ethyl-5-fluorophenyl)-1-((2-(trimethylsilyl)ethoxy)methyl)-3-(1-((2-(trimethylsilyl)ethoxy)methyl)-1-1,4,5,6-tetrahydropyrrolo[3,4-d]imidazol-2-yl)-1H-indazole (20 mg, 0.028 mmol) in dichloromethane (5 ml) was added dropwise at 0°C, followed by addition of triethylamine (55.2 mg, 0.55 mmol). The mixture was stirred at room temperature for 5 minutes, and 1-ethylpiperazine (41.5 mg, 0.364 mmol) in dichloromethane was added. The resulting mixture was stirred at room temperature for 10 minutes. Water was added and the resulting mixture was extracted twice with ethyl acetate, and the organic phases were combined, washed with water and saturated brine, dried over anhydrous sodium

sulfate, concentrated, and purified by silica gel column to afford 100 mg of intermediate MDI-236-1, with a yield of 64.3%.

$^1$H NMR (400 MHz, $CDCl_3$) δ 8.44 (d, *J* = 8 Hz, 1H), 8.28 (s, 1H), 7.50 - 7.33 (m, 5H), 7.22 (d, *J* = 8 Hz, 1H), 7.03 (d, *J* = 12 Hz, 1H), 6.95 (d, *J* = 8 Hz, 1H), 5.93 (s, 2H), 5.74 (s, 2H), 5.20 (s, 2H), 4.77 (s, 2H), 4.63 (s, 2H), 3.63-3.61 (m, 4H), 3.43 - 3.42 (m, 4H), 2.53 - 2.46 (m, 8H), 1.03 (t, *J* = 6 Hz, 3H), 0.93 - 0.86 (m, 7H), -0.06 - -0.08 (m, 18H).

**Synthesis of compound MDI-236: (2-(6-(2-ethyl-5-fluoro-4-hydroxyphenyl)-1H-indazol-3-yl)-4,6-dihydropyrrolo[3,4-d]imidazol-5-(1H)-yl)(4-ethylpiperazin-1-yl)ketone**

Intermediate MDI-236-1 (100 mg, 0.117 mmol) was dissolved in methanol (10 ml), to which 10 mg Pd/C was added and concentrated hydrochloric acid (5 ml) was added dropwise. The mixture was heated to 50 °C, reacted for 6 hours, filtered, and concentrated to give a residue. The residue was dissolved in methanol and was concentrated to dryness, which was repeated 3 times. The resulting residue was dissolved in methanol, and 1 ml of ammonia was added. The resulting mixture was concentrated, and purified by a preparation plate to afford 21 mg of the final product with a yield of 35.6%.

$^1$H NMR (400 MHz, MeOD-d4) δ 8.27 (d, *J* = 8 Hz, 1H), 7.43 (s, 1H), 7.17 (d, *J* = 8 Hz, 1H), 6.96 (d, *J* = 12 Hz, 1H), 6.91 (d, *J*= 8 Hz, 1H), 4.75 - 4.60 (m, 4H), 3.48 - 3.44 (m, 4H), 2.61 - 2.48 (m, 8H), 1.17 (t, *J* = 8 Hz, 3H), 1.08 (t, *J* = 8 Hz, 3H).

**Example 34: cyclopropyl (2-(6-(2-ethyl-5-fluoro-4-hydroxyphenyl)-1H-pyrazolo[4,3-b]pyridine-3-yl)-4,6-dihydropyrrolo[3,4-]imidazol-5(1H)-yl)ketone (MDI-237)**

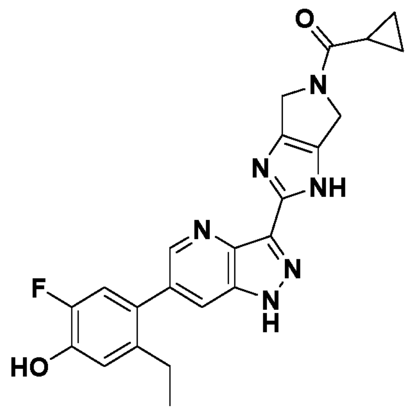

**MDI-237**

**Synthetic route of MDI-237:**

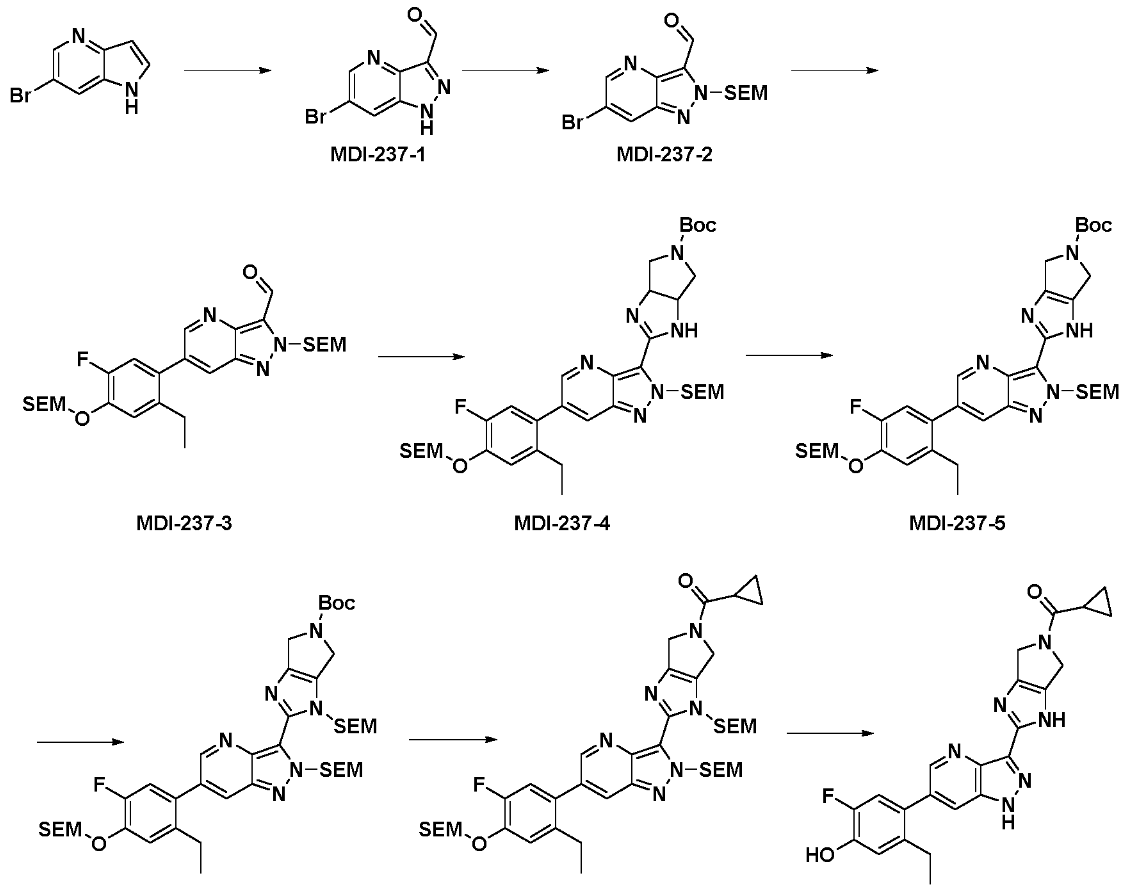

MDI-237-1 MDI-237-2

MDI-237-3 MDI-237-4 MDI-237-5

MDI-237-6 MDI-237-7 MDI-237

**Synthesis method:**

**Synthesis of intermediate MDI-237-1: 6-bromo-1H-pyrazolo[4,3-b]pyridine-3-formaldehyde**

Sodium nitrite (2.81 g, 40.72 mmol) was dissolved in 12 ml DMF and 16 ml water, and cooled to 0°C. Under nitrogen protection, 2N HCl (17.7 ml, 35.4 mmol) was slowly added dropwise, and after the addition was completed, the reaction continued for 10 minutes. At 0°C, 6-bromo-4-azaindole (1.0 g, 5.08 mmol) in DMF (8 ml) was slowly added to the reaction solution dropwise. After the addition was completed, it was allowed to react at room temperature overnight. After the reaction was completed, 50 ml of water was added to the reaction. The resulting mixture was stirred at room temperature for 0.5 hours, and filtered with suction to afford 580 mg of intermediate MDI-237-1 with a yield of 50.5%.

$^1$H NMR (400 MHz, DMSO) δ 14.52 (s, 1H), 10.27 (s, 1H), 8.80 (d, J = 2.0 Hz, 1H), 8.55 (d, J = 2.0 Hz, 1H).

**Synthesis of intermediate MDI-237-2: 6-bromo-1-((2-(trimethylsilyl) ethoxy)methyl)-2H-pyrazolo[4,3-b]pyridine-3-formaldehyde**

The intermediate MDI-237-1 (250 mg, 1.11 mmol) was dissolved in 5 ml DMF, and then was cooled to 0°C. NaH (60%) (53.1 mg, 1.33 mmol) was added in batches at 0°C. After the addition was completed, it was allowed to react for 30 minutes, and then SEMCl (276.6 mg, 1.66 mmol) was added dropwise to the reaction. After the dropwise addition was completed, the temperature was raised to room temperature for reaction. After the reaction was completed, it was quenched with water, and the resulting mixture was extracted with ethyl acetate, and the organic phase was washed with saturated brine, dried over anhydrous sodium sulfate, and concentrated by column chromatography to afford 157.4 mg of intermediate MDI-237-2 with a yield of 39.9%.

$^1$H NMR (400 MHz, CDCl3) δ 10.57 (s, 1H), 8.82 (d, J = 2.0 Hz, 1H), 8.39 (d, J = 2.0 Hz, 1H), 6.14 (s, 2H), 3.69-3.65 (m, 2H), 0.98-0.92 (m, 2H), -0.03 (s, 9H).

**Synthesis of intermediate MDI-237-3: 6-(2-ethyl-5-fluoro-4-((2-(trimethylsilanyl)ethoxy)methoxy)phenyl)-2-((2-(trimethylsilanyl)ethoxy)methyl)-2H-pyrazolo [4,3-b] pyridine-3-formaldehyde**

The intermediate MDI-237-2 (176 mg, 0.49 mmol), (2-((5-ethyl-2-fluoro-4-(4,4,5,5-tetramethyl-1,3,2 -dioxaborolan-2-yl)phenoxy)methoxy)ethyl)trimethylsilane (196 mg, 0.49 mmol), Pd(dppf)Cl2 (36.1 mg, 0.05 mmol) and potassium carbonate ( 205 mg, 1.48 mmol) were dissolved in 1,4-dioxane (20 ml) and water (4 ml). The atmosphere was replaced with nitrogen, which was repeated 3 times. The mixture was heated to 100 °C, reacted overnight, and cooled to room temperature. Water was added, the resulting mixture was extracted with ethyl acetate twice, and the organic phases were combined, washed with water and saturated brine, dried over anhydrous sodium sulfate, concentrated, and purified by silica gel column to afford 169.5 mg of intermediate MDI-237-3 with a yield of 62.7%.

$^1H$ NMR (400 MHz, CDCl3) δ 10.58 (s, 1H), 8.76 (d, J = 2.0 Hz, 1H), 8.08 (d, J = 2.0 Hz, 1H), 7.21 (d, J = 8.3 Hz, 1H ), 7.02 (d, J = 11.3 Hz, 1H), 6.19 (s, 2H), 5.33 (s, 2H), 3.88-3.83 (m, 2H), 3.72-3.69 (m, 2H), 2.59-2.53 (m, 2H), 1.10 (t, J = 8.0 Hz, 3H), 1.02-0.94 (m, 4H), 0.03 (s, 9H), -0.03 (s, 9H).

**Synthesis of intermediate MDI-237-4: tert-butyl 2-(6-(2-ethyl-5-fluoro-4-((2-(trimethylsilanyl)ethoxy)methoxy)phenyl)-2-((2-(trimethylsilanyl)ethoxy)methyl)-2H-pyrazolo[4,3-b]pyridine-3-yl)-3a,4,6,6a-tetrahydropyrrolo[3,4-d]imidazol-5(1H)-carboxylate**

The intermediate MDI-237-3 (170 mg, 0.31 mmol) and tert-butyl 3,4-diaminopyrroline-1-carboxylate (69.0 mg, 0.34 mmol) were dissolved in 10 ml tert-butanol, to which $I_2$ (98.8 mg, 0.39 mmol) and $K_2CO_3$ (129 mg, 0.93 mmol) were added. The mixture was heated to 70°C and reacted for 3 hours. After the reaction was completed, aqueous sodium thiosulfate was added to quench the reaction. The resulting mixture was extract twice with ethyl acetate, and the organic phases were combined, washed with water and saturated brine, dried with anhydrous sodium sulfate, concentrated, and purified by silica gel column to afford 150 mg of intermediate MDI-237-4 with a yield of 66.2%.

$^1H$ NMR (400 MHz, CDCl3) δ 8.54 (d, J = 1.9 Hz, 1H), 7.99 (d, J = 1.9 Hz, 1H), 7.20 (d, J = 8.3 Hz, 1H), 7.01 (d, J = 11.3 Hz, 1H), 6.60-6.20 (m, 2H), 5.33 (s, 2H), 5.00-4.87 (m, 1H), 4.58-4.46 (m, 1H), 3.88-3.83 (m, 2H), 3.79-3.64 (m, 6H), 2.58-2.52 (m, 2H), 1.57 (s, 9H), 1.09 (t, J = 8.0 Hz, 3H), 1.02-0.96 (m, 4H), 0.03 (s, 9H), -0.03 (s, 9H).

**Synthesis of intermediate MDI-237-5: Tert-butyl 2-(6-(2-ethyl-5-fluoro-4-((2-(trimethylsilanyl)ethoxy)methoxy)phenyl)-2-((2-(trimethylsilanyl)ethoxy)methyl)-2H-pyrazolo [4,3-b]pyridine-3-yl)-4,6-dihydropyrrolo[3,4-d]imidazol-5(1H)-carboxylate**

Intermediate MDI-237-4 (100 mg, 0.14 mmol) and 2-iodoyl benzoic acid (77.0 mg, 0.28 mmol) were dissolved in 10 ml DMSO, heated to 45 °C and reacted for 5 hours. After the reaction was completed, the reaction was quenched by aqueous sodium thiosulfate. The resulting mixture was extracted twice with ethyl acetate, and the organic phases were combined, washed with water and saturated brine, dried over anhydrous sodium sulfate, concentrated, and purified by silica gel column to afford 75.0 mg of intermediate MDI-237-5, with a yield 75.2%.

$^1H$ NMR (400 MHz, CDCl3) δ 11.78 (d, J = 8.8 Hz, 1H), 8.52-8.50 (m, 1H), 7.99-7.98 (m, 1H), 7.21 (d, J = 8.4 Hz, 1H) , 7.03 (d, J = 11.3 Hz, 1H), 6.44 (s, 2H), 5.33 (s, 2H), 4.66-4.51 (m, 4H), 3.88-3.79 (m, 4H), 2.60-2.54 (m , 2H), 1.54 (s, 9H), 1.11 (t, J = 8.0 Hz, 3H), 1.03-0.98 (m, 4H), 0.03 (s, 9H), -0.05 (s, 9H).

**Synthesis of intermediate MDI-237-6: tert-butyl 2-(6-(2-ethyl-5-fluoro-4-((2-(trimethylsilanyl)ethoxy)methoxy)phenyl)-2-((2-(trimethylsilanyl)ethoxy)methyl)-2H-pyrazolo[4,3-b]pyridine-3-yl)-1-((2-(trimethylsilyl)ethoxy)methyl)-4,6-dihydropyrrolo[3,4-d]imidazol-5(1H)-carboxylate**

The intermediate MDI-237-5 (20.0 mg, 0.03 mmol) was dissolved in 10 ml of THF, the temperature was reduced to 0 °C, and then NaH (60%) (1.2 mg, 0.03 mmol) was added. The reaction mixture was stirred for 0.5 hours. SEMCl (5.1 mg, 0.03 mmol) was added to the mixture, which was warmed to room temperature and stirred for 1 hour. After the reaction was completed, water was added to quench the reaction. The resulting mixture was extracted twice with ethyl acetate, and the organic phases were combined, washed with water and saturated brine, dried over anhydrous sodium sulfate, concentrated and purified by silica gel column to afford 15.0mg of intermediate MDI-237-6 with a yield of 63.5%.

$^1H$ NMR (400 MHz, CDCl3) δ 8.54 (d, J = 2.0 Hz, 1H), 8.00 (t, J = 2.1 Hz, 1H), 7.20 (d, J = 8.3 Hz, 1H), 7.03 (d, J = 11.4 Hz, 1H), 6.13 (d, J = 4.5 Hz, 2H), 5.57 (d, J = 3.7 Hz, 2H), 5.33 (s, 2H), 4.66-4.50 (m, 4H), 3.88-3.84 (m, 2H), 3.75-3.64 (m, 2H), 3.43-3.38 (m, 2H), 2.60-2.54 (m, 2H), 1.54 (s, 9H), 1.11 (t, J = 8.0 Hz, 3H), 1.02-0.98 (m, 2H), 0.93-0.88 (m, 2H), 0.82-0.77 (m, 2H), 0.03 (s, 9H), -0.03 (s, 9H), -0.06 (s, 9H).

**Synthesis of intermediate MDI-237-7:cyclopropyl (2-(6-(2-ethyl-5-fluoro-4-((2-(trimethylsilanyl)ethoxy)methoxy)phenyl)-2-((2-(trimethylsilanyl)ethoxy)methyl)-2H-pyrazolo[4,3-b]pyridine-3-yl)-1-((2-(trimethylsilyl)ethoxy)methyl)-4,6-dihydropyrrolo[3,4-d]imidazol-5(1H)-yl)ketone**

Intermediate MDI-237-6 (30.0 mg, 0.04 mmol) was dissolved in 10 ml DCM, to which zinc bromide (31.6 mg, 0.14 mmol) was added. The mixture was stirred for 5 hours, and water was added to the reaction to quench the reaction. The resulting mixture was extracted twice with DCM, and the organic phases were combined, washed with aqueous ammonia, then washed with water and saturated brine, dried over anhydrous sodium sulfate, and concentrated. The crude product was dissolved in 10 ml DCM, to which DIPEA (5.4 mg, 0.04 mmol) was added and then the mixture was cooled down 0°C. Then, cyclopropylformyl chloride (4.4 mg, 0.04 mmol) was added dropwise. After the addition was completed, the temperature was raised to room temperature for reaction. After the reaction was completed, water was added to quench the reaction, and the resulting mixture was extracted twice with DCM, and the organic phases were combined, wash with water and saturated brine, dried over anhydrous sodium sulfate, and concentrate to afford 23.0 mg of crude MDI-237-7, which was directly used in the next reaction. The crude yield was 79.6%.

**Synthesis of compound MDI-237: cyclopropyl (2-(6-(2-ethyl-5-fluoro-4-hydroxyphenyl)-1H-pyrazolo[4,3-b]pyridine-3-yl)-4,6-dihydropyrrolo[3,4-d] imidazol-5(1H)-yl)ketone**

The intermediate MDI-237-7 (23.0 mg, 0.03 mmol) was dissolved in 4 ml MeOH, to which 2 ml concentrated hydrochloric acid was added. After the addition, the temperature was raised to 50°C for reaction. After 6 hours of reaction, the temperature was lowered to room temperature, and the reaction solvent was evaporated by concentration under reduced pressure. After that, 4 ml methanol and 0.5 ml aqueous ammonia were added. After concentration, the residue was subject to thin layer chromatography to afford 3.2 mg of white solid MDI-237 with a yield of 26.5%.

$^1$H NMR (400 MHz, DMSO) δ 13.60 (s, 1H), 12.60-12.48 (m, 1H), 10.02 (s, 1H), 8.53 (d, J = 1.6 Hz, 1H), 7.95 (s, 1H) , 7.16 (d, J = 11.8 Hz, 1H), 6.98 (d, J = 9.1 Hz, 1H), 4.91-4.41 (m, 4H), 2.51-2.47 (m, 2H), 1.96-1.84 (m, 1H) ), 1.03 (t, J = 8.0 Hz, 3H), 0.87-0.80 (m, 4H). LC-MS m/z (ESI) $[M+H]^+$ calculated value for $C_{23}H_{22}FN_6O_2$: 433.2; measured value: 433.2.

**Example 35: cyclopropyl (2-(6-(2-ethyl-5-fluoro-4-hydroxyphenyl)-4-methyl-1H-indazol-3-yl)-4,6-dihydropyrrolo[3,4-d]imidazol-5(1H)-yl)ketone(MDI-239)**

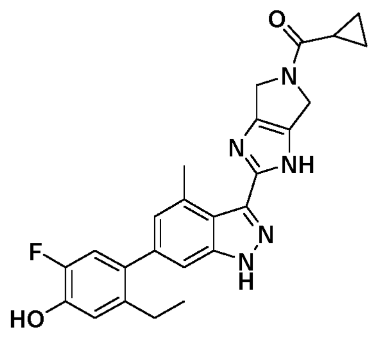

MDI-239

**Synthetic route of MDI-239:**

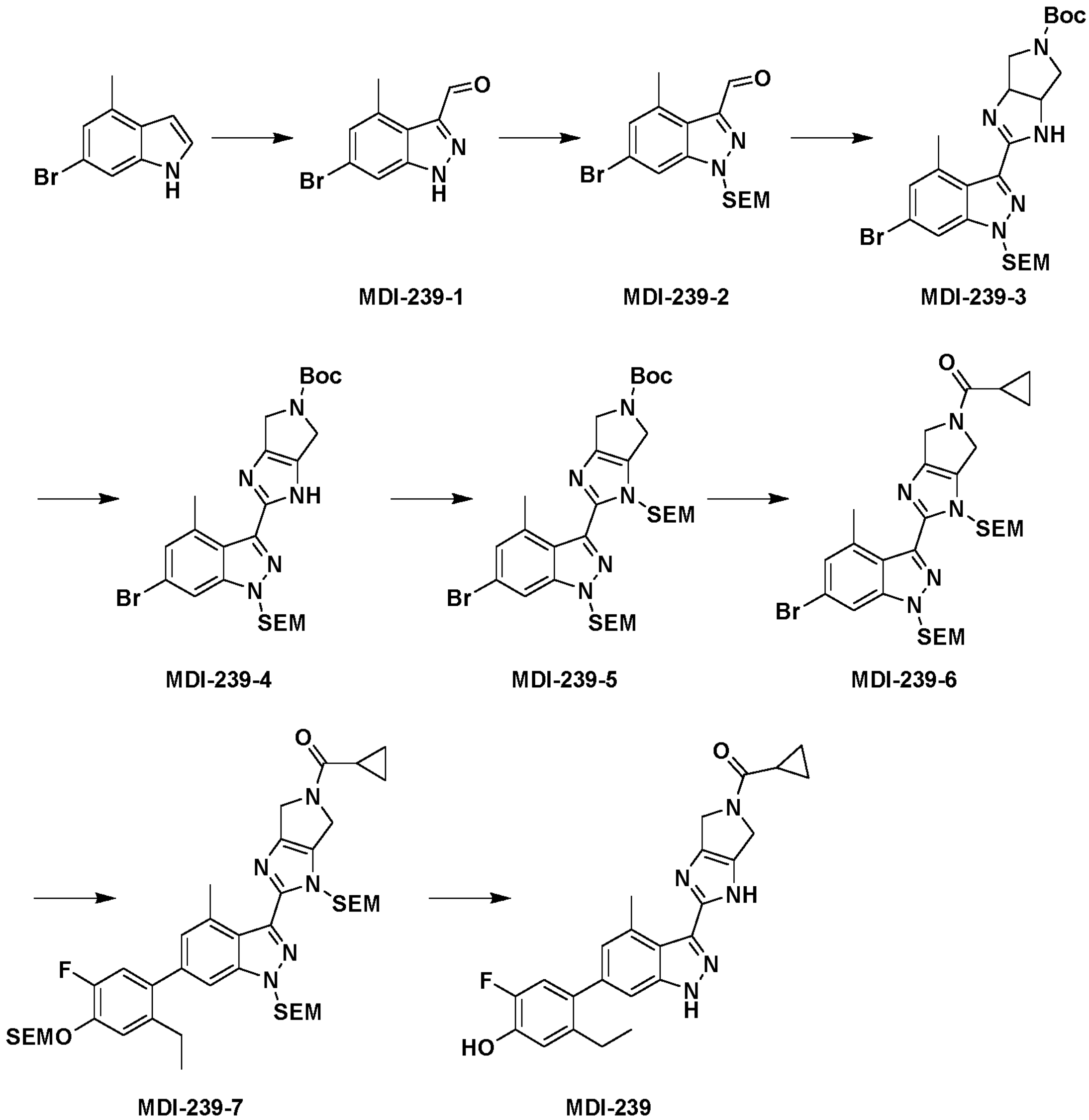

MDI-239-1 MDI-239-2 MDI-239-3

MDI-239-4 MDI-239-5 MDI-239-6

MDI-239-7 MDI-239

**Synthesis method:**

**Synthesis of intermediate MDI-239-1: 6-bromo-4-methyl-1H-indazol-3-formaldehyde**

Sodium nitrite (1.05 g, 15.2 mmol) was dissolved in 5 ml DMF and 5 ml water, and cooled to 0°C. Under nitrogen protection, 3N HCl (4.5 ml, 13.3 mmol) was slowly added dropwise, and the reaction was completed dropwise for 10 minutes. At 0°C, 6-bromo-4-methyl-1H-indole (400 mg, 1.90 mmol) in DMF (20 ml) was slowly added to the reaction solution dropwise. After the dropwise addition was completed, it was allowed to react at room temperature overnight. The mixture was extracted with ethyl acetate 3 times, and the organic phases were combined, washed 3 times with water, washed with saturated brine, dried over anhydrous sodium sulfate, concentrated, and purified by silica gel column to afford 388 mg of intermediate MDI-239-1 with a yield of 84.3%.

$^1H$ NMR (400 MHz, CDCl3) $\delta$ 10.61 (s, 1H), 10.24 (s, 1H), 7.58(d, J = 1.3 Hz, 1H), 7.27 (d, J = 1.2 Hz, 1H), 2.90 (s, 3H).

**Synthesis of intermediate MDI-239-2: 6-bromo-4-methyl-1-((2-(trimethylsilyl)ethoxy)methyl)-1H-indazol-3-formaldehyde**

Intermediate MDI-239-1 (388 mg, 1.62 mmol) was dissolved in 25 ml of dry tetrahydrofuran, and cooled to 0°C. Sodium hydride (60%) (117 mg, 4.86 mmol) was slowly added, and the mixture was stirred for 10 minutes. 2-(Trimethylsilyl)ethoxymethyl chloride (540 mg, 3.24 mmol) was added slowly dropwise, and the reaction was carried out at room temperature for 1 hour. Water was added to quench the reaction, and the resulting mixture was extracted twice with

ethyl acetate, and the organic phases were combined, washed with water and saturated brine, dried over anhydrous sodium sulfate, concentrated, and purified by silica gel column to afford 371 mg of intermediate MDI-239-2 with a yield of 61.9%.

$^1$H NMR (400 MHz, CDCl3) δ 10.20 (s, 1H), 7.68 (s, 1H), 7.30 (s, 1H), 5.78 (s, 2H), 3.61-3.57 (m, 2H), 2.89 (s, 3H), 0.96-0.89 (m, 2H), -0.02 (s, 9H).

**Synthesis of intermediate MDI-239-3: tert-butyl 2-(6-bromo-4-methyl-1-((2-(trimethylsilyl)ethoxy)methyl)-1H-indazol-3-yl)-3a,4,6,6a-tetrahydropyrrolo[3,4-d]imidazol-5(1H)-carboxylate**

The intermediate MDI-239-2 (371 mg, 1.00 mmol) and tert-butyl 3,4-diaminopyrroline-1-carboxylate (242 mg, 1.20 mmol) were dissolved in 10 ml tert-butanol, followed by addition of iodine ( 317 mg, 1.25 mmol) and potassium carbonate (414 mg, 3.00 mmol), and the reaction was carried out at 70°C for 3 hours. The reaction was quenched by adding a saturated aqueous solution of sodium thiosulfate and the resulting mixture was extracted twice with ethyl acetate. The organic phases were combined, washed with water and saturated brine, dried over anhydrous sodium sulfate, concentrated, and purified by silica gel column to afford 330 mg of intermediate MDI-239-3 with a yield of 60.0%.

$^1$H NMR (400 MHz, CDCl3) δ 7.62 (s, 1H), 7.20 (s, 1H), 5.68 (s, 2H), 4.77-4.66 (m, 2H), 3.77-3.60 (m, 4H), 3.57- 3.53 (m, 2H), 2.89 (s, 3H), 1.46 (s, 9H), 0.94-0.89 (m, 2H), -0.02 (s, 9H).

**Synthesis of intermediate MDI-239-4: tert-butyl 2-(6-bromo-4-methyl-1-((2-(trimethylsilyl)ethoxy)methyl)-1H-indazol-3-yl)-4,6-dihydropyrrolo[3,4-d]imidazol-5(1H)-carboxylate**

MDI-239-3 (330 mg, 0.60 mmol) was dissolved in 15 ml DMSO, and IBX (252 mg, 0.90 mmol) was added. It was allowed to react at 50°C for 16 hours. The reaction was quenched by adding water, and resulting mixture was extracted twice with ethyl acetate. The organic phases were combined, washed with water and saturated brine, dried over anhydrous sodium sulfate, concentrated, and purified on a silica gel column to afford 240 mg of intermediate MDI-239-4 with a yield of 73.0%.

$^1$H NMR (400 MHz, CDCl3) δ 7.60 (s, 1H), 7.19 (s, 1H), 5.67 (s, 2H), 4.62-4.50 (m, 4H), 3.67-3.54 (m, 2H), 2.98 (d, J = 7.2 Hz, 3H), 1.55 (s, 9H), 0.98-0.89 (m, 2H), - 0.03 (s, 9H).

**Synthesis of intermediate MDI-239-5: tert-butyl 2-(6-bromo-4-methyl-1-((2-(trimethylsilyl)ethoxy)methyl)-1H-indazol-3-yl)-1-((2-(trimethylsilyl)ethoxy)methyl) -4,6-dihydropyrrolo[3,4-d]imidazol-5(1H)-carboxylate**

Intermediate MDI-239-4 (145 mg, 0.26 mmol) was dissolved in 15 ml of dry tetrahydrofuran, and cooled to 0°C. Sodium hydride (60%) (19.0 mg, 0.79 mmol) was slowly added, and the mixture was stirred for 10 minutes. 2-(Trimethylsilyl)ethoxymethyl chloride (86.7 mg, 0.52 mmol) was added slowly dropwise. After the addition, the reaction was carried out at room temperature for 1 hour. The reaction was quenched by adding water, the resulting mixture was extracted twice with ethyl acetate, and the organic phases were combined, washed with water and saturated brine, dried over anhydrous sodium sulfate, concentrated, and purified by silica gel column to afford 148 mg of intermediate MDI-239-5 with a yield of 84.0%.

$^1$H NMR (400 MHz, CDCl3) δ 7.67 (s, 1H), 7.17 (s, 1H), 5.73 (s, 2H), 5.44 (d, J = 4.7 Hz, 2H), 4.65-4.51 (m, 4H) , 3.61-3.57 (m, 2H), 3.38 -3.34 (m, 2H), 2.54 (d, J = 5.8 Hz, 3H), 1.56 (s, 9H), 0.97-0.88 (m, 4H), -0.02 ( s, 9H), -0.11(s, 9H).

**Synthesis of intermediate MDI-239-6: (2-(6-bromo-4-methyl-1-((2-(trimethylsilyl)ethoxy)methyl)-1H-indazol-3-yl)-1-((2-(trimethylsilyl)ethoxy)methyl)-4, 6-dihydropyrrolo[3,4-d]imidazol-5(1H)-yl)(cyclopropyl)ketone**

Intermediate MDI-239-5 (148 mg, 0.22 mmol) was dissolved in 15 ml of dichloromethane, and zinc bromide (197 mg, 0.87 mmol) was added. The mixture was stirred at 25°C for 4 hours, and 10 ml of aqueous ammonia was added to the reaction solution. After liquid separation, the organic phase was washed with saturated sodium bicarbonate, and saturated sodium chloride, dried over anhydrous sodium sulfate, and concentrated. The concentrate was dissolved in 10 ml of dichloromethane and triethylamine (66.8 mg, 0.66 mmol), and cooled to 0°C. Cyclopropionyl chloride (46.0 mg, 0.44 mmol) was slowly added, and it was allowed to react at room temperature for 1 hour. The reaction was quenched by adding water. The resulting mixture was extracted with dichloromethane twice, and the organic phases were combined, washed with water and saturated brine, dried over anhydrous sodium sulfate, concentrated, and purified by silica gel column to afford intermediate MDI-239-6 94 mg, with a yield of 65.8%.

$^1$H NMR (400 MHz, CDCl3) δ 7.70 (s, 1H), 7.20 (s, 1H), 5.75 (s, 2H), 5.48 (d, J = 15.8 Hz, 2H), 5.00-4.69 (m, 4H) , 3.63-3.59 (m, 2H), 3.46-3.34 (m, 2H), 2.57 (d, J = 7.7 Hz, 3H), 2.09-2.05 (m, 1H), 1.09-1.00 (m, 4H), 0.98 -0.89 (m, 4H), 0.00-0.05 (m, 18H).

**Synthesis of intermediate MDI-239-7: cyclopropyl(2-(6-(2-ethyl-5-fluoro-4-((2-(trimethylsilanyl)ethoxy)methoxy)phenyl)-4-methyl-1-((2-(trimethylsilanyl) ethoxy)methyl)-1H-indazol-3-yl)-1-((2-(trimethylsilyl)ethoxy)methyl)-4,6-dihydropyrrolo[3,4-d]imidazol-5(1H)-yl)ketone**

The intermediate MDI-239-6 (40.0 mg, 0.06 mmol), (2-((5-ethyl-2-fluoro-4-(4,4,5,5-tetramethyl-1,3,2-dioxaborolan-2-yl)phenoxy)methoxy)ethyl)trimethylsilane (36.8 mg, 0.09 mmol), tetrakistriphenylphosphine palladium (6.9 mg, 0.01 mmol) and potassium phosphate (39.4 mg, 0.19 mmol) were dissolved in 1,4-dioxane (10 ml) and water (2 ml). The atmosphere was replaced with nitrogen, which was repeated 3 times. The mixture was heated to 100 °C, reacted for 16 hours, and cooled to room temperature. Water was added, the resulting mixture was extracted twice with ethyl acetate, and the organic phases were combined, washed with water and saturated brine, dried over anhydrous sodium sulfate, concentrated, and purified by a silica gel column to afford 27.3 mg of intermediate MDI-239-7 with a yield of 52.6%.

$^1$H NMR (400 MHz, CDCl3) δ 7.36 (s, 1H), 7.17 (d, J = 8.4 Hz, 1H), 7.06-6.92 (m, 2H), 5.79 (d, J = 7.0 Hz, 2H), 5.53 (d, J = 14.4 Hz, 2H), 5.33 (d, J = 5.3 Hz, 2H), 5.00-4.69 (m, 4H), 3.90-3.86 (m, 2H), 3.64-3.58 (m, 2H), 3.42-3.37 (m, 2H), 2.64-2.56 (m, 5H), 2.06-2.03 (m, 1H), 1.13- 1.07 (m, 4H), 1.05-1.01 (m, 3H), 0.95-0.89 (m , 6H), 0.02 (s, 9H), -0.03-0.12 (m, 18H).

**Synthesis of compound MDI-239: cyclopropyl (2-(6-(2-ethyl-5-fluoro-4-hydroxyphenyl)-4-methyl-1H-indazol-3-yl)-4,6-dihydropyrrolo[3,4-d]imidazol-5(1H)-yl)ketone**

Intermediate MDI-239-7 (27.3 mg, 0.03 mmol) was dissolved in methanol (6 ml), to which concentrated hydrochloric acid (3 ml) was added. The mixture was heated to 50 °C, reacted for 6 hours, and concentrated to give a residue. The residue was dissolved in methanol, and was concentrated to dryness, which was repeated 3 times. The resulting residue was dissolved in methanol, and 1 ml aqueous ammonia was added. The mixture was concentrated, and filtered. The resulting filtrate was concentrated, and purified by a preparation plate to afford 5.1 mg of the final product with a yield of 34.7%.

$^1$H NMR (400 MHz, MeOD) δ 7.27 (s, 1H), 6.95-6.88 (m, 3H), 4.96 (s, 2H), 4.66 (s, 2H), 2.63 (s, 3H), 2.55 (q, J = 7.5 Hz, 2H), 1.98-1.92 (m, 1H), 1.07 (t, J = 7.5 Hz, 3H), 1.04-0.92 (m, 4H).

**Example 36: (S)-(2-(6-(2-ethyl-5-fluoro-4-hydroxyphenyl)-4-methyl-1H-indazol-3-yl)-4,6-dihydropyrrolo[3,4-d]imidazol-5(1H)-yl)(3-hydroxypyrrolidin-1-yl)ketone(MDI-240)**

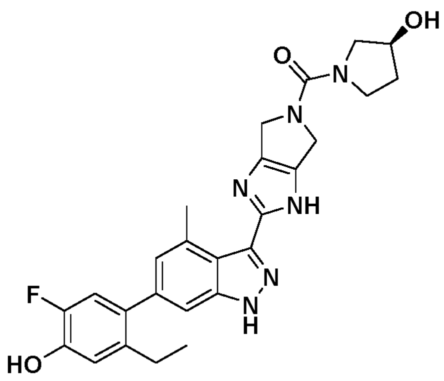

MDI-240

**Synthetic route of MDI-240**

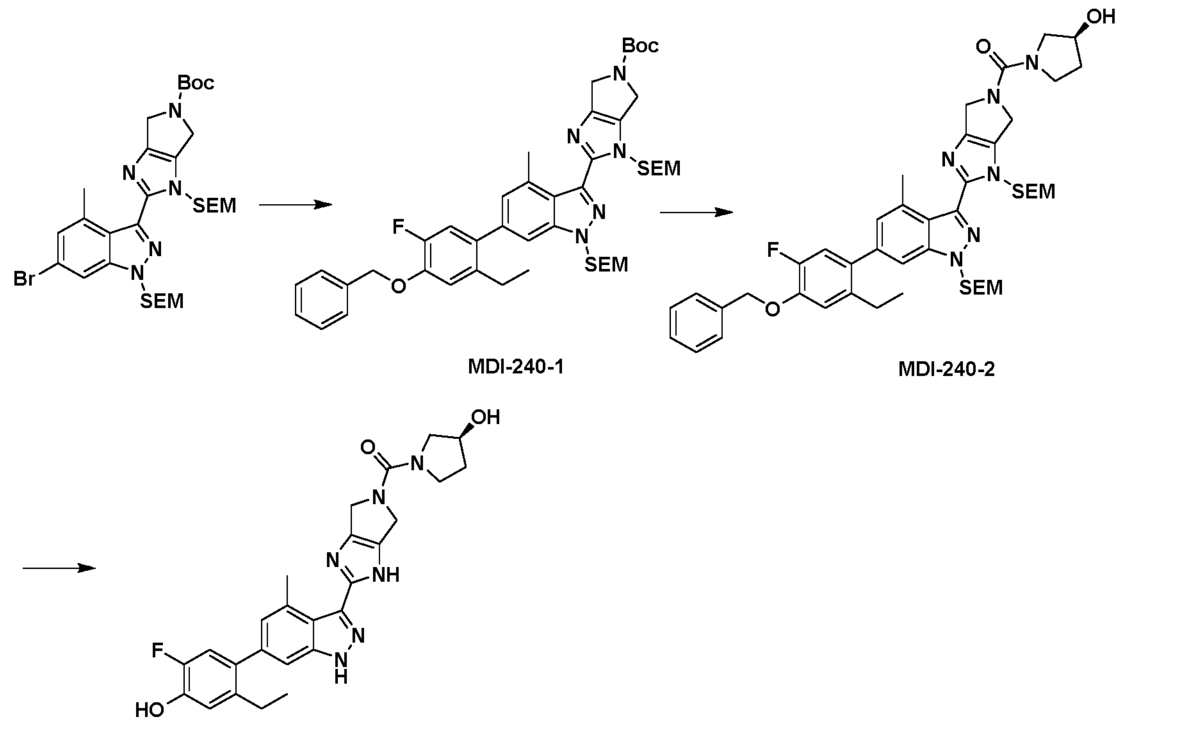

MDI-240-1

MDI-240-2

MDI-240

**Synthesis method:**

**Synthesis of intermediate MDI-240-1: tert-butyl 2-(6-(4-(benzyloxy)-2-ethyl-5-fluorophenyl)-4-methyl-1-((2-(trimethylsilyl)ethoxy)methyl)-1H-indazol-3-yl)-1-((2-(trimethylsilyl)ethoxy)methyl)-4,6-dihydropyrrolo[3,4-d]imidazol-5(1H)-carboxylate**

Tert-butyl 2-(6-bromo-4-methyl-1-(((2-(trimethylsilanyl)ethoxy)methyl)-1H-indazol-3-yl)-1-(((2-(trimethylsilanyl)ethoxy)methyl)-4,6-dihydropyrrolo[3,4-d]imidazol-5(1H)-carboxylate (60.0 mg, 0.09 mmol), 2-(4-(benzyloxy)-2-ethyl-5-fluorophenyl)-4,4,5,5-tetramethyl-1,3,2-dioxaborane (47.2 mg, 0.13 mmol), tetrakistriphenylphosphine palladium (10.4 mg, 0.01 mmol) and potassium phosphate (55.9 mg, 0.26 mmol) were dissolved in 1,4-dioxane (10 ml) and water (2 ml). The atmosphere was replaced with nitrogen, which was repeated 3 times. The mixture was heated to 100 °C, reacted for 16 hours, and cooled to room temperature. Water was added, the resulting mixture was extracted 2 times with ethyl acetate, and the organic phases were combined, washes with water and saturated brine, dried over anhydrous sodium sulfate, concentrated and purified by a silica gel column to afford 63.2 mg of the intermediate MDI-240-1 with a yield of 86.7%.

$^1$H NMR (400 MHz, CDCl3) δ 7.52 (d, J = 7.4 Hz, 2H), 7.46-7.34 (m, 4H), 7.03-6.96 (m, 3H), 5.77 (s, 2H), 5.50 (d, J = 4.1 Hz, 2H), 5.22 (s, 2H), 4.67-4.53 (m, 4H), 3.64-3.60 (m, 2H), 3.40-3.35 (m, 2H), 2.59-2.51 (m, 5H), 1.56 (s, 9H), 1.06 (t, J = 7.5 Hz, 3H), 0.98-0.89 (m, 4H), -0.04 (s, 9H), -0.12 (s, 9H).

**Synthesis of intermediateMDI-240-2: (S)-(2-(6-(4-(benzyloxy)-2-ethyl-5-fluorophenyl)-4-methyl-1-((2-(trimethylsilyl)ethoxy)methyl)-1H-indazol-3-yl)-1-((2-(trimethylsilyl)ethoxy)methyl)-4,6-dihydropyrrolo[3,4-d]imidazol-5(1H)-yl)(3-hydroxyl pyrrolidin-1-yl)ketone**

Intermediate MDI-240-1 (63.2 mg, 0.08 mmol) was dissolved in 10 ml of dichloromethane, and zinc bromide (68.7 mg, 0.31 mmol) was added. The mixture was stirred at 25°C for 4 hours, and 6 ml of aqueous ammonia was added to the reaction solution. After liquid separation, the organic phase was washed with saturated sodium bicarbonate and saturated sodium chloride, dried over anhydrous sodium sulfate, and concentrated. The concentrate was dissolved in 8 ml dichloromethane, at 0°C triphosgene (22.5 mg, 0.08 mmol) was added, and triethylamine (76.7 mg, 0.76 mmol) was slowly added dropwise. The mixture was stirred at room temperature for 10 minutes, and (S)-pyrrolidine butan-3-ol (13.2 mg, 0.15 mmol) in dichloromethane was added. The mixture was stirred at room temperature for 20 minutes. Water was added, the resulting mixture was extracted twice with ethyl acetate, and the organic phases were combined, washed with water and saturated brine, dried over anhydrous sodium sulfate, concentrated and purified by silica gel

column to afford 44.0 mg of intermediate MDI-240-2 with a yield of 68.9%.

$^1$H NMR (400 MHz, CDCl3) δ 7.51 (d, J = 7.0 Hz, 2H), 7.45-7.34 (m, 4H), 7.03- 6.95 (m, 3H), 5.77 (s, 2H), 5.49 (s, 2H), 5.22 (s, 2H), 4.76 -4.53 (m, 4H), 4.46-4.44 (m, 1H), 3.64-3.54 (m, 4H), 3.44-3.33 (m, 4H), 2.57-2.51 (m, 5H), 2.06-1.90 (m, 2H), 1.05 (t, J = 7.5 Hz, 3H), 1.00- 0.88 (m, 4H), -0.04 (s, 9H), -0.13 (s, 9H).

**Synthesis of compound MDI-240: (S)-(2-(6-(2-ethyl-5-fluoro-4-hydroxyphenyl)-4-methyl-1H-indazol-3-yl)-4,6-dihydropyrrolo [3,4-d]imidazol-5(1H)-yl)(3-hydroxylpyrrolidin-1-yl)ketone**

MDI-240-2 (44.0 mg, 0.05 mmol) was dissolved in 6 ml methanol, to which 5 mg 10% palladium on carbon was added. The atmosphere was replaced with hydrogen. It was allowed to react at 40°C for 1 hour. After the reaction was completed, the resulting mixture was filtered, and the filtrate was concentrated. The concentrate was dissolved in 6 ml methanol, and 3 ml concentrated hydrochloric acid was added. It was allowed to react for 7 hours at 50°C, and the mixture was concentrated to give a residue. The residue was dissolved in methanol and was concentrated to dryness, which was repeated 3 times. The resulting residue was dissolved in 5 ml methanol, and 0.5 ml aqueous ammonia was added. The resulting mixture was concentrated, and purified by a preparation plate to afford 5.7 mg of the final product with a yield of 22.3%.

$^1$H NMR (400 MHz, MeOD) δ 7.27 (s, 1H), 6.95-6.88 (m, 3H), 4.85 -4.82 (m, 2H), 4.62-4.59 (m, 2H), 4.46-4.45 (m, 1H), 3.79-3.69 (m, 2H), 3.64-3.57 (m, 1H), 3.46-3.42 (m, 1H), 2.61 (s, 3H), 2.56 (q, J = 7.5 Hz, 2H), 2.09- 1.98 (m, 2H), 1.07 (t, J = 7.5 Hz, 3H).

**Example 37: cyclopropyl(2-(6-(2-ethyl-5-fluoro-4-hydroxyphenyl)-1H-pyrazolo[4,3-c]pyridine-3-yl)-4,6-dihydropyrrolo[3,4-d]imidazol-5(1H)-yl)ketone (MDI-242)**

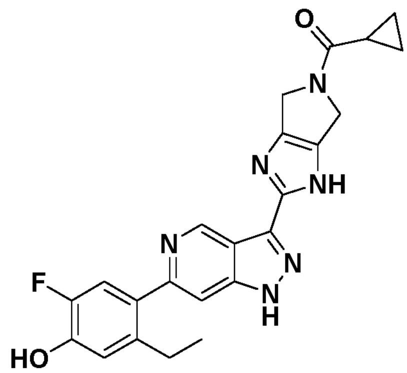

**MDI-242**

**Synthetic route of MDI-242:**

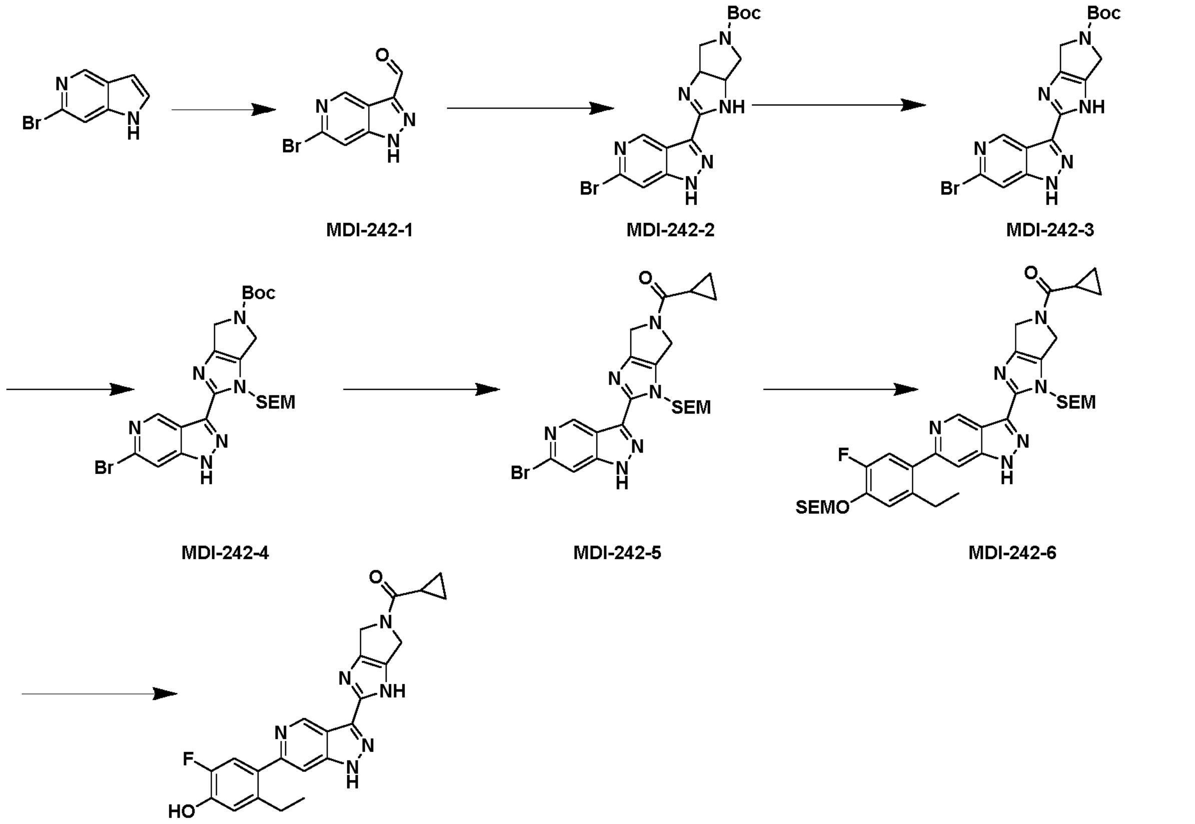

MDI-242-1 MDI-242-2 MDI-242-3

MDI-242-4 MDI-242-5 MDI-242-6

MDI-242

## Synthesis method:

### Synthesis of intermediate MDI-242-1: 6-bromo-1H-pyrazolo[4,3-c]pyridine-3-formaldehyde

Sodium nitrite (1.68 g, 24.4 mmol) was dissolved in 15 ml DMF and 15 ml water, and 3N HCl (7.1 ml, 21.3 mmol) was added at 0°C. The mixture was stirred for 10 minutes, and 6-bromo-1H-pyrrolo[3,2-c]pyridine (600 mg, 3.04 mmol) in DMF (15 ml) was added dropwise at 0°C. It was allowed to react at room temperature for 30 minutes, and to react at 50 °C for 3 hours. The resulting mixture was extracted 3 times with ethyl acetate, and the organic phases were combined, washed with water and saturated brine, dried over anhydrous sodium sulfate, concentrated, and purified by silica gel column to afford 350 mg of intermediate MDI-242-1 with a yield of 50.9%.

$^1$H NMR (400 MHz, CDCl3) δ 10.40 (s, 1H), 9.25 (s, 1H), 7.88 (s, 1H).

### Synthesis of intermediate MDI-242-2: tert-butyl 2-(6-bromo-1H-pyrazolo[4,3-c]pyridine-3-yl)-3a,4,6,6a-tetrahydropyrrolo[3,4-d]imidazol-5(1H)-carboxylate

Intermediate MDI-242-1 (350 mg, 1.55 mmol) was dissolved in 15 ml tert-butanol, followed by addition of tert-butyl 3,4-diaminopyrrolidine-1-carboxylate (3734 mg, 1.86 mmol), potassium carbonate (775 mg, 5.57 mmol) and iodine (590 mg, 2.32 mmol). The mixture was stirred at 60°C for 3 hours, and aqueous saturated sodium thiosulfate was added. The resulting mixture was extracted 3 times with ethyl acetate, and the organic phases were combined, washed with water and saturated brine, dried over anhydrous sodium sulfate, concentrated, and purified by silica gel column to afford 240 mg of intermediate MDI-242-2 with a yield of 38.1%.

$^1$H NMR (400 MHz, CDCl3) δ 9.29 (d, J = 0.9 Hz, 1H), 7.78 (d, J = 1.0 Hz, 1H), 5.00-4.95 (m, 1H), 4.52-4.49 (m, 1H), 3.77-3.70 (m, 3H), 3.57-3.53 (m, 1H), 1.42 (s, 9H).

### Synthesis of intermediate MDI-242-3: Tert-butyl 2-(6-bromo-1H-pyrazolo[4,3-c]pyridine-3-yl)-4,6-dihydropyrrolo[3,4-d]imidazol-5(1H)-carboxylate

Intermediate MDI-242-2 (240 mg, 0.59 mmol) was dissolved in 15 ml DMSO, and IBX (330 mg, 1.18 mmol) was added. The mixture was stirred overnight at 45 °C, and extracted 3 times with ethyl acetate. The organic phases

were combined, washed with water and saturated brine, dried over anhydrous sodium sulfate, concentrated, and purified by silica gel column to afford 100 mg of intermediate MDI-242-3, with a yield of 41.9%.

$^1$H NMR (400 MHz, $CDCl_3$) δ 9.46 (d, J = 6.1 Hz, 1H), 7.78 (s, 1H), 4.65-4.53(m, 4H), 1.54 (s, 9H).

**Synthesis of intermediate MDI-242-4: Tert-butyl 2-(6-bromo-1H-pyrazolo[4,3-c]pyridine-3-yl)-1-((2-(trimethylsilyl)ethoxy)methyl)-4,6-dihydropyrrolo[3,4 -d]imidazol-5(1H)-carboxylate**

Intermediate MDI-242-3 (100 mg, 0.25 mmol) was dissolved in 15 ml THF and cooled to 0°C. NaH (60%) (21.7 mg, 0.54 mmol) was added and the mixture was stirred at 0°C for 20 minutes. After that, SEM-Cl (103 mg, 0.62 mmol) was added. It was allowed to react for 2 hours. The resulting mixture was extracted 3 times with ethyl acetate, and the organic phases were combined, washed with water and saturated brine, dried over anhydrous sodium sulfate, concentrated, and purified on silica gel column to afford 100 mg of intermediate MDI-242-4 with a yield of 75.7%.

$^1$H NMR (400 MHz, CDCl3) δ 9.53-9.51 (m, 1H), 7.77 (d, J = 1.2 Hz, 1H), 5.85 (d, J = 7.2 Hz, 2H), 4.65-4.52 (m, 4H), 3.66-3.61 (m, 2H), 1.54 (s, 9H), 0.90-0.86 (m, 2H), -0.02 (s, 9H).

**Synthesis of intermediate MDI-242-5: (2-(6-bromo-1H-pyrazolo[4,3-c]pyridine-3-yl)-1-((2-(trimethylsilyl)ethoxy)methyl)-4,6-dihydropyrrolo[3,4-d]imidazol-5(1H)-yl)(cyclopropyl)ketone**

Intermediate MDI-242-4 (100 mg, 0.19 mmol) was dissolved in 5 ml of dichloromethane, and zinc bromide (168 mg, 0.75 mmol) was added. The mixture was stirred at room temperature for 4 hours and aqueous ammonia was added. The resulting mixture was extracted twice with dichloromethane, and the organic phases were combined, washed with water and saturated brine, dried over anhydrous sodium sulfate, and concentrated. The obtained compound was dissolved in 5 ml of dichloromethane, and triethylamine (56.6 mg, 0.56 mmol) was added. The mixture was cooled to 0°C, and cyclopropylformyl chloride (29.3 mg, 0.28 mmol) was added. It was allowed to react at room temperature for 2 hours. Water was added to quench the reaction, and the resulting mixture was extracted twice with dichloromethane, and the organic phases were combined, washed with water and saturated brine, dried over anhydrous sodium sulfate, concentrated, and purified by silica gel column to afford 60.0 mg of intermediate MDI-242-5 with a yield of 63.8 %.

$^1$H NMR (400 MHz, $CDCl_3$) δ 9.53 (d, J = 7.4 Hz, 1H), 7.78 (d, J = 2.4 Hz, 1H), 5.86 (s, 2H), 4.97-4.67 (m, 4H), 3.68 -3.61 (m, 2H), 1.77-1.72 (m, 1H), 1.13-1.09 (m, 2H), 0.99-0.89 (m, 4H), -0.05 (s, 9H).

**Synthesis of intermediate MDI-242-6: cyclopropyl (2-(6-(2-ethyl-5-fluoro-4-((2-(trimethylsilyl)ethoxy)methoxy)phenyl)-1H-pyrazolo [4,3-c] pyridine-3-yl)-1-((2-(trimethylsilyl)ethoxy)methyl)-4,6-dihydropyrrolo[3,4-d]imidazol-5(1H)-yl)ketone**

Intermediate MDI-242-5 (60 mg, 0.12 mmol) was dissolved in 5 ml dioxane and 1 ml water, followed by addition of (2-((5-ethyl-2-fluoro-4-(4,4,5,5-tetramethyl-1,3,2-dioxaborolan-2-yl)phenoxy)methoxy)ethyl)trimethylsilane (56.7 mg, 0.14 mmol), $Pd(PPh_3)_4$ (13.8 mg, 0.01 mmol) and potassium carbonate (49.4 mg, 0.36 mmol). The atmosphere was replaced with nitrogen. The mixture was stirred at 100°C for 2 hours. Water was added to quench the reaction, and the resulting mixture was extracted twice with ethyl acetate, and the organic phases were combined, washed with water and saturated brine, dried over anhydrous sodium sulfate, concentrated, and purified by silica gel column to afford 40 mg of intermediate MDI-242-6 with a yield of 48.4%.

$^1$H NMR (400 MHz, $CDCl_3$) δ 9.79 (d, J = 8.7 Hz, 1H), 7.58 (d, J = 1.2 Hz, 1H), 7.26-7.18 (m, 2H), 5.95-5.90 (m, 2H), 5.33 (s, 2H), 5.00-4.70 (m, 4H), 3.86-3.82 (m, 2H), 3.69-3.62 (m, 2H), 2.74-2.68 (m, 2H), 1.79-1.70 (m, 1H), 1.14-1.08 (m, 5H), 1.02-0.96 (m, 4H), 0.92-0.88 (m, 2H), 0.03 (s, 9H), -0.02-0.04 (m, 9H).

**Synthesis of compound MDI-242: cyclopropyl (2-(6-(2-ethyl-5-fluoro-4-hydroxyphenyl)-1H-pyrazolo[4,3-c]pyridine-3-yl)-4,6-dihydropyrrolo[3,4-d]imidazol-5(1H)-yl)ketone(MDI-242)**

Intermediate MDI-242-6 (40 mg, 0.06 mmol) was dissolved in 4 ml of methanol, and 2 ml of concentrated hydrochloric acid was added. The mixture was heated to 50°C, reacted for 6 hours, and concentrated. The solid was dissolved in 1 ml of methanol, and pH was adjusted with aqueous ammonia to 8-9. The resulting mixture was concentrated and purified by a preparation plate to afford 8.0 mg of the final product with a yield of 32.0%.

$^1$H NMR (400 MHz, MeOD) δ 9.61 (d, J = 1.0 Hz, 1H), 7.77 (d, J = 1.1 Hz, 1H), 7.16 (d, J = 11.6 Hz, 1H), 6.93 (d, J = 8.8 Hz, 1H), 5.07-4.88 (m, 2H), 4.68-4.62 (m, 2H), 2.69-2.64 (m, 2H), 1.98-1.89 (m, 1H), 1.09-1.05 (m, 3H) ), 1.01-0.98 (m, 2H), 0.96-0.94 (m, 2H).

**Example 38: (R)-(2-(6-(2-ethyl-5-fluoro-4-hydroxyphenyl)-1H-indazol-3-yl)-4,6-dihydropyrrolo[3,4-d]imidazol-5(1H)-yl)(3-hydroxylpyrrolidin-1-yl)ketone (MDI-243)**

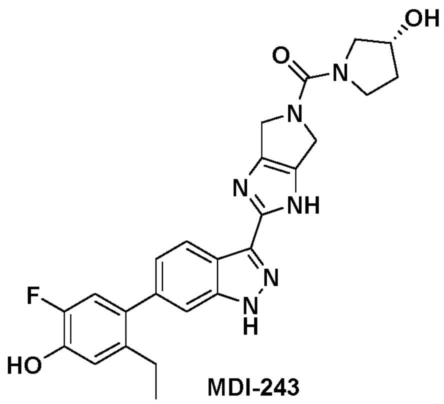

MDI-243

**Synthetic route of MDI-243:**

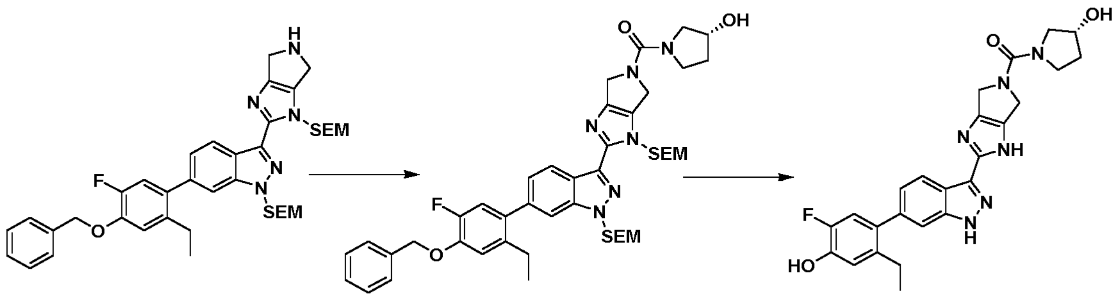

MDI-243-1

MDI-243

**Synthesis method:**

**Synthesis of intermediate MDI-243-1: (R)-(2-(6-(4-(benzyloxy)-2-ethyl-5-fluorophenyl)-1-((2-(trimethylsilyl)ethoxy)methyl)-1H-indazol-3-yl)-1-((2-(trimethylsilyl)ethoxy)methyl)-4,6-dihydropyrrolo[3,4-d]imidazol-5-(1H)-yl)(3-hydroxylpyrrolidin-1-yl)ketone**

Triphosgene (54.1 mg, 0.18 mmol) was dissolved in 10 ml of dichloromethane, and at 0°C, the intermediate 6-(4-(benzyloxy)-2-ethyl-5-fluorophenyl)-1-((2-(trimethylsilyl)ethoxy)methyl)-3-(1-((2-(trimethylsilyl)ethoxy)methyl)-1-1,4,5,6-tetrahydropyrrolo[3,4-d]imidazol-2-yl)-1H-indazole (130 mg, 0.18 mmol) in dichloromethane (5 ml), was added dropwise. After the addition, anhydrous triethylamine (55.2 mg, 0.55 mmol) was added dropwise. The mixture was stirred at room temperature for 10 minutes. TLC monitored that the raw materials disappeared. (R)-Pyrrolidin-3-ol (31.8 mg, 0.36 mmol) in dichloromethane (5ml) was added. The resulting mixture was stirred at room temperature for 20 minutes. Water was added to quench the reaction and the resulting mixture was extracted twice with ethyl acetate, and the organic phases were combined, washed with saturated brine, dried over anhydrous sodium sulfate, concentrated, and purified by silica gel column to afford 118 mg of intermediate MDI-243-1, with a yield of 78.4%.

$^1$H NMR (400 MHz, CDCl3) δ 8.47 (d, *J* = 8.3 Hz, 1H), 7.75 - 7.73 (m, 2H), 7.47 - 7.35 (m, 4H), 7.25 (d, *J* = 8.4 Hz, 1H), 7.06-6.96 (m, 2H), 5.96 (s, 2H), 5.78 (s, 2H), 5.23 (s, 2H), 4.95 - 4.56 (m, 4H), 4.50 - 4.45 (m, 1H), 3.79 - 3.72 (m, 2H), 3.66 - 3.58 (m, 5H), 3.46 - 3.42 (m, 1H), 2.54 (q, *J* = 7.6 Hz, 2H), 2.06 - 2.01 (m, 2H), 1.06 (t, *J* = 7.5 Hz, 3H), 0.99 - 0.89 (m, 4H), 0.02 (s, 9H), -0.05 (d, *J* = 3.4 Hz, 9H).

**Synthesis of compound MDI-243 : (R)-(2-(6-(2-ethyl-5-fluoro-4-hydroxyphenyl)-1H-indazol-3-yl)-4,6-dihydropyrrolo[3,4-d]imidazol-5-(1H)-yl)(3-hydroxylpyrrolidin-1-yl)ketone**

MDI-243-1 (118 mg, 0.14 mmol) was dissolved in 20 ml methanol, and 20 mg palladium on carbon was added. The atmosphere was replaced hydrogen. It was allowed to react at 40 °C for 1 hour. After the reaction was completed, the mixture was filtered, and the filtrate was concentrated. The concentrate was dissolved in 12 ml methanol and 6ml concentrated hydrochloric acid was added. It was allowed to react at 50°C for 7 hours and the mixture was concentrated to give a residue. The residue was dissolved in methanol and was concentrated to dryness, which was repeated 3 times. The resulting residue was dissolved in 8ml methanol, and 0.8ml aqueous ammonia was added. The resulting mixture was concentrated, and purified to afford 28 mg of the final product with a yield of 41.2%.

$^1$H NMR (400 MHz, MeOD) δ 8.27 (d, *J* = 8.4 Hz, 1H), 7.43 (d, *J* = 1.0 Hz, 1H), 7.17 (d, *J* = 8.4 Hz, 1H), 6.98 - 6.90 (m, 2H), 4.82 - 4.60 (m, 4H), 4.47 - 4.45 (m, 1H), 3.79 - 3.70 (m, 2H), 3.60 - 3.57 (m, 1H), 3.46 - 3.43 (m, 1H), 2.56 (q, *J* = 7.5 Hz, 2H), 2.09 - 1.98 (m, 2H), 1.08 (t, *J* = 7.5 Hz, 3H).

**Example 39: (2-(6-(2-ethyl-5-fluoro-4-hydroxyphenyl)-1H-indazol-3-yl)-4,6-dihydropyrrolo[3,4-d]imidazol-5(1H)-yl)(4-hydroxylpiperidin-1-yl)ketone(MDI-245)**

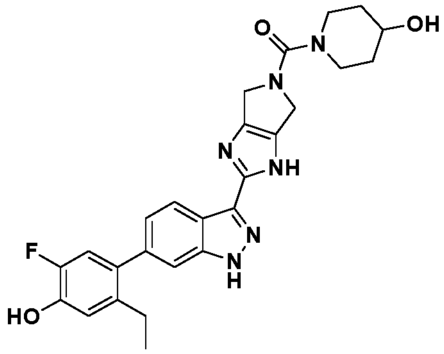

MDI-245

**Synthetic route of MDI-245:**

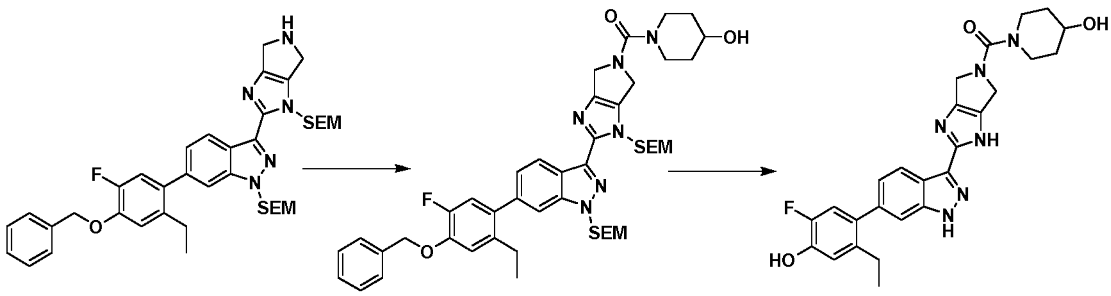

MDI-245-1

MDI-245

**Synthesis method:**

**Synthesis of intermediate MDI-245-1: (2-(6-(4-(benzyloxy)-2-ethyl-5-fluorophenyl)-1-((2-(trimethylsilyl)ethoxy)methyl)-1H-indazol-3-yl)-1-((2-(trimethylsilyl)ethoxy)methyl)-4,6-dihydropyrrolo[3,4-d]imidazol-5-(1H)-yl)(4-hydroxylpiperidin-1-yl)ketone**

Triphosgene (54.1 mg, 0.18 mmol) was dissolved in 10 ml of dichloromethane, to which the intermediate 6-(4-(benzyloxy)-2-ethyl-5-fluorophenyl)-1-((2-(trimethylsilyl)ethoxy)methyl)-3-(1-((2-(trimethylsilyl)ethoxy)methyl)-1-1,4,5,6-tetrahydropyrrolo[3,4-d]imidazol-2-yl)-1H-indazole (130 mg, 0.18 mmol) in dichloromethane (5 ml) was added

dropwise at 0°C, followed by addition of anhydrous triethylamine (185 mg, 1.8 mmol). The mixture was stirred at room temperature for 10 minutes. TLC monitored that the raw materials disappeared. Piperidin-4-ol (36.9 mg, 0.36 mmol) in dichloromethane (5ml) was added. The resulting mixture was stirred at room temperature for 20 minutes. Water was added to quench the reaction and the resulting mixture was extracted twice with ethyl acetate, and the organic phases were combined, washed with saturated brine, dried over anhydrous sodium sulfate, concentrated, and purified by silica gel column to afford 116 mg of intermediate MDI-245-1, with a yield of 75.7%.

$^1$H NMR (400 MHz, CDCl3) δ 8.46 (d, *J* = 8.3 Hz, 1H), 7.53 - 7.51 (m, 2H), 7.47 - 7.35(m, 4H), 7.25 (d, *J* = 8.4 Hz, 1H), 7.06-6.97(m, 2H), 5.96 (s, 2H), 5.75 (s, 2H), 5.37 (s, 2H), 4.79 - 4.66 (m, 4H), 3.95 - 3.92 (m, 1H), 3.75 - 3.72 (m, 2H), 3.66-3.52 (m, 4H), 3.12 - 3.07 (m, 2H), 2.54 (q, *J* = 7.6 Hz, 2H), 2.02 - 1.91 (m, 2H), 1.68 - 1.63 (m, 2H), 1.06 (t, *J* = 7.5 Hz, 3H), 0.99 - 0.89 (m, 4H), 0.02 (s, 9H),-0.05 (d, *J* = 3.4 Hz, 9H).

**Synthesis of compound MDI-245: (2-(6-(2-ethyl-5-fluoro-4-hydroxyphenyl)-1H-indazol-3-yl)-4,6-dihydropyrrolo[3,4-d]imidazol-5-(1H)-yl)(4-hydroxylpiperidin-1-yl)ketone**

MDI-243-1 (116 mg, 0.14 mmol) was dissolved in 20 ml methanol, and 20 mg palladium on carbon was added. The atmosphere was replaced hydrogen. It was allowed to react at 40 °C for 1 hour. After the reaction was completed, the mixture was filtered, and the filtrate was concentrated. The concentrate was dissolved in 12 ml methanol and 6ml concentrated hydrochloric acid was added. It was allowed to react at 50°C for 7 hours and the mixture was concentrated to give a residue. The residue was dissolved in methanol and was concentrated to dryness, which was repeated 3 times. The resulting residue was dissolved in 8ml methanol, and 0.8ml aqueous ammonia was added. The resulting mixture was concentrated, and purified to afford 30 mg of the final product with a yield of 44.4%.

$^1$H NMR (400 MHz, MeOD) δ 8.27 (d, *J* = 8.4 Hz, 1H), 7.43 (s, 1H), 7.18 (d, *J* = 8.4 Hz, 1H), 6.97 - 6.90 (m, 2H), 4.72 - 4.65 (m, 4H), 3.88 - 3.82 (m, 1H), 3.76 - 3.73 (m, 2H), 3.13 - 3.06 (m, 2H), 2.56 (q, *J* = 7.5 Hz, 2H), 1.97 - 1.95 (m, 2H), 1.63 - 1.55 (m, 2H), 1.08 (t, *J* = 7.5 Hz, 3H).

## Example 40: 2-(6-(2-ethyl-5-fluoro-4-hydroxyphenyl)-1H-indazol-3-yl)-N-methyl-4,6-dihydropyrrolo[3,4-d]imidazol-5-(1H)-carboxamide(MDI-246)

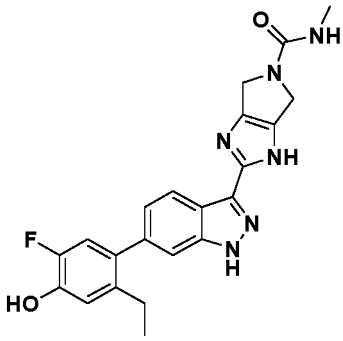

MDI-246

**MDI-246 的 Synthetic route:**

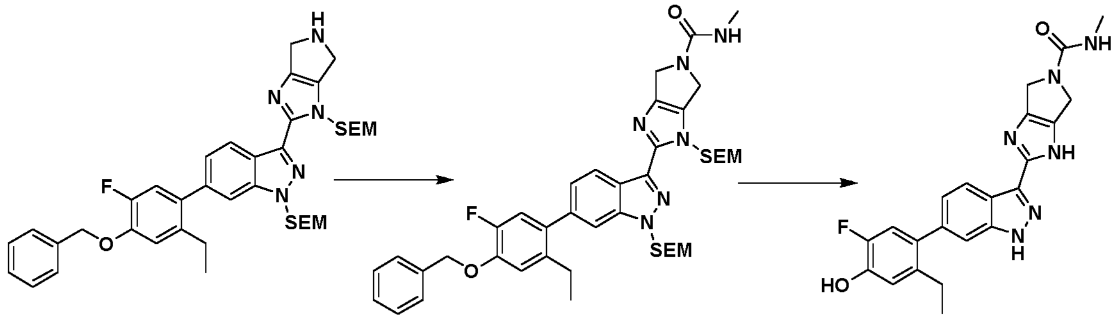

MDI-246-1 MDI-246

**Synthesis method:**

**Synthesis of intermediate MDI-246-1: 2-(6-(4-(benzyloxy)-2-ethyl-5-fluorophenyl)-1-((2-(trimethylsilyl)ethoxy)methyl)-1H-indazol-3-yl)-N-methyl-1-((2-(trimethylsilyl)ethoxy)methyl)-4,6-dihydropyrrolo[3,4-d]imidazol-5-(1H)-carboxamide**

Triphosgene (20.8 mg, 0.07 mmol) was dissolved in 5 ml of dry dichloromethane, to which the intermediate 6-(4-(benzyloxy)-2-ethyl-5-fluorophenyl)-1-((2-(trimethylsilyl)ethoxy)methyl)-3-(1-((2-(trimethylsilyl)ethoxy)methyl)-1-1,4,5,6-tetrahydropyrrolo[3,4-d]imidazol-2-yl)-1H-indazole (50 mg, 0.07 mmol) in dichloromethane (5 ml) was added dropwise at 0°C, then anhydrous triethylamine (70.9 mg, 0.70 mmol) was added slowly. The mixture was stirred at room temperature for 10 minutes. TLC monitored that the raw materials disappeared. Methylamine hydrochloride (9.5 mg, 0.14 mmol) was added. The resulting mixture was stirred at room temperature for 2 hours. Water was added to quench the reaction and the resulting mixture was extracted twice with dichloromethane, and the organic phases were combined, washed with saturated brine, dried over anhydrous sodium sulfate, concentrated, and purified by silica gel column to afford 42 mg of intermediate MDI-246-1, with a yield of 77.8%.

$^1$H NMR (400 MHz, CDCl3) δ 8.47 (d, *J* = 8.3 Hz, 1H), 7.53 - 7.51 (m, 2H), 7.47 - 7.37(m, 4H), 7.25 (d, *J* = 8.4 Hz, 1H), 7.07 - 6.96(m, 2H), 5.96 (s, 2H), 5.78 (s, 2H), 5.23 (s, 2H), 4.72 - 4.54 (m, 4H), 3.65 - 3.58 (m, 4H), 2.64 (s, 3H), 2.56 (q, *J* = 7.6 Hz, 2H), 1.08 (t, *J* = 7.5 Hz, 3H), 0.95 - 0.89 (m, 4H), 0.02 (s, 9H), -0.04 - -0.05 (m, 9H).

**Synthesis of compound MDI-246: 2-(6-(2-ethyl-5-fluoro-4-hydroxyphenyl) -1H-indazol-3-yl)-N-methyl-4,6-dihydropyrrolo[3,4-d]imidazol-5-(1H)-carboxamide**

MDI-246-1 (42 mg, 0.055 mmol) was dissolved in 10 ml methanol, and 8 mg palladium on carbon was added. The atmosphere was replaced hydrogen. It was allowed to react at 40 °C for 1 hour. After the reaction was completed, the mixture was filtered, and the filtrate was concentrated. The concentrate was dissolved in 6 ml methanol and 3ml concentrated hydrochloric acid was added. It was allowed to react at 50°C for 7 hours and the mixture was concentrated to give a residue. The residue was dissolved in methanol and was concentrated to dryness, which was repeated 3 times. The resulting residue was dissolved in 5ml methanol, and 0.5ml aqueous ammonia was added. The resulting mixture was concentrated, and purified to afford 10.3 mg of the final product with a yield of 45.1%.

$^1$H NMR (400 MHz, MeOD) δ 8.27 (d, *J* = 8.4 Hz, 1H), 7.43 (s, 1H), 7.18 (d, *J* = 8.4 Hz, 1H), 6.97 - 6.90 (m, 2H), 4.56 (s, 4H), 2.84 (s, 3H), 2.56 (q, *J* = 7.5 Hz, 2H), 1.08 (t, *J* = 7.5 Hz, 3H).

**Example 41: 2-(6-(2-ethyl-5-fluoro-4-hydroxyphenyl)-1H-indazol-3-yl)-N-ethyl-4,6-dihydropyrrolo[3,4-d]imidazol-5-(1H)-carboxamide(MDI-247)**

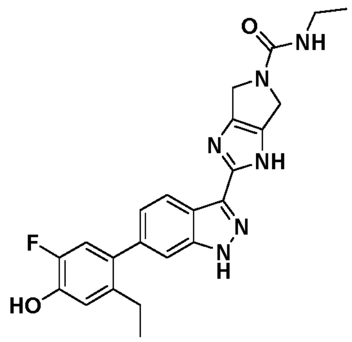

MDI-247

**Synthetic route of MDI-247:**

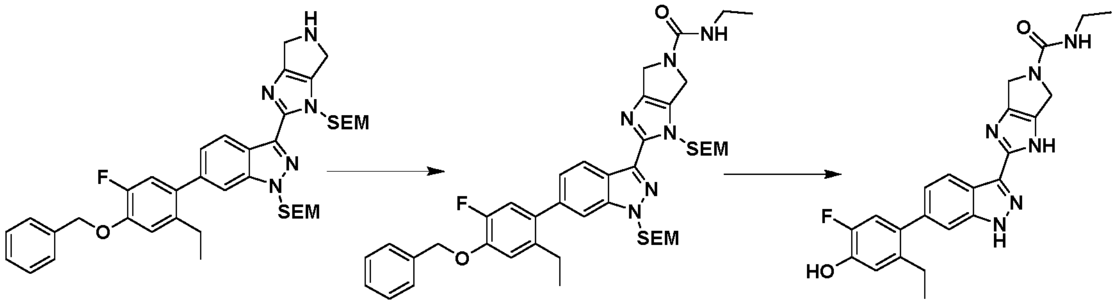

MDI-247-1 MDI-247

**Synthesis method:**

**Synthesis of intermediate MDI-247-1: 2-(6-(4-(benzyloxy)-2-ethyl-5-fluorophenyl)-1- ((2-(trimethylsilyl)ethoxy)methyl)-1H-indazol-3-yl)-N-ethyl-1-((2-(trimethylsilyl)ethoxy)methyl)-4,6-dihydropyrrolo[3,4-d]imidazol-5-(1H)-carboxamide**

Triphosgene (22.9 mg, 0.08 mmol) was dissolved in 6 ml of dry dichloromethane, to which the intermediate 6-(4-(benzyloxy)-2-ethyl-5-fluorophenyl)-1-((2-(trimethylsilyl)ethoxy)methyl)-3-(1-((2-(trimethylsilyl)ethoxy)methyl)-1-1,4,5,6-tetrahydropyrrolo[3,4-d]imidazol-2-yl)-1H-indazole (55 mg, 0.08 mmol) in dichloromethane (5 ml) was added dropwise at 0°C, then anhydrous triethylamine (78.0 mg, 0.8 mmol) was added slowly. The mixture was stirred at room temperature for 10 minutes. TLC monitored that the raw materials disappeared. Ethylamine hydrochloride (12.6 mg, 0.16 mmol) was added. The resulting mixture was stirred at room temperature for 20 hours. Water was added to quench the reaction and the resulting mixture was extracted twice with ethyl acetate, and the organic phases were combined, washed with saturated brine, dried over anhydrous sodium sulfate, concentrated, and purified by silica gel column to afford 47 mg of intermediate MDI-247-1, with a yield of 77.7%.

$^1$H NMR (400 MHz, CDCl3) δ 8.46 (d, *J* = 8.3 Hz, 1H), 7.53 - 7.51 (m, 2H), 7.47 - 7.35(m, 4H), 7.25 (d, *J* = 8.4 Hz, 1H), 7.07 - 6.97(m, 2H), 5.96 (s, 2H), 5.78 (s, 2H), 5.23 (s, 2H), 4.72 - 4.54 (m, 4H), 3.65 - 3.58 (m, 4H), 3.45 - 3.38 (m, 2H), 2.54 (q, *J* = 7.6 Hz, 2H), 1.18 - 1.14 (m, 3H), 1.05 (t, *J* = 7.5 Hz, 3H), 0.95 - 0.89 (m, 4H), 0.02 (s, 9H),-0.05 (d, *J* = 3.4 Hz, 9H).

**Synthesis of compound MDI-247: 2-(6-(2-ethyl-5-fluoro-4-hydroxyphenyl)-1H-indazol-3-yl)-N-ethyl-4,6-dihydropyrrolo[3,4-d]imidazol-5-(1H)-carboxamide**

MDI-247-1 (47 mg, 0.06 mmol) was dissolved in 10 ml methanol, and 8 mg palladium on carbon was added. The atmosphere was replaced hydrogen. It was allowed to react at 40 °C for 1 hour. After the reaction was completed, the mixture was filtered, and the filtrate was concentrated. The concentrate was dissolved in 6 ml methanol and 3ml concentrated hydrochloric acid was added. It was allowed to react at 50°C for 7 hours and the mixture was concentrated to give a residue. The residue was dissolved in methanol and was concentrated to dryness, which was repeated 3 times.

The resulting residue was dissolved in 5ml methanol, and 0.5ml aqueous ammonia was added. The resulting mixture was concentrated, and purified to afford 11 mg of the final product with a yield of 42.4%.

$^1$H NMR (400 MHz, MeOD) δ 8.27 (d, *J* = 8.4 Hz, 1H), 7.43 (s, 1H), 7.18 (d, *J* = 8.4 Hz, 1H), 6.98 - 6.90 (m, 2H), 4.57 (s, 4H), 3.38 - 3.28 (m, 2H), 2.56 (q, *J* = 7.5 Hz, 2H), 1.21 (t, *J* = 7.2 Hz, 3H), 1.08 (t, *J* = 7.5 Hz, 3H).

**Example 42: 2-(6-(2-ethyl-5-fluoro-4-hydroxyphenyl)-1H-indazol-3-yl)-N-(2-hydroxylethyl)-4,6-dihydropyrrolo[3,4-d]imidazol-5(1H)-carboxamide(MDI-248)**

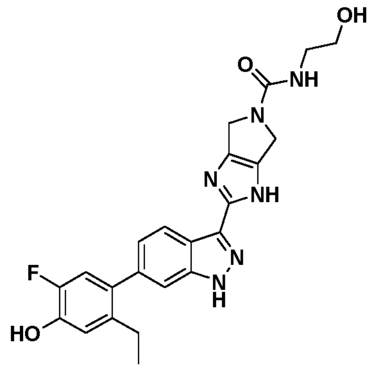

MDI-248

**Synthetic route of MDI-248:**

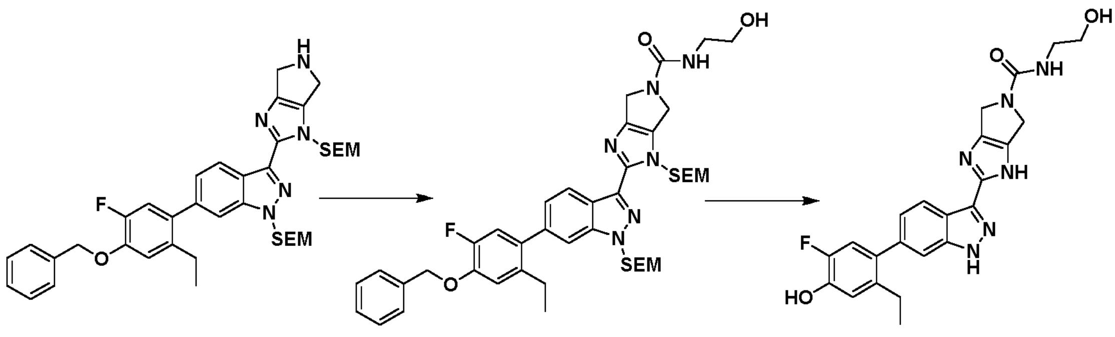

MDI-248-1

MDI-248

**Synthesis method:**

**Synthesis of intermediate MDI-248-1: 2-(6-(4-(benzyloxy)-2-ethyl-5-fluorophenyl)-1-((2-(trimethylsilyl)ethoxy)methyl)-1H-indazol-3-yl)-N-(2-hydroxylethyl)-1-((2-(trimethylsilyl)ethoxy)methyl)-4,6-dihydropyrrolo[3,4-d]imidazol-5-(1H)-carboxamide**

Triphosgene (22.9 mg, 0.08 mmol) was dissolved in 6 ml of dichloromethane, to which the intermediate 6-(4-(benzyloxy)-2-ethyl-5-fluorophenyl)-1-((2-(trimethylsilyl)ethoxy)methyl)-3-(1-((2-(trimethylsilyl)ethoxy)methyl)-1-1,4,5,6-tetrahydropyrrolo[3,4-d]imidazol-2-yl)-1H-indazole (55 mg, 0.08 mmol) in dichloromethane (5 ml) was added dropwise at 0°C, then anhydrous triethylamine (78 mg, 0.8 mmol) was added slowly. The mixture was stirred at room temperature for 10 minutes. TLC monitored that the raw materials disappeared. Ethanolamine (9.4 mg, 0.16 mmol) in dichloromethane (5ml) was added. The resulting mixture was stirred at room temperature for 1 hour. Water was added to quench the reaction and the resulting mixture was extracted twice with dichloromethane, and the organic phases were combined, washed with saturated brine, dried over anhydrous sodium sulfate, concentrated, and purified by silica gel column to afford 44 mg of intermediate MDI-248-1, with a yield of 71.3%.

$^1$H NMR (400 MHz, $CDCl_3$) δ 8.46 (d, *J* = 8.3 Hz, 1H), 7.73 - 7.51 (m, 2H), 7.48 - 7.35(m, 4H), 7.27 - 7.24 (m, 1H), 7.07 - 6.97(m, 2H), 5.96 (s, 2H), 5.78 (s, 2H), 5.23 (s, 2H), 4.73 - 4.57 (m, 4H), 3.65 - 3.53 (m, 6H), 3.33 - 3.29 (m,

2H), 2.54 (q, *J* = 7.6 Hz, 2H), 1.05 (t, *J* = 7.5 Hz, 3H), 0.95 - 0.89 (m, 4H), 0.02 (s, 9H),-0.05 (s, 9H).

**Synthesis of compound MDI-248: 2-(6-(2-ethyl-5-fluoro-4-hydroxyphenyl)-1H-indazol-3-yl)-N-(2-hydroxylethyl)-4,6-dihydropyrrolo[3,4-d]imidazol-5(1H)-carboxamide**

MDI-248-1 (44 mg, 0.06 mmol) was dissolved in 10 ml methanol, and 8 mg palladium on carbon was added. The atmosphere was replaced hydrogen. It was allowed to react at 40 °C for 1 hour. After the reaction was completed, the mixture was filtered, and the filtrate was concentrated. The concentrate was dissolved in 6 ml methanol and 3ml concentrated hydrochloric acid was added. It was allowed to react at 50°C for 7 hours and the mixture was concentrated to give a residue. The residue was dissolved in methanol and was concentrated to dryness, which was repeated 3 times. The resulting residue was dissolved in 5ml methanol, and 0.5ml aqueous ammonia was added. The resulting mixture was concentrated, and purified to afford 14 mg of the final product with a yield of 56.6%.

$^1H$ NMR (400 MHz, MeOD) δ 8.28 (d, *J* = 8.4 Hz, 1H), 7.43 (s, 1H), 7.18 (d, *J* = 8.4 Hz, 1H), 6.97 - 6.90 (m, 2H), 4.59 (s, 4H), 3.68 (t, *J* = 5.8 Hz, 2H), 3.40 (t, *J* = 5.8 Hz, 2H), 2.56 (q, *J* = 7.5 Hz, 2H), 1.08 (t, *J* = 7.5 Hz, 3H)

**Example 43: 1-(2-(6-(2-ethyl-5-fluoro-4-hydroxyphenyl)-1H-indazol-3-yl)-1,4,5,6-tetrahydropyrrolo[3,4-d]imidazol-5 -carbonyl)pyrrolidin-3-nitrile(MDI-250)**

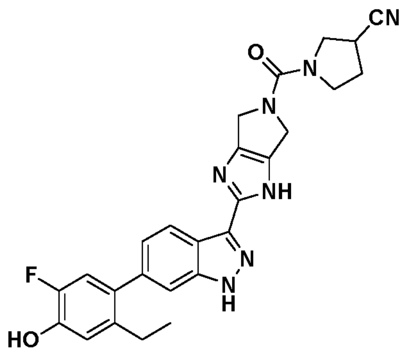

MDI-250

**Synthetic route of MDI-250:**

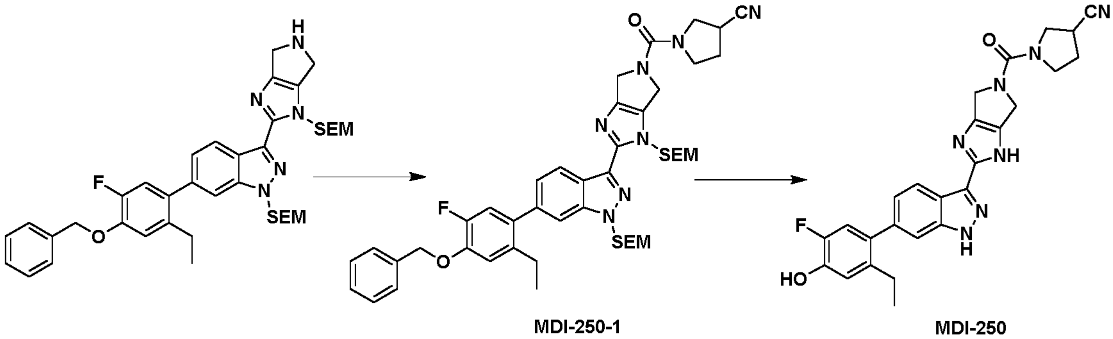

MDI-250-1

MDI-250

**Synthesis method:**

**Synthesis of intermediate MDI-250-1: 1-(2-(6-(4-(benzyloxy)-2-ethyl-5-fluorophenyl)-1-((2-(trimethylsilyl)ethoxy)methyl)-1H-indazol-3-yl)-1-((2-(trimethylsilyl)ethoxy)methyl)-1,4,5,6-tetrahydropyrrolo[3,4-d]imidazole-5-carbonyl)pyrrolidine-3-nitrile**

Triphosgene (14.9 mg, 0.05 mmol) was dissolved in 6 ml of dichloromethane, to which the intermediate 6-(4-(benzyloxy)-2-ethyl-5-fluorophenyl)-1-((2-(trimethylsilyl)ethoxy)methyl)-3-(1-((2-(trimethylsilyl)ethoxy)methyl)-1-1,4,5,6-tetrahydropyrrolo[3,4-d]imidazol-2-yl)-1H-indazole (36 mg, 0.05 mmol) in dichloromethane (5 ml) was added dropwise at 0°C, then anhydrous triethylamine (51.1 mg, 0.50 mmol) was added slowly. The mixture was stirred at room temperature for 10 minutes. TLC monitored that the raw materials disappeared. Pyrrolidine-3-nitrile hydrochloride (9.7 mg, 0.10 mmol) was added. The resulting mixture was stirred at room temperature for 20 minutes. Water was added to quench the reaction and the resulting mixture was extracted twice with ethyl acetate, and the organic phases were combined, washed with saturated brine, dried over anhydrous sodium sulfate, concentrated, and purified by silica gel column to afford 26 mg of intermediate MDI-250-1, with a yield of 61.6%.

$^1$H NMR (400 MHz, CDCl3) δ 8.46 (d, *J* = 8.3 Hz, 1H), 7.53 - 7.51 (m, 2H), 7.47 - 7.35(m, 4H), 7.25 (d, *J* = 8.4 Hz, 1H), 7.06 - 6.97(m, 2H), 5.96 (s, 2H), 5.77 (s, 2H), 5.23 (s, 2H), 4.80 - 4.67 (m, 4H), 3.79 - 3.46 (m, 8H), 3.12 - 3.05 (m, 1H), 2.54 (q, *J* = 7.6 Hz, 2H), 2.38 - 2.16 (m, 2H), 1.05 (t, *J* = 7.5 Hz, 3H), 0.96 - 0.89 (m, 4H), 0.02 (s, 9H),-0.05 (d, *J* = 3.4 Hz, 9H).

**Synthesis of compound MDI-250: 1-(2-(6-(2-ethyl-5-fluoro-4-hydroxyphenyl)-1H-indazol-3-yl)-1,4,5,6-tetrahydropyrrolo[3,4-d]imidazol-5-carbonyl) pyrrolidin-3-nitrile**

MDI-250-1 (26 mg, 0.03 mmol) was dissolved in 10 ml methanol, and 6 mg palladium on carbon was added. The atmosphere was replaced hydrogen. It was allowed to react at 40 °C for 1 hour. After the reaction was completed, the mixture was filtered, and the filtrate was concentrated. The concentrate was dissolved in 4 ml methanol and 3ml concentrated hydrochloric acid was added. It was allowed to react at 50°C for 7 hours and the mixture was concentrated to give a residue. The residue was dissolved in methanol and was concentrated to dryness, which was repeated 3 times. The resulting residue was dissolved in 5ml methanol, and 0.5ml aqueous ammonia was added. The resulting mixture was concentrated, and purified to afford 6 mg of the final product with a yield of 39.8%.

$^1$H NMR (400 MHz, MeOD) δ 8.27 (d, *J*= 8.4 Hz, 1H), 7.43 (s, 1H), 7.18 (d, *J*= 8.4 Hz, 1H), 6.98 - 6.90 (m, 2H), 4.70 (s, 4H), 3.89 - 3.85 (m, 1H), 3.78 - 3.76 (m, 1H), 3.70 - 3.59 (m, 2H), 3.23 - 3.18 (m, 1H), 2.56 (q, *J*= 7.5 Hz, 2H), 2.42 - 2.19 (m, 2H), 1.08 (t, *J* = 7.5 Hz, 3H).

**Example 44: 2-(6-(2-ethyl-5-fluoro-4-hydroxyphenyl)-1H-indazol-3-yl)-N-(tetrahydrofuran-3-yl)-4,6-dihydropyrrolo[3,4-d]imidazol-5(1H)-carboxamide(MDI-251)**

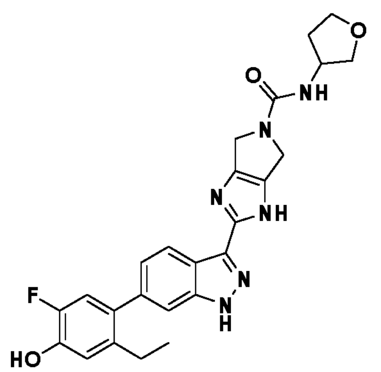

MDI-251

**Synthetic route of MDI-251:**

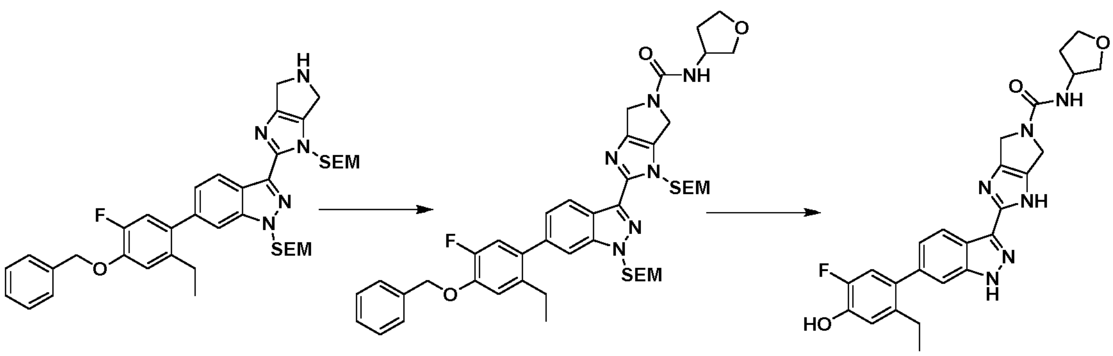

MDI-251-1 MDI-251

**Synthesis method:**

**Synthesis of intermediateMDI-251-1: 2-(6-(4-(benzyloxy)-2-ethyl-5-fluorophenyl)-1-((2-(trimethylsilyl)ethoxy)methyl)-1H-indazol-3-yl)-N-(tetrahydrofuran-3-yl)-1-(((2-(trimethylsilyl)ethoxy)methyl)-4,6-dihydropyrrolo[3,4-d]imidazole-5(1H)-carboxamide**

Triphosgene (19.1 mg, 0.06 mmol) was dissolved in 6 ml of dry dichloromethane, to which the intermediate 6-(4-(benzyloxy)-2-ethyl-5-fluorophenyl)-1-((2-(trimethylsilyl)ethoxy)methyl)-3-(1-((2-(trimethylsilyl)ethoxy)methyl)-1-1,4,5,6-tetrahydropyrrolo[3,4-d]imidazol-2-yl)-1H-indazole (46 mg, 0.06 mmol) in dichloromethane (5 ml) was added dropwise at 0°C, then anhydrous triethylamine (65.3 mg, 0.6 mmol) was added slowly. The mixture was stirred at room temperature for 10 minutes. TLC monitored that the raw materials disappeared. Tetrahydrofuran-3-amine hydrochloride (16.4 mg, 0.13 mmol) was added. It was allowed to react at 38 °C for 5 hours. Water was added to quench the reaction and the resulting mixture was extracted twice with dichloromethane, and the organic phases were combined, washed with saturated brine, dried over anhydrous sodium sulfate, concentrated, and purified by silica gel column to afford 32 mg of intermediate MDI-251-1, with a yield of 60.3%.

$^1H$ NMR (400 MHz, $CDCl_3$) δ 8.46 (d, *J* = 8.3 Hz, 1H), 7.73 - 7.51 (m, 2H), 7.47 - 7.35(m, 4H), 7.27 - 7.24 (m, 1H), 7.07 - 6.97(m, 2H), 5.96 (s, 2H), 5.77 (s, 2H), 5.23 (s, 2H), 4.70 - 4.53 (m, 4H), 4.51 - 4.41 (m, 1H), 4.06 - 3.58 (m, 8H), 2.54 (q, *J* = 7.6 Hz, 2H), 2.40 - 1.99 (m, 2H), 1.05 (t, *J* = 7.5 Hz, 3H), 0.95 - 0.89 (m, 4H), 0.02 (s, 9H),-0.05 (s, 9H).

**Synthesis of compound MDI-251: 2-(6-(2-ethyl-5-fluoro-4-hydroxyphenyl)-1H-indazol-3-yl)-N-(tetrahydrofuran-3-yl)-4,6-dihydropyrrolo[3,4-d]imidazol-5(1H)-carboxamide**

MDI-251-1 (32 mg, 0.04 mmol) was dissolved in 10 ml methanol, and 6 mg palladium on carbon was added. The atmosphere was replaced hydrogen. It was allowed to react at 40 °C for 1 hour. After the reaction was completed, the mixture was filtered, and the filtrate was concentrated. The concentrate was dissolved in 6 ml methanol and 3ml concentrated hydrochloric acid was added. It was allowed to react at 50°C for 7 hours and the mixture was concentrated to give a residue. The residue was dissolved in methanol and was concentrated to dryness, which was repeated 3 times. The resulting residue was dissolved in 5ml methanol, and 0.5ml aqueous ammonia was added. The resulting mixture was concentrated, and purified to afford 3 mg of the final product with a yield of 16.3%.

$^1H$ NMR (400 MHz, MeOD) δ 8.28 (d, *J* = 8.4 Hz, 1H), 7.43 (s, 1H), 7.18 (d, *J* = 8.4 Hz, 1H), 6.97 - 6.90 (m, 2H), 4.63 (s, 4H), 4.45 - 4.40 (m, 1H), 4.03 - 3.94 (m, 2H), 3.87 - 3.81 (m, 1H), 3.71 - 3.68 (m, 1H), 2.56 (q, *J* = 7.5 Hz, 2H), 2.32 - 1.87 (m, 2H), 1.08 (t, *J* = 7.5 Hz, 3H).

**Example 45: Methyl 2-(6-(2-ethyl-5-fluoro-4-hydroxyphenyl)-1H-indazol-3-yl)-4,6-dihydropyrrolo[3,4-d]imidazol-5(1H)-carboxylate (MDI-252)**

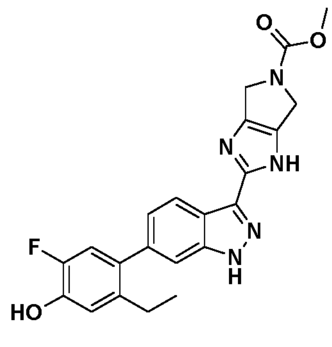

MDI-252

**Synthetic route of MDI-252:**

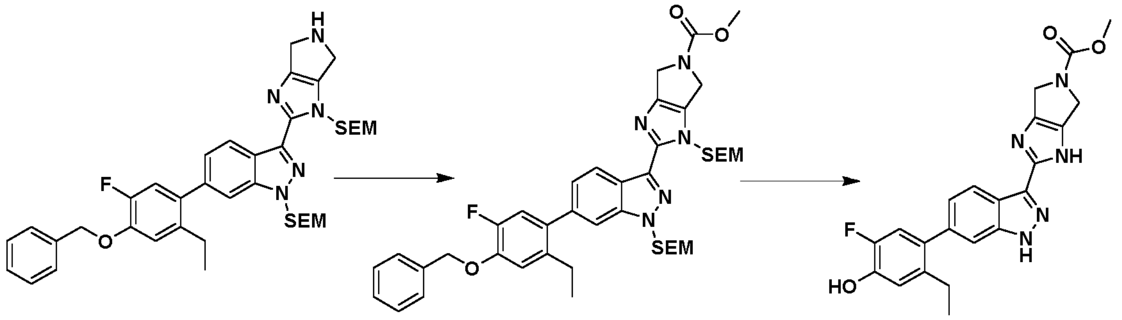

MDI-252-1

MDI-252

**Synthesis method:**

**Synthesis of intermediate MDI-252-1: Methyl 2-(6-(4-(benzyloxy)-2-ethyl-5-fluorophenyl)-1-((2-(trimethylsilyl)ethoxy)methyl)-1H-indazol-3-yl)-1-((2-((trimethylsilyl)ethoxy)methyl)-4,6-dihydropyrrolo[3,4-d]imidazol-5(1H)-carboxylate**

Triphosgene (16.6 mg, 0.06 mmol) was dissolved in 5 ml of dry dichloromethane, to which the intermediate 6-(4-(benzyloxy)-2-ethyl-5-fluorophenyl)-1-((2-(trimethylsilyl)ethoxy)methyl)-3-(1-((2-(trimethylsilyl)ethoxy)methyl)-1-1,4,5,6-tetrahydropyrrolo[3,4-d]imidazol-2-yl)-1H-indazole (40 mg, 0.06 mmol) in dichloromethane (5 ml) was added dropwise at 0°C, then anhydrous triethylamine (56.8 mg, 0.56 mmol) was added slowly. The mixture was stirred at room temperature for 10 minutes. TLC monitored that the raw materials disappeared. The reaction mixture was concentrated and was dissolved in 10 ml of methanol. DMAP (6.9 mg, 0.06 mmol) was added. It was allowed to react at 70 °C for 4 hours. The reaction mixture was concentrated to which water was added. The resulting mixture was extracted twice with dichloromethane, and the organic phases were combined, washed with saturated brine, dried over anhydrous sodium sulfate, concentrated, and purified by silica gel column to afford 30 mg of intermediate MDI-252-1, with a yield of 69.4%.

$^1$H NMR (400 MHz, CDCl3) δ 8.44 - 8.39 (m, 1H), 7.53 - 7.51 (m, 2H), 7.48 - 7.36(m, 4H), 7.23 (d, *J* = 8.4 Hz, 1H), 7.05 - 6.96(m, 2H), 5.95 (s, 2H), 5.78 (s, 2H), 5.23 (s, 2H), 4.75 - 4.59 (m, 4H), 3.85 (s, 3H), 3.66 - 3.57 (m, 4H), 2.53 (q, *J* = 7.6 Hz, 2H), 1.04 (t, *J* = 7.5 Hz, 3H), 0.95 - 0.88 (m, 4H), 0.02 (s, 9H), -0.04 - -0.05 (m, 9H).

**Synthesis of compound MDI-252: Methyl 2-(6-(2-ethyl-5-fluoro-4-hydroxyphenyl)-1H-indazol-3-yl)-4,6-dihydropyrrolo[3,4-d]imidazol-5(1H)-carboxylate**

MDI-252-1 (30 mg, 0.04 mmol) was dissolved in 10 ml methanol, and 6 mg palladium on carbon was added. The atmosphere was replaced hydrogen. It was allowed to react at 40 °C for 1 hour. After the reaction was completed, the mixture was filtered, and the filtrate was concentrated. The concentrate was dissolved in 6 ml methanol and 3ml concentrated hydrochloric acid was added. It was allowed to react at 50°C for 7 hours and the mixture was concentrated to give a residue. The residue was dissolved in methanol and was concentrated to dryness, which was repeated 3 times.

The resulting residue was dissolved in 5ml methanol, and 0.5ml aqueous ammonia was added. The resulting mixture was concentrated, and purified to afford 8 mg of the final product with a yield of 48.9%.

$^1$H NMR (400 MHz, MeOD) δ 8.28 (d, *J* = 8.4 Hz, 1H), 7.43 (s, 1H), 7.18 (d, *J* = 8.4 Hz, 1H), 6.97 - 6.90 (m, 2H), 4.61 (s, 4H), 3.83 (s, 3H), 2.56 (q, *J* = 7.5 Hz, 2H), 1.08 (t, *J* = 7.5 Hz, 3H).

**Example 46: Ethyl 2-(6-(2-ethyl-5-fluoro-4-hydroxyphenyl)-1H-indazol-3-yl)-4,6-dihydropyrrolo[3,4-d]imidazol-5(1H)-carboxylate (MDI-253)**

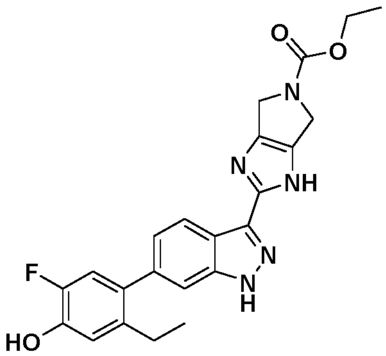

MDI-253

**Synthetic route of MDI-253:**

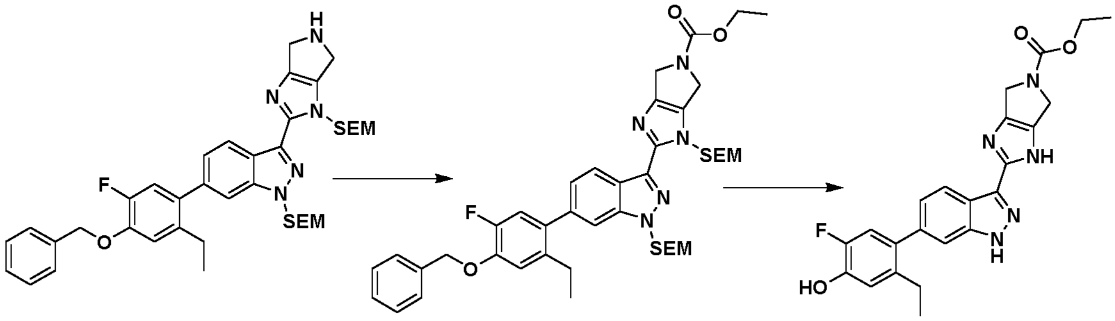

MDI-253-1

MDI-253

**Synthesis method:**

**Synthesis of intermediate MDI-253-1: Ethyl 2-(6-(4-(benzyloxy)-2-ethyl-5-fluorophenyl)-1-((2-(trimethylsilyl)ethoxy)methyl)-1H-indazol-3-yl)-1-((2 - (trimethylsilyl)ethoxy)methyl)-4,6-dihydropyrrolo[3,4-d]imidazole-5(1H)-carboxylate**

Triphosgene (20.1 mg, 0.07 mmol) was dissolved in 5 ml of dry dichloromethane, to which the intermediate 6-(4-(benzyloxy)-2-ethyl-5-fluorophenyl)-1-((2-(trimethylsilyl)ethoxy)methyl)-3-(1-((2-(trimethylsilyl)ethoxy)methyl)-1-1,4,5,6-tetrahydropyrrolo[3,4-d]imidazol-2-yl)-1H-indazole (48 mg, 0.07 mmol) in dichloromethane (5 ml) was added dropwise at 0°C, then anhydrous triethylamine (68.1 mg, 0.67 mmol) was added slowly. The mixture was stirred at room temperature for 10 minutes. TLC monitored that the raw materials disappeared. The reaction mixture was concentrated and was dissolved in 10 ml ethanol. DMAP (8.2 mg, 0.07 mmol) was added. It was allowed to react at 80 °C for 4 hours. The reaction mixture was concentrated to which water was added. The resulting mixture was extracted twice with dichloromethane, and the organic phases were combined, washed with saturated brine, dried over anhydrous sodium sulfate, concentrated, and purified by silica gel column to afford 33 mg of intermediate MDI-253-1, with a yield of 62.5%.

$^1$H NMR (400 MHz, CDCl3) δ 8.50 - 8.45 (m, 1H), 7.53 - 7.51 (m, 2H), 7.47 - 7.37(m, 4H), 7.25 (d, *J* = 8.4 Hz, 1H), 7.07 - 6.96(m, 2H), 5.96 (s, 2H), 5.77 (s, 2H), 5.23 (s, 2H), 4.72 - 4.59 (m, 4H), 4.29 - 4.25 (m, 2H), 3.65 - 3.58 (m,

4H), 2.54 (q, *J* = 7.6 Hz, 2H), 1.38 - 1.34 (m, 3H), 1.05 (t, *J* = 7.5 Hz, 3H), 0.95 - 0.89 (m, 4H), 0.02 (s, 9H),-0.05 (s, 9H).

**Synthesis of compound MDI-253: Ethyl 2-(6-(2-ethyl-5-fluoro-4-hydroxyphenyl)-1H-indazol-3-yl)-4,6-dihydropyrrolo[3,4-d]imidazole-5(1H)-carboxylate**

MDI-253-1 (33 mg, 0.04 mmol) was dissolved in 10 ml ethanol, and 6 mg palladium on carbon was added. The atmosphere was replaced hydrogen. It was allowed to react at 40 °C for 1 hour. After the reaction was completed, the mixture was filtered, and the filtrate was concentrated. The concentrate was dissolved in 6 ml ethanol and 3ml concentrated hydrochloric acid was added. It was allowed to react at 50°C for 7 hours and the mixture was concentrated to give a residue. The residue was dissolved in ethanol and was concentrated to dryness, which was repeated 3 times. The resulting residue was dissolved in 5ml ethanol, and 0.5ml aqueous ammonia was added. The resulting mixture was concentrated, and purified to afford 10 mg of the final product with a yield of 54.8%.

$^1H$ NMR (400 MHz, MeOD) δ 8.27 (d, *J* = 8.4 Hz, 1H), 7.43 (s, 1H), 7.18 (d, *J* = 8.4 Hz, 1H), 6.97 - 6.90 (m, 2H), 4.60 (s, 4H), 4.26 (q, *J* = 7.1 Hz, 2H), 2.57 (q, *J* = 7.5 Hz, 2H), 1.36 (t, *J* = 7.1 Hz, 3H), 1.08 (t, *J* = 7.5 Hz, 3H).

**Example 47: (S)-(2-(6-(2-ethyl-5-fluoro-4-hydroxyphenyl)-1H-pyrazolo [3,4-b]pyridine-3-yl)-4,6-dihydropyrrolo[3,4-d]imidazol-5(1H)-vl)(3-hydroxylpyrrolidin-1-yl)ketone (MDI-255)**

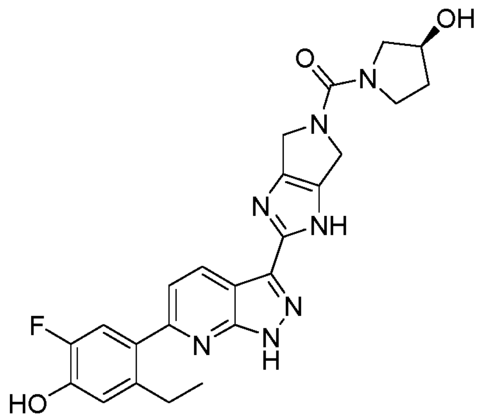

**MDI-255**

**Synthetic route of MDI-255:**

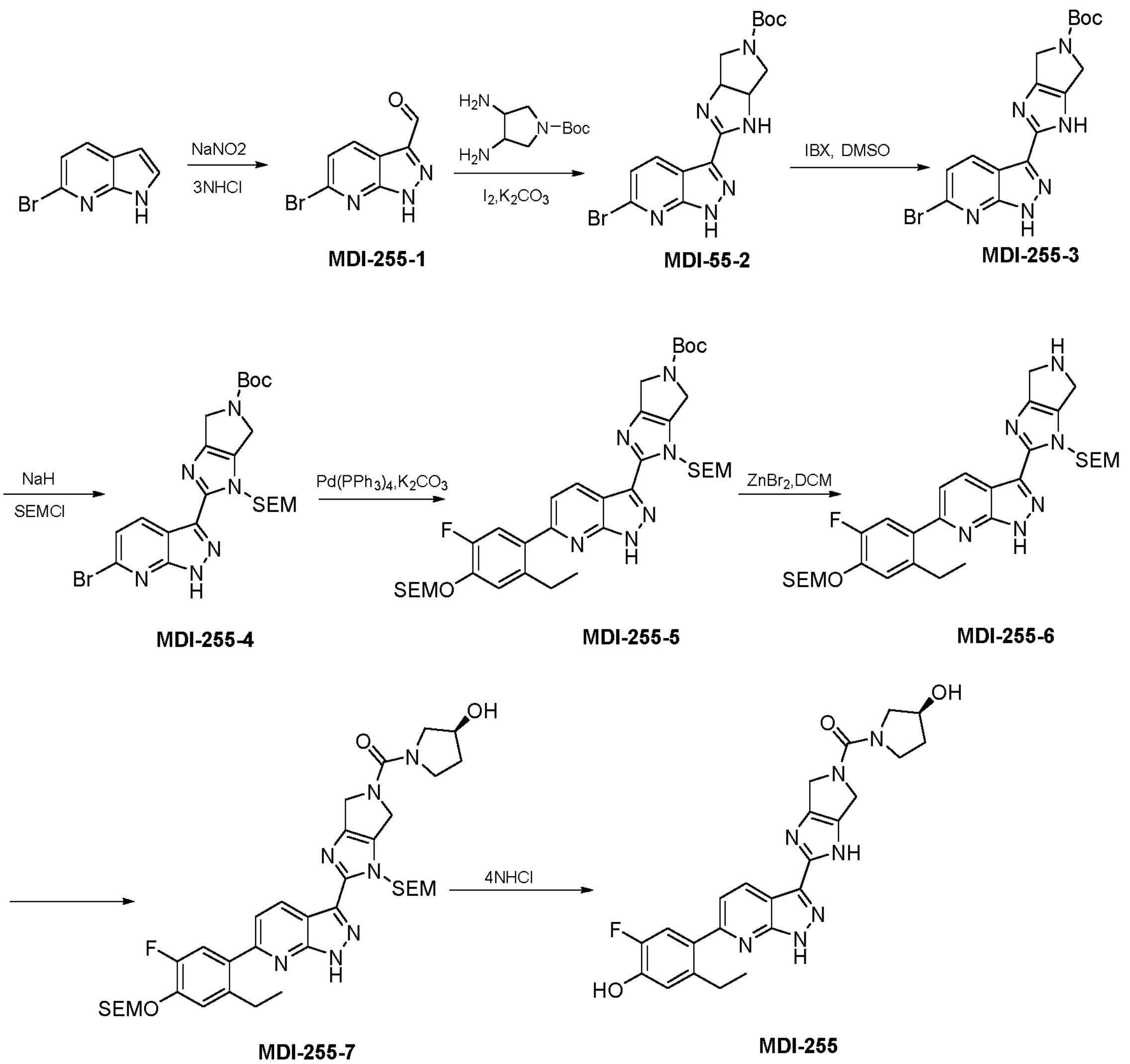


MDI-255-1 MDI-55-2 MDI-255-3

MDI-255-4 MDI-255-5 MDI-255-6

MDI-255-7 MDI-255

**Synthesis method:**

**Synthesis of intermediate MDI-551-1: 6-bromo-1H-pyrazolo[3,4-b]pyridine-3-formaldehyde**

Sodium nitrite (2.80 g, 40.6 mmol) was dissolved in 15 ml DMF and 20 ml water, and cooled to 0°C. 3N HCl (11.9 ml, 35.6 mmol) was slowly added dropwise, and after the addition, the reaction was carried out for 10 minutes. At 0°C, 6-bromo-1H-pyrrolo[2,3-b]pyridine (1.00 g, 5.08 mmol) in DMF (15 ml) was slowly added to the reaction solution dropwise. After the addition, the mixture was heated to 50°C. It was allowed to react for 5 hours. The resulting mixture was extracted with ethyl acetate 3 times, and the organic phases were combined, washed 3 times with water, washed with saturated brine, dried over anhydrous sodium sulfate, concentrated, and purified by silica gel column to afford 540 mg of intermediate MDI-255-1 with a yield of 47.0%.

$^1$H NMR (400 MHz, CDCl3) δ 10.36 (s, 1H), 8.40 (d, J = 8.0 Hz, 1H), 7.70 (d, J = 8.0 Hz, 1H).

**Synthesis of intermediate MDI-255-2: Tert-butyl 2-(6-bromo-1H-pyrazolo[3,4-b]pyridine-3-yl)-3a,4,6,6a-tetrahydropyrrolo[3,4-d]imidazol-5(1H)-carboxylate**

Intermediate MDI-255-1 (540 mg, 2.39 mmol) and tert-butyl 3,4-diaminopyrroline-1-carboxylate (529 mg, 2.63 mmol) were dissolved in 30 ml tert-butanol and stirred at room temperature for 30 minutes, followed by addition of $I_2$ (759 mg, 2.99 mmol) and $K_2CO_3$ (989.1 mg, 7.17 mmol). The mixture was heated to 70°C for 3 hours, and cooled to room temperature. Saturated sodium thiosulfate was added and the mixture was stirred for 20 minutes until the color of iodine disappeared. The resulting mixture was extracted twice with ethyl acetate, and the organic phases were combined, washed with water and saturated brine, dried over anhydrous sodium sulfate, concentrated, and purified by silica gel column to afford 396 mg of intermediate MDI-255-2 with a yield of 40.7%.

$^1$H NMR (400 MHz, CDCl3) δ 8.46 (d, J = 8.0 Hz, 1H), 7.62 (d, J = 8.0 Hz, 1H), 4.99-4.94 (m, 1H), 4.54-4.50 (m, 1H), 3.76-3.68 (m, 3H), 3.60-3.58 (m, 1H), 1.45 (s, 9H).

**Synthesis of intermediate MDI-255-3: Tert-butyl 2-(6-bromo-1H-pyrazolo[3,4-b]pyridine-3-yl)-4,6-dihydropyrrolo[3,4-d]imidazol-5(1H)-carboxylate**

MDI-255-2 (396 mg, 0.97 mmol) was dissolved in 6 ml DMSO, and IBX (543 mg, 1.94 mmol) was added. It was allowed to react at 50°C for 6 hours. The reaction was quenched by adding water. The resulting mixture was extracted twice with ethyl acetate and the organic phases were combined, washed with water and saturated brine, dried over anhydrous sodium sulfate, concentrated, and purified on a silica gel column to afford 227 mg of intermediate MDI-255-3 with a yield of 57.6%.

$^1$H NMR (400 MHz, $CDCl_3$) δ 10.59 (s, 1H), 8.64 (dd, J = 8.0 Hz, J = 12.0 Hz, 1H), 7.64 (d, J = 8.0 Hz, 1H), 4.67-4.53 (m, 4H), 1.56 (s, 9H).

**Synthesis of intermediate MDI-255-4: Tert-butyl 2-(6-bromo-1H-pyrazolo[3,4-b]pyridine-3-yl)-1-((2-(trimethylsilyl)ethoxy)methyl)-4,6-dihydropyrrolo [3,4-d]imidazol-5(1H)-carboxylate**

Intermediate MDI-255-3 (227 mg, 0.56 mmol) was dissolved in 15 ml of dry tetrahydrofuran, and cooled to 0°C, to which sodium hydride (60%) (67.2 mg, 1.68 mmol) was slowly added. The mixture was stirred for 10 minutes. 2-(Trimethylsilyl)ethoxymethyl chloride (280.3 mg, 1.68 mmol) was added slowly dropwise, and after the addition, the reaction was carried out at room temperature for 1 hour. Water was added to quench the reaction. The resulting mixture was extracted twice with ethyl acetate, and the organic phases were combined, washed with water and saturated brine, dried over anhydrous sodium sulfate, concentrated, and purified by silica gel column to afford 126 mg of intermediate MDI-255-4 with a yield of 42.0%.

$^1$H NMR (400 MHz, CDCl3) δ 8.72 (dd, J = 8.0 Hz, J = 12.0 Hz, 1H), 7.63 (dd, J = 8.0 Hz, J = 4.0 Hz, 1H), 5.90 (d, J = 8.0 Hz, 2H), 4.67-4.52 (m, 4H), 3.69-3.64 (m, 2H), 1.56 (s, 9H), 1.01-0.97 (m, 2H), 0.02 (s, 9H).

**Synthesis of intermediate MDI-255-5: Tert-butyl 2-(6-(2-ethyl-5-fluoro-4-((2-(trimethylsilyl)ethoxy)methoxy)phenyl)-1H-pyrazolo [3,4-b] pyridine-3-yl)-1-((2-(trimethylsilyl)ethoxy)methyl)-4,6-dihydropyrrolo[3,4-d]imidazol-5(1H)-carboxylate**

Intermediate MDI-255-4 (126 mg, 0.24 mmol), (2-((5-ethyl-2-fluoro-4-(4,4,5,5-tetramethyl-1,3,2-dioxaborolan-2-yl)phenoxy)methoxy)ethyl)trimethylsilane (143 mg, 0.36 mmol), Pd(PPh3)4 (27.2 mg, 0.02 mmol) and potassium carbonate ( 99.4 mg, 0.72 mmol) were dissolved in 1,4-dioxane (20 ml) and water (4 ml). The atmosphere was replaced with nitrogen, which was repeated 3 times. The mixture was heated to 100 °C, reacted for 3 hours, cooled to room temperature. Water was added, and the resulting mixture was extracted twice with ethyl acetate. The organic phases were combined, washed with water and saturated brine, dried over anhydrous sodium sulfate, concentrated, and purified on a silica gel column to afford 83 mg of intermediate MDI-255-5 with a yield of 47.7%.

$^1$H NMR (400 MHz, CDCl3) δ 8.88 (dd, J = 8.0 Hz, J = 12.0 Hz, 1H), 7.52 (d, J = 8.0 Hz, 1H), 7.25-7.21 (m, 2H), 5.95 (d, J = 8.0 Hz, 2H), 5.36 (s, 2H), 4.69-4.54 (m, 4H), 3.89-3.84 (m, 2H), 3.72-3.65 (m, 2H), 2.82-2.76 (m, 2H) ), 1.59 (s, 9H), 1.18-1.12 (m, 3H), 1.04-0.99 (m, 4H), 0.05 (s, 9H), 0.02 (s, 9H).

**Synthesis of intermediate MDI-255-6: 6-(2-ethyl-5-fluoro-4-((2-(trimethylsilanyl)ethoxy)methoxy)phenyl)-3-(1-((2-(trimethylsilanyl)ethoxy)methyl)-1,4,5,6-tetrahydropyrrolo[3,4-d]imidazol-2-yl)-1H-pyrazolo[3,4-b]pyridine**

Intermediate MDI-255-5 (83 mg, 0.11 mmol) was dissolved in 15 ml of dichloromethane, and zinc bromide (103 mg, 0.46 mmol) was added. The mixture was stirred at 25°C for 4 hours, and 10 ml of aqueous ammonia was added to the reaction solution. After liquid separation, the organic phase was washed with saturated sodium bicarbonate and saturated sodium chloride, dried over anhydrous sodium sulfate, and concentrated to afford 65 mg of intermediate MDI-255-6, with a yield of 95.6%. The crude product was directly used in the next step.

**Synthesis of intermediate MDI-255-7: (S)-(2-(6-(2-ethyl-5-fluoro-4-((2-(trimethylsilyl)ethoxy)methoxy)phenyl)-1H-pyrazolo [3,4-b] pyridine-3-yl)-1-(((2-(trimethylsilyl)ethoxy)methyl)-4,6-dihydropyrrolo[3,4-d]imidazol-5(1H)-yl)(3-hydroxyl pyrrolidin-1-yl)ketone**

Intermediate MDI-241-6 (20 mg, 0.03 mmol) was dissolved in 5 ml of dry dichloromethane, and cooled to 0°C, to which triphosgene (9.5 mg, 0.03 mmol) was added, and triethylamine (32.3 mg, 0.32 mmol) was added dropwise.

After the addition, the mixture was stirred at room temperature for 10 minutes, to which (S)-3-hydroxypyrrolidine hydrochloride (7.7 mg, 0.06 mmol) was added. The mixture was stirred at room temperature for 1 hour. Water was added and the resulting mixture was extracted twice with dichloromethane. The organic phases were combined, washed with water and saturated brine, dried over anhydrous sodium sulfate, and concentrated to afford 19 mg of intermediate MDI-255-7. The crude product was directly used in the next step.

**Synthesis of compound MDI-255: (S)-(2-(6-(2-ethyl-5-fluoro-4-hydroxyphenyl)-1H-pyrazolo[3,4-b]pyridine-3-yl)-4,6-dihydropyrrolo[3,4-d]imidazol-5(1H)-yl)(3-hydroxylpyrrolidin-1-yl)ketone**

Intermediate MDI-255-1 (19 mg, 0.03 mmol) was dissolved in 4 ml of methanol, to which 2 ml of concentrated hydrochloric acid was added. The mixture was heated to 50 °C, reacted for 6 hours, and concentrated to give a residue. The residue was dissolved in methanol and was concentrated to dryness, which was repeated 3 times. The resulting residue was dissolved in methanol to which 1 ml of aqueous ammonia was added to neutralize. The resulting mixture was concentrated and purified by a preparation plate to afford 4.9 mg of the final product 4.9 mg with a total yield of the two steps of 32.0%.

$^1$H NMR (400 MHz, DMSO) δ 13.61 (s, 1H), 10.22 (s, 1H), 8.78 (d, J = 8.0 Hz, 1H), 7.71 (d, J = 8.0 Hz, 1H), 7.34 (d, J = 12.0 Hz, 1H), 6.97 (d, J = 8.0 Hz, 1H), 4.93 (d, J = 4.0 Hz, 1H), 4.75-4.42 (m, 4H), 4.30-4.27 (m, 1H) , 3.58-3.53 (m, 2H), 3.41-3.40 (m, 1H), 3.26-3.23 (m, 1H), 2.73-2.71 (m, 2H), 2.01-1.79 (m, 2H), 1.09 (t, J = 8.0 Hz, 3H).

**Example 48: 3-(2-(6-(2-ethyl-5-fluoro-4-hydroxyphenyl)-1H-indazol-3-yl) -4,6-dihydropyrrolo[3,4-dlimidazol-5(1H)-yl)-3-oxypropionitrile (MDI-256)**

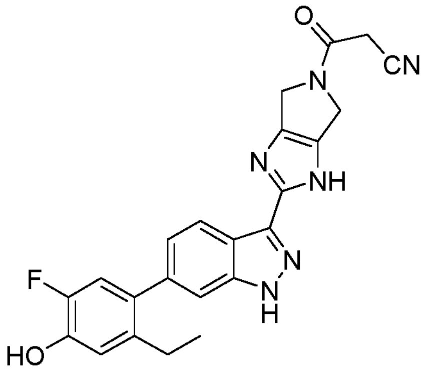

**MDI-256**

Synthetic route of MDI-256:

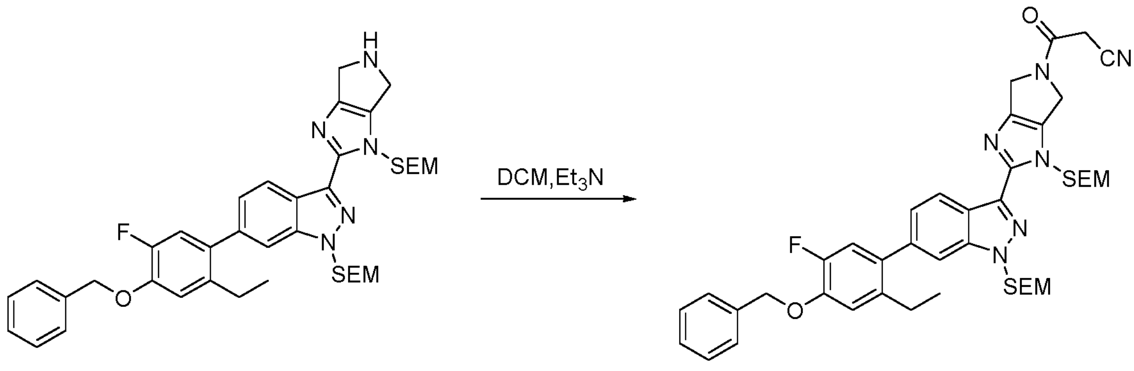


**MDI-256-1**

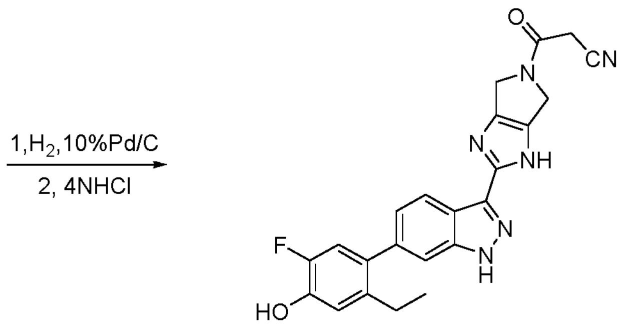


**MDI-256**

**Synthesis method:**

**Synthesis of intermediate MDI-256-1: 3-(2-(6-(4-(benzyloxy)-2-ethyl-5-fluorophenyl)-1-((2-(trimethylsilyl)ethoxy)methyl)-1H-indazol-3-yl)-1-((2-(trimethylsilyl)ethoxy)methyl)-4,6-dihydropyrrolo[3,4-d]imidazol-5(1H)-yl)-3-oxypropionitrile**

6-(4-(benzyloxy)-2-ethyl-5-fluorophenyl)-1-((2-(trimethylsilyl)ethoxy) methyl)-3-(1-((2-(trimethylsilyl)ethoxy)methyl)-1,4,5,6-tetrahydropyrrolo[3,4-d]imidazol-2-yl)-1H-indole (50 mg, 0.07 mmol) was dissolved in 5 ml of dichloromethane, to which Et3N (21.2 mg, 0.21 mmol) was added. The mixture was cooled to 0°C and to which 2-cyanoacetyl chloride (8.7 mg, 0.08 mmol) was slowly added. It was allowed to react at room temperature for 1 hour, and water was added to quench the reaction. The resulting mixture was extracted twice with dichloromethane, and the organic phases were combined, washed with water and saturated brine, dried over anhydrous sodium sulfate, concentrated, and purified by silica gel column to afford 31 mg of intermediate MDI-256-1 with a yield of 56.7%.

**Synthesis of compound MDI-256: 3-(2-(6-(2-ethyl-5-fluoro-4-hydroxyphenyl)-1H-indazol-3-yl)-4,6-dihydropyrrolo[3,4-d]imidazol-5(1H)-yl)-3-oxypropionitrile**

Intermediate MDI-256-1 (31 mg, 0.04 mmol) was dissolved in methanol (6 ml), and 6 mg 10% Pd/C was added. The atmosphere was replaced with hydrogen 3 times. The mixture was heated to 40 °C, reacted for 1 hour, filtered, and concentrated, to which 4 ml of methanol and 1 ml of concentrated hydrochloric acid were added. The mixture was heated to 50 °C, reacted for 6 hours, and concentrated to give a residue. The residue was dissolved in methanol and was concentrated to dryness, which was repeated 3 times. The resulting residue was dissolved in methanol, to which 1 ml of aqueous ammonia was added to neutralize. The resulting mixture was concentrated and purified by a preparation plate to afford 3 mg of the final product with a yield of 17.4%.

$^1$H NMR (400 MHz, MeOD) $\delta$ 8.27 (d, J = 8.0 Hz, 1H), 7.43 (s, 1H), 7.18 (d, J = 8.0 Hz, 1H), 6.97 (dd, J = 8.0 Hz, J = 20.0 Hz, 2H), 4.77-4.70 (m, 4H), 3.62 (s, 2H), 2.59-2.53 (m, 2H), 1.09 (t, J = 8.0 Hz, 3H).

**Example 49: 2-(6-(2-ethyl-5-fluoro-4-hydroxyphenyl)-1H-indazol-3-yl)-N,N-dimethyl-4,6-dihydropyrrolo[3,4-d]imidazol-5(1H)-carboxamide(MDI-257)**

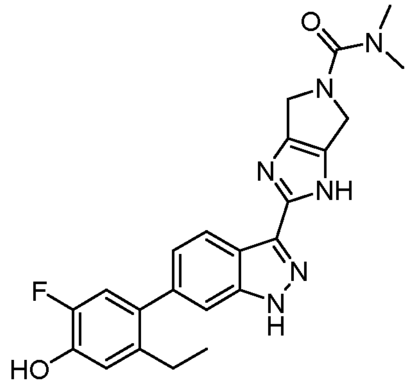

**MDI-257**

**Synthetic route of MDI-257:**

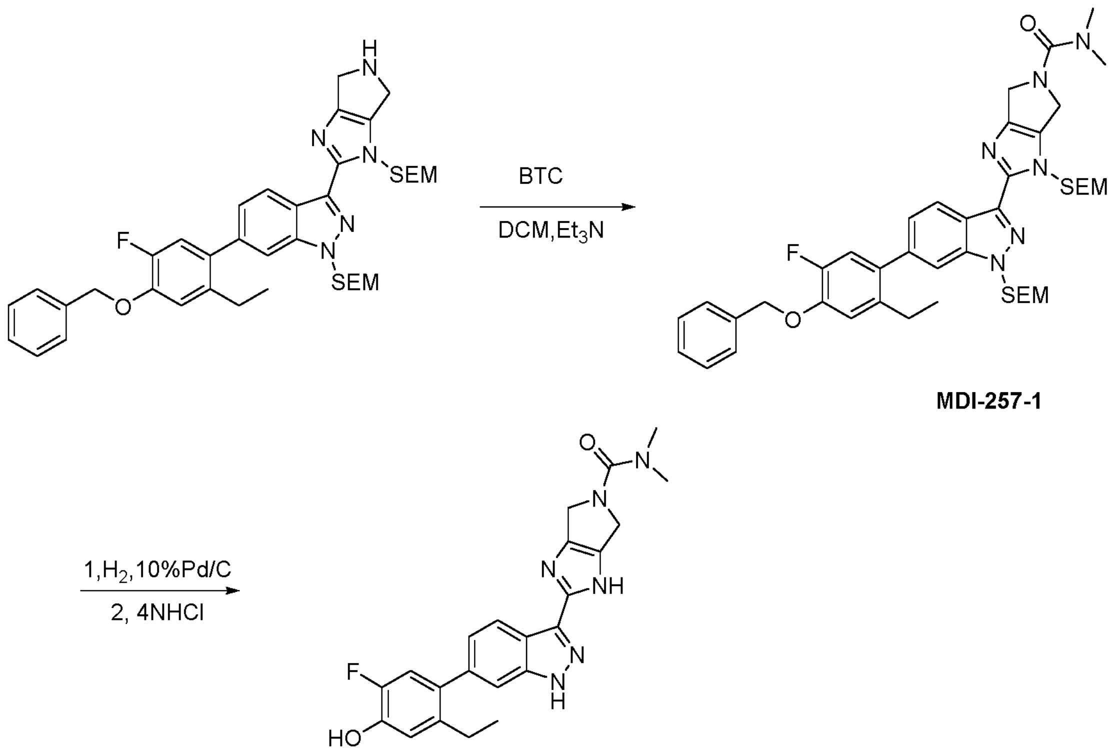


**MDI-257-1**

**MDI-257**

**Synthesis method:**

**Synthesis of intermediate MDI-257-1: 2-(6-(4-(benzyloxy)-2-ethyl-5-fluorophenyl)-1-((2-(trimethylsilyl)ethoxy)methyl)-1H-indazol-3-yl)-N,N-dimethyl-1-((2-(trimethylsilyl)ethoxy)methyl)-4,6-dihydropyrrolo[3,4-d]imidazol-5(1H)-carboxamide**

The synthesis process was similar to that of the intermediate MDI-246-1 with the exception that dimethylamine hydrochloride was used instead of methylamine hydrochloride.

**Synthesis of compound MDI-257: 2-(6-(2-ethyl-5-fluoro-4-hydroxyphenyl)-1H-indazol-3-yl)-N,N-dimethyl-4,6-dihydropyrrolo[3,4-d]imidazol-5(1H)-carboxamide**

Intermediate MDI-257-1 (41 mg, 0.05 mmol) was dissolved in methanol (6 ml), and 8 mg 10% Pd/C was added.

The atmosphere was replaced with hydrogen 3 times. The mixture was heated to 40 °C, reacted for 1 hour, filtered, and concentrated, to which 4 ml of methanol and 1 ml of concentrated hydrochloric acid were added. The mixture was heated to 50 °C, reacted for 6 hours, and concentrated to give a residue. The residue was dissolved in methanol, and was concentrated to dryness, which was repeated 3 times. The resulting residue was dissolved in methanol, to which 1 ml of ammonia was added to neutralize. The resulting mixture was concentrated and purified by a preparation plate to afford 8 mg of the final product with a yield of 35.2%.

$^{1}$H NMR (400 MHz, DMSO) δ 13.28 (s, 1H), 9.85 (s, 1H), 8.32 (d, J = 8.0 Hz, 1H), 7.40 (s, 1H), 7.13 (d, J = 8.0 Hz, 1H), 7.03 (d, J = 12.0 Hz, 1H), 6.93 (d, J = 12.0 Hz, 1H), 4.54-4.53 (m, 4H), 2.85 (s, 6H), 2.50-2.46 (m, 2H), 1.04 (t, J = 8.0 Hz, 3H).

**Example 50: N-(2-cyanoethyl)-2-(6-(2-ethyl-5-fluoro-4-hydroxyphenyl)-1H-indazol-3-yl)-4,6-dihydropyrrolo[3,4-d]imidazole-5(1H)-carboxamide(MDI-258)**

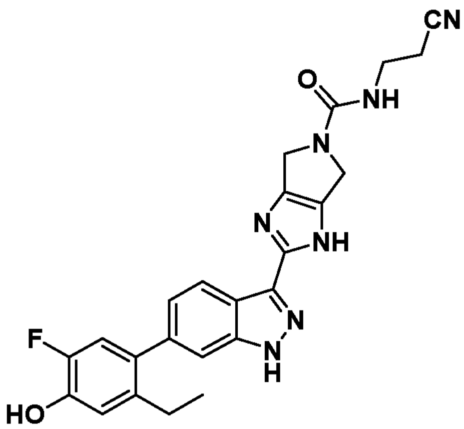

**MDI-258**

**Synthetic route of MDI-258:**

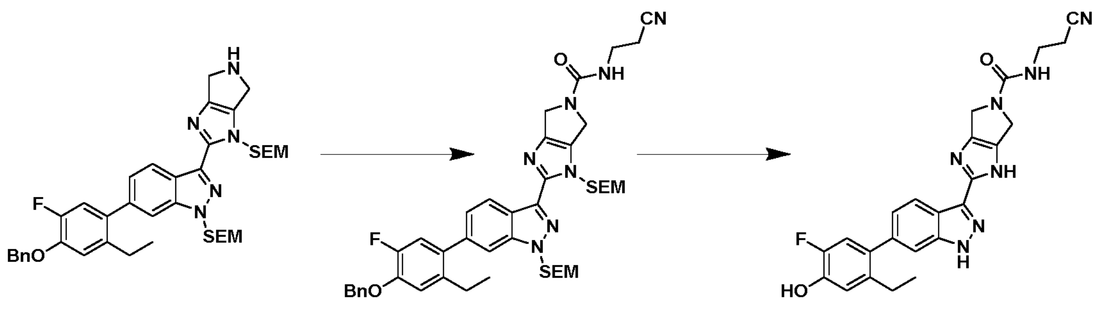

MDI-258-1 MDI-258

**Synthesis method:**

**Synthesis of intermediate MDI-258-1: 2-(6-(4-(benzyloxy)-2-ethyl-5-fluorophenyl)-1-((2-(trimethylsilyl)ethoxy)methyl)-1H-indazol-3-yl)-N-(2-cyanoethyl)-1-((2-(trimethylsilyl)ethoxy)methyl)-4,6-dihydropyrrolo[3,4-d]imidazol-5(1H)-carboxamide**

The synthesis process was similar to that of the intermediate MDI-246-1 with the exception that 3-aminopropionitrile was used instead of methylamine hydrochloride.

**Synthesis of compound MDI-258: N-(2-cyanoethyl)-2-(6-(2-ethyl-5-fluoro-4-hydroxyphenyl)-1H-indazol-3-yl)-4,6-dihydropyrrolo[3,4-d]imidazol-5(1H)-carboxamide**

Intermediate MDI-258-1 (36 mg, 0.04 mmol) was dissolved in methanol (4 ml), and 3.6 mg 10% Pd/C was added. The atmosphere was replaced with hydrogen 3 times. The mixture was heated to 40 °C, reacted for 1 hour, filtered, and concentrated. The concentrated product was dissolved in 4 ml of methanol to which 2 ml of concentrated hydrochloric acid was added. The mixture was heated to 60 °C, reacted for 6 hours, and concentrated. The solid was dissolved in methanol, which was adjusted with aqueous ammonia to pH=8-9. The resulting mixture was concentrated and purified by a preparation plate to afford 7.0 mg of the final product with a yield of 34.2%.

$^1$H NMR (400 MHz, MeOD) δ 8.25 (d, $J$ = 8.4 Hz, 1H), 7.41 (s, 1H), 7.16 (d, $J$ = 8.4 Hz, 1H), 6.91 (dd, $J$ = 20.8, 10.3 Hz, 2H), 4.61 - 4.54 (m, 4H), 3.55 - 3.50 (m, 2H), 2.66 - 2.51 (m, 4H), 1.06 (t, $J$ = 7.5 Hz, 3H).

**Example 51: N-cyclopropyl-2-(6-(2-ethyl-5-fluoro-4-hydroxyphenyl)-1H-indazol-3-yl)-4,6-dihydropyrrolo[3,4-d]imidazol-5(1H) -carboxamide(MDI-259)**

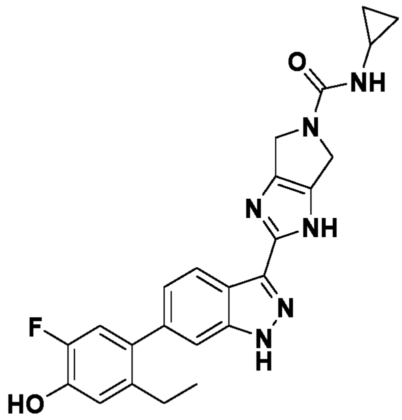

**MDI-259**

**Synthetic route of MDI-259:**

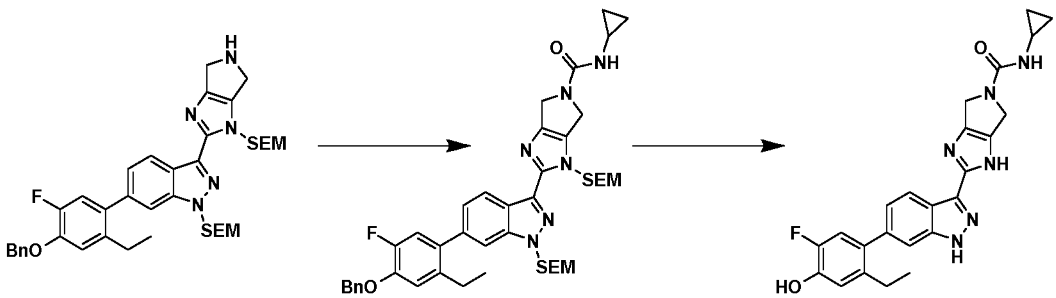

MDI-259-1 MDI-259

**Synthesis method:**

**Synthesis of intermediate MDI-259-1: 2-(6-(4-(benzyloxy)-2-ethyl-5-fluorophenyl)-1-((2-(trimethylsilyl)ethoxy)methyl)-1H-indazol-3-yl)-N-cyclopropyl-1-(((2-(trimethylsilyl)ethoxy)methyl)-4,6-dihydropyrrolo[3,4-d]imidazol-5(1H)-carboxamide**

The synthesis process was similar to that of the intermediate MDI-246-1 with the exception that cyclopropylamine was used instead of methylamine hydrochloride.

**Synthesis of compound MDI-259: N-cyclopropyl-2-(6-(2-ethyl-5-fluoro-4-hydroxyphenyl)-1H-indazol-3-yl)-4,6-dihydropyrrolo[3,4-d]imidazol-5(1H)-carboxamide**

Intermediate MDI-259-1 (36 mg, 0.04 mmol) was dissolved in methanol (4 ml), and 3.6 mg 10% Pd/C was added. The atmosphere was replaced with hydrogen 3 times. The mixture was heated to 40 °C, reacted for 1 hour, filtered, and concentrated. The concentrated product was dissolved in 4 ml of methanol to which 2 ml of concentrated hydrochloric acid was added. The mixture was heated to 60 °C, reacted for 6 hours, and concentrated. The solid was dissolved in methanol, which was adjusted with aqueous ammonia to pH=8-9. The resulting mixture was concentrated and purified by a preparation plate to afford 8.0 mg of the final product with a yield of 39.6%.

$^1$H NMR (400 MHz, MeOD) δ 8.25 (d, $J$ = 8.4 Hz, 1H), 7.43 (s, 1H), 7.16 (dd, $J$ = 8.4, 1.4 Hz, 1H), 6.91 (dd, $J$ = 20.6, 10.4 Hz, 2H), 4.66 - 4.48 (m, 4H), 2.68 - 2.62 (m, 1H), 2.59 - 2.53 (m, 2H), 1.08 (t, $J$ = 7.5 Hz, 3H), 0.76 - 0.71 (m, 2H), 0.60 - 0.56 (m, 2H).

**Example 52: N-cyclobutyl-2-(6-(2-ethyl-5-fluoro-4-hydroxyphenyl)-1H-indazol-3-yl)-4,6-dihydropyrrolo[3,4-d]imidazol-5(1H)-carboxamide (MDI-260)**

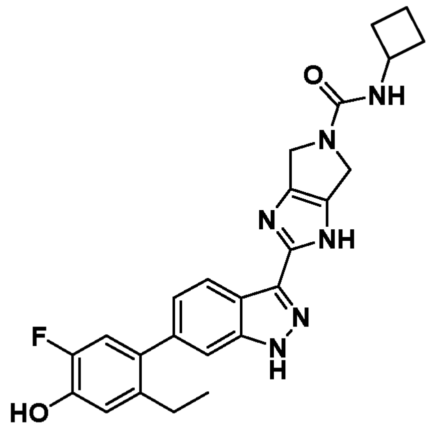

**MDI-260**

**Synthetic route of MDI-260:**

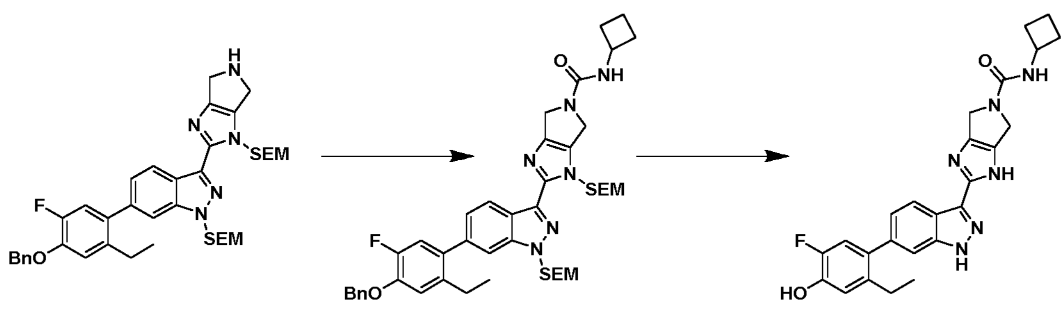

MDI-260-1

MDI-260

**Synthesis method:**

**Synthesis of intermediate MDI-260-1: 2-(6-(4-(benzyloxy)-2-ethyl-5-fluorophenyl)-1-((2-(trimethylsilyl)ethoxy)methyl)-1H-indazol-3-yl)-N-cyclobutyl-1-(((2-(trimethylsilyl)ethoxy)methyl)-4,6-dihydropyrrolo[3,4-d]imidazol-5(1H)-carboxamide**

The synthesis process was similar to that of the intermediate MDI-246-1 with the exception that cyclobutylamine

was used instead of methylamine hydrochloride.

**Synthesis of compoundMDI-260:N-cyclobutyl-2-(6-(2-ethyl-5-fluoro-4-hydroxyphenyl)-1H-indazol-3-yl) -4,6-dihydropyrrolo[3,4-d]imidazol-5(1H)-carboxamide**

Intermediate MDI-260-1 (37 mg, 0.04 mmol) was dissolved in methanol (4 ml), and 3.7 mg 10% Pd/C was added. The atmosphere was replaced with hydrogen 3 times. The mixture was heated to 40 °C, reacted for 1 hour, filtered, and concentrated. The concentrated product was dissolved in 4 ml of methanol to which 2 ml of concentrated hydrochloric acid was added. The mixture was heated to 60 °C, reacted for 6 hours, and concentrated. The solid was dissolved in methanol, which was adjusted with aqueous ammonia to pH=8-9. The resulting mixture was concentrated and purified by a preparation plate to afford 4.0 mg of the final product with a yield of 19.0%.

$^1H$ NMR (400 MHz, MeOD) δ 8.27 (d, J = 8.4 Hz, 1H), 7.43 (s, 1H), 7.18 (d, J = 8.4 Hz, 1H), 7.01-6.85 (m, 2H), 4.57 (s, 4H), 4.35-4.31 (m, 1H), 2.59-2.53 (m, 2H), 2.36-2.30 (m, 2H), 2.11-2.04 (m, 2H), 1.76-1.69 (m, 2H), 1.08 (t, J = 7.5 Hz, 3H).

**Example 53: (S)-6-(2-ethyl-5-fluoro-4-hydroxyphenyl)-3-(5-prolyl-1,4,5,6-tetrahydropyrrolo[3,4-d]imidazol-2-yl)-1H-indazol (MDI-262)**

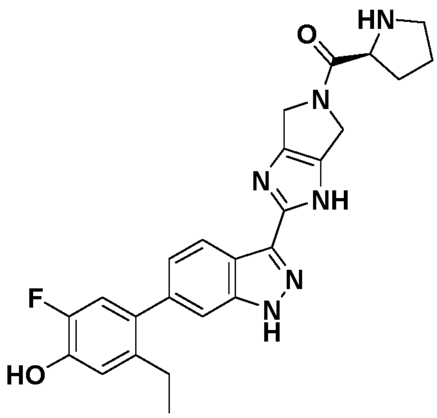

**MDI-262**

**Synthetic route of MDI-262:**

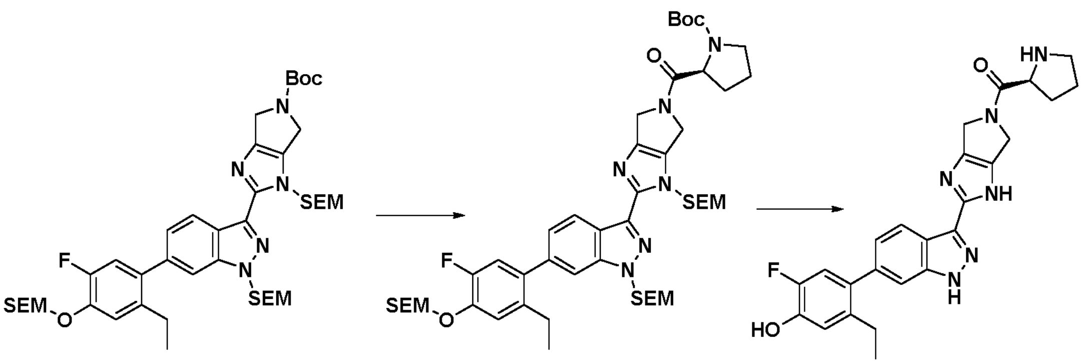

MDI-262-1 MDI-262

Synthesis method:

Synthesis of intermediate MDI-262-1: Tert-butyl (S)-2-(2-(6-(2-ethyl-5-fluoro-4-((2-(trimethylsilyl)ethoxy)methoxy)phenyl)-1-((2-(trimethylsilyl)ethoxy) methyl)-1H-indazol-3-yl)-1-((2-(trimethylsilyl)ethoxy)methyl)-1,4,5,6-**tetrahydropyrrolo[3,4-d]imidazol-5-carbonyl)pyrrolidin-1-carboxylate**

Tert-butyl 2-(6-(2-ethyl-5-fluoro-4-((2-(trimethylsilyl)ethoxy)methoxy) phenyl) 1-((2-(trimethylsilyl)ethoxy)methyl)-1H-indazol-3-yl)-1-((2-(trimethylsilyl)ethoxy) methyl)-4,6-dihydropyrrolo[3,4-d]imidazol-5(1H)-carboxylate (65.0 mg, 0.08 mmol) was dissolved in 10 ml DCM, to which zinc bromide (68.6 mg, 0.31 mmol) was added. The mixture was stirred for 5 hours, and water was added to quench the reaction. The resulting mixture was extracted with DCM twice, and the organic phases were combined, washed with aqueous ammonia, then washed with water and saturated brine, dried over anhydrous sodium sulfate, and concentrated. The obtained crude product was dissolved in 10 ml DMF, to which Boc-L-proline (19.7 mg, 0.09 mmol), HATU (34.71mg, 0.09 mmol), and DIPEA (11.8 mg, 0.09 mmol) were added. After the addition, it was allowed to react at room temperature. Water was added to quench the reaction. The resulting mixture was extracted twice with ethyl acetate, and the organic phases were combined, washed with water and saturated brine, dried over anhydrous sodium sulfate and concentrated to afford 43.0 mg of intermediate MDI-262-1 with a yield of 59.4%.

$^1$H NMR (400 MHz, CDCl3) δ 8.50-8.41 (m, 1H), 7.47-7.45 (m, 1H), 7.25-7.22 (m, 1H), 7.16 (d, J = 8.0 Hz, 1H), 7.01 ( d, J = 10.3 Hz, 1H), 6.05-5.87 (m, 2H), 5.76-5.75 (m, 2H), 5.31 (s, 2H), 5.02-4.23 (m, 5H), 3.89-3.83 (m, 2H), 3.70-3.42 (m, 6H), 2.57-2.51 (m, 2H), 2.37-1.88 (m, 3H), 1.73-1.70 (m, 1H), 1.47 (s, 9H), 1.07-0.98 ( m, 5H), 0.94-0.88 (m, 4H), 0.03(s, 9H), -0.06-0.08 (m, 18H).

**Synthesis of compound MDI-262: (S)-6-(2-ethyl-5-fluoro-4-hydroxyphenyl)-3-(5-prolyl-1,4,5,6-tetrahydropyrrolo[3,4-d]imidazol-2-yl)-1H-indazole**

The intermediate MDI-262-1 (33.0 mg, 0.04 mmol) was dissolved in 4 ml MeOH, to which 2 ml concentrated hydrochloric acid was added. After the addition, the temperature was raised to 50°C for reaction. After 6 hours of reaction, the temperature was reduced to room temperature, and the reaction solvent was evaporated by concentration under reduced pressure, followed by addition of 4 ml methanol and 0.5 ml aqueous ammonia. After concentration, the residue was subject to thin layer chromatography to afford 1.8 mg of white solid MDI-262 with a yield of 11.3%.

$^1$H NMR (400 MHz, MeOD) δ 8.28 (d, J = 8.0 Hz, 1H), 7.44 (s, 1H), 7.18 (d, J = 8.5 Hz, 1H), 6.96 (d, J = 11.7 Hz, 1H), 6.91 (d, J = 8.9 Hz, 1H), 4.80-4.64 (m, 4H), 4.09-4.05 (m, 1H), 3.26-3.22 (m, 2H), 2.59-2.53 (m, 2H), 2.06-1.86 (m, 4H), 1.08 (t, J = 8.0 Hz, 3H). LC-MS m/z (ESI) $[M+H]^+$ calculated value for $C_{25}H_{26}FN_6O_2$: 461.2; measured value: 461.2 .

**Example 54: (R)-6-(2-ethyl-5-fluoro-4-hydroxyphenyl)-3-(5-prolyl-1,4,5,6-tetrahydropyrrolo[3,4-d]imidazol-2-yl)-1H-indazol(MDI-263)**

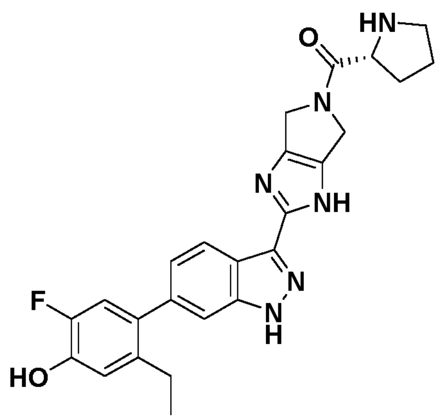

**MDI-263**

The synthesis process was similar to that of MDI-262, with the exception that Boc-D-proline was used instead of Boc-L-proline.

$^1$H NMR (400 MHz, MeOD) δ 8.27 (d, J = 8.0 Hz, 1H), 7.44 (s, 1H), 7.18 (d, J = 8.4 Hz, 1H), 6.96 (d, J = 12.2 Hz, 1H), 6.91 (d, J = 8.8 Hz, 1H), 4.82-4.60 (m, 4H), 4.21-4.15 (m, 1H), 3.33-3.23 (m, 1H), 3.08-2.99 (m, 1H), 2.59-2.53 (m, 2H), 2.08-1.86 (m, 4H), 1.08 (t, J = 8.0 Hz, 3H).

**Example 55: Evaluation of Pharmacological Activity - I**

**1. Experimental principle**

A drug screening system based on kinases JAK1, JAK2, JAK3, and TYK2 was used to detect the inhibitory ability of small molecule compounds on kinase activity. A kinase undergoes an enzymatic reaction with its substrates IRS1, IGF1Rtide, and Poly (4:1 Glu, Tyr), consuming ATP to produce ADP, wherein the ADP-Glo reagent and luminescence method can be used to detect the amount of the product to reflect the activity of the kinase.

**2. Experimental scheme**

**2.1 Experimental materials and instruments**

| Item | Name | Source/Supplier | Catalogue No. |
|---|---|---|---|
| 1 | HEPES | Life Technologies | 15630-080 |
| 2 | BRIJ 35 detergent (10%) | Merck | 203728 |
| 3 | MgCl2 | Sigma | M1028 |
| 4 | EGTA | Sigma | E3889 |
| 5 | ADP-Glo Kinase Assay | Promega | V9101 |
| 6 | JAK1 | Invitrogen | PV4774 |
| 7 | JAK2 | Invitrogen | PV4210 |
| 8 | JAK3 | Invitrogen | PV3855 |
| 9 | TYK2 | Invitrogen | PV4790 |
| 10 | ATP | Promega | V915B |
| 11 | IRS1 | Signalchem | I40-58-1000 |
| 12 | IGF1Rtide | Signalchem | I15-58 |
| 13 | Poly (4:1 Glu, Tyr) | Sigma | P0275 |
| 14 | Topseal A | PerkinElmer | E5341 |
| 15 | OptiPlate-384 | PerkinElmer | 6007290 |
| 16 | 384-Well Polypropylene microplate | Labcyte | PP-0200 |
| 17 | Envision | Perkin Elmer | 2104 |
| 18 | Echo | Labcyte | 550 |
| 19 | Centrifuge | Eppendorf | 5810R |

**2.2 Experimental methods**

**2.2.1 Kinase reaction reagent formulation**

**2.2.1.1 1X Kinase Reaction Buffer (400 mL)**

| Name | Stock Concentration | Volume | Final Concentration |
|---|---|---|---|
| HEPES | 1 M (20X) | 20 mL | 50 mM |
| $MgCl_2$ | 1 M (100X) | 4 mL | 10 mM |
| BRIJ-35 | 10%(1000X) | 400 μL | 0.01% |
| EGTA | Powder | 152 mg | 1 mM |
| ddH2O | | 375.6mL | |
| 2 mM DTT, ready to use | | | |

**2.2.1.2 2X** Kinase **Formulation**

| JAK1 kinase solution | | | | |
|---|---|---|---|---|
| Name | Stock concentration | Volume | 2X Final Concentration | Final Concentration |
| JAK1 | 7072.4 nM (884X) | 0.8 μL | 8 nM | 4 nM |
| 1X Kinase Reaction Buffer | | 706.5 μL | | |

| JAK2 kinase solution | | | | |
|---|---|---|---|---|
| Name | Stock concentration | Volume | 2X Final Concentration | Final Concentration |
| JAK2 | 4955nM (4955X) | 0.2μL | 1nM | 0.5 nM |
| 1X Kinase Reaction Buffer | | 990.8μL | | |

| JAK3 kinase solution | | | | |
|---|---|---|---|---|
| Name | Stock concentration | Volume | 2X Final Concentration | Final Concentration |
| JAK3 | 5341.2nM (5341.2X) | 0.2 μL | 1nM | 0.5 nM |
| 1X Kinase Reaction Buffer | | 1068μL | | |

| TYK2 kinase solution | | | | |
|---|---|---|---|---|
| Name | Stock concentration | Volume | 2X Final Concentration | Final Concentration |
| TYK2 | 6104.7nM (763X) | 1μL | 8nM | 4nM |
| 1X Kinase Reaction Buffer | | 762μL | | |

**2.2.1.3 2X Substrate Mixture Formulation**

| JAK1 substrate mixture solution | | | | |
|---|---|---|---|---|
| Name | Stock concentration | Volume | 2X Final Concentration | Final Concentration |
| ATP | 10 mM (250X) | 2.8μL | 40μM | 20μM |
| IRS1 | 1 mg/mL (10X) | 70μL | 0.1 mg/mL | 0.05 mg/mL |

(continued)

| JAK1 substrate mixture solution | | | | |
|---|---|---|---|---|
| Name | Stock concentration | Volume | 2X Final Concentration | Final Concentration |
| 1X Kinase Reaction Buffer | | 627.2μL | | |

| JAK2 substrate mixture solution | | | | |
|---|---|---|---|---|
| Name | Stock concentration | Volume | 2X Final Concentration | Final Concentration |
| ATP | 10 mM (500X) | 1.4μL | 20 μM | 10 μM |
| IGF1Rtide | 1 mg/mL (50X) | 14μL | 0.02 mg/mL | 0.01 mg/mL |
| 1X Kinase Reaction Buffer | | 684.6μL | | |

| JAK3 substrate mixture solution | | | | |
|---|---|---|---|---|
| Name | Stock concentration | Volume | 2X Final Concentration | Final Concentration |
| ATP | 10 mM (500X) | 1.4μL | 20μM | 10μM |
| Poly (4:1 Glu, Tyr) Peptide | 5 mg/mL (83.3X) | 8.4μL | 0.06 mg/mL | 0.03 mg/mL |
| 1X Kinase Reaction Buffer | | 690.2μL | | |

| TYK2 substrate mixture solution | | | | |
|---|---|---|---|---|
| Name | Stock concentration | Volume | 2X Final Concentration | Final Concentration |
| ATP | 10 mM (500X) | 1.4 μL | 20 μM | 10μM |
| IRS1 | 1 mg/mL (16.67X) | 42 μL | 0.06 mg/mL | 0.03 mg/mL |
| 1X Kinase Reaction Buffer | | 656.6μL | | |

**2.2.1.4 Compounds to be tested**

| Name | Mass /mg | Molecular weight | Concentration /mM |
|---|---|---|---|
| Filgotinib | 5.0 | 420.5 | 10 |
| MDI-2 | 3.3 | 552.24 | 10 |
| MDI-201 | 2.0 | 554.59 | 10 |
| MDI-202 | 1.9 | 471.50 | 10 |
| MDI-206 | 2.0 | 503.55 | 10 |
| MDI-203 | 1.8 | 488.57 | 10 |
| MDI-204 | 2.1 | 567.63 | 10 |
| MDI-205 | 1.9 | 549.64 | 10 |
| MDI-207 | 1.5 | 455.50 | 10 |
| MDI-209 | 1.9 | 431.47 | 10 |
| MDI-211 | 1.6 | 445.50 | 10 |

(continued)

| Name | Mass /mg | Molecular weight | Concentration /mM |
|---|---|---|---|
| MDI-213 | 1.5 | 461.50 | 10 |
| MDI-217 | 1.6 | 461.54 | 10 |

**2.2.2 Kinase Reaction Experiment Procedure**

**2.2.2.1 JAK1 & JAK2 Kinase Reaction Experimental Procedure**

a) Diluting Filgotinib (10 mM stock solution) by 10 times, and diluting a compound solution to be tested by 10 times with 100% DMSO, and then performing a series of dilutions at a ratio of 1:3 in a 384-well dilution plate (Labcyte, PP-0200). Concentrations of Filgotinib: 1000, 333.33, 111.11, 37.04, 12.35, 4.12, 1.37, 0.46, 0.15, 0.05, 0.02, and 0 μM; and concentrations of the compound to be tested: 1000, 333.33, 111.11, 37.04, 12.35, 4.12, 1.37, 0.46, 0.15, 0.05, and 0 μM.
b) Using Echo to transfer 0.1 μL of the compound solution to be tested (prepared in step a) to a 384-well reaction plate (PE, 6007290), and centrifuging it at 1000 rpm/min for 1 min.
c) Transferring 5μL of kinase (prepared according to 2.2.1.2) to the 384-well reaction plate (prepared in step b), centrifuging it at 1000 rpm/min for 1 min, and incubating it at 25°C for 15 min.
d) Transferring 5μL of the substrate mixture (prepared according to 2.2.1.3) to the 384-well reaction plate, centrifuging it at 1000 rpm/min for 1 min, and incubating it at 25°C for 60 min. In the reaction system, the final concentrations of Filgotinib are 10, 3.33, 1.11, 0.37, 0.12, 0.04, 0.014, 0.0046, 0.0015, 0.0005, and 0 μM. The final concentrations of the compound to be tested are: 10, 3.33, 1.11, 0.37, 0.12, 0.04, 0.014, 0.0046, 0.0015, 0.0005, and 0 μM. The final concentration of DMSO is 1%.
e) Transferring 10μL of ADP-Glo to the 384-well reaction plate, centrifuging it at 1000 rpm/min for 1 min, and incubating it at 25°C for 40 min.
f) Transferring 20 μL of Detection solution to the 384-well reaction plate, centrifuging it at 1000 rpm/min for 1 min, and incubating it at 25°C for 40 min.
g) Using Envision multi-function plate reader to read the RLU (Relative luminescence unit) signal. The signal intensity is used to characterize the degree of kinase activity.

2.2.2.2 JAK3 Kinase Reaction Experimental Procedure

a) Diluting Filgotinib (10 mM stock solution) and a compound solution to be tested by 10 times with 100% DMSO, and then performing a series of dilutions at a ratio of 1:3 in a 384-well dilution plate (Labcyte, PP-0200). Filgotinib concentrations are: 10000, 3333.33, 1111.11, 370.37, 123.46, 41.15, 13.72, 4.57, 1.52, 0.51, 0.17, and 0 μM; and concentrations of the compound to be tested are: 1000, 333.33, 111.11, 37.04, 12.35, 4.12, 1.37, 0.46, 0.15, 0.05, and 0 μM.
b) Using Echo to transfer 0.1 μL of the compound solution to be tested (prepared in step a) to a 384-well reaction plate (PE, 6007290), and centrifuging it at 1000 rpm/min for 1 min.
c) Transferring 5μL of kinase (prepared according to 2.2.1.2) to the 384-well reaction plate (prepared in step b), centrifuging it at 1000 rpm/min for 1 min, and incubating it at 25°C for 15 min.
d) Transferring 5μL of the substrate mixture (prepared according to 2.2.1.3) to the 384-well reaction plate, centrifuging it at 1000 rpm/min for 1 min, and incubating it at 25°C for 60 min. In the reaction system, the final concentrations of Filgotinib are 100, 33.33, 11.11, 3.70, 1.23, 0.412, 0.137, 0.046, 0.015, 0.005, 0.002, and 0 μM. The final concentrations of the compound to be tested are: 10, 3.33, 1.11, 0.37, 0.12, 0.04, 0.014, 0.0046, 0.0015, 0.0005, and 0 μM. The final concentration of DMSO is 1%.
e) Transferring 10μLADP-Glo to the 384-well reaction plate, centrifuging it at 1000 rpm/min for 1 min, and incubating it at 25°C for 40 min.
f) Transferring 20 μL of Detection solution to the 384-well reaction plate, centrifuging it at 1000 rpm/min for 1 min, and incubating it at 25°C for 40 min.
g) Using Envision multi-function plate reader to read the RLU (Relative luminescence unit) signal. The signal intensity

is used to characterize the degree of kinase activity.

2.2.2.3 TYK2 Kinase Reaction Experimental Procedure

a) Diluting Filgotinib (10 mM stock solution) by 3.3 times, and a compound solution to be tested by 10 times with 100% DMSO, and then performing a series of dilutions at a ratio of 1:3 in a 384-well dilution plate (Labcyte, PP-0200). The concentrations of Filgotinib are: 3000, 1000, 333.33, 111.11, 37.04, 12.35, 4.12, 1.37, 0.46, 0.15, 0.05, and 0 μM; and the concentrations of the compound to be tested are: 1000, 333.33, 111.11, 37.04, 12.35, 4.12, 1.37, 0.46, 0.15, 0.05, and 0 μM.
b) Using Echo to transfer 0.1 μL of the compound solution to be tested (prepared in step a) to a 384-well reaction plate (PE, 6007290), and centrifuging it at 1000 rpm/min for 1 min.
c) Transferring 5μL of kinase (prepared according to 2.2.1.2) to the 384-well reaction plate (prepared in step b), centrifuging it at 1000 rpm/min for 1 min, and incubating it at 25°C for 15 min.
d) Transferring 5μL of the substrate mixture (prepared according to 2.2.1.3) to the 384-well reaction plate, centrifuging it at 1000 rpm/min for 1 min, and incubating it at 25°C for 60 min. In the reaction system, the final concentrations of Filgotinib are 30, 10, 3.3333, 1.1111, 0.3704, 0.1235, 0.0412, 0.0137, 0.0046, 0.0015, 0.0005, and 0 μM. The final concentrations of the compound to be tested are: 10, 3.33, 1.11, 0.37, 0.12, 0.04, 0.014, 0.0046, 0.0015, 0.0005, and 0 μM. The final concentration of DMSO is 1%.
e) Transferring 10μL of ADP-Glo to the 384-well reaction plate, centrifuging it at 1000 rpm/min for 1 min, and incubating it at 25°C for 40 min.
f) Transferring 20 μL of Detection solution to the 384-well reaction plate, centrifuging it at 1000 rpm/min for 1 min, and incubating it at 25°C for 40 min.
g) Using Envision multi-function plate reader to read the RLU (Relative luminescence unit) signal. The signal intensity is used to characterize the degree of kinase activity.

**2.2.3 Experimental data processing method**

Compound inhibition rate (% inh) = (negative control - compound) / (negative control-positive control) * 100%

Negative control: DMSO
Positive control: 10 μM/100 μM/30 μM Filgotinib
IC50 (half inhibitory concentration) of the compound can be obtained using the following nonlinear fitting formula:

$$Y=Bottom + (Top-Bottom)/(1+10^{((LogIC50-X)*HillSlope)})$$

X: log value of the compound concentration
Y: Compound inhibition rate (% inh)
**Z' factor calculation equation:**

$$Z'=1-3(SDmin+SDmax)/(AVEmax-AVEmin)$$

in which:

Min is the RLU value of the positive control 10 μM/100 μM/30 μM Filgotinib, and Max is the RLU value of the negative control; and

SD is the standard error, and AVE is the average value of RLU.

### 3. Experimental Results

### 3.1 Quality control results of binding experiment

#### 3.1.1 Quality control result of JAK1 binding experiment

$$Z'=0.77 CV\%(min) = 0\% CV\%(max) = 6.2\%$$

#### 3.1.2 Quality control result of JAK2 binding experiment

$$Z'=0.78 CV\%(min) = 2.9\% CV\%(max) = 5.7\%$$

#### 3.1.3 Quality control result of JAK3 binding experiment

$$Z'=0.71 CV\%(min) = 7.0\% CV\%(max) = 11.3\%$$

#### 3.1.4 Quality control result of TYK2 binding experiment

$$Z'=0.77 CV\%(min) = 3.9\% CV\%(max) = 6.8\%$$

### 3.2 Summary of test results as obtained

| Item | Tested compound | Hillslope | IC50 (nM) |
|---|---|---|---|
| JAK1 Experiment | Filgotinib | 1.067 | 25.550 |
| | MDI-2 | 1.356 | 0.8056 |
| | MDI-201 | 3.487 | 0.138 |
| | MDI-202 | 5.052 | 0.125 |
| | MDI-206 | 1.091 | 0.943 |
| JAK2 Experiment | Filgotinib | 1.142 | 67.920 |
| | MDI-2 | 1.271 | 0.7723 |
| | MDI-201 | 1.633 | 0.217 |
| | MDI-202 | 2.385 | 0.279 |
| | MDI-206 | 1.457 | 0.556 |

(continued)

| Item | Tested compound | Hillslope | IC50 (nM) |
|---|---|---|---|
| JAK3 Experiment | Filgotinib | 1.318 | 1343 |
| | MDI-2 | 1.569 | 0.7649 |
| | MDI-201 | 1.989 | 0.187 |
| | MDI-202 | 2.038 | 0.160 |
| | MDI-206 | 1.216 | 0.628 |
| TYK2 Experiment | Filgotinib | 1.037 | 128.0 |
| | MDI-2 | | 1.630 |
| | MDI-201 | 1.411 | 0.281 |
| | MDI-202 | 1.416 | 0.318 |
| | MDI-206 | 0.744 | 7.229 |

For brevity, only IC50 values are shown for the below tested compounds.

| Item | Tested compound | IC50 (nM) |
|---|---|---|
| JAK1 Experiment | Filgotinib | 25.550 |
| | MDI-203 | 0.160 |
| | MDI-204 | 0.152 |
| | MDI-205 | 0.121 |
| | MDI-207 | 0.120 |
| | MDI-209 | 0.128 |
| | MDI-211 | 0.162 |
| | MDI-213 | 0.146 |
| | MDI-217 | 0.122 |
| JAK2 Experiment | Filgotinib | 67.920 |
| | MDI-203 | 0.208 |
| | MDI-204 | 0.176 |
| | MDI-205 | 0.158 |
| | MDI-207 | 0.160 |
| | MDI-209 | 0.165 |
| | MDI-211 | 0.198 |
| | MDI-213 | 0.166 |
| | MDI-217 | 0.217 |

(continued)

| Item | Tested compound | IC50 (nM) |
|---|---|---|
| JAK3 Experiment | Filgotinib | 1343 |
| | MDI-203 | 0.212 |
| | MDI-204 | 0.238 |
| | MDI-205 | 0.178 |
| | MDI-207 | 0.132 |
| | MDI-209 | 0.158 |
| | MDI-211 | 0.160 |
| | MDI-213 | 0.116 |
| | MDI-217 | 0.137 |
| TYK2 Experiment | Filgotinib | 128.0 |
| | MDI-203 | 0.328 |
| | MDI-204 | 0.200 |
| | MDI-205 | 0.194 |
| | MDI-207 | 0.474 |
| | MDI-209 | 0.281 |
| | MDI-211 | 0.266 |
| | MDI-213 | 0.146 |
| | MDI-217 | 0.391 |

The above experimental results demonstrate that: MDI-2, MDI-201, MDI-202, MDI-206, MDI-203, MDI-204, MDI-207, MDI-209, MDI-211, MDI-213, and MDI-217 can inhibit JAK1, JAK2, JAK3, and TYK2 at an extremely low concentration, and the inhibitory activities of the compounds in these examples are much higher than that of Filgotinib.

**Example 56: Evaluation of Pharmacological Activity - II**

**1. Experimental principle**

The experimental principle of the pharmacological activity evaluation in this example is the same as that described in Example 55, but the experimental materials or instruments as used, and/or some specific test condition parameters (such as the kinase formulation, substrate formulation, kinase reaction experiment procedures, and the like) were varied and adjusted.

**2. Experimental scheme**

**2.1 Experimental materials and instruments**

| No. | Name | Source/Supplier | Catalogue No. |
|---|---|---|---|
| 1 | HEPES | Life Technologies | 15630-080 |
| 2 | BRIJ 35 detergent (10%) | Sigma | 1018940100 |
| 3 | MgCl2 | Sigma | M1028 |
| 4 | EGTA | Sigma | E3889 |
| 5 | ADP-Glo Kinase Assay | Promega | V9101 |

(continued)

| No. | Name | Source/Supplier | Catalogue No. |
|---|---|---|---|
| 6 | JAK1 | Carna | 08-144 |
| 7 | JAK2 | Carna | 08-045 |
| 8 | JAK3 | Carna | 08-046 |
| 9 | TYK2 | Carna | 08-147 |
| 10 | ATP | Promega | V915B |
| 11 | IRS1 | Signalchem | I40-58-1000 |
| 12 | IGFIRtide | Signalchem | I15-58 |
| 13 | Poly (4:1 Glu, Tyr) | Sigma | P0275 |
| 15 | 384-Well polystyrene shallow flat white | Greiner | 784075 |
| 16 | 384-Well Polypropylene microplate | Labcyte | PP-0200 |
| 17 | Biotek Microplate Reader | Biotek | Synergy 4 |
| 18 | Microplate Low Speed Centrifuge | XiangZhi | TD5B |

**2.2 Experimental methods**

**2.2.1 Kinase reaction reagent formulation**

**2.2.1.1 1X Kinase Reaction Buffer (400 mL)**

It was the same as the formulation of the 1X kinase reaction buffer in Example 55.

**2.2.1.2 2X Kinase Formulation**

| JAK1 kinase solution | | | | |
|---|---|---|---|---|
| Name | Stock concentration | Volume | 2X Final Concentration | Final Concentration |
| JAK1 | 3225 nM (884X) | 5.21 μL | 40 nM | 20 nM |
| 1X Kinase Reaction Buffer | | 414.79 μL | | |

| JAK2 kinase solution | | | | |
|---|---|---|---|---|
| Name | Stock concentration | Volume | 2X Final Concentration | Final Concentration |
| JAK2 | 4256 nM (4955X) | 0.2 μL | 2nM | 1nM |
| 1X Kinase Reaction Buffer | | 419.8 μL | | |

| JAK3 kinase solution | | | | |
|---|---|---|---|---|
| Name | Stock concentration | Volume | 2X Final Concentration | Final Concentration |
| JAK3 | 3195 nM (5341.2X) | 0.5 μL | 4nM | 2nM |
| 1X Kinase Reaction Buffer | | 419.5 μL | | |

| TYK2 kinase solution | | | | |
|---|---|---|---|---|
| Name | Stock concentration | Volume | 2X Final Concentration | Final Concentration |
| TYK2 | 3174nM (763X) | 2.65 μL | 20 nM | 10 nM |
| 1X Kinase Reaction Buffer | | 417.35 μL | | |

**2.2.1.3 4X Substrate Mixture Formulation**

| JAK1 substrate mixture solution | | | | |
|---|---|---|---|---|
| Name | Stock concentration | Volume | 4X Final Concentration | Final Concentration |
| ATP | 10 mM (125X) | 2.4 μL | 80 μM | 20 μM |
| IRS1 | 1 mg/mL (5X) | 60 μL | 0.2 mg/mL | 0.05 mg/mL |
| 1X Kinase Reaction Buffer | | 237.6 μL | | |

| JAK2 substrate mixture solution | | | | |
|---|---|---|---|---|
| Name | Stock concentration | Volume | 4X Final Concentration | Final Concentration |
| ATP | 10 mM (500X) | 6 μL | 20 μM | 5 μM |
| IGF1Rtide | 1 mg/mL (25X) | 12 μL | 0.04 mg/mL | 0.01 mg/mL |
| 1X Kinase Reaction Buffer | | 287.4 μL | | |

| JAK3 substrate mixture solution | | | | |
|---|---|---|---|---|
| Name | Stock concentration | Volume | 4X Final Concentration | Final Concentration |
| ATP | 10 mM (250X) | 1.2 μL | 40 μM | 10 μM |
| Poly (4:1 Glu, Tyr) Peptide | 5 mg/mL (41.6X) | 6 μL | 0.12 mg/mL | 0.03 mg/mL |
| 1X Kinase Reaction Buffer | | 292.8 μL | | |

| TYK2 substrate mixture solution | | | | |
|---|---|---|---|---|
| Name | Stock concentration | Volume | 4X Final Concentration | Final Concentration |
| ATP | 10 mM (250X) | 1.2 μL | 40 μM | 10 μM |
| IRS1 | 1 mg/mL (5X) | 60 μL | 0.08 mg/mL | 0.02 mg/mL |
| 1X Kinase Reaction Buffer | | 238.8 μL | | |

**2.2.1.4 Compounds to be tested**

| Name | Mass /mg | Molecular weight | Concentration /mM |
|---|---|---|---|
| Filgotinib | 5.0 | 420.5 | 10 |
| MDI-208 | 1.6 | 417.49 | 10 |

(continued)

| Name | Mass /mg | Molecular weight | Concentration /mM |
|---|---|---|---|
| MDI-210 | 1.4 | 431.52 | 10 |
| MDI-214 | 1.5 | 469.48 | 10 |
| MDI-215 | 1.5 | 469.48 | 10 |
| MDI-218 | 1.5 | 467.52 | 10 |
| MDI-219 | 1.7 | 481.55 | 10 |
| MDI-220 | 1.5 | 495.57 | 10 |
| MDI-221 | 1.5 | 457.51 | 10 |
| MDI-224 | 1.5 | 431.52 | 10 |
| MDI-225 | 1.6 | 447.51 | 10 |
| MDI-216 | 1.5 | 476.51 | 10 |
| MDI-226 | 1.7 | 405.43 | 10 |
| MDI-227 | 1.6 | 419.46 | 10 |
| MDI-228 | 1.5 | 433.49 | 10 |
| MDI-229 | 1.5 | 445.50 | 10 |
| MDI-230 | 1.4 | 447.51 | 10 |
| MDI-233 | 1.6 | 474.54 | 10 |
| MDI-235 | 1.8 | 489.56 | 10 |
| MDI-231 | 1.5 | 460.51 | 10 |
| MDI-234 | 1.5 | 476.51 | 10 |
| MDI-236 | 1.8 | 503.58 | 10 |
| MDI-237 | 2.3 | 432.5 | 10 |
| MDI-239 | 1.5 | 445.5 | 10 |
| MDI-240 | 1.6 | 490.5 | 10 |
| MDI-242 | 1.4 | 432.5 | 10 |
| MDI-243 | 1.7 | 476.5 | 10 |
| MDI-245 | 1.6 | 490.5 | 10 |
| MDI-246 | 1.5 | 420.5 | 10 |
| MDI-247 | 1.8 | 434.5 | 10 |
| MDI-248 | 1.5 | 450.5 | 10 |
| MDI-250 | 1.6 | 485.5 | 10 |
| MDI-251 | 2.1 | 476.5 | 10 |
| MDI-252 | 1.8 | 421.4 | 10 |
| MDI-253 | 1.6 | 435.5 | 10 |
| MDI-255 | 1.4 | 477.5 | 10 |
| MDI-256 | 1.4 | 430.4 | 10 |
| MDI-257 | 1.6 | 434.5 | 10 |
| MDI-258 | 1.5 | 459.5 | 10 |
| MDI-259 | 1.7 | 446.5 | 10 |

(continued)

| Name | Mass /mg | Molecular weight | Concentration /mM |
|---|---|---|---|
| MDI-260 | 1.7 | 460.5 | 10 |
| MDI-262 | 1.7 | 460.5 | 10 |
| MDI-263 | 1.3 | 460.5 | 10 |

### 2.2.2 Kinase Reaction Experiment Procedure

#### 2.2.2.1 JAK1 & JAK2 Kinase Reaction Experimental Procedure

a) Diluting a compound solution to be tested by 5 times with 100% DMSO. Then, using 100% DMSO as diluent, performing a series of dilutions at a ratio of 1:3 for Filgotinib (10 mM stock solution) and the compound solution to be tested in a 96-well dilution plate. Taking out 1 μL of the compound solution and adding it to 49 μL of kinase reaction buffer, and shaking the resulting mixture on a microplate shaker for 20 minutes.
b) Transferring 2 μL of kinase (prepared according to 2.2.1.2) to a 384-well reaction plate, add 1 μL of the compound solution to be tested (prepared in step a) to the 384-well reaction plate (Greiner, 784075), centrifuging it at 1000 rpm/min for 1 min and incubating it at 25°C for 10 min.
c) Transferring 1 μL of the substrate mixture (prepared according to 2.2.1.3) to the 384-well reaction plate, centrifuging it at 1000 rpm/min for 1 min, and incubating it at 25°C for 60 min. In the reaction system, the final concentrations of Filgotinib are 50, 12.5, 3.125, 0.7812, 0.1953, 0.0488, 0.0122, 0.003, 0.00076, 0.00019, and 0.000047μM. The final concentrations of the compound to be tested are: 10, 2.5, 0.625, 0.15625, 0.039, 0.0097, 0.0024, 0.0006, 0.0015, 0.000038, and 0.0000095μM. The final concentration of DMSO is 0.5%.
d) Transferring 4 μL of ADP-Glo to the 384-well reaction plate, centrifuging it at 1000 rpm/min for 1 min, and incubating it at 25°C for 40 min.
e) Transferring 8 μL of Detection solution to the 384-well reaction plate, centrifuging it at 1000 rpm/min for 1 min, and incubating it at 25°C for 40 min.
f) Using Biotek multi-function plate reader to read the RLU (Relative luminescence unit) signal. The signal intensity is used to characterize the degree of kinase activity.

#### 2.2.2.2 JAK3 & TYK2 Kinase Reaction Experimental Procedure

a) Dilute a compound solution to be tested by 5 times with 100% DMSO. Then, using 100% DMSO as diluent, performing a series of dilutions at a ratio of 1:3 for Filgotinib (10 mM stock solution) and the compound solution to be tested in a 96-well dilution plate. Taking out 1 μL of the compound solution and add it to 49 μL of kinase reaction buffer, and shaking the resulting mixture on a microplate shaker for 20 minutes.
b) Transferring 2 μL of kinase (prepared according to 2.2.1.2) to a 384-well reaction plate, and add 1 μL of the compound solution to be tested (prepared in step a) to the 384-well reaction plate (Greiner, 784075), centrifuging it at 1000 rpm/min for 1 min and incubating it at 25°C for 10 min.
c) Transferring 1 μL of the substrate mixture (prepared according to 2.2.1.3) to the 384-well reaction plate, centrifuging it at 1000 rpm/min for 1 min, and incubating it at 25°C for 60 min. In the reaction system, the final concentrations of Filgotinib are 50, 16.67, 5.555, 1.851, 0.617, 0.205, 0.0686, 0.0228, 0.00762, and 0.0025μM. The final concentrations of the compound to be tested are 10, 3.33, 1.11, 0.37, 0.12, 0.04, 0.014, 0.0046, 0.0015, and 0.0005μM. The final concentration of DMSO is 0.5%.
d) Transferring 4 μL of ADP-Glo to the 384-well reaction plate, centrifuging it at 1000 rpm/min for 1 min, and incubating it at 25°C for 40 min.
e) Transferring 8 μL of Detection solution to the 384-well reaction plate, centrifuging it at 1000 rpm/min for 1 min, and incubating it at 25°C for 40 min.
f) Using Biotek multi-function plate reader to read the RLU (Relative luminescence unit) signal. The signal intensity is used to characterize the degree of kinase activity.

### 2.2.3 Experimental data processing method

The same as the experimental data processing method used in Example 55.

### 3. Experimental Results

| Item | Tested Compound | IC50 (nM) |
|---|---|---|
| JAK1 Experiment | Filgotinib | 88 |
| | MDI-208 | 0.153 |
| | MDI-210 | 0.347 |
| | MDI-214 | 0.303 |
| | MDI-215 | 0.197 |
| | MDI-218 | 0.825 |
| | MDI-219 | 1.38 |
| | MDI-220 | 2.02 |
| | MDI-221 | 0.128 |
| | MDI-224 | 0.248 |
| | MDI-225 | 0.226 |
| | MDI-216 | 0.134 |
| | MDI-226 | 0.308 |
| | MDI-227 | 0.224 |
| | MDI-228 | 0.398 |
| | MDI-229 | 0.753 |
| | MDI-230 | 0.819 |
| | MDI-233 | 1.31 |
| | MDI-235 | 0.0395 |
| | MDI-231 | 0.530 |
| | MDI-234 | 0.206 |
| | MDI-236 | 0.0403 |

(continued)

| Item | Tested Compound | IC50 (nM) |
|---|---|---|
| JAK2 Experiment | Filgotinib | 71 |
| | MDI-208 | 0.440 |
| | MDI-210 | 1.11 |
| | MDI-214 | 0.273 |
| | MDI-215 | 0.277 |
| | MDI-218 | 0.614 |
| | MDI-219 | 1.38 |
| | MDI-220 | 1.38 |
| | MDI-221 | 0.363 |
| | MDI-224 | 0.754 |
| | MDI-225 | 0.390 |
| | MDI-216 | 0.233 |
| | MDI-226 | 0.371 |
| | MDI-227 | 0.246 |
| | MDI-228 | 0.355 |
| | MDI-229 | 0.356 |
| | MDI-230 | 0.555 |
| | MDI-233 | 1.33 |
| | MDI-235 | 0.166 |
| | MDI-231 | 1.17 |
| | MDI-234 | 0.737 |
| | MDI-236 | 0.329 |
| JAK3Experiment | Filgotinib | 1463 |

(continued)

| Item | Tested Compound | IC50 (nM) |
|---|---|---|
| | MDI-208 | 1.11 |
| | MDI-210 | 0.979 |
| | MDI-214 | 0.352 |
| | MDI-215 | 0.308 |
| | MDI-218 | 0.948 |
| | MDI-219 | 2.29 |
| | MDI-220 | 3.15 |
| | MDI-221 | 0.379 |
| | MDI-224 | 2.01 |
| | MDI-225 | 0.487 |
| | MDI-216 | 0.247 |
| | MDI-226 | 0.676 |
| | MDI-227 | 0.441 |
| | MDI-228 | 0.565 |
| | MDI-229 | 0.481 |
| | MDI-230 | 0.821 |
| | MDI-233 | 2.60 |
| | MDI-235 | 0.183 |
| | MDI-231 | 0.893 |
| | MDI-234 | 0.375 |
| | MDI-236 | 0.141 |

(continued)

| Item | Tested Compound | IC50 (nM) |
| --- | --- | --- |
| TYK2Experiment | Filgotinib | 532 |
| | MDI-208 | 9.31 |
| | MDI-210 | 31.5 |
| | MDI-214 | 2.8 |
| | MDI-215 | 1.58 |
| | MDI-218 | 1.75 |
| | MDI-219 | 1.88 |
| | MDI-220 | 5.56 |
| | MDI-221 | 7.60 |
| | MDI-224 | 16.1 |
| | MDI-225 | 3.50 |
| | MDI-216 | 1.62 |
| | MDI-226 | 4.18 |
| | MDI-227 | 3.89 |
| | MDI-228 | 4.76 |
| | MDI-229 | 4.71 |
| | MDI-230 | 6.57 |
| | MDI-233 | 3.50 |
| | MDI-235 | 0.142 |
| | MDI-231 | 1.31 |
| | MDI-234 | 0.438 |
| | MDI-236 | 0.0954 |

| Item | Tested Compound | IC50 (nM) |
| --- | --- | --- |
| JAK1 Experiment | Filgotinib | 46.2 |

(continued)

| Item | Tested Compound | IC50 (nM) |
|---|---|---|
| | MDI-237 | 0.758 |
| | MDI-239 | 1.15 |
| | MDI-240 | 0.450 |
| | MDI-242 | 67.2 |
| | MDI-243 | 0.118 |
| | MDI-245 | 0.178 |
| | MDI-246 | 0.241 |
| | MDI-247 | 0.557 |
| | MDI-248 | 0.093 |
| | MDI-250 | 0.395 |
| | MDI-251 | 0.144 |
| | MDI-252 | 1.8 |
| | MDI-253 | 2.9 |
| | MDI-255 | 57.2 |
| | MDI-256 | 0.700 |
| | MDI-257 | 0.185 |
| | MDI-258 | 0.939 |
| | MDI-259 | 0.659 |
| | MDI-260 | 2.28 |
| | MDI-262 | 0.319 |
| | MDI-263 | 0.120 |

(continued)

| Item | Tested Compound | IC50 (nM) |
|---|---|---|
| JAK2 Experiment | Filgotinib | 47.6 |
| | MDI-237 | 0.588 |
| | MDI-239 | 1.20 |
| | MDI-240 | 0.842 |
| | MDI-242 | 28.6 |
| | MDI-243 | 0.499 |
| | MDI-245 | 0.648 |
| | MDI-246 | 0.973 |
| | MDI-247 | 1.88 |
| | MDI-248 | 0.560 |
| | MDI-250 | 0.974 |
| | MDI-251 | 0.818 |
| | MDI-252 | 2.6 |
| | MDI-253 | 1.6 |
| | MDI-255 | 37.4 |
| | MDI-256 | 3.06 |
| | MDI-257 | 0.600 |
| | MDI-258 | 3.31 |
| | MDI-259 | 1.30 |
| | MDI-260 | 2.99 |
| | MDI-262 | 1.54 |
| | MDI-263 | 0.717 |
| JAK3 Experiment | Filgotinib | 1051 |
| | MDI-237 | 1.39 |
| | MDI-239 | 4.94 |
| | MDI-240 | 2.02 |

(continued)

| Item | Tested Compound | IC50 (nM) |
|---|---|---|
| | MDI-242 | 152 |
| | MDI-243 | 0.269 |
| | MDI-245 | 0.306 |
| | MDI-246 | 0.709 |
| | MDI-247 | 1.30 |
| | MDI-248 | 0.303 |
| | MDI-250 | 0.497 |
| | MDI-251 | 0.406 |
| | MDI-252 | 2.2 |
| | MDI-253 | 1.5 |
| | MDI-256 | 2.71 |
| | MDI-257 | 0.381 |
| | MDI-258 | 2.36 |
| | MDI-259 | 1.29 |
| | MDI-260 | 2.47 |
| | MDI-262 | 1.22 |
| | MDI-263 | 0.458 |
| TYK2 Experiment | Filgotinib | 233 |
| | MDI-237 | 9.68 |
| | MDI-239 | 19.2 |
| | MDI-240 | 5.34 |
| | MDI-242 | 583 |
| | MDI-243 | 0.167 |
| | MDI-245 | 0.365 |
| | MDI-246 | 1.52 |
| | MDI-247 | 2.35 |
| | MDI-248 | 0.578 |
| | MDI-250 | 0.993 |
| | MDI-251 | 1.33 |
| | MDI-252 | 22 |
| | MDI-253 | 31 |
| | MDI-256 | 6.14 |
| | MDI-257 | 0.684 |
| | MDI-258 | 6.27 |
| | MDI-259 | 2.94 |
| | MDI-260 | 8.98 |
| | MDI-262 | 2.59 |
| | MDI-263 | 0.717 |

The above experimental results show that among the compounds of the present disclosure tested in this Example, most of the tested compounds can inhibit JAK1, JAK2, JAK3, and TYK3 at very low concentrations and the inhibitory activities of these compounds are much higher than that of Filgotinib, with the exception that a few compounds have an activity comparable to Filgotinib,.

Example 57: Effects of compounds on LPS-induced lung inflammation model in mice

The LPS-induced lung inflammation model in mice is one of the commonly used animal models in the development of new drugs for acute pneumonia and cytokine storms, whose pathogenesis and clinical signs are similar to those of severe pneumonia in humans.

**1. Experimental materials and methods**

1.1 Experimental reagent

LPS, Sigma, Cat. No.: L2880; Saline, Zongyi, Cat. No. HA002507; Tween 80, Sigma, Cat. No. P1754; and CBA

| Reagents | Cat. No. | Supplier |
|---|---|---|
| Mouse INF-γ Flex Set | 558296 | BD |
| Mouse TNF Flex Set | 558299 | BD |
| Mouse IL-6 Flex Set | 558301 | BD |
| Mouse IL-1β Flex Set | 560232 | BD |
| CBA mouse/Rat soluble protein master buffer kit | 558267 | BD |
| Cytometer setup and tracking beads | 642412 | BD |
| 96-well V-bottom plate | WIPP02280 | Axygen |

1.2 Compounds to be tested

Name: MDI-209, MDI-216 and Tofacitinib
Forms: White powder
Storage: Being stored at 4°C away from light after receipt.
Solvent: physiological saline solution with 0.2% of TWEEN 80

Formulation method of compounds: To a compound to be tested, a corresponding volume of solvent was added directly after calculation, and the resulting mixture was mixed by vortexing, and then sonicated until a homogeneous suspension was presented. The prepared compound solution with the highest concentration was then diluted proportionally to obtain compound solution with the desired dosing concentration, which were stored at 4°C.

1.3 Experimental apparatus

Anesthesia machine: Raymain, RM-AS-I (RMAS-111207025)
Compound weighing balance: Sartorius, CPA225D
Animal Weighing Balance: Changzhou Tianzhi Ping Electronic Balance, YH2000
CBA Flow Assay: LSRFortessa (BD)

1.4 Experimental animals and housing environment

Strain: C57BL/6J mice
Gender and weight: female, 8 weeks (at the start of the experiment), free of specific pathogens (SPF)
Place of housing: WuXi AppTec Animal Breeding Center
Environment: SPF animal housing
Temperature: 20-25 °C
Humidity: 40-70%
Light: fluorescent light, 12 hours of light (08:00-20:00) and 12 hours of dark (20:00-08:00)
Feeding density: 4 animals/cage
Food: ad libitum (radiation sterilized chow, purchased from Jackson Laboratory, USA)
Water: ad libitum (prepared by a water purifier)
The animal handling described in this experiment was reviewed and approved by the Laboratory Animal Use and Management (Ethics) Committee (IACUC) of WuXi AppTec, Inc.

1.5 Experimental Methods

All mice were randomly divided into 8 groups, and then were administrated separately. Specific information is shown in Table 1:

Table 1: Animal grouping and dosing regimen

| Group No. | Number of animals | Treatment | dosage (mg/kg) | Route of administration | Frequency of dosing regimen |
|---|---|---|---|---|---|
| **1** | 5 | Normal | - | - | - |
| **2** | 8 | Solvent | - | transnasally | 2 times daily for 3 days |
| **3** | 8 | Tofacitinib | 4 | transnasally | 2 times daily for 3 days |
| **4** | 8 | MDI-209 | 2 | transnasally | 2 times daily for 3 days |
| **5** | 8 | MDI-216 | 2 | transnasally | 2 times daily for 3 days |

1.6 Data collection

After the start of the experiment, in order to induce lung inflammation, mice were stimulated with lipopolysaccharide (LPS) (10 μg LPS per mouse dissolved in 20 μL saline) transnasally on D0, D1 and D2. The compounds were formulated and stored at 4°C and administered at D0 for three consecutive days.

At the end of the experiment on D3, lung lavage fluid was collected and four cytokines, IL-1β, IL-6, TNF-α and IFN-γ, were measured by cytokine microsphere assay technique (CBA).

1.7 CBA assay.

1) Removing a sample from the refrigerator and allowing it to stand at room temperature for about 10 min.
2) Removing the standard pellets from each of the four cytokine CBA kits, taking one 15 mL centrifuge tube, adding the standard pellets to the corresponding tube, adding 4 mL of Assay Diluent, and allowing it to stand at room temperature for more than 15 min
3) After the supernatant sample being dissolved and mixed well, taking a sample and adding it to a 96-well V-bottom plate in an amount of 30 μL per well.
4) Pre-filling an EP tube with Assay Diluent, 200 μL/tube, taking a tube with 200 μL diluent and adding another 200 μL diluent thereto to obtain a series of diluted standards with 10 gradients, and then adding the standard s sequentially to a 96-well V-bottom plate in 30 μL/well.
5) Preparation of Capture beads reagent: the capture beads from the 4 cytokine CBA kits are vortexed for 30 seconds, and 51 μl IL-6 beads + 51 μl IL-1β beads + 51 μl INF-Y beads + 51 μl TNF-α beads + 2346 μl capture beads diluent are taken and mixed well. The resulting mixture is added to the respective assay plate in 30 μL/well. The plates are shaken at 200 g for 5 minutes and incubated at room temperature for 1 hour.
6) Preparation of Detection reagent: 51 μl IL-6 PE Detection Reagent + 51 μl IL-1β PE Detection Reagent + 51 μl INF-Y PE Detection Reagent + 51 μl TNF-α PE Detection Reagent + 2346 μl detection reagent are taken and mixed well.
7) Adding the Detection reagent as prepared to the standards and samples to be tested in 30 μL/well; and then shaking the plate at 200 g for 1 min and incubating it at room temperature for 1 hr.

8) Washing: adding a wash buffer in 180 μL/well and centrifuging it at 400 g for 5 min.
9) Removing the supernatant and add a washing buffer in 150 μL/well, which is ready for detection.
10) Performing detection.

1.8 Statistical processing

Experimental data were plotted using GraphPad Prism 7 and expressed as mean ± standard error (Mean ± SEM). The cytokine levels were analyzed using one-way ANOVA with $p < 0.05$ being considered significantly different from the solvent control.

**2. Results**

The levels of four cytokines, IL-1β, IL-6, TNF-α and IFN-γ, were measured by CBA in lung lavage fluids collected from all mice, and the results are shown in FIGs. 1-4. At the doses level administered in the experiment, the levels of all four cytokines were significantly decreased in the MDI-209 and MDI-216 groups compared with the solvent control group, wherein the MDI-216 group showed significant differences in IL-1β and IFN-γ levels compared with the solvent control group (FIG. 1 and FIG. 2), while the MDI-209 group showed significant differences in IFN-γ levels compared with the solvent control group (FIG. 2).

Although specific embodiments of the present disclosure have been illustrated and described, it does not mean that these embodiments illustrate and describe all possible implementation forms of the present disclosure. More precisely, the language used in this specification are only descriptive words and not restrictive. It will be obvious to those skilled in the art that various kinds of changes and modifications can be made without departing from the general scope of the present disclosure. Therefore, the appended claims are intended to include all these changes and modifications within the scope of the present disclosure.

**Claims**

**1.** Use of a compound of Formula (G),

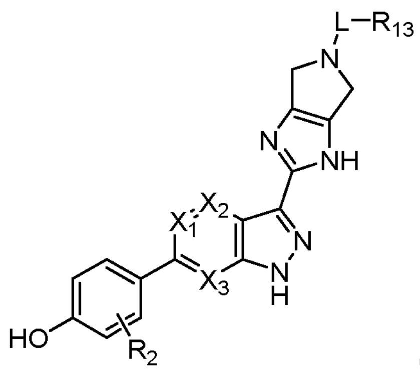

(G)

or an isotopically labeled compound thereof, or an optical isomer thereof, a geometric isomer thereof, a tautomer thereof or a mixture of various isomers, or a pharmaceutically acceptable salt thereof, or a prodrug thereof, or a metabolite thereof in the manufacture of a medicament for treating severe pneumonia,
in which

L is C=O, O=S=O, $CH_2$ or a covalent bond; and
$X_1$ is N or $CR_{14}$; and
$X_2$ is N or $CR_{15}$; and
$X_3$ is N or $CR_{16}$; and
$R_{14}$, $R_{15}$, $R_{16}$ are each independently selected from H, -OH, -SH, -CN, halogen, $-NO_2$, - $SF_5$, -S-$C_{1-4}$ alkyl, $C_{1-6}$ alkyl, $C_{2-8}$ alkenyl, $C_{2-8}$ alkynyl, $C_{1-6}$ alkoxy, $C_{3-7}$ cycloalkyl, 3-7 membered heterocycloalkyl, $C_{5-7}$ aryl, 5-7 membered heteroaryl, $-N(R_9)(R_{10})$, $-N(R_{11})(C(=O)R_{12})$, $-C(=O)-N(R_9)(R_{10})$, $-C(=O)-R_{12}$, $-C(=O)-OR_{12}$, $-OC(=O)R_{12}$, $-N(R_{11})(S(=O)_2R_{12})$, $-S(=O)_2-N(R_9)(R_{10})$, $-SR_{12}$ and $-OR_{12}$, in which the -S-$C_{1-4}$ alkyl, $C_{1-6}$ alkyl, $C_{1-6}$ alkoxy, $C_{3-7}$ cycloalkyl, and 3-7 membered heterocycloalkyl are optionally substituted with 1, 2 or 3 substitutes selected from halogen, -OH, $-NH_2$, $-NH(CH_3)$, $-N(CH_3)_2$, -CN, $C_{1-4}$ alkyl, $C_{3-7}$ cycloalkyl, $C_{1-4}$ hydroxy-

alkyl, -S-$C_{1-4}$ alkyl, -C(=O)H, -C(=O)-$C_{1-4}$ alkyl, -C(=O)-O-$C_{1-4}$ alkyl, -C(=O)-$NH_2$, -C(=O)-N($C_{1-4}$ alkyl)$_2$, -N($C_{1-4}$ alkyl)(C(=O) $C_{1-4}$ alkyl), $C_{1-4}$ haloalkyl, $C_{1-4}$ alkoxy and $C_{1-4}$ haloalkoxy; and

$R_{13}$ is H, -N($R_{17}$)($R_{18}$), $C_{1-6}$ alkoxy, -$SR_{12}$, -$OR_{12}$, -CN, halogen, -$NO_2$, -$SF_5$, -S-$C_{1-4}$ alkyl, $C_{1-6}$ alkyl or $C_{3-7}$ cycloalkyl, 3-7 membered heterocycloalkyl, $C_{5-7}$ aryl, 5-7 membered heteroaryl, $C_{7-11}$ bicyclic aryl, 7-11membered bicyclic heteroaryl, 11-15 membered tricyclyl, Cs-nbicycloalkyl, or 5-11 membered bicyclic heteroalkyl, and $R_{13}$ is substituted with 0, 1, 2, 3 or 4 $R_1$(s), in which $R_{17}$, and $R_{18}$ are each independently selected from H, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, $C_{3-7}$ heterocycloalkyl, $C_{5-7}$ aryl, 5-7 membered heteroaryl, $C_{7-11}$ bicyclic aryl, 7-1 1membered bicyclic heteroaryl, 11-15membered tricyclyl, $C_{5-11}$bicycloalkyl, and 5-11 membered bicyclic heteroalkyl and are optionally substituted with one or more substitutes each independently selected from -OH, - CN, -SH, halogen, -$NO_2$, -$SF_5$, -S-$C_{1-4}$ alkyl, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 4-10 membered heterocycloalkyl, $C_{5-7}$ aryl, 5-7 membered heteroaryl, $C_{7-11}$ bicyclic aryl, 7-11membered bicyclic heteroaryl, -N($R_9$)($R_{10}$), -N($R_{11}$)(C(=O)$R_{12}$), -C(=O)-N($R_9$)($R_{10}$), -C(=O)-$R_{12}$, -C(=O)-$OR_{12}$, -OC(=O)$R_{12}$, - N($R_{11}$)(S(=O)$_2R_{12}$), -S(=O)$_2$-N($R_9$)($R_{10}$), -$SR_{12}$ and -$OR_{12}$, wherein the -S-$C_{1-4}$ alkyl, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 4-10 membered heterocycloalkyl, $C_{5-7}$ aryl, 5-7 membered heteroaryl, $C_{7-11}$ bicyclic aryl, and 7-11membered bicyclic heteroaryl are optionally substituted with 1, 2 or 3 substitutes each independently selected from halogen, -CN, -OH, $C_{1-4}$ alkyl, $C_{1-4}$ alkoxy, $C_{1-4}$ haloalkyl, $C_{1-4}$ haloalkoxy, $C_{3-6}$ cycloalkyl, -N($R_9$)($R_{10}$), -N($R_{11}$)(C(=O)$R_{12}$), -C(=O)-$OR_{12}$, -C(=O)H, -C(=O)$R_{12}$, -C(= O)-N($R_9$)($R_{10}$), -N($R_{11}$)(S(=O)$_2R_{12}$), -S(=O)$_2$-N($R_9$)($R_{10}$), -$SR_{12}$ and -$OR_{12}$; or $R_{17}$, $R_{18}$ and the N atom connected thereto together form a 3-14 membered ring; and

0, 1, 2, 3 or 4 $R_2$(s) are present in formula (G), and $R_2$ is selected from H, halogen, -OH, -$NO_2$, -CN, -$SF_5$, -SH, -S-$C_{1-4}$ alkyl, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 4-10 membered heterocycloalkyl, $C_{5-7}$ aryl, 5-7 membered heteroaryl, $C_{7-11}$ bicyclic aryl, 7-1 1membered bicyclic heteroaryl, -N($R_9$)($R_{10}$), -N($R_{11}$)(C(= O)$R_{12}$), -C(=O)-N($R_9$)($R_{10}$), -C(=O)-$R_{12}$, -C(=O)-$OR_{12}$, -OC(=O)$R_{12}$, -N($R_{11}$)(S(=O)$_2R_{12}$), -S(=O)$_2$-N($R_9$)($R_{10}$), -$SR_{12}$ and -$OR_{12}$, in which the -S-$C_{1-4}$ alkyl, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 4-10 membered heterocycloalkyl, $C_{5-7}$ aryl, 5-7 membered heteroaryl, $C_{7-11}$ bicyclic aryl, and 7-11membered bicyclic heteroaryl are each optionally substituted with 1, 2 or 3 substituent(s) each independently selected from the group consisting of halogen, -CN, -OH, $C_{1-4}$ alkyl, $C_{1-4}$ alkoxy, $C_{1-4}$ haloalkyl, $C_{1-4}$ haloalkoxy, $C_{3-6}$ cycloalkyl, -N($R_9$)($R_{10}$), -N($R_{11}$)(C(=O)$R_{12}$), - C(=O)-$OR_{12}$, -C(=O)H, -C(=O)$R_{12}$, -C(=O)-N($R_9$)($R_{10}$), -N($R_{11}$)(S(=O)$_2R_{12}$), -S(=O)$_2$-N($R_9$)($R_{10}$), -$SR_{12}$ and -$OR_{12}$; and

$R_1$ is selected from H, halogen, -OH, -$NO_2$, -CN, -$SF_5$, -SH, -S-$C_{1-4}$ alkyl, $C_{1-8}$ alkyl, $C_{2-8}$ alkenyl, $C_{2-8}$ alkynyl, $C_{1-8}$ alkoxy, $C_{3-7}$ cycloalkyl, 3-10 membered heterocycloalkyl, $C_{5-7}$ aryl, 5-7 membered heteroaryl, $C_{7-11}$ bicyclic aryl, 7-11membered bicyclic heteroaryl, 11-15membered tricyclyl, Cs-nbicycloalkyl, 5-11 membered bicyclic heteroalkyl, -N($R_9$)($R_{10}$), - N($R_{11}$)(C(=O)$R_{12}$), -C(=O)-N($R_9$)($R_{10}$), -C(=O)-$R_{12}$, -C(=O)-$OR_{12}$, -OC(=O)$R_{12}$, -N($R_{11}$)(S(=O)$_2R_{12}$), -S(=O)$_2$-N($R_9$)($R_{10}$), -$SR_{12}$ and -$OR_{12}$, in which the -S-$C_{1-4}$ alkyl, $C_{1-8}$ alkyl, $C_{2-8}$ alkenyl, $C_{2-8}$ alkynyl, and $C_{1-8}$ alkoxy are optionally substituted with 1, 2, 3, or 4 $R_3$(s), and in which the $C_{3-7}$ cycloalkyl, 3-10 membered heterocycloalkyl, $C_{5-7}$ aryl, 5-7 membered heteroaryl, $C_{7-11}$ bicyclic aryl, and 7-11membered bicyclic heteroaryl are optionally substituted with 1, 2, 3, or 4 $R_4$(s); and

$R_3$ and $R_4$ are each independently selected from H, halogen, -OH, -$NO_2$, -CN, -$SF_5$, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 3-10 membered heterocycloalkyl, $C_{5-7}$ aryl, 5-7 membered heteroaryl, $C_{7-11}$ bicyclic aryl, 7-11membered bicyclic heteroaryl, -N($R_5$)($R_6$), -N($R_{11}$)(C(=O)$R_{12}$), -CON($R_7$)($R_8$), -C(=O)-$R_{12}$, -C(=O)-$OR_{12}$, -OC(=O)$R_{12}$, -N($R_{11}$)(S(=O)$_2R_{12}$), -S(=O)$_2$-N($R_9$)($R_{10}$), -$SR_{12}$ and -$OR_{12}$, in which the $C_{1-6}$ alkyl, $C_{3-7}$ cycloalkyl, 3-10 membered heterocycloalkyl, $C_{5-7}$ aryl, 5-7 membered heteroaryl, $C_{7-11}$ bicyclic aryl, and 7-11membered bicyclic heteroaryl are each optionally substituted with 1, 2, 3 or 4 substituent(s) each independently selected from the group consisting of halogen, -CN, -OH, $C_{1-4}$ alkyl, $C_{1-6}$ alkoxy, $C_{1-4}$ haloalkyl, $C_{1-4}$ haloalkoxy, $C_{3-6}$ cycloalkyl, -N($R_9$)($R_{10}$), -N($R_{11}$)(C(=O)$R_{12}$), -C(=O)-$OR_{12}$, -C(=O)H, -C(=O)$R_{12}$, -C(= O)-N($R_9$)($R_{10}$), -N($R_{11}$)(S(=O)$_2R_{12}$), -S(=O)$_2$-N($R_9$)($R_{10}$), -$SR_{12}$ and -$OR_{12}$; and

$R_5$, $R_6$, $R_7$, Rs, $R_9$, $R_{10}$, $R_{11}$, and $R_{12}$ are each independently H or selected from the group consisting of $C_{1-6}$ alkyl, $C_{1-4}$ haloalkyl, $C_{3-7}$ cycloalkyl, 4-14 membered heterocycloalkyl, $C_{6-10}$ aryl, 5-10 membered heteroaryl, ($C_{3-7}$ cycloalkyl)-$C_{1-4}$ alkyl-, (4-10 membered heterocycloalkyl)-$C_{1-4}$ alkyl-, ($C_{6-10}$ aryl)-$C_{1-4}$ alkyl- and (5-10 membered heteroaryl)-$C_{1-4}$ alkyl-, wherein the substituents included in the above group are each optionally substituted with 1, 2, 3 or 4 substituent(s) each independently selected from the group consisting of halogen, -$CF_3$, -OH, -$NH_2$, -NH($CH_3$), -N($CH_3$)$_2$, -CN, oxo, $C_{1-4}$ alkyl, $C_{2-6}$ alkenyl, $C_{2-6}$ alkynyl, $C_{3-7}$ cycloalkyl, $C_{1-4}$ hydroxyalkyl, -S-$C_{1-4}$ alkyl, -C(=O)H, -C(=O)-$C_{1-4}$ alkyl, - C(=O)-O-$C_{1-4}$ alkyl, -C(=O)-$NH_2$, -C(=O)-N($C_{1-4}$ alkyl)$_2$, $C_{1-4}$ haloalkyl, $C_{1-4}$ alkoxy and $C_{1-4}$ haloalkoxy.

**2.** The use according to claim 1, wherein the compound of formula (G) is an isotopically labeled compound of the compound of formula (G), in which all Hs are each independently and optionally substituted with D.

**3.** The use according to claim 1, wherein in formula (G), $X_1$ is N, $X_2$ is N, or $X_3$ is N.

**4.** The use according to claim 1, wherein in formula (G), $X_1$ is $CR_{14}$, $X_2$ is N or $CR_{15}$, or $X_3$ is $CR_{16}$.

**5.** The use according to claim 1, wherein in formula (G), $X_1$, $X_2$ and $X_3$ are the same.

**6.** The use according to claim 5, wherein in formula (G), $X_1$, $X_2$ and $X_3$ are CH or N.

**7.** The use according to any one of claims 1 to 6, wherein in formula (G), L is C=O, O=S=O or $CH_2$.

**8.** The use according to any one of claims 1 to 6, wherein in formula (G), $R_{13}$ is H, - $N(R_{17})(R_{18})$, $C_{1-6}$ alkoxy, -OH, -SH, -CN, halogen, $-NO_2$, $-SF_5$, $-S-C_{1-4}$ alkyl, $C_{1-6}$ alkyl, or $C_{3-7}$ cycloalkyl, 3-7 membered heterocycloalkyl, $C_{5-7}$ aryl, 5-7 membered heteroaryl, $C_{7-11}$ bicyclic aryl, 7-11membered bicyclic heteroaryl, 11-15 membered tricyclyl, $C_{5-11}$bicycloalkyl, or 5-11 membered bicyclic heteroalkyl, and $R_{13}$ is substituted with 0, 1, 2, 3 or 4 $R_1$(s), in which $R_{17}$ and $R_{18}$ are each independently selected from H, $C_{1-6}$ alkyl, $C_{1-6}$ alkoxy, $C_{3-7}$ cycloalkyl, $C_{3-7}$ heterocycloalkyl, $C_{5-7}$ aryl, and 5-7 membered heteroaryl, and are optionally substituted with one or more of -OH, -CN, -SH, halogen, $-NO_2$, -and $SF_5$.

**9.** The use according to any one of claims 1 to 6, wherein in formula (G), $R_{13}$ is H, - $N(R_{17})(R_{18})$, $C_{1-6}$ alkoxy, -OH, -SH, -CN, halogen, $-NO_2$, $-SF_5$, $-S-C_{1-4}$ alkyl, $C_{1-6}$ alkyl, $C_{3-7}$ cycloalkyl, 3-7 membered heterocycloalkyl, $C_{5-7}$ aryl, 5-7 membered heteroaryl, $C_{7-11}$ bicyclic aryl, 7-11membered bicyclic heteroaryl, or 11-15 membered tricyclyl, and $R_{13}$ is substituted with 0, 1, 2, 3 or 4 $R_1$(s).

**10.** The use according to any one of claims 1 to 6, wherein in formula (G), $R_{13}$ is - $N(R_{17})(R_{18})$, $C_{1-6}$ alkoxy, $C_{1-6}$ alkyl, or $C_{3-7}$ cycloalkyl, 4-6 membered heterocycloalkyl, phenyl, or 5-6 membered heteroaryl, and $R_{13}$ is substituted with 0, 1, 2, 3, or 4 Ri(s)in which $R_{17}$ and $R_{18}$ are each independently selected from H, $C_{1-6}$ alkyl, $C_{3-7}$ cycloalkyl, and $C_{3-7}$ heterocycloalkyl, and are optionally substituted with one or more of -OH, -CN, -SH, halogen, $-NO_2$, and $SF_5$, or $R_{17}$, $R_{18}$ and the N atom connected thereto together form a 4-10 membered ring.

**11.** The use according to any one of claims 1 to 6, wherein in formula (G), 1, 2 or 3 $R_2$(s) are present and $R_2$ is selected from H, halogen, -OH, $-NO_2$, -CN, $-SF_5$, -SH, $-S-C_{1-4}$ alkyl, $C_{1-6}$ alkyl, $C_{1-6}$ alkoxy, $C_{1-6}$ haloalkoxy, $C_{2-6}$ alkenyl, $C_{2-6}$ alkynyl, $C_{3-7}$ cycloalkyl, and 4-10 membered heterocycloalkyl, in which the $-S-C_{1-4}$ alkyl, $C_{1-6}$ alkyl, $C_{3-7}$ cycloalkyl, and 4-10 membered heterocycloalkyl are each optionally substituted with 1, 2 or 3 substituent(s) each independently selected from the group consisting of halogen, -OH, $-NH_2$, $-NH(CH_3)$, - $N(CH_3)_2$, -CN, $C_{1-4}$ alkyl, $C_{1-4}$ haloalkyl, $C_{1-4}$ alkoxy, and $C_{1-4}$ haloalkoxy.

**12.** The use according to claim 11, wherein in formula (G), 1 or 2 $R_2$(s) are present, and $R_2$ is selected from halogen, and $C_{1-6}$ alkyl.

**13.** The use according to claim 1, wherein the compound of formula (G) is a compound of Formula (I),

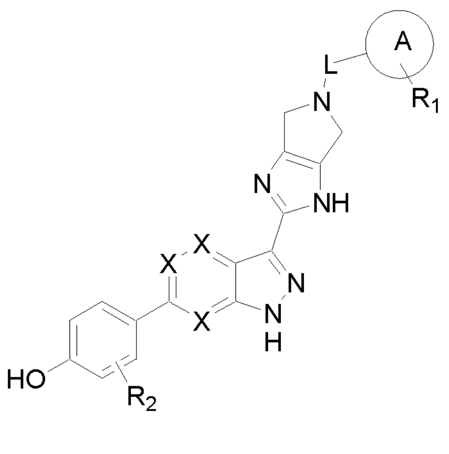

(I)

in which

L is C=O, O=S=O, $CH_2$ or a covalent bond; and
X is CH or N;
the ring A is $C_{3-7}$ cycloalkyl, 3-7 membered heterocycloalkyl, $C_{5-7}$ aryl, 5-7 membered heteroaryl, $C_{7-11}$ bicyclic aryl, 7-11 membered bicyclic heteroaryl, or 11-15 membered tricyclyl;
0, 1, 2, 3 or 4 $R_1$(s) are present in formula (I), and $R_1$ is selected from H, halogen, $C_{1-8}$ alkyl, $C_{2-8}$ alkenyl, $C_{2-8}$ alkynyl, $C_{1-8}$ alkoxy, $C_{3-7}$ cycloalkyl, 3-7 membered heterocycloalkyl, $C_{5-7}$ aryl, 5-7 membered heteroaryl, $C_{7-11}$ bicyclic aryl, and 7-11 membered bicyclic heteroaryl, in which the $C_{1-8}$ alkyl, $C_{2-8}$ alkenyl, $C_{2-8}$ alkynyl, and $C_{1-8}$ alkoxy are optionally substituted with 1, 2, 3 or 4 $R_3$(s), and in which the $C_{3-7}$ cycloalkyl, 3-7 membered heterocycloalkyl, $C_{5-7}$ aryl, 5-7 membered heteroaryl, $C_{7-11}$ bicyclic aryl, 7-11 membered bicyclic heteroaryl are optionally substituted with 1, 2, 3 or 4 $R_4$(s),
0, 1, 2, 3 or 4 $R_2$(s) are present in formula (I), and $R_2$ is selected from H, halogen, -OH, $-NO_2$, -CN, $-SF_5$, $C_{1-6}$alkyl, $C_{1-6}$ alkoxy, $C_{1-6}$ haloalkoxy, $C_{2-6}$ alkenyl, $C_{2-6}$ alkynyl, $C_{3-7}$ cycloalkyl, 4-10 membered heterocycloalkyl, $-N(R_9)(R_{10})$, $-N(R_{11})(C(=O)R_{12})$, $-C(=O)-N(R_9)(R_{10})$, $-C(=O)-R_{12}$, $-C(=O)-OR_{12}$, $-OC(=O)R_{12}$, $-N(R_{11})(S(=O)_2R_{12})$, $-S(=O)_2-N(R_9)(R_{10})$, $-SR_{12}$ and $-OR_{12}$, in which the $C_{1-6}$ alkyl, $C_{3-7}$ cycloalkyl and 4-10 membered heterocycloalkyl are each optionally substituted with 1, 2 or 3 substituent(s) each independently selected from the group consisting of halogen, -CN, -OH, $C_{1-4}$ alkyl, $C_{1-4}$ alkoxy, $C_{1-4}$ haloalkyl, $C_{1-4}$ haloalkoxy, $C_{3-6}$ cycloalkyl, $-N(R_9)(R_{10})$, $-N(R_{11})(C(=O)R_{12})$, - $C(=O)-OR_{12}$, -C(=O)H, $-C(=O)R_{12}$, $-C(=O)-N(R_9)(R_{10})$, $-N(R_{11})(S(=O)_2R_{12})$, $-S(=O)_2-N(R_9)(R_{10})$, $-SR_{12}$ and $-OR_{12}$;
$R_3$ is selected from halogen, cyano, $C_{1-3}$ alkyl, hydroxyl, $C_{1-6}$ alkoxy, $-N(R_5)(R_6)$, - $CON(R_7)(R_8)$ or 3-7 membered heterocycloalkyl, in which the 3-7 membered heterocycloalkyl is optionally substituted with 1, 2, 3 or 4 $R_4$(s);
$R_4$ is selected from halogen, $C_{1-3}$ alkyl, hydroxyl, $C_{1-6}$ alkoxy, $-NH_2$, $-NHCH_3$ or - $N(CH_3)_2$;
$R_5$, $R_6$, $R_7$, $R_8$ are each independently hydrogen or $C_{1-4}$ alkyl;
$R_9$ is selected from H, $C_{1-4}$ alkyl, $C_{1-4}$ haloalkyl or $C_{3-7}$ cycloalkyl;
$R_{10}$ is H or selected from the group consisting of $C_{1-4}$ alkyl, $C_{1-4}$ haloalkyl, $C_{3-7}$ cycloalkyl, 4-10 membered heterocycloalkyl, $C_{6-10}$ aryl, 5-10 membered heteroaryl, ($C_{3-7}$ cycloalkyl)-$C_{1-4}$ alkyl-, (4-10 membered heterocycloalkyl)-$C_{1-4}$ alkyl-, ($C_{6-10}$ aryl)-$C_{1-4}$ alkyl- and (5-10 membered heteroaryl)-$C_{1-4}$ alkyl-, wherein each substituent included in the above group is optionally substituted with 1, 2, 3 or 4 substituent(s) each independently selected from the group consisting of -OH, $-NH_2$, $-NH(CH_3)$, $-N(CH_3)_2$, -CN, $C_{1-4}$ alkyl, $C_{3-7}$ cycloalkyl, $C_{1-4}$ hydroxyalkyl, -S-$C_{1-4}$ alkyl, -C(=O)H, -C(=O)-$C_{1-4}$ alkyl, -C(=O)-O-$C_{1-4}$ alkyl, $-C(=O)-NH_2$, -C(=O)-N($C_{1-4}$ alkyl)$_2$, $C_{1-4}$ haloalkyl, $C_{1-4}$ alkoxy and $C_{1-4}$ haloalkoxy;
$R_{11}$ is selected from H, $C_{1-4}$ alkyl and $C_{3-7}$ cycloalkyl; and
$R_{12}$ is selected from the group consisting of $C_{1-6}$ alkyl, $C_{3-7}$ cycloalkyl, 4- to 14-membered heterocycloalkyl, $C_{6-10}$ aryl, 5-10 membered heteroaryl, ($C_{3-7}$ cycloalkyl)-$C_{1-4}$ alkyl-, (4-10 membered heterocycloalkyl)-$C_{1-4}$ alkyl-, ($C_{6-10}$ aryl)-$C_{1-4}$ alkyl- and (5-10 membered heteroaryl)-$C_{1-4}$ alkyl-, wherein each substituent included in the above group is optionally substituted with 1, 2 or 3 substituent(s) each independently selected from the group consisting of halogen, $-CF_3$, -CN, -OH, $-NH_2$, $-NH(CH_3)$, $-N(CH_3)_2$, oxo, -S-$C_{1-4}$ alkyl, $C_{1-4}$ alkyl, $C_{1-4}$ haloalkyl, $C_{2-6}$ alkenyl, $C_{2-6}$ alkynyl , $C_{3-7}$ cycloalkyl, $C_{1-4}$ alkoxy and $C_{1-4}$ haloalkoxy.

**14.** The use according to claim 13, wherein in formula (I), the ring A is $C_{3-7}$ cycloalkyl, 3-7 membered heterocycloalkyl, $C_{5-7}$ aryl, and 5-7 membered heteroaryl.

**15.** The use according to claim 13, wherein in formula (I), the ring A is 5-6 membered heteroaryl or phenyl.

**16.** The use according to any one of claims 13 to 15, wherein in formula (I), 0 or 1 $R_1$ is present and $R_1$ is selected from $C_{1-6}$ alkyl, and 5-7 membered heterocycloalkyl, wherein said $C_{1-6}$ alkyl is optionally substituted with 1 or 2 $R_3$ and wherein said 5-7 membered heterocycloalkyl is optionally substituted with 1, 2, 3 or 4 $C_{1-3}$ alkyl.

**17.** The use according to any one of claims 13-15, wherein in formula (I),1 or 2 $R_2$ are present and $R_2$ is selected from halogen, $C_{1-6}$ alkyl, and $C_{3-6}$ cycloalkyl, wherein said $C_{1-6}$ alkyl and $C_{3-6}$ cycloalkyl are each optionally substituted with 1, 2, or 3 substituents each independently selected from the group consisting of: halogen, -OH, $-NH_2$, $-NH(CH_3)$ , - $N(CH_3)_2$, -CN, $C_{1-4}$ alkyl, $C_{1-4}$ haloalkyl, $C_{1-4}$ alkoxy, and $C_{1-4}$ haloalkoxy.

**18.** The use according to claim 1, wherein the compound is selected from a group consisting of:

(2-(6-(2-ethyl-5-fluoro-4-hydroxyphenyl)-1H-indazol-3-yl)-4,6-dihydropyrrolo[3,4-d]imidazol-5(1H)-yl)(5-(piperidin-1-yl)pyrazin-2-yl)ketone;
(2-(6-(2-ethyl-5-fluoro-4-hydroxyphenyl)-1H-indazol-3-yl)-4,6-dihydropyrrolo[3,4-d]imidazol-5(1H)-yl)(5-morpholinylpyrazin-2-yl)ketone;

(2-(6-(2-ethyl-5-fluoro-4-hydroxyphenyl)-1H-indazol-3-yl)-4,6-dihydropyrrolo[3,4-d]imidazol-5(1H)-yl)(1-methyl-1H-pyrazol-4-yl)ketone;
(2-(6-(2-ethyl-5-fluoro-4-hydroxyphenyl)-1H-indazol-3-yl)pyrrolo[3,4-d]imidazol-5(1H, 4H,6H)-yl)(1-methylpiperidin-4-yl)ketone;
(2-(6-(2-ethyl-5-fluoro-4-hydroxyphenyl)-1H-indazol-3-yl)pyrrolo[3,4-d]imidazol-5(1H,4H,6H)-yl)(5-(4-methylpiperzin-1-yl)pyrazin-2-yl)ketone;
(2-(6-(2-ethyl-4-hydroxyphenyl)-1H-indazol-3-yl)pyrrolo[3,4-d]imidazol-5(1H,4H,6H)-yl)(5-(4-methylpiperzin-1-yl)pyrazin-2-yl)ketone;
5-ethyl-2-fluoro-4-(3-(5-(benzenesulfonyl)-1,4,5,6-tetrahydropyrrolo[3,4-d]imidazol-2-yl)-1H-indazol-6-yl)phenol;
5-ethyl-2-fluoro-4-(3-(5-(pyrazin-2ylmethyl)-1,4,5,6-tetrahydropyrrolo[3,4-d]imidazol-2-yl)-1H-indazol-6-yl)phenol;
4-(3-(5-(cyclopropylmethyl)-1,4,5,6-tetrahydropyrrolo[3,4-d]imidazol-2-yl)-1H-indazol-6-yl)-5-ethyl-2-fluorophenol;
Cyclopropyl(2-(6-(2-ethyl-5-fluoro-4-hydroxyphenyl)-1H-indazol-3-yl)pyrrolo[3,4-d]imidazol-5(1H,4H,6H)-yl)ketone;
4-(3-(5-(cyclobutylmethyl)-1,4,5,6-tetrahydropyrrolo[3,4-d]imidazol-2-yl)-1H-indazol-6-yl)-5-ethyl-2-fluorophenol;
Cyclobutyl(2-(6-(2-ethyl-5-fluoro-4-hydroxyphenyl)-1H-indazol-3-yl)pyrrolo[3,4-d]imidazol-5(1H,4H,6H)-yl)ketone;
(2-(6-(2-ethyl-5-fluoro-4-hydroxyphenyl)-1H-indazol-3-yl)pyrrolo[3,4-d]imidazol-5(1H,4H,6H)-yl)(3-hydroxylcyclobutyl)ketone;
(2-(6-(2-ethyl-5-fluoro-4-hydroxyphenyl)-1H-indazol-3-yl)-pyrrolo[3,4-d]imidazol-5-(1H,4H,6H)-yl)(pyridazin-4-yl)ketone;
(2-(6-(2-ethyl-5-fluoro-4-hydroxyphenyl)-1H-indazol-3-yl)-pyrrolo[3,4-d]imidazol-5-(1H,4H,6H)-yl)(pyridazin-3-yl)ketone;
(S)-(2-(6-(2-ethyl-5-fluoro-4-hydroxyphenyl)-1H-indazol-3-yl)-4,6-dihydropyrrolo[3,4-d]imidazol-5-(1H)-yl)(3-hydroxylpyrrolidin-1-yl)ketone;
5-ethyl-2-fluoro-4-(3 -(5-(4-hydroxylcyclohexyl)-1,4, 5, 6-tetrahydropyrrolo[3,4-d]imidazol-2-yl)-1H-indazol-6-yl)phenol;
4-(3-(5-(cyclopropanesulfonyl)-1,4,5,6-tetrahydropyrrolo[3,4-d]imidazol-2-yl)-1H-indazol-6-yl)-5-ethyl-2-fluorophenol;
4-(3-(5-(cyclobutylsulfonyl)-1,4,5,6-tetrahydropyrrolo[3,4-d]imidazol-2-yl)-1H-indazol-6-yl)-5-ethyl-2-fluorophenol;
4-(3-(5-(cyclopentylsulfonyl)-1,4,5,6-tetrahydropyrrolo[3,4-d]imidazol-2-yl)-1H-indazol-6-yl)-5-ethyl-2-fluorophenol;
5-ethyl-2-fluoro-4-(3-(5-((1-methyl-1H-pyrazol-4-yl)methyl)-1,4,5,6-tetrahydropyrrolo[3,4-d]imidazol-2-yl)-1H-indazol-6-yl)phenol;
4-(3-(5-(cyclopentyl-1,4,5,6-tetrahydropyrrolo[3,4-d]imidazol-2-yl)-1H-indazol-6-yl)-5-ethyl-2-fluorophenol;
5-ethyl-2-fluoro-4-(3 -(5-(tetrahydro-2H-pyran-4-yl)-1,4, 5, 6-tetrahydropyrrolo[3,4-d]imidazol-2-yl)-1H-indazol-6-yl)phenol;
1-(2-(6-(2-ethyl-5-fluoro-4-hydroxyphenyl)-1H-indazol-3-yl)-4,6-dihydropyrrolo[3,4-d]imidazol-5-(1H)-yl)ethan-1-one;
1-(2-(6-(2-ethyl-5-fluoro-4-hydroxyphenyl)-1H-indazol-3-yl)-4,6-dihydropyrrolo[3,4-d]imidazol-5-(1H)-yl)propan-1-one;
1-(2-(6-(2-ethyl-5-fluoro-4-hydroxyphenyl)-1H-indazol-3-yl)-4,6-dihydropyrrolo[3,4-d]imidazol-5-(1H)-yl)-2-methylpropan-1-one;
2-cyclopropyl-1-(2-(6-(2-ethyl-5-fluoro-4-hydroxyphenyl)-1H-indazol-3-yl)-4,6-dihydropyrrolo[3,4-d]imidazol-5-(1H)-yl)ethan-1-one;
1-(2-(6-(2-ethyl-5-fluoro-4-hydroxyphenyl)-1H-indazol-3-yl)-4,6-dihydropyrrolo[3,4-d]imidazol-5-(1H)-yl)-3-methylbutan-1-one;
(2-(6-(2-ethyl-5-fluoro-4-hydroxyphenyl)-1H-indazol-3-yl)-4,6-dihydropyrrolo[3,4-d]imidazol-5-(1H)-yl)(pyrrolidin-1-yl)ketone;
(2-(6-(2-ethyl-5-fluoro-4-hydroxyphenyl)-1H-indazol-3-yl)-4,6-dihydropyrrolo[3,4-d]imidazol-5-(1H)-yl)(piperidin-1-yl)ketone;
(2-(6-(2-ethyl-5-fluoro-4-hydroxyphenyl)-1H-indazol-3-yl)-4,6-dihydropyrrolo[3,4-d]imidazol-5-(1H)-yl)(morpholino)ketone;
(2-(6-(2-ethyl-5-fluoro-4-hydroxyphenyl)-1H-indazol-3-yl)-4,6-dihydropyrrolo[3,4-d]imidazol-5-(1H)-yl)(4-methylpiperzin-1-yl)ketone;
(2-(6-(2-ethyl-5-fluoro-4-hydroxyphenyl)-1H-indazol-3-yl)-4,6-dihydropyrrolo[3,4-d]imidazol-5-(1H)-yl)(4-ethylpiperzin-1-yl)ketone;
Cyclopropyl(2-(6-(2-ethyl-5-fluoro-4-hydroxyphenyl)-1H-pyrazolo [4,3-b]pyridin-3-yl)-4,6-dihydropyrrolo[3,4-d]imidazol-5(1H)-yl)ketone;
Cyclopropyl(2-(6-(2-ethyl-5-fluoro-4-hydroxyphenyl)-4-methyl-1H-indazol-3-yl)-4,6-dihydropyrrolo[3,4-d]imidazol-5(1H)-yl)ketone;
(S)-(2-(6-(2-ethyl-5-fluoro-4-hydroxyphenyl)-4-methyl-1H-indazol-3-yl)-4,6-dihydropyrrolo[3,4-d]imidazol -5(1H)-yl)(3-hydroxylpyrrolidin-1-yl)ketone;
Cyclopropyl(2-(6-(2-ethyl-5-fluoro-4-hydroxyphenyl)-1H-pyrazolo[4,3-c]pyridin-3-yl)-4,6-dihydropyrrolo[3,4-d]imidazol-5(1H)-yl)ketone;
(R)-(2-(6-(2-ethyl-5-fluoro-4-hydroxyphenyl)-1H-indazol-3-yl)-4,6-dihydropyrrolo[3,4-d]imidazol-5(1H )-yl)(3-hydroxylpyrrolidin-1-yl)ketone;
(2-(6-(2-ethyl-5-fluoro-4-hydroxyphenyl)-1H-indazol-3-yl)-4,6-dihydropyrrolo[3,4-d]imidazol-5(1H)-yl)(4-hydroxylpiperidin-1-yl)ketone;
2-(6-(2-ethyl-5-fluoro-4-hydroxyphenyl)-1H-indazol-3-yl)-N-methyl-4,6-dihydropyrrolo[3,4-d]imidazol-5-(1H)-carboxamide;
2-(6-(2-ethyl-5-fluoro-4-hydroxyphenyl)-1H-indazol-3-yl)-N-ethyl-4,6-dihydropyrrolo[3,4-d]imidazol-5-(1H)-carboxamide;
2-(6-(2-ethyl-5-fluoro-4-hydroxyphenyl)-1H-indazol-3-yl)-N-(2-hydroxylethyl)-4,6-dihydropyrrolo[3,4-d]imidazol-5(1H)-carboxamide;
1-(2-(6-(2-ethyl-5-fluoro-4-hydroxyphenyl)-1H-indazol-3 -yl)-1,4, 5, 6-tetrahydropyrrolo[3,4-d]imidazol-5 -carbonyl) pyrrolidin-3-nitrile;
2-(6-(2-ethyl-5-fluoro-4-hydroxyphenyl)-1H-indazol-3-yl)-N-(tetrahydrofuran-3-yl)-4,6-dihydropyrrolo[3,4-d]imidazol-5(1H)-carboxamide;
Methyl 2-(6-(2-ethyl-5-fluoro-4-hydroxyphenyl)-1H-indazol-3-yl)-4,6-dihydropyrrolo[3,4-d]imidazol-5(1H)-carboxylate;
Ethyl 2-(6-(2-ethyl-5-fluoro-4-hydroxyphenyl)-1H-indazol-3-yl)-4,6-dihydropyrrolo[3,4-d]imidazol-5(1H)-carboxylate;
(S)-(2-(6-(2-ethyl-5-fluoro-4-hydroxyphenyl)-1H-pyrazolo[3,4-b]pyridin-3-yl)-4,6-dihydropyrrolo[3,4-d]imidazol-5(1H)-yl)(3-hydroxylpyrrolidin-1-yl)ketone;
3-(2-(6-(2-ethyl-5-fluoro-4-hydroxyphenyl)-1H-indazol-3-yl) -4,6-dihydropyrrolo[3,4-d]imidazol-5(1H)-yl)-3-oxypropionitrile;
2-(6-(2-ethyl-5-fluoro-4-hydroxyphenyl)-1H-indazol-3-yl)-N,N-dimethyl-4,6-dihydropyrrolo[3,4-d]imidazol-5(1H)-carboxamide;
N-(2-cyanoethyl)-2-(6-(2-ethyl-5-fluoro-4-hydroxyphenyl)-1H-indazol-3-yl)-4,6-dihydropyrrolo[ 3,4-d]imidazol-5(1H)-carboxamide;
N-cyclopropyl-2-(6-(2-ethyl-5-fluoro-4-hydroxyphenyl)-1H-indazol-3-yl)-4,6-dihydropyrrolo[3,4-d]imidazol-5(1H)-carboxamide;
N-cyclobutyl-2-(6-(2-ethyl-5-fluoro-4-hydroxyphenyl)-1H-indazol-3-yl) -4,6-dihydropyrrolo[3,4- d]imidazol-5(1H)-carboxamide;
(S)-6-(2-ethyl-5-fluoro-4-hydroxyphenyl)-3-(5-prolyl-1,4,5,6-tetrahydropyrrolo[3,4-d]imidazol-2-yl)- 1H-indazol;
(R)-6-(2-ethyl-5-fluoro-4-hydroxyphenyl)-3-(5-prolyl-1,4,5,6-tetrahydropyrrolo[3,4-d]imidazol-2-yl)- 1H-indazol;
(2-(6-(2-ethyl-5-fluoro-4-hydroxyphenyl)-1H-indazol-3-yl)--4,6-dihydropyrrolo[3,4-d]imidazol-5(1H)-yl)(5-(piperidin-1-yl)pyrazin-2-yl)ketone;
(2-(6-(2-ethyl-5-fluoro-4-hydroxyphenyl)-1H-indazol-3-yl)--4,6-dihydropyrrolo[3,4-d]imidazol-5(1H)-yl)(5-morpholinpyrazin-2-yl)ketone;
(2-(6-(2-ethyl-5-fluoro-4-hydroxyphenyl)-1H-indazol-3-yl)--4,6-dihydropyrrolo[3,4-d]imidazol-5(1H)-yl)(1-methyl-1H-pyrazol-4-yl)ketone;
5-ethyl-2-fluoro-4-{3-[5-(1-methylpiperidin-4-carbonyl)-1H,4H,5H,6H-pyrrolo[3,4-d]imidazol-2-yl]-1H-indazol-6-yl}phenol;
5-ethyl-2-fluoro-4-{3-[5-(4-methylpiperazin-1-carbonyl)-1H,4H,5H,6H-pyrrolo[3,4-d]imidazol-2-yl]-1H-indazol-6-yl}phenol;
3-ethyl-4-{3-[5-(4-methylpiperazin-1-carbonyl)-1H,4H,5H,6H-pyrrolo[3,4-d]imidazol-2-yl]-1H-indazol-6-yl}phenol;
5-ethyl-2-fluoro-4-(3-(5-(bemzenesulfonyl)-1,4,5,6-tetrahydropyrrolo[3,4-d]imidazol-2-yl)-1H-indazol-6-yl)phenol; and
5-ethyl-2-fluoro-4-(3-(5-(pyrazin-2-methyl)-1,4,5,6-tetrahydropyrrolo[3,4-d]imidazol-2-yl)-1H-indazol-6-yl)phenol.

**19.** The use according to any one of claims 1 to 18, wherein said medicament is used to inhibit a cytokine storm in severe pneumonia.

**20.** The use according to claim 19, wherein said severe pneumonia is caused by a virus.

**21.** The use according to claim 20, wherein said virus is severe acute respiratory syndrome coronaviruses (SARS-CoV), severe acute respiratory syndrome coronaviruses-2 (SARS-CoV-2), influenza viruses or Middle East respiratory syndrome coronaviruses (MERS-CoV).

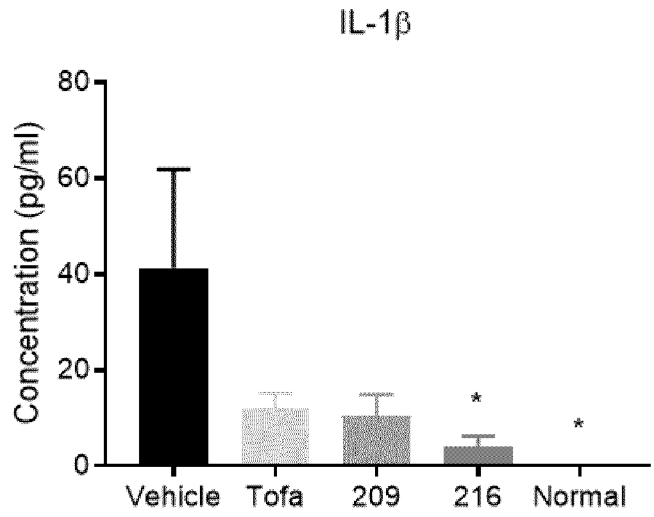
IL-1β
Concentration (pg/ml)
80
60
40
20
0
*
*
Vehicle
Tofa
209
216
Normal

FIG. 1

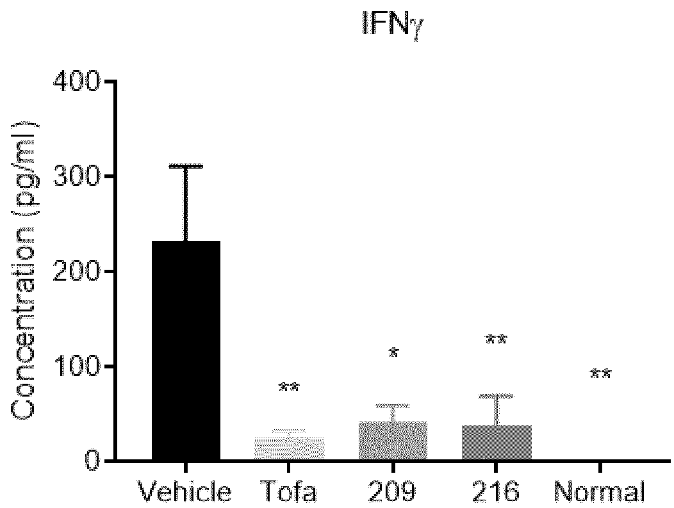
IFNγ
Concentration (pg/ml)
400
300
200
100
0
**
*
**
**
Vehicle
Tofa
209
216
Normal

FIG. 2

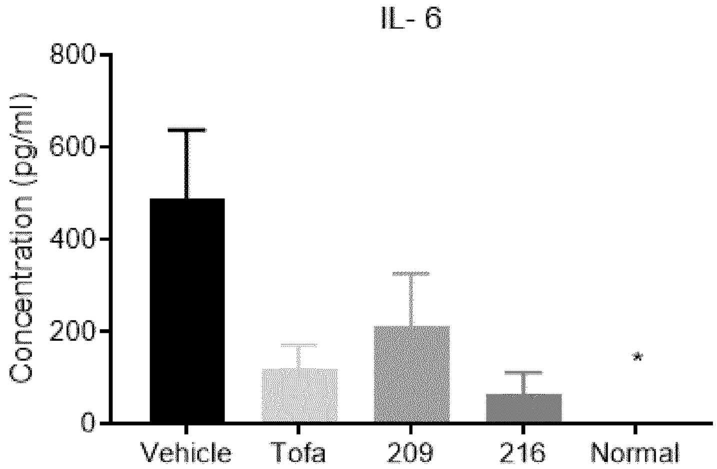
IL- 6
Concentration (pg/ml)
800
600
400
200
0
*
Vehicle
Tofa
209
216
Normal

**FIG. 3**

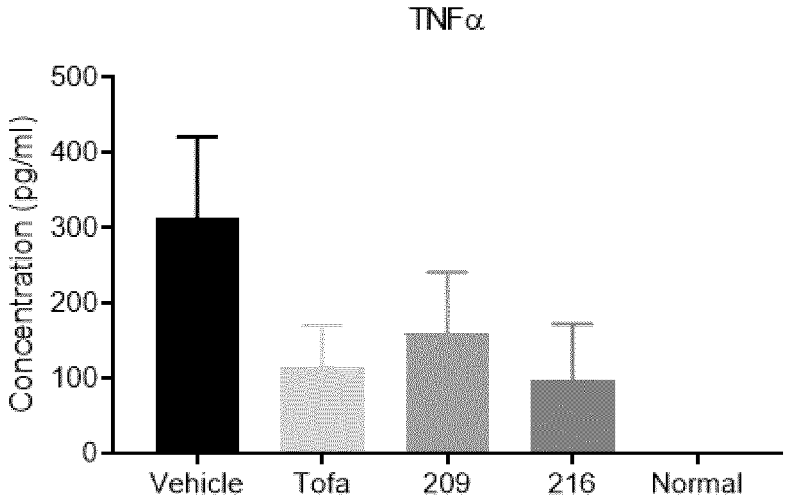
TNFα
Concentration (pg/ml)
500
400
300
200
100
0
Vehicle
Tofa
209
216
Normal

**FIG. 4**

**INTERNATIONAL SEARCH REPORT**

International application No. **PCT/CN2021/112351**

**A. CLASSIFICATION OF SUBJECT MATTER**

C07D 487/04(2006.01)i; C07D 519/00(2006.01)i; A61K 31/497(2006.01)i; A61K 31/5377(2006.01)i; A61P 17/00(2006.01)i; A61P 19/02(2006.01)i; A61P 25/00(2006.01)i

According to International Patent Classification (IPC) or to both national classification and IPC

**B. FIELDS SEARCHED**

Minimum documentation searched (classification system followed by classification symbols)

C07D; A61K; A61P

Documentation searched other than minimum documentation to the extent that such documents are included in the fields searched

Electronic data base consulted during the international search (name of data base and, where practicable, search terms used)

DWPI; SIPOABS; WOTXT; USTXT; EPTXT; CNABS; CNTXT; CNKI; STN-REGISTRY; STN-CAPLUS, STN-MARPAT; Web of Science: 河南迈英诺医药科技, 路良, 黄海, 张龙争, 赵赛赛, 张霁旋, 吲唑, 吡咯, 咪唑, 蛋白激酶, Janus, JAK, STAT, 炎, 免疫, 肺炎, +indazol+, +imidazo+, +pyrrol+, inflammatory, immun+, filgotinib, tofacitinib

**C. DOCUMENTS CONSIDERED TO BE RELEVANT**

| Category* | Citation of document, with indication, where appropriate, of the relevant passages | Relevant to claim No. |
|---|---|---|
| PX | CN 111606908 A (HENAN MAIYINGNUO PHARMACEUTICAL TECHNOLOGY CO., LTD.) 01 September 2020 (2020-09-01)<br>entire document | 1-21 |
| A | CN 103717599 A (PFIZER INC.) 09 April 2014 (2014-04-09)<br>description paragraphs [0008]-[0027], [0043]-[0112] | 1-21 |
| A | WO 2017077283 A1 (TOPIVERT PHARMA LTD) 11 May 2017 (2017-05-11)<br>description page 3 line 4 from the bottom - page 5 line 35, description page 29 line 40 - page 39 line 5 | 1-21 |
| A | WO 2017077288 A1 (TOPIVERT PHARMA LTD) 11 May 2017 (2017-05-11)<br>entire document | 1-21 |

☐ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **09 October 2021** | **04 November 2021** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**
**Information on patent family members**

International application No.
**PCT/CN2021/112351**

| Patent document cited in search report | | | Publication date (day/month/year) | Patent family member(s) | | | Publication date (day/month/year) |
|---|---|---|---|---|---|---|---|
| CN | 111606908 | A | 01 September 2020 | WO | 2020173400 | A1 | 03 September 2020 |
| CN | 103717599 | A | 09 April 2014 | MA | 35285 | B1 | 03 July 2014 |
| | | | | UA | 108442 | C2 | 27 April 2015 |
| | | | | NI | 201400005 | A | 23 April 2014 |
| | | | | TN | 2014000033 | A1 | 01 July 2015 |
| | | | | PE | 20141059 | A1 | 30 August 2014 |
| | | | | CL | 2014000122 | A1 | 18 August 2014 |
| | | | | KR | 20140026627 | A | 05 March 2014 |
| | | | | MX | 2014001004 | A | 13 May 2014 |
| | | | | GT | 201400016 | A | 19 February 2015 |
| | | | | UY | 34220 | A | 28 February 2013 |
| | | | | CU | 20140009 | A7 | 26 March 2014 |
| | | | | US | 2013029968 | A1 | 31 January 2013 |
| | | | | EP | 2736907 | A1 | 04 June 2014 |
| | | | | EA | 201490357 | A1 | 30 May 2014 |
| | | | | CR | 20140042 | A | 19 March 2014 |
| | | | | AU | 2012288491 | A1 | 30 January 2014 |
| | | | | WO | 2013014567 | A1 | 31 January 2013 |
| | | | | US | 2014024634 | A1 | 23 January 2014 |
| | | | | US | 8575336 | B2 | 05 November 2013 |
| | | | | EC | SP14013222 | A | 31 March 2014 |
| | | | | AP | 2014007372 | A0 | 31 January 2014 |
| | | | | TW | 201313712 | A | 01 April 2013 |
| | | | | BR | 112014001801 | A2 | 14 February 2017 |
| | | | | CA | 2841882 | A1 | 31 January 2013 |
| | | | | AR | 087348 | A1 | 19 March 2014 |
| | | | | JP | 2014522865 | A | 08 September 2014 |
| | | | | EP | 2736907 | B1 | 07 October 2015 |
| | | | | CN | 103717599 | B | 25 November 2015 |
| | | | | US | 8895544 | B2 | 25 November 2014 |
| | | | | AP | 201407372 | A0 | 31 January 2014 |
| | | | | JP | 5579351 | B1 | 27 August 2014 |
| | | | | CO | 6862160 | A2 | 10 February 2014 |
| | | | | DO | P2014000016 | A | 15 July 2014 |
| | | | | TW | I462922 | B | 01 December 2014 |
| | | | | MD | 20140002 | A2 | 30 June 2014 |
| | | | | IN | 201400112 | P1 | 01 July 2016 |
| | | | | GE | 201506398 | B | 10 November 2015 |
| | | | | SG | 196469 | B | 24 July 2015 |
| | | | | ID | 201501040 | A | 20 March 2015 |
| | | | | HK | 1194369 | A0 | 17 October 2014 |
| | | | | VN | 37873 | A | 26 May 2014 |
| | | | | PH | 12014500199 | A1 | 03 March 2014 |
| | | | | SG | 196469 | A1 | 13 February 2014 |
| | | | | IL | 230662 | A | 31 March 2014 |
| WO | 2017077283 | A1 | 11 May 2017 | EP | 3371184 | A1 | 12 September 2018 |
| | | | | DK | 3712152 | T3 | 22 March 2021 |
| | | | | PT | 3712152 | T | 15 April 2021 |
| | | | | EP | 3712152 | B1 | 13 January 2021 |
| | | | | CA | 3037243 | A1 | 11 May 2017 |

Form PCT/ISA/210 (patent family annex) (January 2015)

**INTERNATIONAL SEARCH REPORT**
**Information on patent family members**

International application No.
**PCT/CN2021/112351**

| Patent document cited in search report | Publication date (day/month/year) | Patent family member(s) | Publication date (day/month/year) |
|---|---|---|---|
| | | US 10851097 B2 | 01 December 2020 |
| | | EP 3712152 A1 | 23 September 2020 |
| | | US 2018319791 A1 | 08 November 2018 |
| WO 2017077288 A1 | 11 May 2017 | US 10556901 B2 | 11 February 2020 |
| | | CA 3037248 A1 | 11 May 2017 |
| | | EP 3371185 A1 | 12 September 2018 |
| | | US 2018370963 A1 | 27 December 2018 |
| | | US 10882859 B2 | 05 January 2021 |
| | | DK 3371185 T3 | 30 November 2020 |
| | | PT 3371185 T | 28 December 2020 |
| | | EP 3371185 B1 | 30 September 2020 |
| | | EP 3831830 A1 | 09 June 2021 |
| | | US 2020223843 A1 | 16 July 2020 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202010818470


### Non-patent literature cited in the description


- **STAHL ; WERMUTH.** Handbook of Pharmaceutical Salts: Properties, Selection and Use. Wiley-VCH, 2002